# EUROPEAN PATENT SPECIFICATION

(11) **EP 3 305 323 B1**
(45) Date of publication and mention of the grant of the patent: **17.02.2021**
(21) Application number: 17188389.5
(22) Date of filing: 17.04.2013
(51) Int. Cl.: A61K 39/395

(54) **HUMAN ANTIBODIES AND SPECIFIC BINDING SEQUENCES THEREOF FOR USE IN STROKE AND ISCHEMIA OR ISCHEMIC CONDITIONS**
MENSCHLICHE ANTIKÖRPER UND SPEZIFISCHE BINDUNGSSEQUENZEN DAVON ZUR VERWENDUNG BEI APOPLEXIE UND ISCHÄMIE ODER ISCHÄMISCHEN ZUSTÄNDEN
ANTICORPS HUMAINS ET LEURS SÉQUENCES DE LIAISON SPÉCIFIQUE POUR UNE UTILISATION DANS UN ACCIDENT VASCULAIRE CÉRÉBRAL ET LE ISCHÉMIE OU DES ÉTATS ISCHÉMIQUES

(30) Priority: 17.04.2012 US 201261625628 P
(43) Date of publication of application: 11.04.2018
(62) Divisional of application: 13778780.0
(73) Proprietor: Mayo Foundation for Medical Education and Research, Rochester, MN 55905 (US)
(72) Inventor: RODRIGUEZ, Moses, Rochester, MN 55902 (US); WARRINGTON, Arthur E., Rochester, MN 55906 (US); PEASE, Larry R., Rochester, MN 55906 (US)
(74) Representative: Carpmaels & Ransford LLP

(56) References cited:
- WO-A2-2009/052374
- WO-A2-2012/054077
- BHARATH WOOTLA ET AL: "Need for a paradigm shift in therapeutic approaches to CNS injury", EXPERT REVIEW OF NEUROTHERAPEUTICS, vol. 12, no. 4, 1 April 2012 (2012-04-01), pages 409-420, XP055209560, ISSN: 1473-7175, DOI: 10.1586/ern.12.24
- ALEKSANDAR DENIC ET AL: "A Single Dose of Neuron-Binding Human Monoclonal Antibody Improves Spontaneous Activity in a Murine Model of Demyelination", PLOS ONE, vol. 6, no. 10, 12 October 2011 (2011-10-12), page e26001, XP055209558, DOI: 10.1371/journal.pone.0026001

### SEQUENCE LISTING

<110> Rodriguez, Moses Warrington, Arthur E. Pease, Larry R.
<120> Human Antibodies and Specific Binding
   Sequences Thereof For Use In Stroke and Ishemia or Ischemic Conditions
<130> 1199-1-016PCT3
<140> PCT/US2013/036947
   <141> 2013-04-17
<150> 61/625,628
   <151> 2012-04-17
<160> 93
<170> FastSEQ for Windows Version 4.0
<210> 1
   <211> 118
   <212> PRT
   <213> Homo sapiens
<400> 1
   Gln Val Gln Leu Gln Glu Ser Gly Pro Gly Leu Leu Lys Pro Ser Glu
    1               5                  10                  15
   Thr Leu Ser Leu Thr Cys Thr Val Ser Gly Gly Ser Val Ser Leu Tyr
               20                  25                  30
   Tyr Trp Ser Trp Ile Arg Gln Ser Pro Gly Lys Glu Pro Glu Trp Ile
           35                  40                  45
   Gly Tyr Ile Tyr Ser Ser Gly Ser Thr Asp Tyr Asn Pro Ser Leu Arg
       50                  55                  60
   Ser Arg Val Thr Ile Ser Leu Asp Thr Ser Asn Asn Arg Phe Ser Leu
   65                  70                  75                  80
   Asn Leu Arg Ser Val Thr Ala Ala Asp Thr Ala Val Tyr Trp Cys Ala
                   85                  90                  95
   Arg Ser Ala Ser Ile Arg Gly Trp Phe Asp Pro Trp Gly Gln Gly Thr
               100                 105                 110
   Leu Val Thr Val Ser Ser
           115
<210> 2
   <211> 354
   <212> DNA
   <213> Homo sapiens
<400> 2
   caggtgcagc tgcaggagtc gggcccagga ctgctgaagc cttcggagac cctgtccctc 60
   acatgcactg tctctggtgg ttccgtcagt ctttactact ggagctggat ccggcagtcc 120
   ccagggaagg aaccggagtg gattggatat atctattcca gtggaagcac cgattacaac 180
   ccttccctca ggagtcgagt caccatatca ctggacacgt caaacaaccg gttttcccta 240
   aacctgaggt ctgtgaccgc cgcagataca gcggtctatt ggtgtgcgag aagtgcgtca 300
   attaggggct ggttcgaccc ctggggccag ggaaccctgg tcaccgtctc ctca       354
<210> 3
   <211> 104
   <212> PRT
   <213> Homo sapiens
<400> 3
   Gly Ser Ala Ser Ala Pro Thr Leu Phe Pro Leu Val Ser Cys Glu Asn
    1               5                  10                  15
   Ser Pro Ser Asp Thr Ser Ser Val Ala Val Gly Cys Leu Ala Gln Asp
               20                  25                  30
   Phe Leu Pro Asp Ser Ile Thr Phe Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro
               100
<210> 4
   <211> 310
   <212> DNA
   <213> Homo sapiens
<400> 4
   gagtgcatcc gccccaaccc ttttccccct cgtctcctgt gagaattccc cgtcggatac 60
   gagcagcgtg gccgttggct gcctcgcaca ggacttcctt cccgactcca tcactttctc 120
   ctggaaatac aagaacaact ctgacatcag cagcacccgg ggcttcccat cagtcctgag 180
   agggggcaag tacgcagcca cctcacaggt gctgctgcct tccaaggacg tcatgcaggg 240
   cacagacgaa cacgtggtgt gcaaagtcca gcaccccaac ggcaacaaag aaaagaacgt 300
   gcctcttcca                                                        310
<210> 5
   <211> 112
   <212> PRT
   <213> Homo sapiens
<400> 5
   Val Ile Ala Glu Leu Pro Pro Lys Val Ser Val Phe Val Pro Pro Arg
    1               5                  10                  15
   Asp Gly Phe Phe Gly Asn Pro Arg Lys Ser Lys Leu Ile Cys Gln Ala
               20                  25                  30
   Thr Gly Phe Ser Pro Arg Gln Ile Gln Val Ser Trp Leu Arg Glu Gly
           35                  40                  45
   Lys Gln Val Gly Ser Gly Val Thr Thr Asp Gln Val Gln Ala Glu Ala
       50                  55                  60
   Lys Glu Ser Gly Pro Thr Thr Tyr Lys Val Thr Ser Thr Leu Thr Ile
   65                  70                  75                  80
   Lys Glu Ser Asp Trp Leu Ser Gln Ser Met Phe Thr Cys Arg Val Asp
                   85                  90                  95
   His Arg Gly Leu Thr Phe Gln Gln Asn Ala Ser Ser Met Cys Val Pro
               100                 105                 110
<210> 6
   <211> 336
   <212> DNA
   <213> Homo sapiens
<400> 6
   gtgattgctg agctgcctcc caaagtgagc gtcttcgtcc caccccgcga cggcttcttc 60
   ggcaaccccc gcaagtccaa gctcatctgc caggccacgg gtttcagtcc ccggcagatt 120
   caggtgtcct ggctgcgcga ggggaagcag gtggggtctg gcgtcaccac ggaccaggtg 180
   caggctgagg ccaaagagtc tgggcccacg acctacaagg tgaccagcac actgaccatc 240
   aaagagagcg actggctcag ccagagcatg ttcacctgcc gcgtggatca caggggcctg 300
   accttccagc agaatgcgtc ctccatgtgt gtcccc                           336
<210> 7
   <211> 106
   <212> PRT
   <213> Homo sapiens
<400> 7
   Asp Gln Asp Thr Ala Ile Arg Val Phe Ala Ile Pro Pro Ser Phe Ala
    1               5                  10                  15
   Ser Ile Phe Leu Thr Lys Ser Thr Lys Leu Thr Cys Leu Val Thr Asp
               20                  25                  30
   Leu Thr Thr Tyr Asp Ser Val Thr Ile Ser Trp Thr Arg Gln Asn Gly
           35                  40                  45
   Glu Ala Val Lys Thr His Thr Asn Ile Ser Glu Ser His Pro Asn Ala
       50                  55                  60
   Thr Phe Ser Ala Val Gly Glu Ala Ser Ile Cys Glu Asp Asp Trp Asn
   65                  70                  75                  80
   Ser Gly Glu Arg Phe Thr Cys Thr Val Thr His Thr Asp Leu Pro Ser
                   85                  90                  95
   Pro Leu Lys Gln Thr Ile Ser Arg Pro Lys
               100                 105
<210> 8
   <211> 318
   <212> DNA
   <213> Homo sapiens
<400> 8
   gatcaagaca cagccatccg ggtcttcgcc atccccccat cctttgccag catcttcctc 60
   accaagtcca ccaagttgac ctgcctggtc acagacctga ccacctatga cagcgtgacc 120
   atctcctgga cccgccagaa tggcgaagct gtgaaaaccc acaccaacat ctccgagagc 180
   caccccaatg ccactttcag cgccgtgggt gaggccagca tctgcgagga tgactggaat 240
   tccggggaga ggttcacgtg caccgtgacc cacacagacc tgccctcgcc actgaagcag 300
   accatctccc ggcccaag                                                318
<210> 9
   <211> 131
   <212> PRT
   <213> Homo sapiens
<400> 9
   Gly Val Ala Leu His Arg Pro Asp Val Tyr Leu Leu Pro Pro Ala Arg
    1               5                  10                  15
   Glu Gln Leu Asn Leu Arg Glu Ser Ala Thr Ile Thr Cys Leu Val Thr
               20                  25                  30
   Gly Phe Ser Pro Ala Asp Val Phe Val Gln Trp Met Gln Arg Gly Gln
           35                  40                  45
   Pro Leu Ser Pro Glu Lys Tyr Val Thr Ser Ala Pro Met Pro Glu Pro
       50                  55                  60
   Gln Ala Pro Gly Arg Tyr Phe Ala His Ser Ile Leu Thr Val Ser Glu
   65                  70                  75                  80
   Glu Glu Trp Asn Thr Gly Glu Thr Tyr Thr Cys Val Val Ala His Glu
                   85                  90                  95
   Ala Leu Pro Asn Arg Val Thr Glu Arg Thr Val Asp Lys Ser Thr Gly
               100                 105                 110
   Lys Pro Thr Leu Tyr Asn Val Ser Leu Val Met Ser Asp Thr Ala Gly
           115                 120                 125
   Thr Cys Tyr
       130
<210> 10
   <211> 392
   <212> DNA
   <213> Homop sapiens
<400> 10
   gggtggccct gcacaggccc gatgtctact tgctgccacc agcccgggag cagctgaacc 60
   tgcgggagtc ggccaccatc acgtgcctgg tgacgggctt ctctcccgcg gacgtcttcg 120
   tgcagtggat gcagaggggg cagcccttgt ccccggagaa gtatgtgacc agcgccccaa 180
   tgcctgagcc ccaggcccca ggccggtact tcgcccacag catcctgacc gtgtccgaag 240
   aggaatggaa cacgggggag acctacacct gcgtggtggc ccatgaggcc ctgcccaaca 300
   gggtcaccga gaggaccgtg gacaagtcca ccggtaaacc caccctgtac aacgtgtccc 360
   tggtcatgtc cgacacagct ggcacctgct ac                               392
<210> 11
   <211> 105
   <212> PRT
   <213> Homo sapiens
<400> 11
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Val Leu Ile
           35                  40                  45
   Tyr Ala Ala Ser Thr Leu Arg Ser Gly Val Pro Ser Arg Phe Ser Gly
       50                  55                  60
   Ser Gly Ser Gly Thr Asp Phe Thr Leu Ser Val Ser Ser Leu Gln Pro
   65                  70                  75                  80
   Glu Asp Phe Ala Thr Tyr Tyr Cys Gln Gln Ser Tyr His Thr Pro Trp
                   85                  90                  95
   Thr Phe Gly Gln Gly Thr Lys Val Glu
               100                 105
<210> 12
   <211> 315
   <212> DNA
   <213> Homo sapiens
<400> 12
   gacatccaga tgacccagtc tccatcctcc ttgtctgcgt ctgtaggaga cagagtcacc 60
   atcacttgcc gggcaagtca gagtattagt agttatctaa attggtatca gcagaaacca 120
   gggaaagccc ctaaggtcct gatctatgct gcatccactt tgcgaagtgg ggtcccgtca 180
   aggttcagtg gcagtggatc tgggacagat ttcactctca gcgtcagcag tctgcaacct 240
   gaagattttg caacttacta ctgtcaacag agttaccata ccccgtggac gttcggtcag 300
   gggaccaagg tggaa                                                  315
<210> 13
   <211> 109
   <212> PRT
   <213> Homo sapiens
<400> 13
   Ile Lys Arg Thr Val Ala Ala Pro Ser Val Phe Ile Phe Pro Pro Ser
    1               5                  10                  15
   Asp Glu Gln Leu Lys Ser Gly Thr Ala Ser Val Val Cys Leu Leu Asn
               20                  25                  30
   Asn Phe Tyr Pro Arg Glu Ala Lys Val Gln Trp Lys Val Asp Asn Ala
           35                  40                  45
   Leu Gln Ser Gly Asn Ser Gln Glu Ser Val Thr Glu Gln Asp Ser Lys
       50                  55                  60
   Asp Ser Thr Tyr Ser Leu Ser Ser Thr Leu Thr Leu Ser Lys Ala Asp
   65                  70                  75                  80
   Tyr Glu Lys His Lys Val Tyr Ala Cys Glu Val Thr His Gln Gly Leu
                   85                  90                  95
   Ser Ser Pro Val Thr Lys Ser Phe Asn Arg Gly Glu Cys
               100                 105
<210> 14
   <211> 330
   <212> DNA
   <213> Homo sapiens
<400> 14
   atcaaacgaa ctgtggctgc accatctgtc ttcatcttcc cgccatctga tgagcagttg 60
   aaatctggaa ctgcctctgt tgtgtgcctg ctgaataact tctatcccag agaggccaaa 120
   gtacagtgga aggtggataa cgccctccaa tcgggtaact cccaggagag tgtcacagag 180
   caggacagca aggacagcac ctacagcctc agcagcaccc tgacgctgag caaagcagac 240
   tacgagaaac acaaagtcta cgcctgcgaa gtcacccatc agggcctgag ctcgcccgtc 300
   acaaagagct tcaacagggg agagtgttag                                  330
<210> 15
   <211> 159
   <212> PRT
   <213> Homo sapiens
<400> 15
   Met Lys Asn His Leu Leu Phe Trp Gly Val Leu Ala Val Phe Ile Lys
    1               5                  10                  15
   Ala Val His Val Lys Ala Gln Glu Asp Glu Arg Ile Val Leu Val Asp
               20                  25                  30
   Asn Lys Cys Lys Cys Ala Arg Ile Thr Ser Arg Ile Ile Arg Ser Ser
           35                  40                  45
   Glu Asp Pro Asn Glu Asp Ile Val Glu Arg Asn Ile Arg Ile Ile Val
       50                  55                  60
   Pro Leu Asn Asn Arg Glu Asn Ile Ser Asp Pro Thr Ser Pro Leu Arg
   65                  70                  75                  80
   Thr Arg Phe Val Tyr His Leu Ser Asp Leu Cys Lys Lys Cys Asp Pro
                   85                  90                  95
   Thr Glu Val Glu Leu Asp Asn Gln Ile Val Thr Ala Thr Gln Ser Asn
               100                 105                 110
   Ile Cys Asp Glu Asp Ser Ala Thr Glu Thr Cys Tyr Thr Tyr Asp Arg
           115                 120                 125
   Asn Lys Cys Tyr Thr Ala Val Val Pro Leu Val Tyr Gly Gly Glu Thr
       130                 135                 140
   Lys Met Val Glu Thr Ala Leu Thr Pro Asp Ala Cys Tyr Pro Asp
   145                 150                 155
<210> 16
   <211> 480
   <212> DNA
   <213> Homo sapiens
<400> 16
   atgaagaacc atttgctttt ctggggagtc ctggcggttt ttattaaggc tgttcatgtg 60
   aaagcccaag aagatgaaag gattgttctt gttgacaaca aatgtaagtg tgcccggatt 120
   acttccagga tcatccgttc ttccgaagat cctaatgagg acattgtgga gagaaacatc 180
   cgaattattg ttcctctgaa caacagggag aatatctctg atcccacctc accattgaga 240
   accagatttg tgtaccattt gtctgacctc tgtaaaaaat gtgatcctac agaagtggag 300
   ctggataatc agatagttac tgctacccag agcaatatct gtgatgaaga cagtgctaca 360
   gagacctgct acacttatga cagaaacaag tgctacacag ctgtggtccc actcgtatat 420
   ggtggtgaga ccaaaatggt ggaaacagcc ttaaccccag atgcctgcta tcctgactaa 480
<210> 17
   <211> 133
   <212> PRT
   <213> Homo sapiens
<400> 17
   Val Gln Leu Leu Glu Ser Gly Gly Gly Leu Val Gln Pro Gly Gly Ser
    1               5                  10                  15
   Leu Arg Leu Ser Cys Ala Ala Ser Gly Phe Thr Phe Ser Thr Tyr Ala
               20                  25                  30
   Met Ser Trp Val Arg Gln Ala Pro Gly Glu Gly Leu Glu Trp Val Ser
           35                  40                  45
   Asp Ile Asn Val Gly Gly Val Thr Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Val Glu Asp Thr Ala Met Tyr Tyr Cys Val
                   85                  90                  95
   Arg Arg Ser Gly Pro Asp Arg Asn Ser Ser Pro Ala Asp Phe Trp Gly
               100                 105                 110
   Gln Gly Ser Leu Val Ile Val Ser Ser Gly Ser Ala Ser Ala Pro Thr
           115                 120                 125
   Leu Phe Pro Leu Val
       130
<210> 18
   <211> 402
   <212> DNA
   <213> Homo sapiens
<400> 18
   gtgcagctgt tggagtctgg gggaggcttg gtacagcctg gggggtccct gagactctcc 60
   tgtgcagcct ctggattcac ctttagcacc tatgccatga gctgggtccg ccaggctcca 120
   ggggaggggc tggagtgggt ctcagatatt aatgttggtg gtgttaccac atactacgca 180
   gactccgtga agggccggtt caccatctcc agagacaatt ccaagaacac gctgtatcta 240
   caaatgaaca gcctgagagt agaggacacg gccatgtatt actgtgtgag gcggtccggg 300
   cccgatcgca actcgtcgcc cgctgacttc tggggccagg gatccctggt catcgtctcc 360
   tcagggagtg catccgcccc aacccttttc cccctcgtct ga                   402
<210> 19
   <211> 104
   <212> PRT
   <213> Homo sapiens
<400> 19
   Gly Ser Ala Ser Ala Pro Thr Leu Phe Pro Leu Val Ser Cys Glu Asn
    1               5                  10                  15
   Ser Pro Ser Asp Thr Ser Ser Val Ala Val Gly Cys Leu Ala Gln Asp
               20                  25                  30
   Phe Leu Pro Asp Ser Ile Thr Phe Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro
                                   100
<210> 20
   <211> 310
   <212> DNA
   <213> Homo sapiens
<400> 20
   gagtgcatcc gccccaaccc ttttccccct cgtctcctgt gagaattccc cgtcggatac 60
   gagcagcgtg gccgttggct gcctcgcaca ggacttcctt cccgactcca tcactttctc 120
   ctggaaatac aagaacaact ctgacatcag cagcacccgg ggcttcccat cagtcctgag 180
   agggggcaag tacgcagcca cctcacaggt gctgctgcct tccaaggacg tcatgcaggg 240
   cacagacgaa cacgtggtgt gcaaagtcca gcaccccaac ggcaacaaag aaaagaacgt 300
   gcctcttcca                                                        310
<210> 21
   <211> 112
   <212> PRT
   <213> Homo sapiens
<400> 21
   Val Ile Ala Glu Leu Pro Pro Lys Val Ser Val Phe Val Pro Pro Arg
    1               5                  10                  15
   Asp Gly Phe Phe Gly Asn Pro Arg Lys Ser Lys Leu Ile Cys Gln Ala
               20                  25                  30
   Thr Gly Phe Ser Pro Arg Gln Ile Gln Val Ser Trp Leu Arg Glu Gly
           35                  40                  45
   Lys Gln Val Gly Ser Gly Val Thr Thr Asp Gln Val Gln Ala Glu Ala
       50                  55                  60
   Lys Glu Ser Gly Pro Thr Thr Tyr Lys Val Thr Ser Thr Leu Thr Ile
   65                  70                  75                  80
   Lys Glu Ser Asp Trp Leu Ser Gln Ser Met Phe Thr Cys Arg Val Asp
                   85                  90                  95
   His Arg Gly Leu Thr Phe Gln Gln Asn Ala Ser Ser Met Cys Val Pro
               100                 105                 110
<210> 22
   <211> 336
   <212> DNA
   <213> Homo sapiens
<400> 22
   gtgattgctg agctgcctcc caaagtgagc gtcttcgtcc caccccgcga cggcttcttc 60
   ggcaaccccc gcaagtccaa gctcatctgc caggccacgg gtttcagtcc ccggcagatt 120
   caggtgtcct ggctgcgcga ggggaagcag gtggggtctg gcgtcaccac ggaccaggtg 180
   caggctgagg ccaaagagtc tgggcccacg acctacaagg tgaccagcac actgaccatc 240
   aaagagagcg actggctcag ccagagcatg ttcacctgcc gcgtggatca caggggcctg 300
   accttccagc agaatgcgtc ctccatgtgt gtcccc                           336
<210> 23
   <211> 106
   <212> PRT
   <213> Homo sapiens
<400> 23
   Asp Gln Asp Thr Ala Ile Arg Val Phe Ala Ile Pro Pro Ser Phe Ala
    1               5                  10                  15
   Ser Ile Phe Leu Thr Lys Ser Thr Lys Leu Thr Cys Leu Val Thr Asp
               20                  25                  30
   Leu Thr Thr Tyr Asp Ser Val Thr Ile Ser Trp Thr Arg Gln Asn Gly
           35                  40                  45
   Glu Ala Val Lys Thr His Thr Asn Ile Ser Glu Ser His Pro Asn Ala
       50                  55                  60
   Thr Phe Ser Ala Val Gly Glu Ala Ser Ile Cys Glu Asp Asp Trp Asn
   65                  70                  75                  80
   Ser Gly Glu Arg Phe Thr Cys Thr Val Thr His Thr Asp Leu Pro Ser
                   85                  90                  95
   Pro Leu Lys Gln Thr Ile Ser Arg Pro Lys
               100                 105
<210> 24
   <211> 318
   <212> DNA
   <213> Homo sapiens
<400> 24
   gatcaagaca cagccatccg ggtcttcgcc atccccccat cctttgccag catcttcctc 60
   accaagtcca ccaagttgac ctgcctggtc acagacctga ccacctatga cagcgtgacc 120
   atctcctgga cccgccagaa tggcgaagct gtgaaaaccc acaccaacat ctccgagagc 180
   caccccaatg ccactttcag cgccgtgggt gaggccagca tctgcgagga tgactggaat 240
   tccggggaga ggttcacgtg caccgtgacc cacacagacc tgccctcgcc actgaagcag 300
   accatctccc ggcccaag                                                318
<210> 25
   <211> 131
   <212> PRT
   <213> Homo sapiens
<400> 25
   Gly Val Ala Leu His Arg Pro Asp Val Tyr Leu Leu Pro Pro Ala Arg
    1               5                   10                  15
   Glu Gln Leu Asn Leu Arg Glu Ser Ala Thr Ile Thr Cys Leu Val Thr
               20                  25                  30
   Gly Phe Ser Pro Ala Asp Val Phe Val Gln Trp Met Gln Arg Gly Gln
           35                  40                  45
   Pro Leu Ser Pro Glu Lys Tyr Val Thr Ser Ala Pro Met Pro Glu Pro
       50                  55                  60
   Gln Ala Pro Gly Arg Tyr Phe Ala His Ser Ile Leu Thr Val Ser Glu
   65                  70                  75                  80
   Glu Glu Trp Asn Thr Gly Glu Thr Tyr Thr Cys Val Val Ala His Glu
                   85                  90                  95
   Ala Leu Pro Asn Arg Val Thr Glu Arg Thr Val Asp Lys Ser Thr Gly
               100                 105                 110
   Lys Pro Thr Leu Tyr Asn Val Ser Leu Val Met Ser Asp Thr Ala Gly
           115                 120                 125
   Thr Cys Tyr
       130
<210> 26
   <211> 392
   <212> DNA
   <213> Homo sapiens
<400> 26
   gggtggccct gcacaggccc gatgtctact tgctgccacc agcccgggag cagctgaacc 60
   tgcgggagtc ggccaccatc acgtgcctgg tgacgggctt ctctcccgcg gacgtcttcg 120
   tgcagtggat gcagaggggg cagcccttgt ccccggagaa gtatgtgacc agcgccccaa 180
   tgcctgagcc ccaggcccca ggccggtact tcgcccacag catcctgacc gtgtccgaag 240
   aggaatggaa cacgggggag acctacacct gcgtggtggc ccatgaggcc ctgcccaaca 300
   gggtcaccga gaggaccgtg gacaagtcca ccggtaaacc caccctgtac aacgtgtccc 360
   tggtcatgtc cgacacagct ggcacctgct ac                               392
<210> 27
   <211> 105
   <212> PRT
   <213> Homo sapiens
<210> 28
   <211> 315
   <212> DNA
   <213> Homo sapiens
<400> 28
   gacatccaga tgacccagtc tccatcctcc ctgtctgcat ctgtaggaga cagagtcacc 60
   atcacttgcc gggcgagtca gggcattggc aattatttag cctggtatca gcagaaacca 120
   gggaaagttc ctaaactcct gatctatact acatccattt tgcaatcagg ggtcccatct 180
   cgattcagtg gcagtggatc tgggacagat ttcactctca ccatcagcag cctgcagcct 240
   gaagattttg caacttatta ctgtcaaaaa tataacagtg ccccgcggac gttcggccaa 300
   gggaccaggg tggac                                                  315
<210> 29
   <211> 109
   <212> PRT
   <213> Homo sapiens
<400> 29
   Ile Lys Arg Thr Val Ala Ala Pro Ser Val Phe Ile Phe Pro Pro Ser
    1               5                  10                  15
   Asp Glu Gln Leu Lys Ser Gly Thr Ala Ser Val Val Cys Leu Leu Asn
               20                  25                  30
   Asn Phe Tyr Pro Arg Glu Ala Lys Val Gln Trp Lys Val Asp Asn Ala
           35                  40                  45
   Leu Gln Ser Gly Asn Ser Gln Glu Ser Val Thr Glu Gln Asp Ser Lys
       50                  55                  60
   Asp Ser Thr Tyr Ser Leu Ser Ser Thr Leu Thr Leu Ser Lys Ala Asp
   65                  70                  75                  80
   Tyr Glu Lys His Lys Val Tyr Ala Cys Glu Val Thr His Gln Gly Leu
                   85                  90                  95
   Ser Ser Pro Val Thr Lys Ser Phe Asn Arg Gly Glu Cys
               100                 105
<210> 30
   <211> 330
   <212> DNA
   <213> Homo sapiens
<400> 30
   atcaaacgaa ctgtggctgc accatctgtc ttcatcttcc cgccatctga tgagcagttg 60
   aaatctggaa ctgcctctgt tgtgtgcctg ctgaataact tctatcccag agaggccaaa 120
   gtacagtgga aggtggataa cgccctccaa tcgggtaact cccaggagag tgtcacagag 180
   caggacagca aggacagcac ctacagcctc agcagcaccc tgacgctgag caaagcagac 240
   tacgagaaac acaaagtcta cgcctgcgaa gtcacccatc agggcctgag ctcgcccgtc 300
   acaaagagct tcaacagggg agagtgttag                                  330
<210> 31
   <211> 8
   <212> PRT
   <213> Artificial Sequence
<220>
   <223> Heavy Chain CDR1
<400> 31
   Gly Gly Ser Val Ser Leu Tyr Tyr
    1               5
<210> 32
   <211> 9
   <212> PRT
   <213> Artificial Sequence
<220>
   <223> Heavy Chain CDR2
<400> 32
   Gly Tyr Ile Tyr Ser Ser Gly Ser Thr
   1               5
<210> 33
   <211> 11
   <212> PRT
   <213> Artificial Sequence
<220>
   <223> Heavy Chain CDR3
<400> 33
   Ala Arg Ser Ala Ser Ile Arg Gly Trp Phe Asp
   1               5                   10
<210> 34
   <211> 6
   <212> PRT
   <213> Artificial Sequence
<220>
   <223> Light Chain CDR1
<400> 34
   Gln Ser Ile Ser Ser Tyr
   1               5
<210> 35
   <211> 3
   <212> PRT
   <213> Artificial Sequence
<220>
   <223> Light Chain CDR2
<400> 35
   Ala Ala Ser
   1
<210> 36
   <211> 8
   <212> PRT
   <213> Artificial Sequence
<220>
   <223> Light Chain CDR3
<400> 36
   Gln Gln Ser Tyr His Thr Pro Trp
   1 5
<210> 37
   <211> 8
   <212> PRT
   <213> Artificial Sequence
<220>
   <223> Heavy Chain CDR1
<400> 37
   Gly Phe Thr Phe Ser Thr Tyr Ala
   1 5
<210> 38
   <211> 8
   <212> PRT
   <213> Artificial Sequence
<220>
   <223> Heavy Chain CDR2
<400> 38
   Ile Asn Val Gly Gly Val Thr Thr
   1 5
<210> 39
   <211> 15
   <212> PRT
   <213> Artificial Sequence
<220>
   <223> Heavy Chain CDR3
<400> 39
   Val Arg Arg Ser Gly Pro Asp Arg Asn Ser Ser Pro Ala Asp Phe
   1 5 10 15
<210> 40
   <211> 4
   <212> PRT
   <213> Artificial Sequence
<220>
   <223> Light Chain CDR1
<400> 40
   Gln Gly Ile Gly
   1
<210> 41
   <211> 3
   <212> PRT
   <213> Artificial Sequence
<220>
   <223> Light Chain CDR2
<400> 41
   Thr Thr Ser
   1
<210> 42
   <211> 9
   <212> PRT
   <213> Artificial Sequence
<220>
   <223> Light Chain CDR3
<400> 42
   Gln Lys Tyr Asn Ser Ala Pro Arg Thr
   1               5
<210> 43
   <211> 119
   <212> PRT
   <213> Homo sapiens
<220>
   <221> VARIANT
   <222> 50
   <223> Xaa can be Val or Ile
<221> VARIANT
   <222> 89
   <223> Xaa can be Asp or Glu
<400> 43
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Ser
               20                  25                  30
   Gly Met His Trp Val Arg Gln Ala Pro Gly Lys Gly Leu Glu Trp Val
           35                  40                  45
   Ala Xaa Ile Ser Tyr Asp Gly Ser Arg Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Thr Ala Xaa Asp Thr Ala Val Tyr Tyr Cys
                           85                  90                  95
   Ala Lys Gly Val Thr Gly Ser Pro Thr Leu Asp Tyr Trp Gly Gln Gly
               100                 105                 110
   Thr Leu Val Thr Val Ser Ser
           115
<210> 44
   <211> 114
   <212> PRT
   <213> Homo sapiens
<220>
   <221> VARIANT
   <222> 46
   <223> Xaa can be Arg or Lys
<221> VARIANT
   <222> 90
   <223> Xaa can be Gly or Glu
<400> 44
   Gln Ser Val Leu Thr Gln Pro Pro Ser Val Ser Ala Ala Pro Gly Gln
    1               5                  10                  15
   Lys Val Thr Ile Ser Cys Ser Gly Ser Ser Ser Asn Ile Gly Asn Asn
               20                  25                  30
   Phe Val Ser Trp Tyr Gln Gln Leu Pro Gly Thr Ala Pro Xaa Leu Leu
           35                  40                  45
   Ile Tyr Asp Ile Thr Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Tyr Tyr Cys Xaa Thr Trp Asp Ser Ser Leu
                   85                  90                  95
   Ser Ala Val Val Phe Gly Gly Gly Thr Lys Leu Thr Val Leu Gly Gln
               100                 105                 110
   Pro Lys
<210> 45
   <211> 124
   <212> PRT
   <213> Homo sapiens
<400> 45
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Thr Trp Val Arg Gln Ala Pro Gly Lys Gly Leu Glu Met Val
           35                  40                  45
   Ala Asn Ile Lys Lys Asp Gly Ser Glu Lys Ser Tyr Val Asp Ser Val
       50                  55                  60
   Lys Gly Arg Phe Thr Thr Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Pro Asn Cys Gly Gly Asp Cys Tyr Leu Pro Trp Tyr Phe Asp
               100                 105                 110
   Leu Trp Gly Arg Gly Thr Leu Val Thr Val Ser Ser
           115                 120
<210> 46
   <211> 122
   <212> PRT
   <213> Homo sapiens
<400> 46
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Asn Thr Pro Gln Ala Phe Gly Gln Gly Thr Lys Val Glu Ile
               100                 105                 110
   Lys Arg Thr Val Ala Ala Pro Ser Val Phe
           115                 120
<210> 47
   <211> 5
   <212> PRT
   <213> Artificial Sequence
<220>
   <223> Heavy Chain CDR1
<400> 47
   Ser Ser Gly Met His
   1               5
<210> 48
   <211> 17
   <212> PRT
   <213> Artificial Sequence
<220>
   <223> Heavy Chain CDR2
<221> VARIANT
   <222> (1) ... (1)
   <223> Xaa can be Val or Ile
<400> 48
   Xaa Ile Ser Tyr Asp Gly Ser Arg Lys Tyr Tyr Ala Asp Ser Val Lys
    1               5                   10                  15
   Gly
<210> 49
   <211> 10
   <212> PRT
   <213> Artificial Sequence
<220>
   <223> Heavy Chain CDR3
<400> 49
   Gly Val Thr Gly Ser Pro Thr Leu Asp Tyr
    1               5                   10
<210> 50
   <211> 13
   <212> PRT
   <213> Artificial Sequence
<220>
   <223> Light Chain CDR1
<400> 50
   Ser Gly Ser Ser Ser Asn Ile Gly Asn Asn Phe Val Ser
    1               5                   10
<210> 51
   <211> 7
   <212> PRT
   <213> Artificial Sequence
<220>
   <223> Light Chain CDR2
<400> 51
   Asp Ile Thr Lys Arg Pro Ser
    1               5
<210> 52
   <211> 11
   <212> PRT
   <213> Artificial Sequence
<220>
   <223> Light Chain CDR3
<221> VARIANT
   <222> (1) ... (1)
   <223> Xaa can be Gly or Glu
<400> 52
   Xaa Thr Trp Asp Ser Ser Leu Ser Ala Val Val
   1 5 10
<210> 53
   <211> 9
   <212> PRT
   <213> Artificial Sequence
<220>
   <223> Heavy Chain CDR1
<400> 53
   Ser Gly Phe Thr Phe Ser Ser Tyr Trp
   1 5
<210> 54
   <211> 8
   <212> PRT
   <213> Artificial Sequence
<220>
   <223> Heavy Chain CDR2
<400> 54
   Ile Lys Lys Asp Gly Ser Glu Lys
   1 5
<210> 55
   <211> 16
   <212> PRT
   <213> Artificial Sequence
<220>
   <223> Heavy Chain CDR3
<400> 55
   Ala Arg Pro Asn Cys Gly Gly Asp Cys Tyr Leu Pro Trp Tyr Phe Asp
   1 5 10 15
<210> 56
   <211> 12
   <212> PRT
   <213> Artificial Sequence
<220>
   <223> Light Chain CDR1
<400> 56
   Gln Ser Val Leu Tyr Ser Ser Asn Asn Lys Asn Tyr
   1 5 10
<210> 57
   <211> 4
   <212> PRT
   <213> Artificial Sequence
<220>
   <223> Light Chain CDR2
<400> 57
   Tyr Trp Ala Ser
   1
<210> 58
   <211> 9
   <212> PRT
   <213> Artificial Sequence
<220>
   <223> Light Chain CDR3
<400> 58
   Gln Gln Tyr Tyr Asn Thr Pro Gln Ala
   1 5
<210> 59
   <211> 16
   <212> DNA
   <213> Artificial Sequence
<220>
   <223> 5' primer
<400> 59
   tccggacggt ccggga 16
<210> 60
   <211> 102
   <212> DNA
   <213> Artificial Sequence
<220>
   <223> primer
<400> 60
   tccggacggt ccgggacctc ctgtgcaaga acatgaaaca tctgtggttc ttccttctcc 60
   tggtggcagc tcccagatgt gagtatctca gggatccaga ca 102
<210> 61
   <211> 114
   <212> DNA
   <213> Artificial Sequence
<220>
   <223> primer
<400> 61
   cagggatcca gacatgggga tatgggaggt gcctctgatc ccagggctca ctgtgggtct 60
   ctctgttcac aggggtcctg tcccaggtgc agctgcagga gtcgggccca ggac 114
<210> 62
   <211> 56
   <212> DNA
   <213> Artificial Sequence
<220>
   <223> 3' primer
<400> 62
   ccttaattaa gacctggaga ggccattctt acctgaggag acggtgacca gggttc 56
<210> 63
   <211> 16
   <212> DNA
   <213> Artificial Sequence
<220>
   <223> 5' primer
<400> 63
   cgaactgtgg ctgcac 16
<210> 64
   <211> 30
   <212> DNA
   <213> Artificial Sequence
<220>
   <223> 3' primer
<400> 64
   ccgctcgagt atctaacact ctcccctgtt 30
<210> 65
   <211> 117
   <212> DNA
   <213> Artificial Sequence
<220>
   <223> primer
<400> 65
   agcattacta gctagctcaa gactcagcct ggacatggac atgagggtcc ccgctcagct 60
   cctggggctc ctgctactct ggctccgagg tgccagatgt gacatccaga tgaccca 117
<210> 66
   <211> 4933
   <212> DNA
   <213> Artificial Sequence
<220>
   <223> vector sequence
<400> 66
   tttccacacc tggttgctga ctaattgaga tgcatgcttt gcatacttct gcctgctggg 60
   gagcctgggg actttccaca ccctaactga cacacattcc acagctgcct cgcgcgtttc 120
   ggtgatgacg gtgaaaacct ctgacacatg cagctcccgg agacggtcac agcttgtctg 180
   taagcggatg ccgggagcag acaagcccgt cagggcgcgt cagcgggtgt tggcgggtgt 240
   cggggcgcag ccatgaccca gtcacgtagc gatagcggag tgtatactgg cttaactatg 300
   cggcatcaga gcagattgta ctgagagtgc accatatgcg gtgtgaaata ccgcacagat 360
   gcgtaaggag aaaataccgc atcaggcgct cttccgcttc ctcgctcact gactcgctgc 420
   gctcggtcgt tcggctgcgg cgagcggtat cagctcactc aaaggcggta atacggttat 480
   ccacagaatc aggggataac gcaggaaaga acatgtgagc aaaaggccag caaaaggcca 540
   ggaaccgtaa aaaggccgcg ttgctggcgt ttttccatag gctccgcccc cctgacgagc 600
   atcacaaaaa tcgacgctca agtcagaggt ggcgaaaccc gacaggacta taaagatacc 660
   aggcgtttcc ccctggaagc tccctcgtgc gctctcctgt tccgaccctg ccgcttaccg 720
   gatacctgtc cgcctttctc ccttcgggaa gcgtggcgct ttctcatagc tcacgctgta 780
   ggtatctcag ttcggtgtag gtcgttcgct ccaagctggg ctgtgtgcac gaaccccccg 840
   ttcagcccga ccgctgcgcc ttatccggta actatcgtct tgagtccaac ccggtaagac 900
   acgacttatc gccactggca gcagccactg gtaacaggat tagcagagcg aggtatgtag 960
   gcggtgctac agagttcttg aagtggtggc ctaactacgg ctacactaga aggacagtat 1020
   ttggtatctg cgctctgctg aagccagtta ccttcggaaa aagagttggt agctcttgat 1080
   ccggcaaaca aaccaccgct ggtagcggtg gtttttttgt ttgcaagcag cagattacgc 1140
   gcagaaaaaa aggatctcaa gaagatcctt tgatcttttc tacggggtct gacgctcagt 1200
   ggaacgaaaa ctcacgttaa gggattttgg tcatgagatt atcaaaaagg atcttcacct 1260
   agatcctttt aaattaaaaa tgaagtttta aatcaatcta aagtatatat gagtaaactt 1320
   ggtctgacag ttaccaatgc ttaatcagtg aggcacctat ctcagcgatc tgtctatttc 1380
   gttcatccat agttgcctga ctccccgtcg tgtagataac tacgatacgg gagggcttac 1440
   catctggccc cagtgctgca atgataccgc gagacccacg ctcaccggct ccagatttat 1500
   cagcaataaa ccagccagcc ggaagggccg agcgcagaag tggtcctgca actttatccg 1560
   cctccatcca gtctattaat tgttgccggg aagctagagt aagtagttcg ccagttaata 1620
   gtttgcgcaa cgttgttgcc attgctgcag gcatcgtggt gtcacgctcg tcgtttggta 1680
   tggcttcatt cagctccggt tcccaacgat caaggcgagt tacatgatcc cccatgttgt 1740
   gcaaaaaagc ggttagctcc ttcggtcctc cgatcgttgt cagaagtaag ttggccgcag 1800
   tgttatcact catggttatg gcagcactgc ataattctct tactgtcatg ccatccgtaa 1860
   gatgcttttc tgtgactggt gagtactcaa ccaagtcatt ctgagaatag tgtatgcggc 1920
   gaccgagttg ctcttgcccg gcgtcaacac gggataatac cgcgccacat agcagaactt 1980
   taaaagtgct catcattgga aaacgttctt cggggcgaaa actctcaagg atcttaccgc 2040
   tgttgagatc cagttcgatg taacccactc gtgcacccaa ctgatcttca gcatctttta 2100
   ctttcaccag cgtttctggg tgagcaaaaa caggaaggca aaatgccgca aaaaagggaa 2160
   taagggcgac acggaaatgt tgaatactca tactcttcct ttttcaatat tattgaagca 2220
   tttatcaggg ttattgtctc atgagcggat acatatttga atgtatttag aaaaataaac 2280
   aaataggggt tccgcgcaca tttccccgaa aagtgccacc tgacgtctaa gaaaccatta 2340
   ttatcatgac attaacctat aaaaataggc gtatcacgag gccctttcgt cttcaagaat 2400
   tcctttgcct aatttaaatg aggacttaac ctgtggaaat attttgatgt gggaagctgt 2460
   tactgttaaa actgaggtta ttggggtaac tgctatgtta aacttgcatt cagggacaca 2520
   aaaaactcat gaaaatggtg ctggaaaacc cattcaaggg tcaaattttc atttttttgc 2580
   tgttggtggg gaacctttgg agctgcaggg tgtgttagca aactacagga ccaaatatcc 2640
   tgctcaaact gtaaccccaa aaaatgctac agttgacagt cagcagatga acactgacca 2700
   caaggctgtt ttggataagg ataatgctta tccagtggag tgctgggttc ctgatccaag 2760
   taaaaatgaa aacactagat attttggaac ctacacaggt ggggaaaatg tgcctcctgt 2820
   tttgcacatt actaacacag caaccacagt gcttcttgat gagcagggtg ttgggccctt 2880
   gtgcaaagct gacagcttgt atgtttctgc tgttgacatt tgtgggctgt ttaccaacac 2940
   ttctggaaca cagcagtgga agggacttcc cagatatttt aaaattaccc ttagaaagcg 3000
   gtctgtgaaa aacccctacc caatttcctt tttgttaagt gacctaatta acaggaggac 3060
   acagagggtg gatgggcagc ctatgattgg aatgtcctct caagtagagg aggttagggt 3120
   ttatgaggac acagaggagc ttcctgggga tccctaggtg ggactgtctc cctttggact 3180
   ttccttcaat atcttctttt aatatctttt gtgtcaagat acagaagtga tagaaagaat 3240
   ttgacagtaa tccatcattt ccttagacat gggaaaagtt atgaagaatt gataaggaat 3300
   tttttttgta agtctgacac attcaacagt gacattctct gttcctaggc ttttggttaa 3360
   ggtgctgctt ttaagaacaa catgaatttc atttaaaatt catgtttttc cattatttac 3420
   accttaagga cttaattcta gtagataata taatattaga gattttcttt tgtccagttt 3480
   atctagcatt gttagagtgt aagtttttat attaattaca attttaattt agagtgtgtg 3540
   tatactgttc agatgaagaa tgagtcaagc aaatgtgact attattatca tttgtagaac 3600
   tattatccag gattactgtg tatatgacca gataccattc aagtgtcagg acaccaattg 3660
   attacccatt tataccttga tgtcttgtga gtctcttcaa gttttgtagc cttacattga 3720
   acaaggcttc agtggatgaa cttggaatga tcagagtcag gaggcacaaa gcctgtgaga 3780
   taagaggtgg gaaaggcaac ttatagcatt aaaggtgcaa gcacattcag tggtatgggg 3840
   ggggcgggaa tactggcaaa gccaggaagt ctgtcagcac ttggatacaa gagtctagag 3900
   gagtgcttgt cctatgaggt gagagcaatg tgtacttaat agctcagaaa agcaagccaa 3960
   tggagaatag tgctaatgat gaggggagga gaaaggcaac atgagagaga agagaaggag 4020
   attacaggaa gaaattaagc tgtgggagag gaaggggctg gactaccaga catgcaggat 4080
   aggcaagttt caatgtgacc agatgggtcc tttttgtgag aaaaagcaga cctagatgtt 4140
   gatgagagaa aggcaagtcc ttggtgatta tcagaacaca aacatatagc atgcaaaatt 4200
   atctttacgt ggtacttagg gaggtcacaa atgagctgga catgcatttg gaatagagag 4260
   gaggtagtct atatggtgga tgagaagttc agcactggag gaactaagtg atacacacag 4320
   acccccagtg tatattcact ttttaaatca agaaataatg tcaagtataa tccatatcta 4380
   ttttatgaag ttctatgatt aaattttaag ctgggcgcgc tataccttat tttggattga 4440
   agccaatatg ataatgaggg ggtggagttt gtgacgtggc gcggggcgtg gaacggggcg 4500
   ggtgacgtag tagtgtggcg gaagtgtgat gttgcaagtg tggcggaaca catgtaagcg 4560
   acggatgtgg caaaagtgac gtttttggtg tgcgccggtg tacacaggaa gtgacaattt 4620
   tcgcgcggtt ttaggcggat gttgtagtaa atttgggcgt aaccgagtaa gatttggcca 4680
   ttttcgcggg aaaactgaat aagaggaagt gaaatctgaa taattttgtg ttactcatag 4740
   cgcgtaatat ttgtctaggg ccgcggggac tttgaccgtt tacgtggaga ctcgcccagg 4800
   tgtttttctc aggtgttttc cgcgttccgg gtcaaagttg gcgttttatt attatagtca 4860
   gcatgcatgc tcgagcggcc gcgacgcgca gtgtatttat atccggacgg tccgggacct 4920
   cctgtgcaag aac                                                    4933
<210> 67
   <211> 45
   <212> DNA
   <213> Artificial Sequence
<220>
   <223> leader sequence
<400> 67
   atgaaacatc tgtggttctt ccttctcctg gtggcagctc ccaga 45
<210> 68
   <211> 15
   <212> PRT
   <213> Artificial Sequence
<220>
   <223> leader sequence
<400> 68
   Met Lys His Leu Trp Phe Phe Leu Leu Leu Val Ala Ala Pro Arg
   1 5 10 15
<210> 69
   <211> 83
   <212> DNA
   <213> Artificial Sequence
<220>
   <223> vector sequence
<400> 69
   gtgagtatct cagggatcca gacatgggga tatgggaggt gcctctgatc ccagggctca 60
   ctgtgggtct ctctgttcac agg 83
<210> 70
   <211> 12
   <212> DNA
   <213> Homo sapiens
<400> 70
   tgggtcctgt cc 12
<210> 71
   <211> 4
   <212> PRT
   <213> Homo sapiens
<400> 71
   Trp Val Leu Ser
   1
<210> 72
   <211> 4345
   <212> **DNA**
   <213> Homo sapiens
<400> 72
<210> 73
   <211> 90
   <212> DNA
   <213> Homo sapiens
<400> 73
   ggtgagggcc gggcccagcc accgggacag agagggagcc gaagggggcg ggagtggcgg 60
   gcaccgggct gacacgtgtc cctcactgca                                   90
<210> 74
   <211> 245
   <212> DNA
   <213> Homo sapiens
<400> 74
   ggtgagtgac ctgtccccag gggcagcacc caccgacaca caggggtcca ctcgggtctg 60
   gcattcgcca ccccggatgc agccatctac tccctgagcc ttggcttccc agagcggcca 120
   agggcagggg ctcgggcggc aggacccctg ggctcggcag aggcagttgc tactctttgg 180
   gtgggaacca tgcctccgcc cacatccaca cctgccccac ctctgactcc cttctcttga 240
   ctcca                                                              245
<210> 75
   <211> 180
   <212> DNA
   <213> Homo sapiens
<400> 75
   ggtaggcccc actcttgccc ctcttcctgc actccctggg acctcccttg gcctctgggg 60
   catggtggaa agcacccctc actcccccgt tgtctgggca actggggaaa aggggactca 120
   accccagccc acaggctggt ccccccactg ccccgccctc accaccatct ctgttcacag 180
<210> 76
   <211> 2037
   <212> DNA
   <213> Artificial Sequence
<220>
   <223> vector sequence
<400> 76
   tgaccctgct ggcctgccca caggctcggg gcggctggcc gctctgtgtg tgcatgcaaa 60
   ctaaccgtgt caacggggtg agatgttgca tcttataaaa ttagaaataa aaagatccat 120
   tcaaaagata ctggtcctga gtgcacgatg ctctggccta ctggggcggc ggctgtgctg 180
   cacccaccct gcgcctcccc tgcagaacac cttcctccac agcccccacc cctgcctcac 240
   ccacctgcgt gcctcagtgg cttctagaaa cccctgaatt ccctgcagct gctcacagca 300
   ggctgacctc agacttgcca ttcctcctac tgcttccaga aagaaagctg aaagcaagac 360
   cacacgtata caggcagcac acaggcatgt gtggatacac atggacagac acggacacac 420
   acaaacacat ggacacacag agacgtgcta acccatgggc acacacatac acagacatgg 480
   acccacacac aaacatatgt ggacacacat gtacaaacat gcacaggcac acaaagagaa 540
   cactgactac aggcacacac acacacgggc acacacatgg atatgtgcac acatggacac 600
   atacatgtgc aggacatgca cacacacaga cacactagca cagaggcata cacacacaga 660
   cacacacatt cacaaacaca catgtgcatg caaacacaca cacatgtaca gacacaagta 720
   catggacaca tgcacaccca gagacacact gacacagaca cacaggagca tgtgatacac 780
   taacacgtgg acacacacgt ctacccacag gcacacaaca gatggacacg cgtacacaga 840
   catgcacaca cccacaggca caacacgtgc gcatgccggc cgagatccct ggccagggat 900
   ccctggccag ggatccctgg ccagggatct tcaatattgg ccattagcca tattattcat 960
   tggttatata gcataaatca atattggcta ttggccattg catacgttgt atctatatca 1020
   taatatgtac atttatattg gctcatgtcc aatatgaccg ccatgttggc attgattatt 1080
   gactagttat taatagtaat caattacggg gtcattagtt catagcccat atatggagtt 1140
   ccgcgttaca taacttacgg taaatggccc gcctggctga ccgcccaacg acccccgccc 1200
   attgacgtca ataatgacgt atgttcccat agtaacgcca atagggactt tccattgacg 1260
   tcaatgggtg gagtatttac ggtaaactgc ccacttggca gtacatcaag tgtatcatat 1320
   gccaagtccg ccccctattg acgtcaatga cggtaaatgg cccgcctggc attatgccca 1380
   gtacatgacc ttacgggact ttcctacttg gcagtacatc tacgtattag tcatcgctat 1440
   taccatggtg atgcggtttt ggcagtacac caatgggcgt ggatagcggt ttgactcacg 1500
   gggatttcca agtctccacc ccattgacgt caatgggagt ttgttttggc accaaaatca 1560
   acgggacttt ccaaaatgtc gtaacaactg cgatcgcccg ccccgttgac gcaaatgggc 1620
   ggtaggcgtg tacggtggga ggtctatata agcagagctc gtttagtgaa ccgtcagatc 1680
   actagaagct ttattgcggt agtttatcac agttaaattg ctaacgcagt cagtgcttct 1740
   gacacaacag tctcgaactt aagctgcagt gactctctta aggtagcctt gcagaagttg 1800
   gtcgtgaggc actgggcagg taagtatcaa ggttacaaga caggtttaag gagaccaata 1860
   gaaactgggc ttgtcgagac agagaagact cttgcgtttc tgataggcac ctattggtct 1920
   tactgacatc cactttgcct ttctctccac aggtgtccac tcccagttca attacagctc 1980
   ttaaggctag agtacttaat acgactcact ataggctagc tcaagactca gccggac    2037
<210> 77
   <211> 66
   <212> DNA
   <213> Homo sapiens
<400> 77
   atggacatga gggtccccgc tcagctcctg gggctcctgc tactctggct ccgaggtgcc 60
   agatgt                                                            66
<210> 78
   <211> 22
   <212> PRT
   <213> Homo sapiens
<400> 78
   Met Asp Met Arg Val Pro Ala Gln Leu Leu Gly Leu Leu Leu Leu Trp
    1               5                  10                  15
   Leu Arg Gly Ala Arg Cys
               20
<210> 79
   <211> 2049
   <212> DNA
   <213> Artificial Sequence
<220>
   <223> vector sequence
<400> 79
   atactcgagt tggccgcttc cctttagtga gggttaatgc ttcgagcaga catgataaga 60
   tacattgatg agtttggaca aaccacaact agaatgcagt gaaaaaaatg ctttatttgt 120
   gaaatttgtg atgctattgc tttatttgta accattataa gctgcaataa acaagttaac 180
   aacaacaatt gcattcattt tatgtttcag gttcaggggg agatgtggga ggttttttaa 240
   agcaagtaaa acctctacaa atgtggtaaa atccgataag gatcgatggc gctctccctt 300
   atgcgactcc tgcattagga agcagcccag tagtaggttg aggccgttga gcaccgccgc 360
   cgcaaggaat ggtgcatgca aggagatggc gcccaacagt cccccggcca cggggcctgc 420
   caccataccc acgccgaaac aagcgctcat gagcccgaag tggcgagccc gatcttcccc 480
   atcggtgatg tcggcgatat aggcgccagc aaccgcacct gtggcgccgg tgatgccggc 540
   cacgatgcgt ccggcgtaga ggatctctga cggaaggaaa gaagtcagaa ggcaaaaacg 600
   agagtaactc cacagtagct ccaaattctt tataagggtc aatgtccatg ccccaaagcc 660
   acccaaggca cagcttggag gcttgaacag tgggacatgt acaagagatg attaggcaga 720
   ggtgaaaaag ttgcatggtg ctggtgcgca gaccaattta tgcctacagc ctcctaatac 780
   aaagaccttt aacctaatct cctcccccag ctcctcccag tccttaaaca cacagtcttt 840
   gaagtaggcc tcaaggtcgg tcgttgacat tgctgggagt ccaagagtcc tcttatgtaa 900
   gaccttgggc aggatctgat gggcgttcac ggtggtctcc atgcaacgtg cagaggtgaa 960
   gcgaagtgca cacggaccgg cagatgagaa ggcacagacg gggagaccgc gtaaagagag 1020
   gtgcgccccg tggtcggctg gaacggcaga cggagaaggg gacgagagag tcccaagcgg 1080
   ccccgcgagg ggtcgtccgc gggattcagc gccgacggga cgtaaacaaa ggacgtcccg 1140
   cgaaggatct aaagccagca aaagtcccat ggtcttataa aaatgcatag ctttaggagg 1200
   ggagcagaga acttgaaagc atcttcctgt tagtctttct tctcgtagac ttcaaactta 1260
   tacttgatgc ctttttcctc ctggacctca gagaggacgc ctgggtattc tgggagaagt 1320
   ttatatttcc ccaaatcaat ttctgggaaa aacgtgtcac tttcaaattc ctgcatgatc 1380
   cttgtcacaa agagtctgag gtggcctggt tgattcatgg cttcctggta aacagaactg 1440
   cctccgacta tccaaaccat gtctacttta cttgccaatt ccggttgttc aataagtctt 1500
   aaggcatcat ccaaactttt ggcaagaaaa tgagctcctc gtggtggttc tttgagttct 1560
   ctactgagaa ctatattaat tctgtccttt aaaggtcgat tcttctcagg aatggagaac 1620
   caggttttcc tacccataat caccagattc tgtttacctt ccactgaaga ggttgtggtc 1680
   attctttgga agtacttgaa ctcgttcctg agcggaggcc agggtcggtc tccgttcttg 1740
   ccaatcccca tattttggga cacggcgacg atgcagttca atggtcgaac catgatggca 1800
   attctagaaa gctttttgca aaagcctagg cctccaaaaa agcctcctca ctacttctgg 1860
   aatagctcag aggccgaggc ggcctcggcc tctgcataaa taaaaaaaat tagtcagcca 1920
   tggggcggag aatgggcgga actgggcgga gttaggggcg ggatgggcgg agttaggggc 1980
   gggactatgg ttgctgacta attgagatgc atgctttgca tacttctgcc tgctggggag 2040
   cctggggac                                                         2049
<210> 80
   <211> 4933
   <212> DNA
   <213> Artificial Sequence
<220>
   <223> vector sequence
<400> 80
   tttccacacc tggttgctga ctaattgaga tgcatgcttt gcatacttct gcctgctggg 60
   gagcctgggg actttccaca ccctaactga cacacattcc acagctgcct cgcgcgtttc 120
   ggtgatgacg gtgaaaacct ctgacacatg cagctcccgg agacggtcac agcttgtctg 180
   taagcggatg ccgggagcag acaagcccgt cagggcgcgt cagcgggtgt tggcgggtgt 240
   cggggcgcag ccatgaccca gtcacgtagc gatagcggag tgtatactgg cttaactatg 300
   cggcatcaga gcagattgta ctgagagtgc accatatgcg gtgtgaaata ccgcacagat 360
   gcgtaaggag aaaataccgc atcaggcgct cttccgcttc ctcgctcact gactcgctgc 420
   gctcggtcgt tcggctgcgg cgagcggtat cagctcactc aaaggcggta atacggttat 480
   ccacagaatc aggggataac gcaggaaaga acatgtgagc aaaaggccag caaaaggcca 540
   ggaaccgtaa aaaggccgcg ttgctggcgt ttttccatag gctccgcccc cctgacgagc 600
   atcacaaaaa tcgacgctca agtcagaggt ggcgaaaccc gacaggacta taaagatacc 660
   aggcgtttcc ccctggaagc tccctcgtgc gctctcctgt tccgaccctg ccgcttaccg 720
   gatacctgtc cgcctttctc ccttcgggaa gcgtggcgct ttctcatagc tcacgctgta 780
   ggtatctcag ttcggtgtag gtcgttcgct ccaagctggg ctgtgtgcac gaaccccccg 840
   ttcagcccga ccgctgcgcc ttatccggta actatcgtct tgagtccaac ccggtaagac 900
   acgacttatc gccactggca gcagccactg gtaacaggat tagcagagcg aggtatgtag 960
   gcggtgctac agagttcttg aagtggtggc ctaactacgg ctacactaga aggacagtat 1020
   ttggtatctg cgctctgctg aagccagtta ccttcggaaa aagagttggt agctcttgat 1080
   ccggcaaaca aaccaccgct ggtagcggtg gtttttttgt ttgcaagcag cagattacgc 1140
   gcagaaaaaa aggatctcaa gaagatcctt tgatcttttc tacggggtct gacgctcagt 1200
   ggaacgaaaa ctcacgttaa gggattttgg tcatgagatt atcaaaaagg atcttcacct 1260
   agatcctttt aaattaaaaa tgaagtttta aatcaatcta aagtatatat gagtaaactt 1320
   ggtctgacag ttaccaatgc ttaatcagtg aggcacctat ctcagcgatc tgtctatttc 1380
   gttcatccat agttgcctga ctccccgtcg tgtagataac tacgatacgg gagggcttac 1440
   catctggccc cagtgctgca atgataccgc gagacccacg ctcaccggct ccagatttat 1500
   cagcaataaa ccagccagcc ggaagggccg agcgcagaag tggtcctgca actttatccg 1560
   cctccatcca gtctattaat tgttgccggg aagctagagt aagtagttcg ccagttaata 1620
   gtttgcgcaa cgttgttgcc attgctgcag gcatcgtggt gtcacgctcg tcgtttggta 1680
   tggcttcatt cagctccggt tcccaacgat caaggcgagt tacatgatcc cccatgttgt 1740
   gcaaaaaagc ggttagctcc ttcggtcctc cgatcgttgt cagaagtaag ttggccgcag 1800
   tgttatcact catggttatg gcagcactgc ataattctct tactgtcatg ccatccgtaa 1860
   gatgcttttc tgtgactggt gagtactcaa ccaagtcatt ctgagaatag tgtatgcggc 1920
   gaccgagttg ctcttgcccg gcgtcaacac gggataatac cgcgccacat agcagaactt 1980
   taaaagtgct catcattgga aaacgttctt cggggcgaaa actctcaagg atcttaccgc 2040
   tgttgagatc cagttcgatg taacccactc gtgcacccaa ctgatcttca gcatctttta 2100
   ctttcaccag cgtttctggg tgagcaaaaa caggaaggca aaatgccgca aaaaagggaa 2160
   taagggcgac acggaaatgt tgaatactca tactcttcct ttttcaatat tattgaagca 2220
   tttatcaggg ttattgtctc atgagcggat acatatttga atgtatttag aaaaataaac 2280
   aaataggggt tccgcgcaca tttccccgaa aagtgccacc tgacgtctaa gaaaccatta 2340
   ttatcatgac attaacctat aaaaataggc gtatcacgag gccctttcgt cttcaagaat 2400
   tcctttgcct aatttaaatg aggacttaac ctgtggaaat attttgatgt gggaagctgt 2460
   tactgttaaa actgaggtta ttggggtaac tgctatgtta aacttgcatt cagggacaca 2520
   aaaaactcat gaaaatggtg ctggaaaacc cattcaaggg tcaaattttc atttttttgc 2580
   tgttggtggg gaacctttgg agctgcaggg tgtgttagca aactacagga ccaaatatcc 2640
   tgctcaaact gtaaccccaa aaaatgctac agttgacagt cagcagatga acactgacca 2700
   caaggctgtt ttggataagg ataatgctta tccagtggag tgctgggttc ctgatccaag 2760
   taaaaatgaa aacactagat attttggaac ctacacaggt ggggaaaatg tgcctcctgt 2820
   tttgcacatt actaacacag caaccacagt gcttcttgat gagcagggtg ttgggccctt 2880
   gtgcaaagct gacagcttgt atgtttctgc tgttgacatt tgtgggctgt ttaccaacac 2940
   ttctggaaca cagcagtgga agggacttcc cagatatttt aaaattaccc ttagaaagcg 3000
   gtctgtgaaa aacccctacc caatttcctt tttgttaagt gacctaatta acaggaggac 3060
   acagagggtg gatgggcagc ctatgattgg aatgtcctct caagtagagg aggttagggt 3120
   ttatgaggac acagaggagc ttcctgggga tccctaggtg ggactgtctc cctttggact 3180
   ttccttcaat atcttctttt aatatctttt gtgtcaagat acagaagtga tagaaagaat 3240
   ttgacagtaa tccatcattt ccttagacat gggaaaagtt atgaagaatt gataaggaat 3300
   tttttttgta agtctgacac attcaacagt gacattctct gttcctaggc ttttggttaa 3360
   ggtgctgctt ttaagaacaa catgaatttc atttaaaatt catgtttttc cattatttac 3420
   accttaagga cttaattcta gtagataata taatattaga gattttcttt tgtccagttt 3480
   atctagcatt gttagagtgt aagtttttat attaattaca attttaattt agagtgtgtg 3540
   tatactgttc agatgaagaa tgagtcaagc aaatgtgact attattatca tttgtagaac 3600
   tattatccag gattactgtg tatatgacca gataccattc aagtgtcagg acaccaattg 3660
   attacccatt tataccttga tgtcttgtga gtctcttcaa gttttgtagc cttacattga 3720
   acaaggcttc agtggatgaa cttggaatga tcagagtcag gaggcacaaa gcctgtgaga 3780
   taagaggtgg gaaaggcaac ttatagcatt aaaggtgcaa gcacattcag tggtatgggg 3840
   ggggcgggaa tactggcaaa gccaggaagt ctgtcagcac ttggatacaa gagtctagag 3900
   gagtgcttgt cctatgaggt gagagcaatg tgtacttaat agctcagaaa agcaagccaa 3960
   tggagaatag tgctaatgat gaggggagga gaaaggcaac atgagagaga agagaaggag 4020
   attacaggaa gaaattaagc tgtgggagag gaaggggctg gactaccaga catgcaggat 4080
   aggcaagttt caatgtgacc agatgggtcc tttttgtgag aaaaagcaga cctagatgtt 4140
   gatgagagaa aggcaagtcc ttggtgatta tcagaacaca aacatatagc atgcaaaatt 4200
   atctttacgt ggtacttagg gaggtcacaa atgagctgga catgcatttg gaatagagag 4260
   gaggtagtct atatggtgga tgagaagttc agcactggag gaactaagtg atacacacag 4320
   acccccagtg tatattcact ttttaaatca agaaataatg tcaagtataa tccatatcta 4380
   ttttatgaag ttctatgatt aaattttaag ctgggcgcgc tataccttat tttggattga 4440
   agccaatatg ataatgaggg ggtggagttt gtgacgtggc gcggggcgtg gaacggggcg 4500
   ggtgacgtag tagtgtggcg gaagtgtgat gttgcaagtg tggcggaaca catgtaagcg 4560
   acggatgtgg caaaagtgac gtttttggtg tgcgccggtg tacacaggaa gtgacaattt 4620
   tcgcgcggtt ttaggcggat gttgtagtaa atttgggcgt aaccgagtaa gatttggcca 4680
   ttttcgcggg aaaactgaat aagaggaagt gaaatctgaa taattttgtg ttactcatag 4740
   cgcgtaatat ttgtctaggg ccgcggggac tttgaccgtt tacgtggaga ctcgcccagg 4800
   tgtttttctc aggtgttttc cgcgttccgg gtcaaagttg gcgttttatt attatagtca 4860
   gcatgcatgc tcgagcggcc gcgacgcgca gtgtatttat atccggacgg tccgggacct 4920
   cctgtgcaag aac                                                    4933
<210> 81
   <211> 45
   <212> DNA
   <213> Artificial Sequence
<220>
   <223> leader sequence
<400> 81
   atggagtttg ggctgagctg gctttttctt gtggctattt taaaa 45
<210> 82
   <211> 15
   <212> PRT
   <213> Artificial Sequence
<220>
   <223> leader sequence
<400> 82
   Met Glu Phe Gly Leu Ser Trp Leu Phe Leu Val Ala Ile Leu Lys
   1               5                   10                  15
<210> 83
   <211> 103
   <212> DNA
   <213> Artificial Sequence
<220>
   <223> vector sequence
<400> 83
   gtaattcatg gagaaataga aaaattgagt gtgaatggat aagagtgaga gaaacagtgg 60
   atacgtgtgg cagtttctga ccagggtttc tttttgtttg cag                   103
<210> 84
   <211> 15
   <212> DNA
   <213> Homo sapiens
<400> 84
   ggtgtccagt gtgag 15
<210> 85
   <211> 5
   <212> PRT
   <213> Homo sapiens
<400> 85
   Gly Val Gln Cys Glu
   1               5
<210> 86
   <211> 4345
   <212> DNA
   <213> Homo sapiens
<400> 86
<210> 87
   <211> 90
   <212> DNA
   <213> Homo sapiens
<400> 87
   ggtgagggcc gggcccagcc accgggacag agagggagcc gaagggggcg ggagtggcgg 60
   gcaccgggct gacacgtgtc cctcactgca                                  90
<210> 88
   <211> 245
   <212> DNA
   <213> Homo sapiens
<400> 88
   ggtgagtgac ctgtccccag gggcagcacc caccgacaca caggggtcca ctcgggtctg 60
   gcattcgcca ccccggatgc agccatctac tccctgagcc ttggcttccc agagcggcca 120
   agggcagggg ctcgggcggc aggacccctg ggctcggcag aggcagttgc tactctttgg 180
   gtgggaacca tgcctccgcc cacatccaca cctgccccac ctctgactcc cttctcttga 240
   ctcca                                                             245
<210> 89
   <211> 180
   <212> DNA
   <213> Homo sapiens
<400> 89
   ggtaggcccc actcttgccc ctcttcctgc actccctggg acctcccttg gcctctgggg 60
   catggtggaa agcacccctc actcccccgt tgtctgggca actggggaaa aggggactca 120
   accccagccc acaggctggt ccccccactg ccccgccctc accaccatct ctgttcacag 180
<210> 90
   <211> 2043
   <212> DNA
   <213> Artificial Sequence
<220>
   <223> vector sequence
<400> 90
   tgaccctgct ggcctgccca caggctcggg gcggctggcc gctctgtgtg tgcatgcaaa 60
   ctaaccgtgt caacggggtg agatgttgca tcttataaaa ttagaaataa aaagatccat 120
   tcaaaagata ctggtcctga gtgcacgatg ctctggccta ctggggcggc ggctgtgctg 180
   cacccaccct gcgcctcccc tgcagaacac cttcctccac agcccccacc cctgcctcac 240
   ccacctgcgt gcctcagtgg cttctagaaa cccctgaatt ccctgcagct gctcacagca 300
   ggctgacctc agacttgcca ttcctcctac tgcttccaga aagaaagctg aaagcaagac 360
   cacacgtata caggcagcac acaggcatgt gtggatacac atggacagac acggacacac 420
   acaaacacat ggacacacag agacgtgcta acccatgggc acacacatac acagacatgg 480
   acccacacac aaacatatgt ggacacacat gtacaaacat gcacaggcac acaaagagaa 540
   cactgactac aggcacacac acacacgggc acacacatgg atatgtgcac acatggacac 600
   atacatgtgc aggacatgca cacacacaga cacactagca cagaggcata cacacacaga 660
   cacacacatt cacaaacaca catgtgcatg caaacacaca cacatgtaca gacacaagta 720
   catggacaca tgcacaccca gagacacact gacacagaca cacaggagca tgtgatacac 780
   taacacgtgg acacacacgt ctacccacag gcacacaaca gatggacacg cgtacacaga 840
   catgcacaca cccacaggca caacacgtgc gcatgccggc cgagatccct ggccagggat 900
   ccctggccag ggatccctgg ccagggatct tcaatattgg ccattagcca tattattcat 960
   tggttatata gcataaatca atattggcta ttggccattg catacgttgt atctatatca 1020
   taatatgtac atttatattg gctcatgtcc aatatgaccg ccatgttggc attgattatt 1080
   gactagttat taatagtaat caattacggg gtcattagtt catagcccat atatggagtt 1140
   ccgcgttaca taacttacgg taaatggccc gcctggctga ccgcccaacg acccccgccc 1200
   attgacgtca ataatgacgt atgttcccat agtaacgcca atagggactt tccattgacg 1260
   tcaatgggtg gagtatttac ggtaaactgc ccacttggca gtacatcaag tgtatcatat 1320
   gccaagtccg ccccctattg acgtcaatga cggtaaatgg cccgcctggc attatgccca 1380
   gtacatgacc ttacgggact ttcctacttg gcagtacatc tacgtattag tcatcgctat 1440
   taccatggtg atgcggtttt ggcagtacac caatgggcgt ggatagcggt ttgactcacg 1500
   gggatttcca agtctccacc ccattgacgt caatgggagt ttgttttggc accaaaatca 1560
   acgggacttt ccaaaatgtc gtaacaactg cgatcgcccg ccccgttgac gcaaatgggc 1620
   ggtaggcgtg tacggtggga ggtctatata agcagagctc gtttagtgaa ccgtcagatc 1680
   actagaagct ttattgcggt agtttatcac agttaaattg ctaacgcagt cagtgcttct 1740
   gacacaacag tctcgaactt aagctgcagt gactctctta aggtagcctt gcagaagttg 1800
   gtcgtgaggc actgggcagg taagtatcaa ggttacaaga caggtttaag gagaccaata 1860
   gaaactgggc ttgtcgagac agagaagact cttgcgtttc tgataggcac ctattggtct 1920
   tactgacatc cactttgcct ttctctccac aggtgtccac tcccagttca attacagctc 1980
   ttaaggctag agtacttaat acgactcact ataggctagc gctagctcaa gactcagccg 2040
   gac                                                               2043
<210> 91
   <211> 88
   <212> DNA
   <213> Homo sapiens
<400> 91
   gctagctcaa gactcagccg gacatggaca tgagggtccc tgctcagctc ctgggactcc 60
   tctgctctgg ctcccagata ccagatgt                                    88
<210> 92
   <211> 28
   <212> PRT
   <213> Homo sapiens
<400> 92
   Leu Lys Thr Gln Pro Asp Met Asp Met Arg Val Pro Ala Gln Leu Leu
    1               5                  10                  15
   Gly Leu Leu Leu Leu Trp Leu Pro Asp Thr Arg Cys
               20                  25
<210> 93
   <211> 2049
   <212> DNA
   <213> Artificial Sequence
<220>
   <223> vector sequence
<400> 93
   atactcgagt tggccgcttc cctttagtga gggttaatgc ttcgagcaga catgataaga 60
   tacattgatg agtttggaca aaccacaact agaatgcagt gaaaaaaatg ctttatttgt 120
   gaaatttgtg atgctattgc tttatttgta accattataa gctgcaataa acaagttaac 180
   aacaacaatt gcattcattt tatgtttcag gttcaggggg agatgtggga ggttttttaa 240
   agcaagtaaa acctctacaa atgtggtaaa atccgataag gatcgatggc gctctccctt 300
   atgcgactcc tgcattagga agcagcccag tagtaggttg aggccgttga gcaccgccgc 360
   cgcaaggaat ggtgcatgca aggagatggc gcccaacagt cccccggcca cggggcctgc 420
   caccataccc acgccgaaac aagcgctcat gagcccgaag tggcgagccc gatcttcccc 480
   atcggtgatg tcggcgatat aggcgccagc aaccgcacct gtggcgccgg tgatgccggc 540
   cacgatgcgt ccggcgtaga ggatctctga cggaaggaaa gaagtcagaa ggcaaaaacg 600
   agagtaactc cacagtagct ccaaattctt tataagggtc aatgtccatg ccccaaagcc 660
   acccaaggca cagcttggag gcttgaacag tgggacatgt acaagagatg attaggcaga 720
   ggtgaaaaag ttgcatggtg ctggtgcgca gaccaattta tgcctacagc ctcctaatac 780
   aaagaccttt aacctaatct cctcccccag ctcctcccag tccttaaaca cacagtcttt 840
   gaagtaggcc tcaaggtcgg tcgttgacat tgctgggagt ccaagagtcc tcttatgtaa 900
   gaccttgggc aggatctgat gggcgttcac ggtggtctcc atgcaacgtg cagaggtgaa 960
   gcgaagtgca cacggaccgg cagatgagaa ggcacagacg gggagaccgc gtaaagagag 1020
   gtgcgccccg tggtcggctg gaacggcaga cggagaaggg gacgagagag tcccaagcgg 1080
   ccccgcgagg ggtcgtccgc gggattcagc gccgacggga cgtaaacaaa ggacgtcccg 1140
   cgaaggatct aaagccagca aaagtcccat ggtcttataa aaatgcatag ctttaggagg 1200
   ggagcagaga acttgaaagc atcttcctgt tagtctttct tctcgtagac ttcaaactta 1260
   tacttgatgc ctttttcctc ctggacctca gagaggacgc ctgggtattc tgggagaagt 1320
   ttatatttcc ccaaatcaat ttctgggaaa aacgtgtcac tttcaaattc ctgcatgatc 1380
   cttgtcacaa agagtctgag gtggcctggt tgattcatgg cttcctggta aacagaactg 1440
   cctccgacta tccaaaccat gtctacttta cttgccaatt ccggttgttc aataagtctt 1500
   aaggcatcat ccaaactttt ggcaagaaaa tgagctcctc gtggtggttc tttgagttct 1560
   ctactgagaa ctatattaat tctgtccttt aaaggtcgat tcttctcagg aatggagaac 1620
   caggttttcc tacccataat caccagattc tgtttacctt ccactgaaga ggttgtggtc 1680
   attctttgga agtacttgaa ctcgttcctg agcggaggcc agggtcggtc tccgttcttg 1740
   ccaatcccca tattttggga cacggcgacg atgcagttca atggtcgaac catgatggca 1800
   attctagaaa gctttttgca aaagcctagg cctccaaaaa agcctcctca ctacttctgg 1860
   aatagctcag aggccgaggc ggcctcggcc tctgcataaa taaaaaaaat tagtcagcca 1920
   tggggcggag aatgggcgga actgggcgga gttaggggcg ggatgggcgg agttaggggc 1980
   gggactatgg ttgctgacta attgagatgc atgctttgca tacttctgcc tgctggggag 2040
   cctggggac                                                          2049

## Description

### STATEMENT OF GOVERNMENT SUPPORT

The invention described herein was supported in whole or in part by the National Institutes of Health (Grant Nos. R01 NS 24180, R01 NS 32129, R01 CA104996, R01 CA096859), and the National Multiple Sclerosis Society (Grant No. CA 1011 A8-3) The United States Government has certain rights in the invention.

### FIELD OF THE INVENTION

The present invention relates to antibodies, particularly human natural antibodies recombinant antibodies derived therefrom and fragments thereof, which are capable of binding to and recognizing neurons in the CNS and eliciting responses in CNS neurons. These antibodies are useful in the diagnosis and treatment of conditions associated with nerve damage, injury or degeneration, neurodegenerative disease, chronic nerve injury or damage and sudden nerve injury or damage. The antibodies, variable regions or CDR domain sequences thereof, and fragments thereof of the present invention may also be used in therapy in combination with chemotherapeutics, immune modulators, or neuroactive agents and/or with other antibodies or fragments thereof. This invention relates particularly to methods for alleviating or treating stroke or ischemia, particularly cerebral ischemia, and associated agents, constructs and compositions for use in stroke or ischemia.

### BACKGROUND OF THE INVENTION

Neuroregeneration refers to the regrowth or repair of nervous tissues, cells or cell products. Such mechanisms may include remyelination, generation of new neurons, glia, axons, myelin, or synapses. Neuroregeneration differs between the Peripheral Nervous System (PNS) and the Central Nervous System (CNS) by both the functional mechanisms involved in and the extent and speed of regeneration.

Axon regeneration in the mature mammalian central nervous system (CNS) is very limited after injury. Consequently, functional deficits persist after spinal cord injury (SCI), traumatic brain injury, stroke, and related conditions that involve axonal disconnection. This situation differs from that in the mammalian peripheral nervous system (PNS), where long-distance axon regeneration and substantial functional recovery can occur in the adult. Both extracellular molecules and the intrinsic growth capacity of the neuron influence regenerative success.

Central nervous system (CNS) axons do not spontaneously regenerate after injury in adult mammals. In contrast, peripheral nervous system (PNS) axons readily regenerate, allowing recovery of function after peripheral nerve damage. Aguayo and colleagues demonstrated that at least some mature CNS neurons retain the capacity to regenerate when provided with a permissive peripheral nerve graft (Richardson PM, McGuinness UM, Aguayo AJ (1980) Nature 284:264-265; Richardson PM, Issa VM, Aguayo AJ (1984) J Neurocytol 13:165-182; David S, Aguayo AJ (1981) Science 214:931-933; Benfey M, Aguayo AJ (1982) Nature 296:150-152). This work suggested that the PNS environment is stimulatory and/or that the CNS environment is inhibitory for axon growth. Subsequent studies identified both growth-promoting factors in the PNS and growth-inhibiting factors in the CNS. Inhibitors of regeneration include specific proteins in CNS myelin and molecules associated with the astroglial scar. In addition, slower debris clearance in the CNS relative to the PNS may impede axonal re-growth. An understanding of factors which influence axon growth is critical for the development of therapeutics to promote CNS regeneration.

After peripheral nerve injury, axons readily regenerate. The distal portion of the axon, which is disconnected from the cell body, undergoes Wallerian degeneration. This active process results in fragmentation and disintegration of the axon. Debris is removed by glial cells, predominantly macrophages. Proximal axons can then regenerate and re-innervate their targets, allowing recovery of function.

The two major classes of CNS regeneration inhibitors are the myelin-associated inhibitors (MAls) and the chondroitin sulfate proteoglycans (CSPGs). These molecules limit axon regeneration, and, by interfering with their function, some degree of growth in the adult CNS is achieved. Cell-autonomous factors are also important determinants of CNS regeneration failure. CNS neurons do not upregulate growth-associated genes to the same extent as do PNS neurons. Consequently, their ability to regenerate is limited even in the absence of inhibitors. Increasing the intrinsic growth capacity of neurons allows modest axon regeneration within the CNS (Bomze HM et al (2001) Nat Neurosci 4:38-43; Neumann S, Woolf CJ (1999) Neuron 23:83-91).

MAls are proteins expressed by oligodendrocytes as components of CNS myelin. MAls impair neurite outgrowth in vitro and are thought to limit axon growth in vivo after CNS damage. MAls include Nogo-A (Chen MS et al (2000) Nature 403:434-439; GrandPre T et al (2000) Nature 403:439-44), myelin-associated glycoprotein (MAG) (McKerracher L et al (1994) Neuron 13:805-811), oligodendrocyte myelin glycoprotein (OMgp) (Kottis V et al (2002) J Neurochem 82:1566-1569), ephrin-B3 (Benson MD et al (2005) Proc Nat Acad Sci USA 102:10694-10699) and Semaphorin 4D (Sema4D) (Moreau-Fauvarque C et al (2003) J Neurosci 23:9229-9239). Three of these (Nogo-A, MAG and OMgp) interact with a neuronal Nogo-66 receptor 1 (NgR1) to limit axon growth. These three structurally unrelated ligands also show affinity for a second axon growth-inhibiting receptor, paired immunoglobulin-like receptor B (PirB) (Atwal JK et al (2008) Science 322:967-970).

Several recognition molecules which act as molecular cues underlying promotion and/or inhibition of neurite growth have been identified. Among the neurite outgrowth promoting recognition molecules, the neural cell adhesion molecule L1 plays a prominent role in mediating neurite outgrowth (Schachner M (1990) Seminars in the Neurosciences 2:497-507). L1-dependent neurite outgrowth is mediated by homophilic interaction. L1 enhances neurite outgrowth on L1 expressing neurites and Schwann cells, and L1 transfected fibroblasts (Bixby et al (1982) Proc Natl Acad. Sci. U.S.A. 84:2555-2559; Chang et al (1987) J Cell Biol 104:355-362; Lagenaur et al. (1987) Proc Natl Acad Sci USA 84:7753-7757; Seilheimer et al (1988) J Cell Biol 107:341-351; Kadmon et al (1990) J Cell Biol 110:193-208; Williams et al (1992) J Cell Biol 119:883-892).

Nervous system injuries affect over 90,000people every year, and a much greater number when cerebro-vascular events such as stroke are included. It is estimated that spinal cord injuries alone affect 10,000 each year. As a result of this high incidence of neurological injuries, nerve regeneration and repair, a subfield of neural tissue engineering, is becoming a rapidly growing field dedicated to the discovery of new ways to recover nerve functionality after injury. The nervous system is divided into two parts: the central nervous system, which consists of the brain and spinal cord, and the peripheral nervous system, which consists of cranial and spinal nerves along with their associated ganglia. While the peripheral nervous system has an intrinsic ability for repair and regeneration, the central nervous system is, comparably and for the most part, restricted in its ability to self-repair and regeneration. There is currently no accepted and approved treatment for recovering human nerve function after injury to the central nervous system.

Axon protection and repair following spinal cord injury (SCI) holds great potential as an effective strategy to prevent motor neuron loss and permanent disability. Neuron protection has been achieved using targeted trophic factors for preventing damage and promoting repair of axons following injury. These molecules were predominantly identified using selection strategies based upon in vitro systems that focused upon targeting specific small molecule neurotrophic factors. Whereas these molecules demonstrated neuroprotective results in preclinical models, the results from clinical trials have been less favorable.

Natural autoreactive monoclonal antibodies have demonstrated beneficial biological functions in CNS cells using multiple models of injury and disease. Antibody-mediated promotion of neuron survival, axon regeneration and functional recovery has been demonstrated *in vivo* using the mouse monoclonal IgM, IN-1 (Bregman BS et al (1995) Nature 378(6556):498-501; Caroni P, Schwab ME (1988) Neuron 1(1):85-96). Similar results were obtained using immunization of spinal cord homogenate (SCH) prior to CNS damage (Ellezam B, Bertrand J, Dergham P, McKerracher L (2003) Neurobiol Dis 12(1):1-10; Huang DW et al (1999) Neuron 24(3):639-647).

Multiple sclerosis (MS) is a chronic, frequently progressive, inflammatory central nervous system (CNS) disease characterized pathologically by primary demyelination, usually without initial axonal injury. The etiology and pathogenesis of MS are unknown. Several immunological features of MS, and its moderate association with certain major histocompatibility complex alleles, have prompted the speculation that MS is an immune-mediated disease. An autoimmune hypothesis is supported by the experimental autoimmune (allergic) encephalomyelitis (EAE) model, where injection of certain myelin components into genetically susceptible animals leads to T cell-mediated CNS demyelination. However, specific autoantigens and pathogenic myelin-reactive T cells have not been definitively identified in the CNS of MS patients, nor is MS associated with other autoimmune diseases. An alternative hypothesis, based upon epidemiological data, is that an environmental factor, perhaps an unidentified virus, precipitates an inflammatory response in the CNS, which leads to either direct or indirect ("bystander") myelin destruction, potentially with an induced autoimmune component. This hypothesis is supported by evidence that several naturally occurring viral infections, both in humans and animals, can cause demyelination. One commonly utilized experimental viral model is induced by Theiler's murine encephalomyelitis virus (TMEV) (Dal Canto, M.C., and Lipton, H.L., Am. J. Path., 88:497-500 (1977)).

In the case of MS, demyelination ultimately results in nerve cell death. However, axon death following demyelination is not immediate. If appropriate support molecules or cells are provided the nervous system has a significant capacity for repair. Protecting axons of the CNS promises to be an effective strategy to limit the loss of surviving axons and prevent permanent disability. Neuroprotection may be achieved by modulating the potentially neurotoxic inflammatory environment in lesions. Reagents designed to limit excitotoxicity, inhibit nitric oxide, or block ion channels are under study as methods to protect axons in danger (Pitt, D., P. Werner, and C. S. Raine (2000) Nat Med 6:67-70; Okuda, Y et al (1997) Journal of neuroimmunology 73:107-116; Waxman, S. G. (2002) .J Rehabil Res Dev 39:233-242). Many of these reagents are small molecules with demonstrated toxicity and act systemically on all cells. Wootla et al., Expert Rev Neurother. 2012 April; 1284:409-420 , indicate that human IgM antibodies have therapeutic potential for CNS injuries.

Ischemia is a restriction in blood supply to tissues, causing a shortage of oxygen and glucose needed for cellular metabolism (to keep tissue alive). Ischemia is generally caused by problems with blood vessels, with resultant damage to or dysfunction of tissue. It also refers to local anemia in a given part of a body sometimes resulting from congestion (such as vasoconstriction, thrombosis or embolism). Hypoxia is a condition in which the body as a whole or a region of the body (tissue hypoxia, or less commonly regional hypoxia) is deprived of adequate oxygen supply. Variations in arterial oxygen concentrations can be part of the normal physiology, for example, during strenuous physical exercise. A mismatch between oxygen supply and its demand at the cellular level may result in a hypoxic condition. Ischemic hypoxia occurs when there is decreased blood flow and can occur for example in heart failure and vasodilatory shock. Cerebral hypoxia occurs when there is not enough oxygen getting to the brain. The brain needs a constant supply of oxygen and nutrients to function.

In cerebral hypoxia, sometimes only the oxygen supply is interrupted. This can be caused by smoke inhalation), carbon monoxide poisoning, choking, diseases that prevent movement of the breathing muscles, high altitudes, pressure on the windpipe (trachea), and strangulation. In other cases, both oxygen and nutrient supply are stopped or interrupted, such as in cardiac arrest, cardiac arrhythmia, complications of general anesthesia, drowning, drug overdose, injuries to a newborn during or after birth such as in cerebral palsy, stroke, very low blood pressure.

Brain ischemia is insufficient blood flow to the brain, and can be acute or chronic. Acute ischemic stroke is a neurologic emergency that may be reversible if treated rapidly. Chronic ischemia of the brain may result in a form of dementia called vascular dementia. A brief episode of ischemia affecting the brain is called a Transient Ischemic Attack (TIA), frequently referred to as a mini-stroke.

A stroke, also known as a cerebrovascular accident (CVA), is the rapid loss of brain function(s) due to disturbance in the blood supply to the brain. This can be due to ischemia (lack of blood flow) caused by blockage (thrombosis, arterial embolism), or a hemorrhage (leakage of blood). Brain ischemia, also known as cerebral ischemia, is a condition in which there is insufficient blood flow to the brain to meet metabolic demand and is a type of stroke along with subarachnoid hemorrhage and intracerebral hemorrhage. Ischemia may be focal ischemia, which is confined to a specific region of the brain, or global ischemia, which encompasses wide areas of brain tissue. Stroke is the leading cause of long-term disability in the United States. Each year, close to 800,000 people suffer a stroke. Following ischemic injury, the disability associated with clinical deficits is primarily due to dysfunction of neurons. The lack of functional recovery is in part attributed to the restriction of regeneration and neuroplasticity (Walmsley, A.R., and Mir, A.K. (2007) Current pharmaceutical design 13:2470-2484). Stroke may result in a altered motor function such as an inability to move one or more limbs on one side of the body, cognitive effects such as an inability to understand or formulate speech, or other deficits including visual effects such as an inability to see one side of the visual field.There are currently no effective treatments to prevent or reverse stroke-related neurologic deficits.

An ischemic stroke is occasionally treated in a hospital with thrombolysis (also known as a "clot buster"), and some hemorrhagic strokes benefit from neurosurgery. Prevention of recurrence can involve the administration of antiplatelet drugs such as aspirin and dipyridamole, control and reduction of hypertension, and the use of statins. Selected patients may benefit from carotid endarterectomy and the use of anticoagulants. Interventional therapy of cerebral ischemia remains limited to enzymatic thrombolysis by use of recombinant tissue plasminogen activator rtPA (Brott, T.G., et al (1992) Stroke 23:632-640; Haley, E.C., et al (1992) Stroke 23:641-645; Group, T.N. (1995) New England J Med 333:1581-1587), and to interventional removal of the blood clot via direct catheterization (Gobin YP et al (2004) Stroke 35:2848-2854). However, rtPA has a short 3 hr therapeutic window and increased incidence of hemorrhagic cerebral ischemia if infused beyond the 3 hr time frame (Clark, WM et al (1999) JAMA 282:2019-2026; Hacke W et al (2008) New England J Med 359:1317-1329).

Human monoclonal autoantibodies have been identified that exhibit activity in the central nervous system, and that were particularly associated with the stimulation of remyelination. It would be desirable to develop human monoclonal antibodies with activity in the CNS, particularly recombinant antibodies with these activities, and with capability to promote neural regeneration and/or to protect neurons from disease, injury, damage and/or death particularly in instances of stroke or brain/cerebral ischemia, or where oxygen or blood flow to the brain or CNS is deprived or affected either acutely or chronically. In particular, use and application of human monoclonal antibodies, with capability to access the CNS and treat, prevent or alleviate cerebral ischemia or stroke would be desirable. It is toward the achievement of that objective that the present invention is directed.

### SUMMARY OF THE INVENTION

The invention relates to neuromodulatory agents with particular effectiveness in the CNS, which agents comprise a material selected from the group consisting of an antibody of the IgM subtype, active fragments thereof, monomers thereof, agonists thereof and combinations thereof. The neuromodulatory agents or antibodies have one or more of the following characteristics: they protect and/or stabilize neurons; they target sites in the CNS or nerve cell damage, compromise or injury; they reduce or block cell death, e.g. hydrogen-peroxide induced cell death. In a particular aspect of the present invention the agents, particularly neuron-binding monoclonal antibodies are effective and have application and use in treating and alleviating stroke and ischemia, particularly cerebral ischemia, and in conditions where ischemia or hypoxia of nerve cells or the brain is involved or has occurred.

The invention is defined by the claims. The disclosure provides neuron-binding monoclonal antibodies with capability to promote neurite extension, to act in neuroregeneration, and/or to protect neurons from damage and for diagnostic and therapeutic purposes in the central nervous system. The present studies demonstrate that when neuron-binding monoclonal antibodies, particularly antibody IgM12 rHIgM12, are administered to animals following stroke (in this instance an animal model for induction of ischemic stroke), it results in a clear improvement of function of the animals and protects tissue integrity.

In particular, specific recombinant antibodies are provided, wherein said antibodies recognize and are capable of binding neurons, including cortical neurons, hippocampal neurons, cerebellar granule cells and retinal ganglion cells, and which target and mediate neurons and neural cells in the event and upon a stroke, ischemic condition, or ischemia or hypoxia in nerve cells or microglia. Recombinant fully human antibodies are provided herein. The antibodies of the present invention have diagnostic and therapeutic use in conditions or diseases associated with nerve compromise, damage or injury.

In a general aspect, the present disclosure provides antibodies directed against and capable of binding one or more epitope on neurons, including cortical neurons, hippocampal neurons, cerebellar granule cells and retinal ganglion cells. In a broad aspect, the present disclosure provides an isolated specific binding member, particularly an antibody or fragment thereof, including a recombinant human antibody, which recognizes, binds, and/or targets neurons. The invention provides an antibody, particularly a human antibody, particularly an IgM antibody, which specifically binds neurons and protects neurons from cell death and which does not promote remyelination. The antibody has use in treating or ameliorating diseases or conditions in mammals where nerves are compromised, injured or damaged or are at risk thereof, including in spinal cord injury (SCI), traumatic brian injury (TBI), Amyolotropic Lateral Sclerosis (ALS), multiple sclerosis (MS), Alzheimer's disease, stroke, Parkinson's disease, Huntington's disease, prenatal anoxia/perinatal ischemia, Cerebral Palsy, encephalopathy, myelopathy, or motor neuron diseases, and particularly in the protection, survival or maintenance of neurons and neurological capacity in these diseases.

In a particular aspect, the antibody of the invention or compositions thereof have use and application in stroke and in cerebral ischemia. In a particular aspect, the antibodies or compositions of the invention have use in conditions in which there is insufficient blood flow and/or oxygen to the brain to meet metabolic demand or wherein there is traumatic injury to the brain resulting in death or injury or risk of death or injury of neurons and associated cells. In a particular aspect, the present invention provides an antibody or fragment thereof, which is antibody 12 or 42, particularly serum derived or recombinant IgM12 or IgM42.

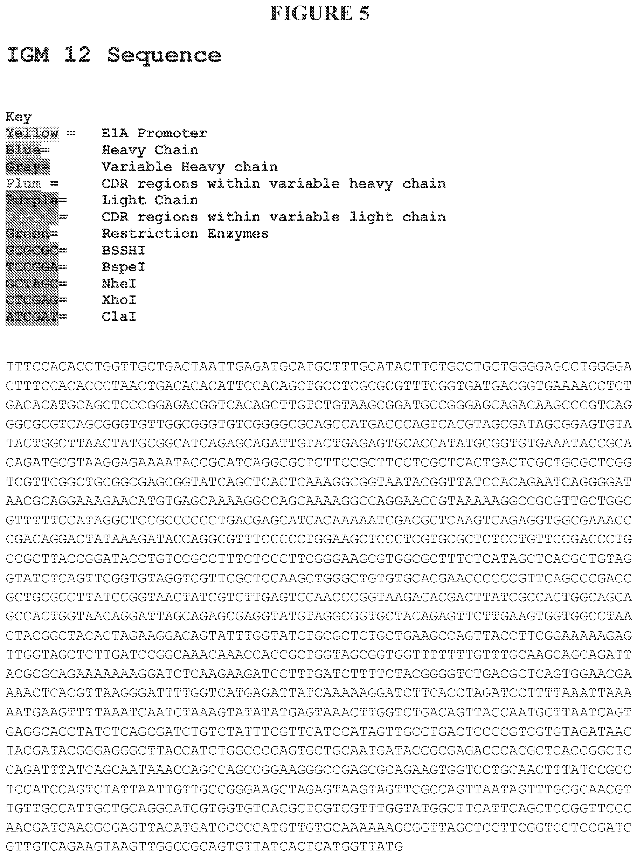

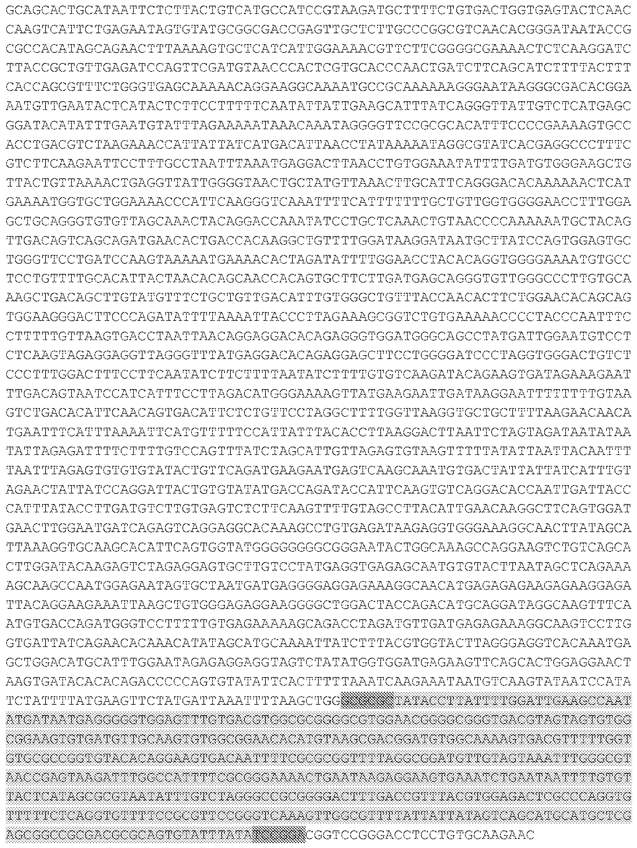

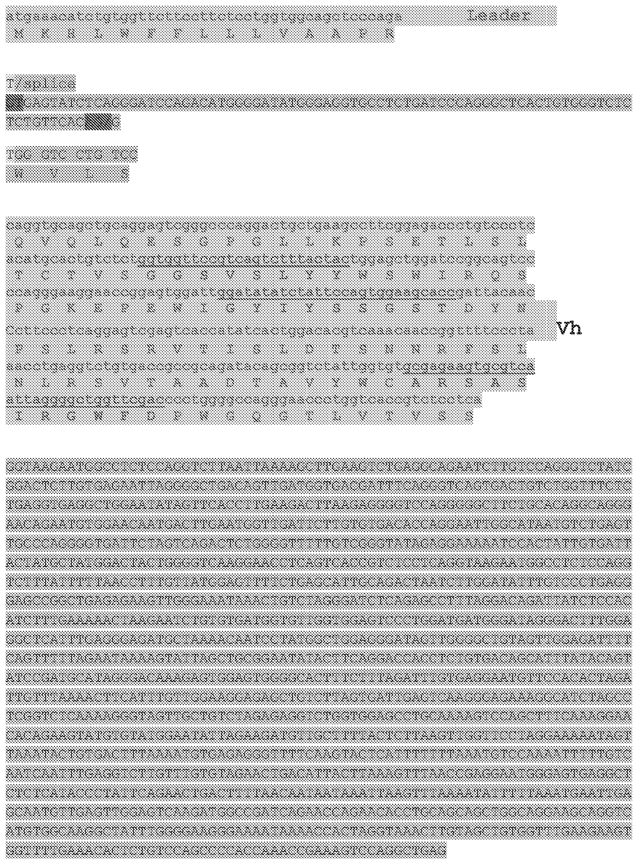

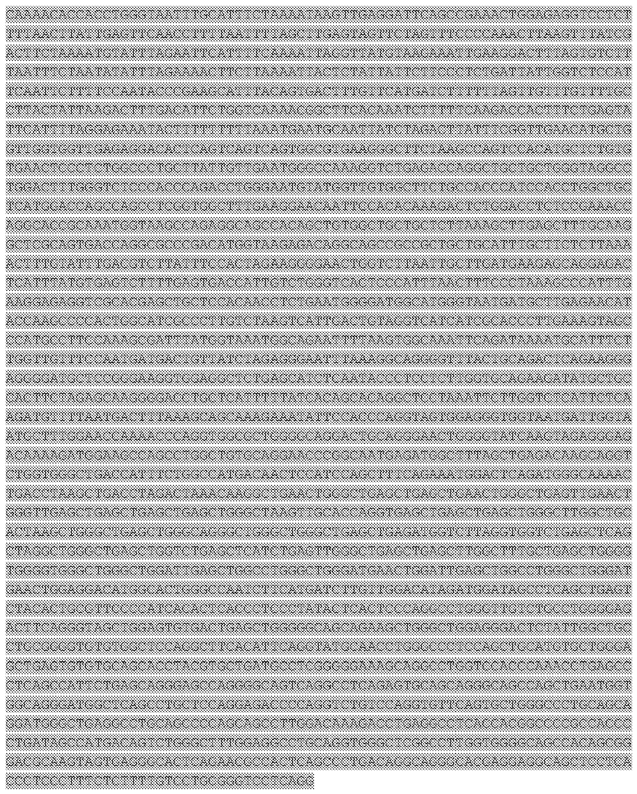

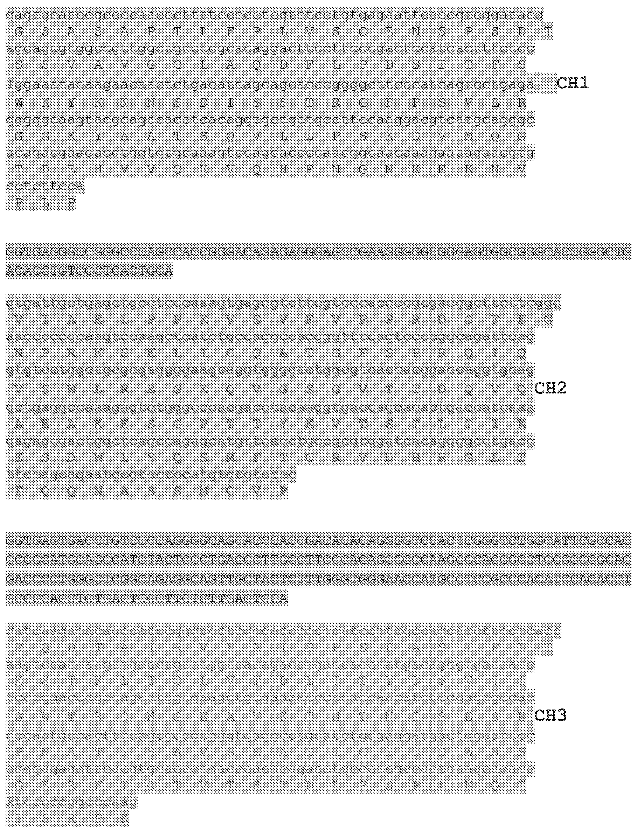

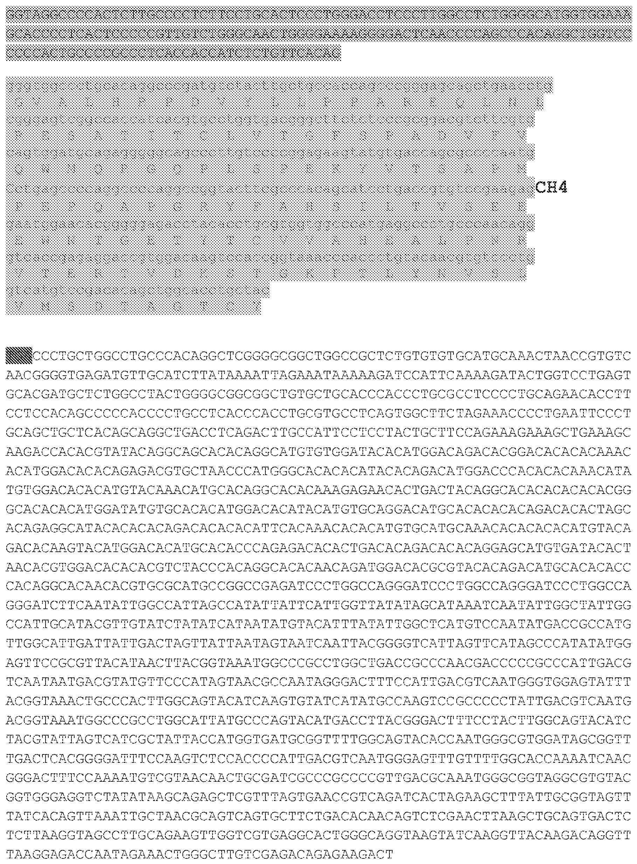

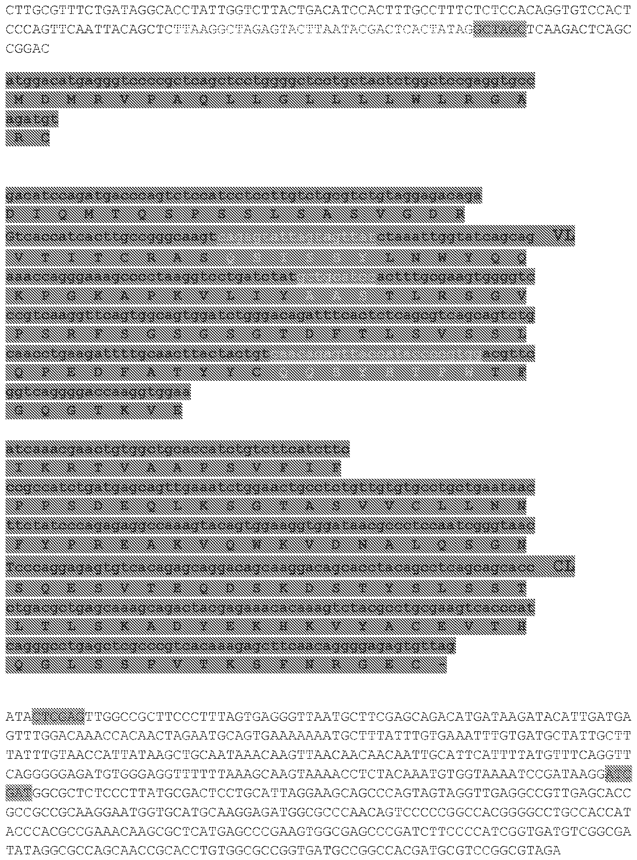

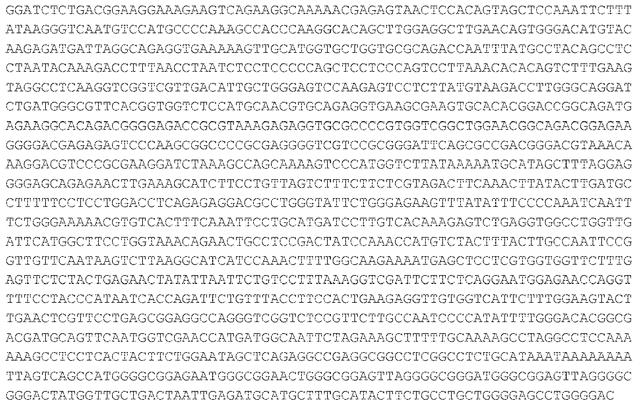

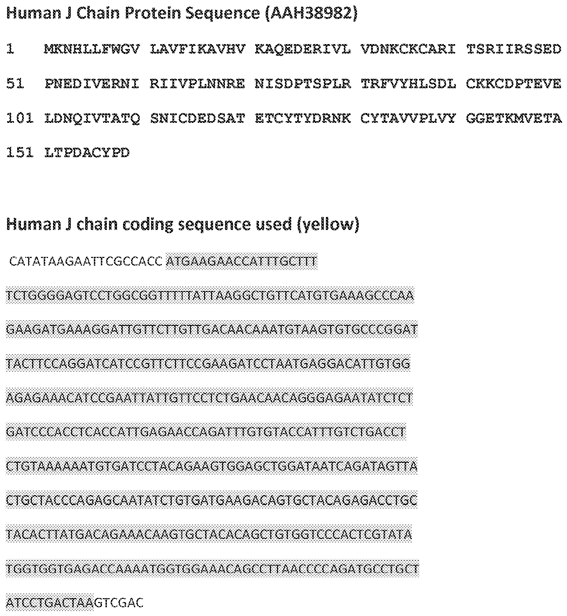

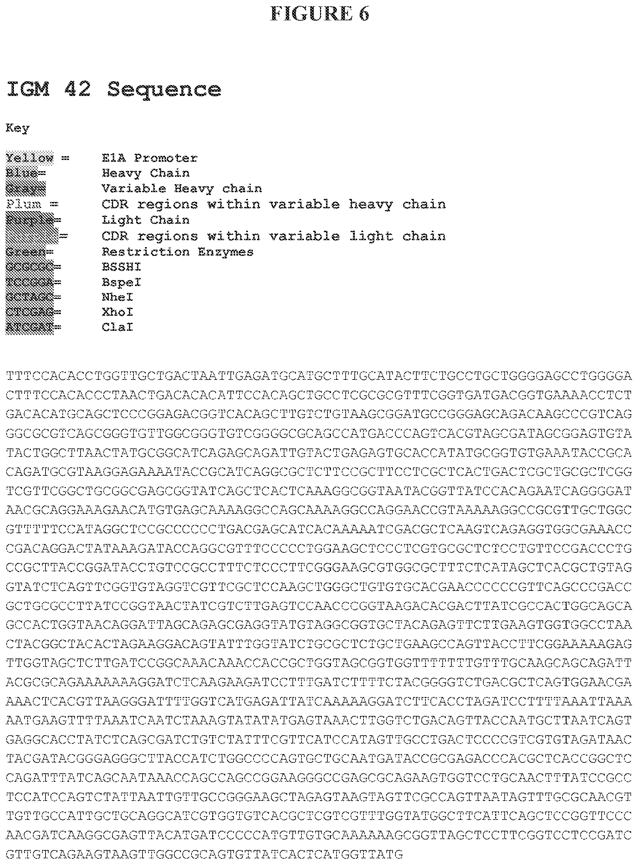

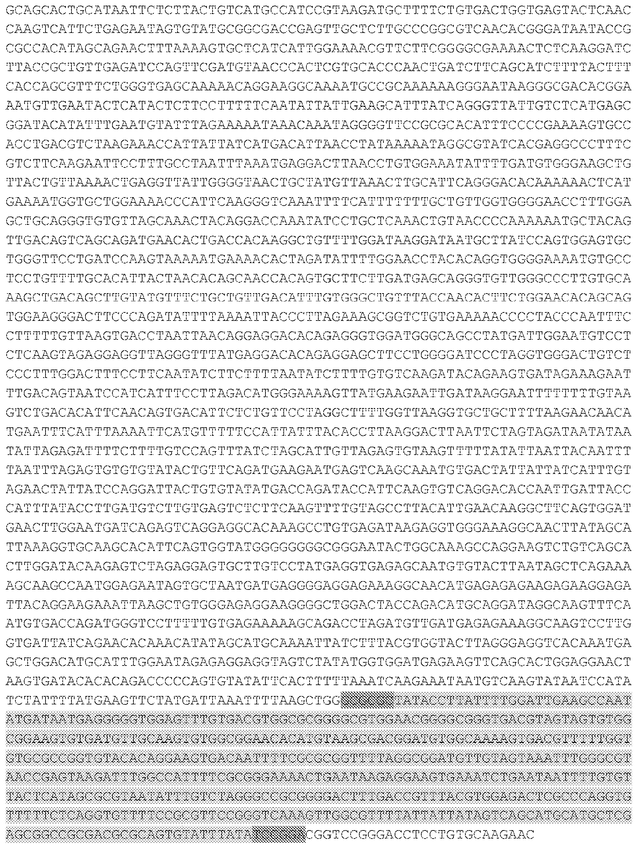

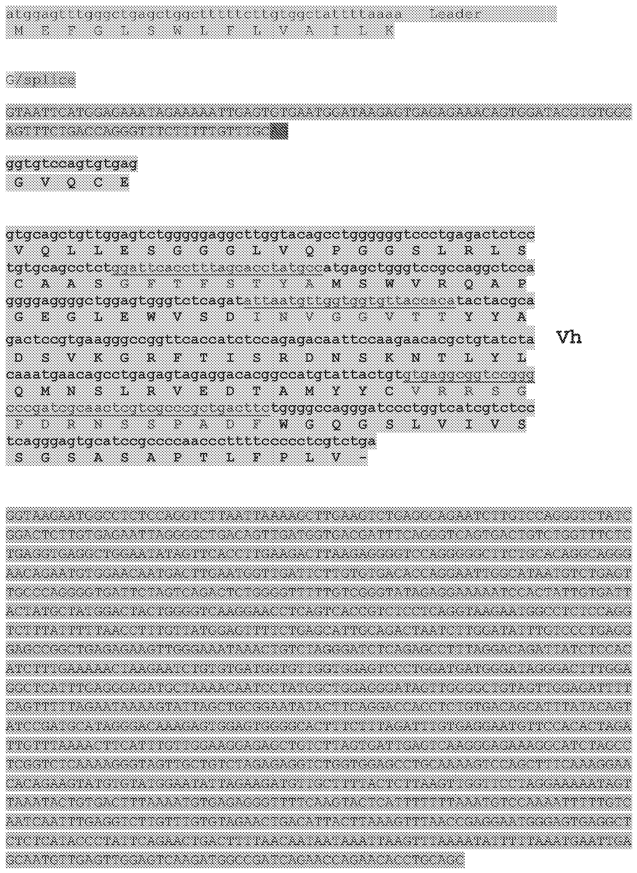

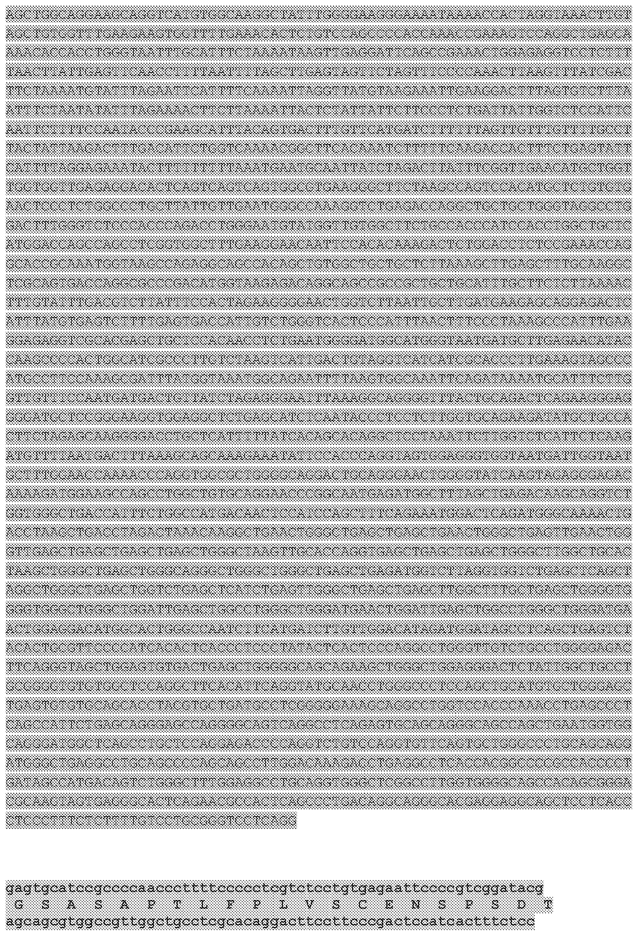

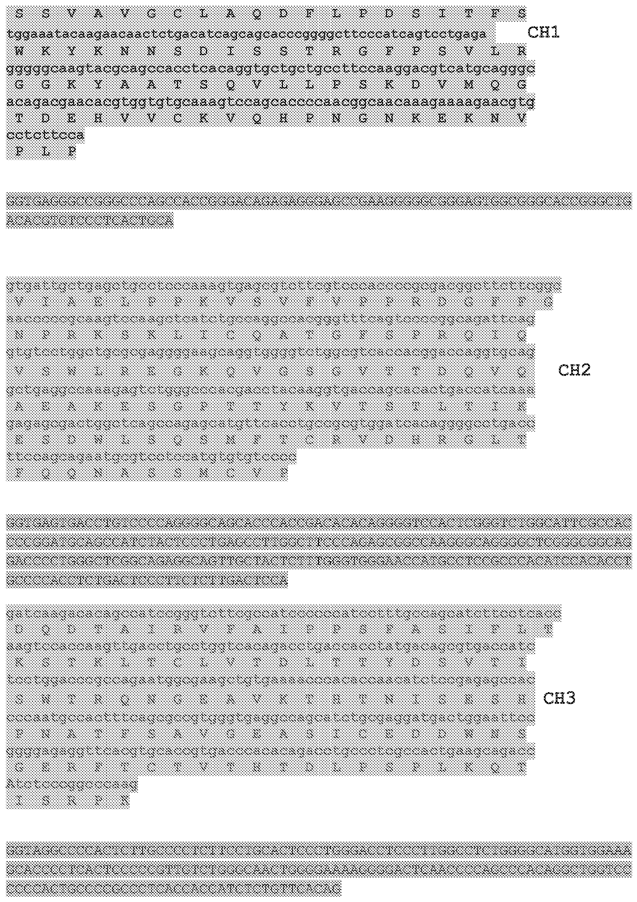

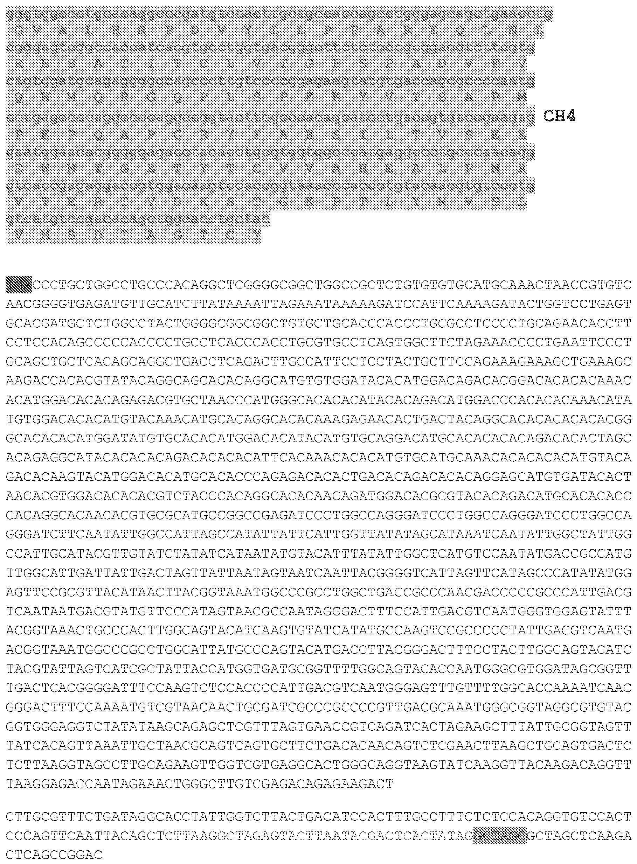

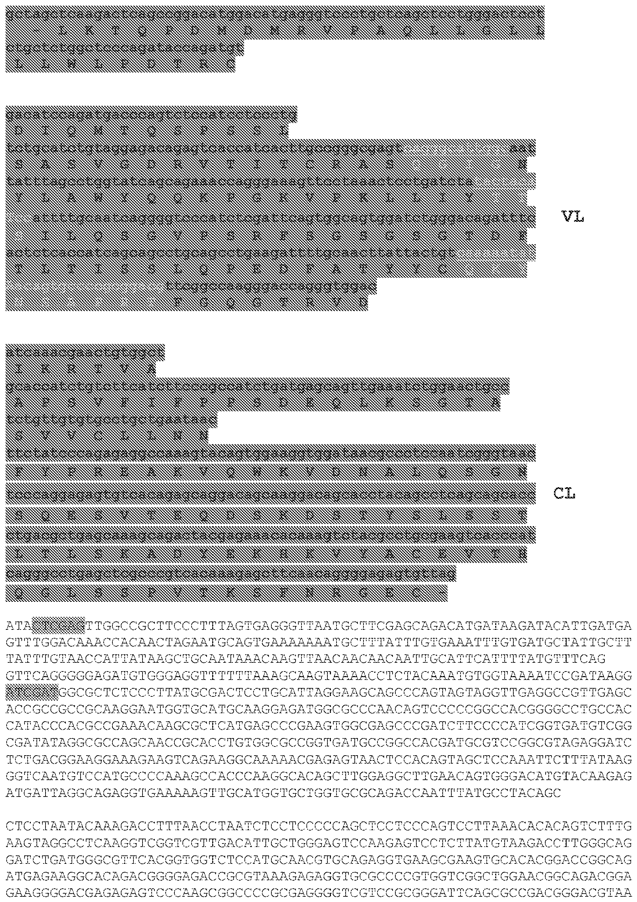

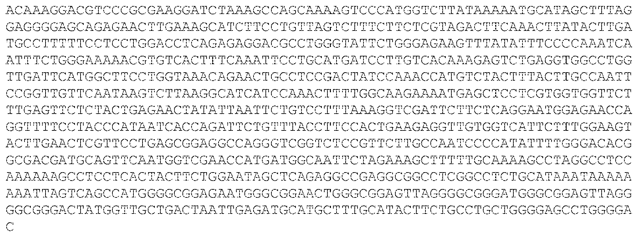

In an aspect of the invention a recombinant or synthetic neuron binding antibody is provided comprising the variable region CDR sequences set out in Figure 5 and/or 6. Antibody 12 comprises heavy chain CDR sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5. Antibody 42 comprises heavy chain CDR sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6. Accordingly, recombinant antibodies which are based on the CDRs of the antibody(ies) identified herein will be useful for targeting and protecting neurons, particularly susceptible, compromised, damaged or injured neurons in diseases or in cancers.

The invention thus provides isolated human IgM antibody(ies) or fragment(s) thereof which specifically binds neurons and protects neurons from cell death and which does not promote remyelination, wherein the antibody or fragment comprises: (a) the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5; or (b) the variable heavy chain amino acid CDR domain sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6, for use in treating or ameliorating cerebral ischemia or stroke.

In a further particular aspect, the antibody comprises the amino acid sequence of antibody 12 or 42 including as set out in Figure 5 and/or Figure 6. Recombinant antibody of the present invention comprises the variable heavy chain sequence (SEQ ID NO: 1) and light chain sequence (SEQ ID NO: 11) as set out in Figure 5. Recombinant antibody IgM42 of the present invention comprises the variable heavy chain sequence (SEQ ID NO: 17) and light chain sequence (SEQ ID NO: 27) as set out in Figure 6. In a particular aspect of the invention, the recombinant antibodies are fully human recombinant antibodies, comprising human heavy chain variable region, constant region and human J chain. Fully human recombinant antibodies are provided herein comprising human immunoglobulin heavy chain comprising variable region (SEQ ID NO: 1), or the CDRs thereof, a human constant region, particularly a kappa sequence (SEQ ID NO: 3, 5,7 and 9) and human J chain (SEQ ID NO:15) as set out in Figure 5.

In a further aspect, the present disclosure provides an isolated antibody or fragment thereof capable of binding an antigen, wherein said antibody or fragment thereof comprises a polypeptide binding domain comprising an amino acid sequence substantially as set out herein and in Figure 6. The invention provides an isolated human antibody or fragment thereof capable of binding neurons, wherein said antibody or fragment thereof comprises a heavy chain variable region (SEQ ID NO: 17), or the CDRs thereof, and light chain variable region (SEQ ID NO: 27), or the CDRS thereof, or comprising an amino acid sequence substantially as set out herein and in Figure 6.

In a further aspect, the present disclosure provides an isolated fully human antibody or fragment thereof capable of binding an antigen, wherein said antibody or fragment thereof comprises a polypeptide binding domain comprising an amino acid sequence substantially as set out herein and in Figure 5. The invention provides an isolated fully human antibody or fragment thereof capable of binding neurons, wherein said antibody or fragment thereof comprises a heavy chain variable region (SEQ ID NO: 1), or the CDRs thereof, and light chain variable region (SEQ ID NO: 11), or the CDRs thereof, or comprising an amino acid sequence substantially as set out herein and in Figure 5. The disclosure particularly provides an isolated human IgM antibody or active fragment thereof, comprising the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5. In a particular aspect, the disclosure provides an isolated fully human recombinant IgM antibody or active fragment thereof, comprising the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), a human constant region and a human J chain sequence as set out in SEQ ID NO: 15.

In further aspects, the disclosure provides an isolated nucleic acid which comprises a sequence encoding a neuron binding polypeptide or antibody as defined above, and methods of preparing polypeptides or antibodies of the invention which comprise expressing said nucleic acids under conditions to bring about expression of said polypeptide or antibody, and recovering the polypeptide or antibody. In one such aspect, a nucleic acid encoding antibody variable region sequence having the amino acid sequences as set out in Figures 5 or 6 is provided or an antibody having CDR domain sequences as set out in Figures 5 or 6 is provided. In one aspect, a nucleic acid comprising the nucleic acid sequence of Figure 5 or 6 is provided. In a further aspect, a heavy chain variable region VH or light chain variable region VL encoding nucleic acid as set out in Figure 5 or 6 is provided. The present invention also relates to a recombinant DNA molecule or cloned gene, or a degenerate variant thereof, which encodes an antibody of the present invention; preferably a nucleic acid molecule, in particular a recombinant DNA molecule or cloned gene, encoding the antibody VH and VL, particularly the CDR region sequences, which has a sequence or is capable of encoding a sequence shown in Figure 5 or 6. In preferred aspects the invention provides nucleic acid encoding heavy chain (SEQ ID NO: 2) and light chain variable region sequence (SEQ ID NO: 12) of IgM12, and nucleic acid encoding heavy chain (SEQ ID NO:18) and light chain variable region sequence (SEQ ID NO: 28) of IgM42. In an aspect of the invention, a nucleic acid is provided encoding an antibody or fragment thereof comprising the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36). In a further aspect, the nucleic acid further encodes a human constant region and a human J chain, particularly the J chain of SEQ ID NO: 15. In an aspect of the invention, a nucleic acid is provided encoding an antibody or fragment thereof comprising the variable heavy chain amino acid CDR domain sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42). In a further aspect, the nucleic acid further encodes a human constant region and a human J chain, particularly the J chain of SEQ ID NO: 15.

The antibodies, fragments thereof and recombinant antibodies comprising the variable region sequences may be used in a method of treatment, prevention or diagnosis of the human or animal body, such as a method of neuroprotection in a mammal which comprises administering to said mammal an effective amount of the antibodies, fragments thereof and recombinant antibodies of the invention. Recombinant antibodies or fragments thereof comprising the CDR domain region sequences may be used in a method of neuroprotection in a mammal which comprises administering to said mammal an effective amount of the antibodies, fragments thereof and recombinant antibodies of the invention.

Recombinant antibodies or fragments thereof may be used as neuroprotective agents in methods for prevention, treatment or amelioration of nerve injury, damage or compromise and complications that can, may or do result in CNS damage. The treatment or prevention methods are applicable where loss of structure, function or survival of neurons is involved or associated including brain injury or trauma, spinal cord injury (SCI), nerve injury, head injury, conditions where blood supply to the brain is reduced or compromised, infectious diseases of the brain, neurodegenerative diseases. Exemplary such diseases or conditions for treatment, prevention or amelioration in accordance with the methods of the invention include spinal cord injury (SCI), traumatic brain injury (TBI), Amyolotropic Lateral Sclerosis (ALS), multiple sclerosis (MS), Alzheimer's disease, stroke, Parkinson's disease, Huntington's disease, prenatal anoxia/perinatal ischemia and/or Cerebral Palsy, encephalopathy, myelopathy, and motor neuron diseases. The present invention thus relates to methods of treating or ameliorating diseases or conditions in mammals where nerves are compromised, injured or damaged or scenarios where nerves or neurons are susceptible or at risk of compromise, injury or damage comprising administering a recombinant antibody or fully human antibody or fragment thereof selected from and IgM42.

In a particular aspect of the invention, the antibodies, fragments thereof and recombinant antibodies comprising the variable region sequences according to the invention, are utilized in methods or administered in compositions for treatment or amelioration of stroke or cerebral ischemia. In a particular aspect of the invention, the antibodies, fragments thereof and recombinant antibodies comprising the variable region sequences according to the invention, may be utilized in methods or administered in compositions for treatment or amelioration of stroke. In a particular aspect of the invention, the antibodies, fragments thereof and recombinant antibodies comprising the variable region sequences according to the invention, may be utilized in methods or administered in compositions for treatment or amelioration of cerebral ischemia. In an embodiment of the invention, the antibodies, fragments thereof and recombinant antibodies comprising the variable region sequences according to the invention, may be utilized in methods or administered in compositions for improving motor function in conditions such as stroke. The antibodies, fragments thereof and recombinant antibodies comprising the variable region sequences according to the invention, may be utilized in methods or administered in compositions for improving motor function in an animal suffering from or suspected of suffering from a stroke. The antibodies, fragments thereof and recombinant antibodies comprising the variable region sequences according to the invention, may be utilized in methods or administered in compositions for improving motor function in an animal that has had a stroke or that has cerebral ischemia. In a particular aspect, IgM12, rIgM12, antibody 12 comprising heavy chain CDR sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5, may be utilized in treatment, prevention or alleviation of stroke or cerebral ischemia. In an aspect thereof, the antibodies, fragments thereof and recombinant antibodies comprising the variable region sequences according to the invention may be administered in instances where stroke and/or cerebral ischemia is determined, suspected or at risk, including in instances wherein enzymatic thrombolytics, such as tissue plasminogen activator (TPA) or other thrombolytic or anti-thrombotic agents, may be administered or utilized.

The antibodies, fragments thereof and recombinant antibodies comprising the variable region sequences according to the invention, may be utilized in methods or administered in compositions for improvement or stabilization of neurological function or of motor function in instances, diseases or conditions wherein nerves are compromised, injured or damaged or scenarios where nerves or neurons are susceptible or at risk of compromise, injury or damage. In a particular aspect, the antibodies or fragments thereof are utilized or administered in early or initial stages of a disease, trauma, or condition, or repeated over the course or duration of a disease, trauma, or condition, so as to facilitate or maintain the improvement or stabilization of neurological function or motor function, including or selected from movement, such as walking, or cognitive function, such as recall or recognition.

In an aspect of the invention, the antibodies, fragments thereof and recombinant antibodies comprising the variable region sequences according to the invention, may be utilized in methods or administered in compositions for mitigating or ameliorating neurological effects, defects, damage or neuronal cell death in the event of a stroke or cerebral ischemia. In accordance with the methods, the antibody(ies) ameliorate one or more functional or assessable neurological or motor parameter, including an evaluable or scalable parameter such as a stroke scale or function scale. Administration of the antibody(ies) is effective to improve or ameliorate one or more of a symptom or parameter associated with stroke, including for example one or more of upper and lower extremity motor function, limb ataxia, sensory function, language, articulation, attention, gaze and visual function, arm, hand and leg power and movement.

Methods may comprise administration of more than one antibody or fragment, including combinations of antibody and 42. In an aspect of the invention, methods are provided comprising administering one or more antibody or fragment thereof, wherein an antibody comprises (a) the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36) or (b) the variable heavy chain amino acid CDR domain sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42). In a further such method, one or more of antibody and/or of antibody IgM42 may be combined with another CNS active antibody, particularly including one or more of antibodies rHIgM22 and/or rHIgM46. rHIgM22 antibody comprises the amino acid sequence set forth in SEQ ID NO: 43 and 44, or the CDRs of these SEQ IDs. rHIgM46 antibody comprises the amino acid sequence set forth in SEQ ID NO: 45 and 46, or the CDRs of these SEQ IDs. Thus, one or more of antibody IgM12 and/or of antibody IgM42 may be combined with one or more remyelinating antibody comprising (a) heavy chain variable region CDRs comprising CDR1 sequence SSGMH CDR2 sequence V(I)ISYDGSRKYYADSVKG and CDR3 sequence GVTGSPTLDY, and light chain variable region CDRs comprising CDR1 sequence SGSSSNIGNNFVS CDR2 sequence DITKRPS and CDR3 sequence G(E)TWDSSLSAV V; or (b) heavy chain variable region CDRs comprising CDR1 sequence SGFTFSSYW CDR2 sequence IKKDGSEK and CDR3 sequence ARPNCGGDCYLPWYFD, and light chain variable region CDRs comprising CDR1 sequence QSVLYSSNNKNY CDR2 sequence YWAS and CDR3 sequence QQYYNTPQA. Combinations of antibodies may be administered collectively or in series, and at various times and various amounts or concentrations. Bi-or multi-specific antibodies may be utilized. In a particular such aspect, antibody 12 and/or 42 is administered in combination with antibody 22 and/or 46 and/or an antibody having the CDR region CDR1, CDR2 and CDR3 sequences of IgM22 or IgM46, by combined administration or in series, separated by a short length of time or longer length of time, including by hours, days or weeks. In one such particular aspect antibody 12 and/or 42 is administered in combination with antibody 22 and/or 46, by combined administration or in series for the treatment of amelioration of a disease or condition involving neurodegeneration, and particularly including demyelination. In a further such aspect antibody 12 and/or 42 is administered in combination with antibody 22 and/or 46, by combined administration or in series for the treatment of amelioration of a demyelinating disease or condition. In a particular aspect, antibody 12 and/or 42 is administered in combination with antibody 22 and/or 46, by combined administration or in series for the treatment of amelioration of multiple sclerosis. In one such aspect, remyelination and neuroprotection is achieved for combined effects, including synergistic effects, on a measurable clinical aspect of MS disease.

In view of the neuroprotective and/or neuroregenerative capabilities of the agents of the invention, this invention further relates to *in vitro* methods of producing and stimulating the proliferation of neurons, including the proliferation of cortical neurons, hippocampal neurons, cerebellar granule cells and retinal ganglion cells. Such proliferated neurons may be suitable for transplantation and nerve cell therapeutic protocols and methods.

The diagnostic utility extends to the use of the antibodies in assays and methods to characterize CNS nerve injury or damage or to screen for or assess disease or conditions involving nerve injury, damage or death (including necrotic or apoptotic death), including *in vitro* and *in vivo* diagnostic and imaging assays. Thus, the antibodies may be utilized in assays and methods to assess stroke or ischemia and/or to evaluate injury in following ischemia or stroke or predicted or suspected ischemia or stroke. In an immunoassay, a control quantity of the antibodies, or the like may be prepared and labeled with an enzyme, a specific binding partner, ligand, a dye, a fluorescent tag and/or a radioactive element, and may then be introduced into a cellular sample. After the labeled material or its binding partner(s) has had an opportunity to react with sites within the sample, the resulting mass may be examined by known techniques, which may vary with the nature of the label attached. In an *in vivo* diagnostic or imaging application, the antibody or a neuron binding fragment thereof may be prepared and labeled with an enzyme, a specific binding partner, ligand, a dye, a fluorescent tag and/or a radioactive element, and may then be introduced into a an animal. After the labeled material or its binding partner(s) has had an opportunity to react with sites within the animal, the animal may be examined by known techniques, which may vary with the nature of the label attached. In an aspect of the diagnostic utility of the invention, the antibodies or active fragments thereof of the invention may be utilized in assays and methods to characterize cerebral ischemia or stroke. In particular, in instances where cerebral ischemia or stroke is suspected or at risk, the antibodies may be utilized to determine or assess for cerebral ischemia and/or stroke.

The radiolabelled specific binding members, particularly antibodies and fragments thereof, are useful in *in vitro* diagnostics techniques and in *in vivo* radioimaging techniques. In a further aspect radiolabelled specific binding members, particularly antibodies and fragments thereof, particularly radioimmunoconjugates, are useful in radioimmunotherapy, particularly as radiolabelled antibodies for nerve injury repair, neuroregeneration, neurodegenerative recovery, cancer or CNS tumor therapy, or alternatively for ablation of damaged nerve tissue or neurons in certain instances. In an *in vivo* aspect, the antibody or neuron binding fragment thereof is labeled and administered to the animal prior to, during or after surgery or a surgical technique, including a stereotactic or minimally invasive technique, for the purpose of locating nerve injury or for assessing remaining damaged or injured neural tissue. In one such aspect, the radiolabelled specific binding members, particularly antibodies and fragments thereof, are useful in radioimmuno-guided surgery techniques, wherein they can identify and indicate the presence and/or location of compromised, damaged, injured or dying nerve cells or neurons or nervous tissue, prior to, during or following surgery to target, identify or remove such cells.

Immunoconjugates or antibody fusion proteins are included in the present invention, wherein the specific binding members, particularly antibodies and fragments thereof, of the present invention are conjugated or attached to other molecules or agents further include, but are not limited to binding members conjugated to a neuroactive drug, chemical ablation agent, toxin, immunomodulator, cytokine, cytotoxic agent, chemotherapeutic agent or drug.

The present disclosure includes an assay system which may be prepared in the form of a test kit for the quantitative analysis of the extent of the presence of, for instance, damaged, compromised or injured neurons or to quantitate neurons in a sample. The system or test kit may comprise a labeled component prepared by one of the radioactive and/or enzymatic techniques discussed herein, coupling a label to the antibody, and one or more additional immunochemical reagents, optionally at least one of which is a free or immobilized component(s) or their binding partner(s).

The antibodies and in a particular embodiment recombinant antibody(ies) comprising the heavy and/or light chain sequences presented in Figure 5 and 6 herein, or active fragments thereof, and single chain, recombinant or synthetic antibodies derived therefrom, particularly comprising the CDR region sequences depicted in Figures 5 and 6, can be prepared in pharmaceutical compositions, including a suitable vehicle, carrier or diluent, for administration in instances wherein therapy is appropriate, such as to treat or ameliorate conditions or diseases involving neuron compromise, damage, injury or death. Such pharmaceutical compositions may also include methods of modulating the half-life of the antibodies or fragments by methods known in the art such as pegylation. Such pharmaceutical compositions may further comprise additional antibodies or therapeutic agents.

The present disclosure also includes antibodies and fragments thereof, which are covalently attached to or otherwise associated with other molecules or agents. These other molecules or agents include, but are not limited to, molecules (including antibodies or antibody fragments) with distinct recognition characteristics, toxins, ligands, neuroactive agents, and chemotherapeutic agents. In an additional aspect the antibodies or fragments may be used to target or direct therapeutic molecules or other agents, for example to target molecules or agents to neurons, for example to cortical neurons, hippocampal neurons, retinal ganglion cells or cerebellar granule cells, including at wound sites, ischemic sites, tumor sites, inflammatory areas or neurodegenerative lesions. The antibodies or fragments may be used to target or direct therapeutic molecules or other agents, for example to target molecules or agents to sites of cerebral ischemia or stroke.

Other objects and advantages will become apparent to those skilled in the art from a review of the ensuing detailed description, which proceeds with reference to the following illustrative drawings, and the attendant claims.

### BRIEF DESCRIPTION OF THE DRAWINGS

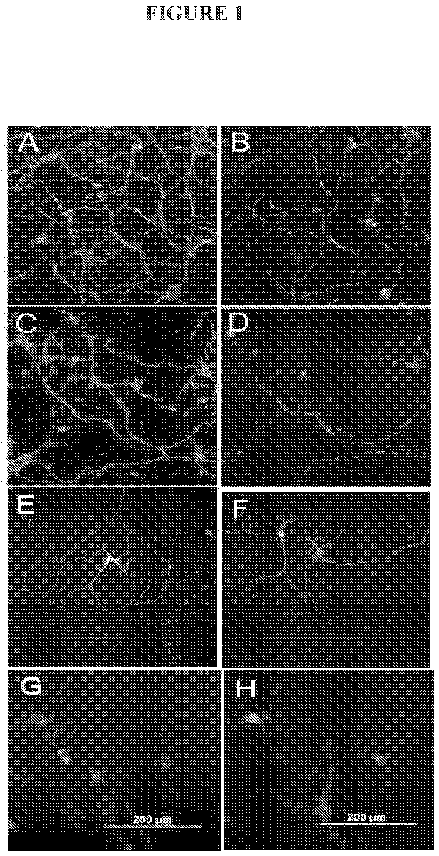

**FIGURE 1****: Human IgMs bind to the surface of multiple types of neurons.** sHIgM42 (**A**) and rHIgM12 (**C**) bind to the surface of live cortical neurons that co-express neurofilament (NF) (**B & D**). Cells were maintained on ice during a 10 min incubation with 10 µg/ml IgMs in media. Cells were fixed, washed, and human IgM detected with an anti-human mu chain Fab'2-FITC secondary Ab. Internal NF is present as a characteristic segmented pattern. Cortical cells were grown for 6 days in Neurobasal B27 media on polyornithine coated glass coverslips. Not all cortical neurons express NF yet, but the IgMs bind to all neurons in the culture. rHIgM12 (**E** green) and sHIgM42 (**F** green) bind to the surface of live mouse hippocampal neurons that co-express neurofilament (NF, red). Hippocampal neurons were grown for 1 wk in Neurobasal B27 media on polylysine coated plastic. Cells isolated from a human temporal lobe resection were grown in DMEM/F12/N2/B27 for 21 days. rHIgM12 bound to the surface of cells (**G**) that were also β III tubulin positive (**F**, anti- β III from Promega).

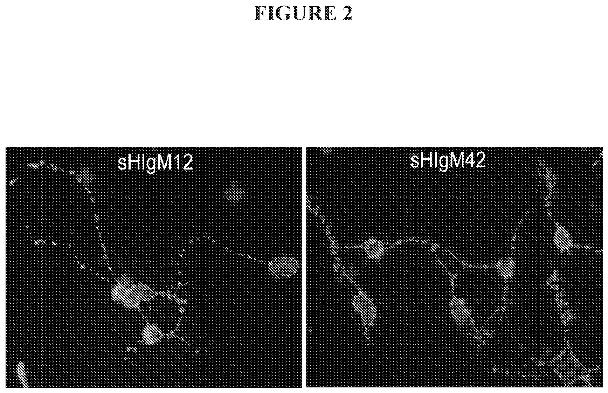

**FIGURE 2****. Human IgMs label the surface of cerebellar granule cells in distinct patterns.** Rat cerebellar granule cells at 1 day in culture were incubated live with 10 µg/ml of the human serum derived IgMs, sHIgM12 or sHIgM42, in culture media for 15 min on ice. After washing and fixation with 4% paraformaldehyde, cells were incubated at room temperature with a FITC conjugated anti- human mu chain antibody and imaged using an immunofluorescence microscope. The pattern of neuronal membrane label using these two antibodies is different. Short areas of neurite membrane are bound by sHIgM12 resulting in a clearly punctate pattern. Larger areas of neurite membrane are bound by sHIgM42 resulting in a segmented pattern. Magnification is 400x.

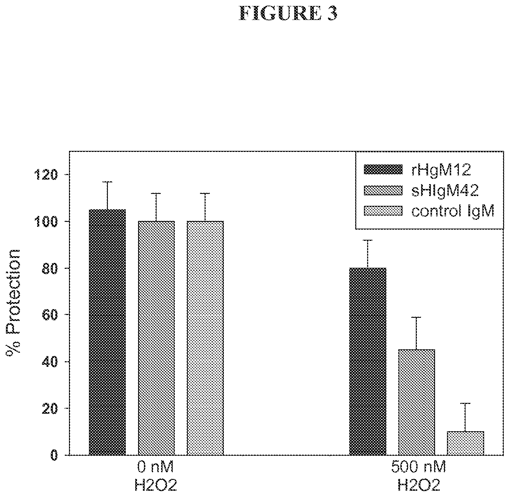

**FIGURE 3****. Neuron binding human IgMs, rHIgM12 and sHIgM42 protect cortical neurons in culture from peroxide induced cell death.** Mouse neurons at 3 days in culture were treated with 500 nM H₂O₂ or saline with or without 10 µg/ml of human IgMs for 30 min. Cells were washed with fresh Neurobasal B27 media and 24 hr later the expression of caspase-3 detected in triplicate treated 96 wells using a FLICA caspase-3 assay kit (Immunochemistry Technologies 935). Bars indicate % protection from caspase 3 activation.

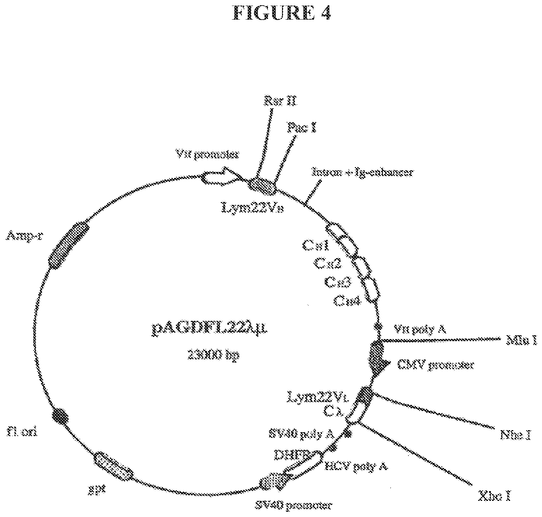

**FIGURE 4** depicts amplifiable vector for producing recombinant antibody. This is a map of the vector used to generate recombinant antibody based on sHIgM22 (rHIgM22), which was modified for production of recombinant and IgM42. For expression in CHO cells, the VH promoter was replaced with E1A promoter. The CA light chain constant region was replaced with a kappa light chain constant region C_{K}.
**FIGURE 5** depicts antibody amino acid and nucleic acid sequence, in the recombinant vector. The heavy chain amino acid sequence and encoding nucleic acid sequence is in gray and each of the Vh, and constant region sequences CH1, CH2, CH3 and CH4 are provided and indicated. The light chain amino acid sequence and encoding nucleic acid sequence is in purple and both variable VL and constant CL (kappa) sequences are indicated. CDR region sequences for the heavy and light chain variable regions are noted. The human J chain is indicated in amino acid and nucleic acid sequence.
**FIGURE 6** depicts IgM42 antibody amino acid and nucleic acid sequence, in the recombinant vector The heavy chain amino acid sequence and encoding nucleic acid sequence is in gray and each of the Vh, and constant region sequences CH1, CH2, CH3 and CH4 are provided and indicated. The light chain amino acid sequence and encoding nucleic acid sequence is in purple and both variable VL and constant CL (kappa) sequences are indicated. CDR region sequences for the heavy and light chain variable regions are noted.

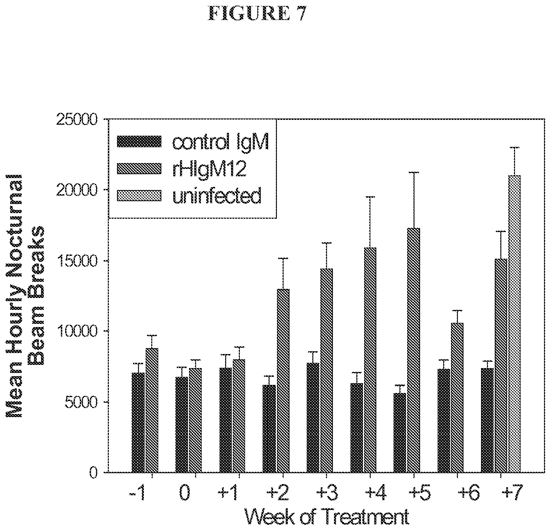

**FIGURE 7****. Recombinant rHIgM12 improves deficits in mice with TMEV induced disease.** Mean hourly spontaneous nocturnal activity (when mice are normally active) is altered by 3 wk after a single i.p. dose of 100 µg of sHIgM12. Groups of 5 SJL mice at 90 days post TMEV infection were treated with rHIgM12 (red bars), or a control human IgM (black bars) and monitored for 3 days weekly as groups in AccuScan activity monitoring boxes. The mean ± SEM of hourly nocturnal horizontal activity over 3 nocturnal periods were compared to nocturnal activity for the 3 nights prior to IgM treatment. An increase in nocturnal activity compared to pre treatment levels was present at 3 wk thru 7 wk after treatment with a single dose of rHIgM12 (P<0.01), but not after treatment with control. Activity levels of uninfected aged matched mice are graphed for comparison (green bar).

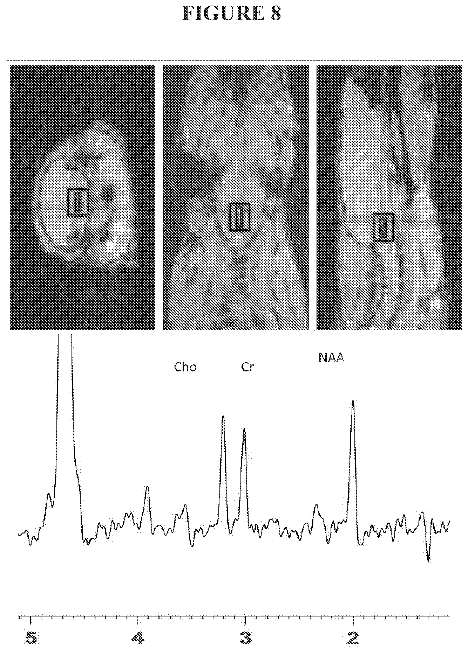

**FIGURE 8****. MRS spectra collected at the mouse brain stem.** Upper panels indicate the voxel from which signal was collected. Positioning this region of interest used anatomic landmarks. A very short MRI scan is used to obtain the images in three orthogonal planes to position the 2.5 mm³ cube over the brainstem (upper panel). A 300MHz, 1H spectrum of the mouse brainstem (lower panel). Choline (Cho), creatine (Cr) and N-acetyl-aspartate (NAA) peaks are easily identified. The spectrum is referenced to water at 4.7 ppm.

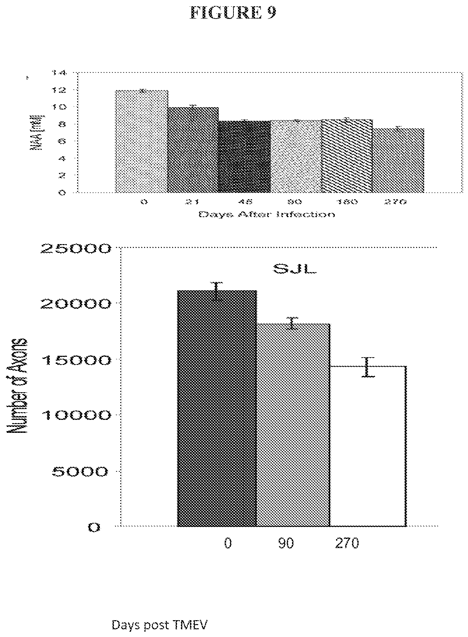

**FIGURE 9****. Decreasing NAA levels at the brainstem correlate with increasing axon loss.** 10 SJL mice were studied prospectively from 0 to 270 days after infection with TMEV. MRS was obtained at the brain stem of each mouse and the NAA concentration determined (**top**). A gradual decrease in NAA was recorded in all mice which reached a statistical significant drop at 90 days after virus infection, the point when large axons are shown to be lost by direct counting in cross sections (2). NAA levels remained depressed for the duration of the disease. Absolute axons counted (**bottom**) at the T6 level of uninfected mice (**black bar**), 90 days (**grey**) and 270 days (**white**) post infection.

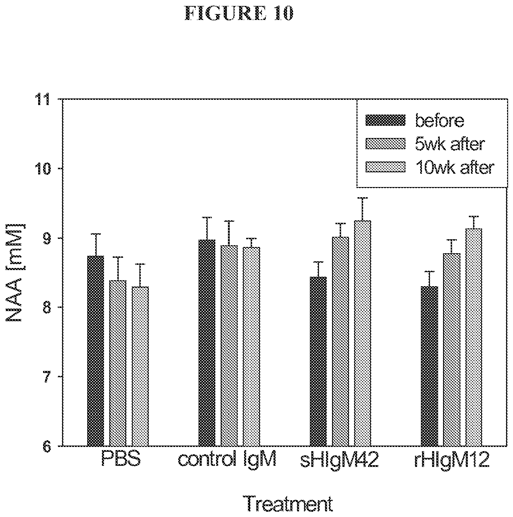

**FIGURE 10****. NAA in the brain stem after a single dose of human IgMs.** Groups of 10-15 SJL mice at 90 days post TMEV infection, the time when NAA levels begin to fall, and large axon loss is detectable were given a single 100 µg dose of sHIgM12, sHIgM42, a control human IgM, or saline (PBS) i.p. MRS at the brainstem was collected before and at 5 and 10 wk after treatment. NAA levels were calculated from these spectra. In the sHIgM12 and sHIgM42 treated groups NAA increased compared to pretreatment levels after 5 wk (P=0.005, P=0.029) and at 10 wk (P<0.001, P=0.037). In groups of mice treated with control reagents were stable or trended down. Graph represents the mean +/- SEM of the brain stem voxel NAA levels.

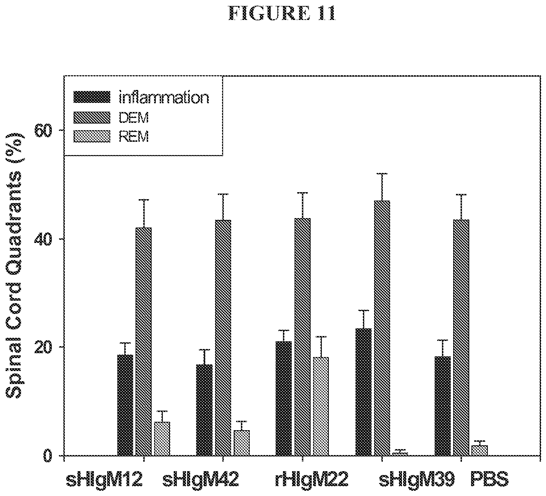

**FIGURE 11****. Human IgMs that bind to neurons do not alter demyelination, remyelination or inflammation.** Groups of 5 chronically TMEV infected mice were treated with a single 100 µg dose of human IgMs. All groups contain the same degree of spinal cord inflammation (**black bars**) and demyelination (**red bars**) at 10 wk after treatment. As expected, mice treated with the oligodendrocyte binding IgM, rHIgM22, showed greater remyelination (**green bars**). sHIgM12 and sHIgM42 do not promote spinal cord remyelination. This suggests there may be multiple ways to protect axons in vivo.

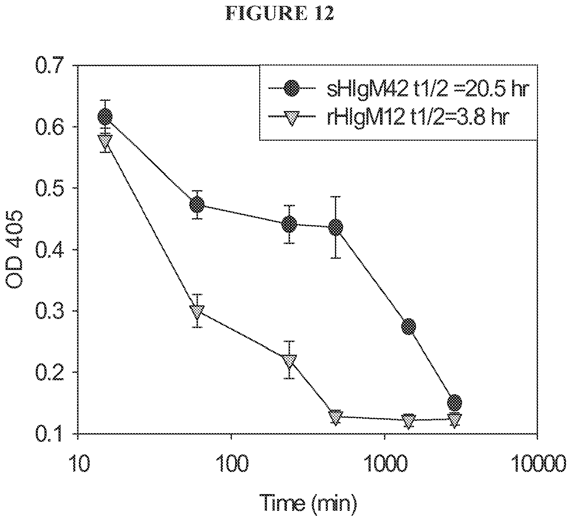

**FIGURE 12****. Serum half life of neuron binding human IgMs in the circulation of CD-1 mice after intravenous injection.** Sandwich ELISA was used to quantify the decay of human mu chain from mouse sera over 48 hr. The mean of the OD405 of alkaline phosphatase substrate (Sigma 104) obtained from the means. Sera from 3 mice per group and standard error are graphed.

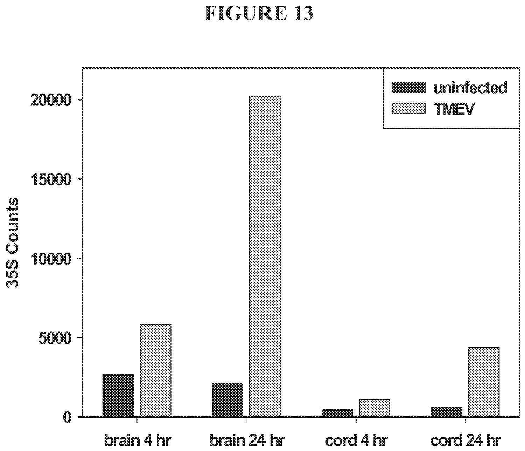

**FIGURE 13****.** **³⁵****S Labeled rHIgM12 entered the brain and spinal cord of TMEV infected and uninfected SJL Mice.** ³⁵S labeled rHIgM12 was administered i.p. to 5 month TMEV infected and age matched uninfected SJL mice. 4 hr later a pair of mice were perfused with saline, tissues acutely harvested, weighed, minced with a razor, and mixed with 10 ml of scintillation fluid (Ultima Gold LSC). The same was repeated at 24 hr. After 48 hr, allowing tissue to solubilize, vials were counted for 1 min in a Beckman scintillation counter. The total counts for the whole brain and spinal cord of each mouse is graphed. Background counts with no tissue in the vials were 12 counts.

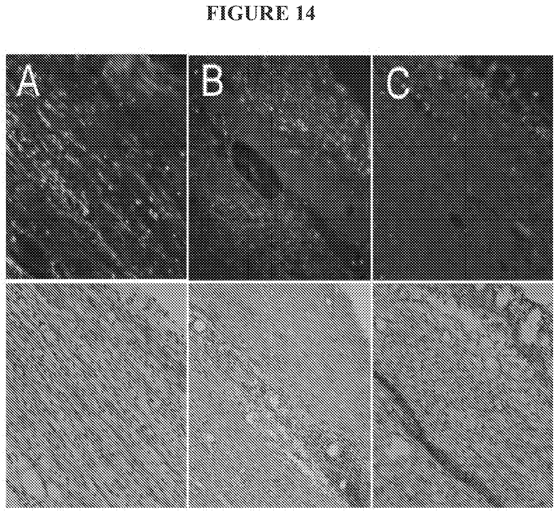

**FIGURE 14****. Neuron binding human IgMs target in vivo to spinal cord lesions.** Mice with chronic TMEV induced spinal cord demyelination were given 1.0 mg intraperitoneally of sHIgM42 (**A**), rHIgM12 (**B**), or control human monoclonal IgM (**C**). 4 hr later mice were perfused with 4% paraformaldehyde, the spinal cords removed, frozen sectioned and immunolabeled using FITC-conjugated Fab'2 fragments directed against human mu chain (Jackson Immnoresearch). Spinal cords from mice receiving sHIgM42 or rHIgM12 contained human IgM in lesions. Little control human IgM was found in lesions. Lower panels are H/E staining to visualize inflammatory cells and lesions. We conclude that certain human IgMs can cross the BBB in the TMEV model.

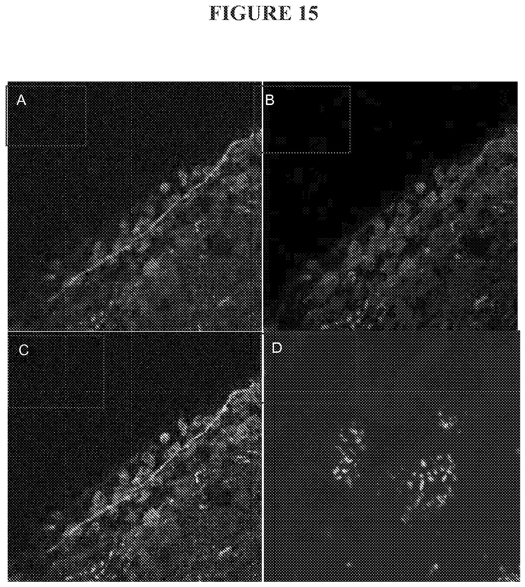

**FIGURE 15****. Neuron binding human IgMs localize to neurofilament+ (NF) axons in spinal cord lesions.** Mice with chronic TMEV induced spinal cord demyelination were administered 1.0 mg intraperitoneally of IgM and killed 4 hr later. Spinal cords were frozen sectioned longitudinally and immunolabeled with FITC conjugated anti-human mu chain Fab'2 fragments (**green**) and ant-NF (SMI-32 and 34, **red**) followed by a TRICT conjugated secondary. Confocal images merged confirm the co-localization of sHIgM42 **(A)** with long NF+ fibers **(B)**, merged in **(C)**. rHIgM12 (**D, green**) also co-localized with NF+ structures (**D, red**) such as a cross cut axon fiber bundles.

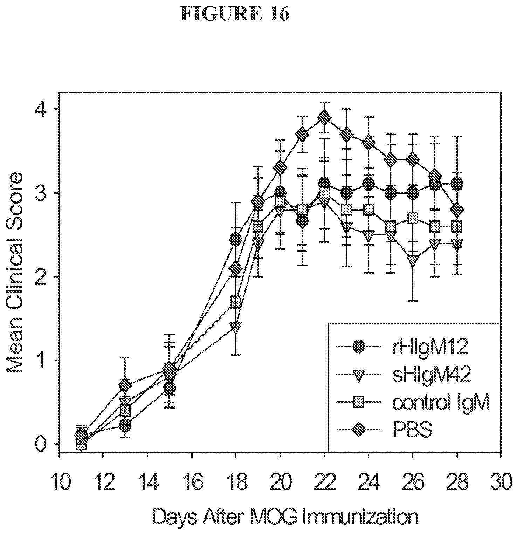

**FIGURE 16****. rHIgM12 and sHIgM42 do not exacerbate EAE clinical scores.** Groups of 10 C57BL6 mice with MOG peptide induced EAE were given a single 100 µg dose i.v. of rHIgM12, sHIgM42, control human IgM or saline at the time each mouse reached a clinical score of 1 (limp tail) and monitored every other day until 28 days post immunization. Graphs are the mean+/- SEM of the mean clinical score of each treatment group. ANOVA on ranks of mean clinical scores indicated no difference between groups (P=0.14).

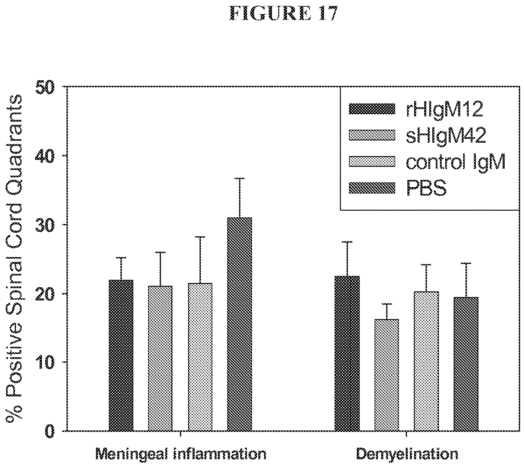

**FIGURE 17****. rHIgM12 and sHIgM42 do not exacerbate EAE spinal cord pathology.** At the end of the study in Fig 13 spinal cords were removed, cut in to 1 mm blocks, cross sections cut from every third consecutive block and stained with erichrome to visualize pathology. 10 cross sections per mouse (40 quadrants) were graded blindly. There were no differences in inflammation (P=0.825) or demyelination (P=0.766) across groups.

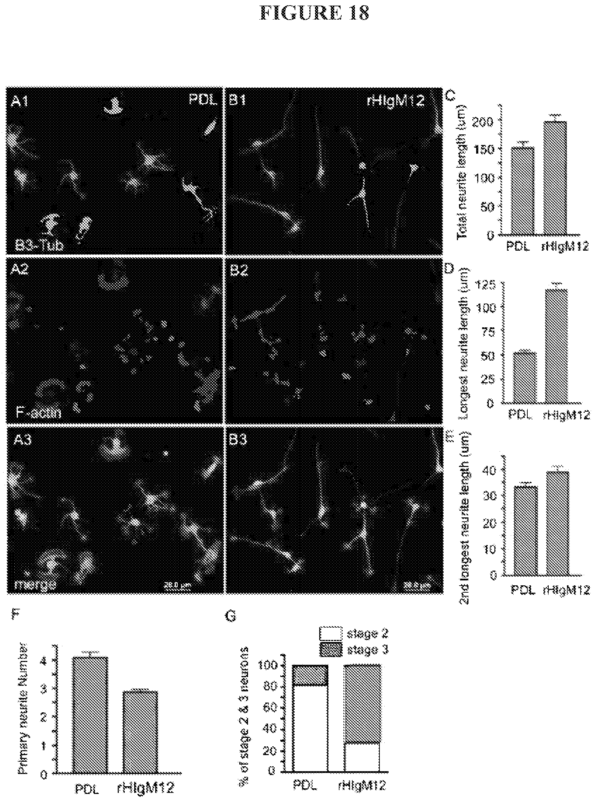

**FIGURE 18****. rHIgM12 promotes neurite outgrowth** E15 hippocampal neurons were grown on poly-D-lysine (PDL) (**A**) or rHIgM12 (**B**) -nitrocellulose coated glass coverslips. 12 hours after plating of cells, these neurons were fixed and stained with β3-tubulin antibody (**A1** & **B1**, green) and Texas-red phalloidin (**A2** & **B2**). **A3-B3** are overlays of **A1-A2** and **B1-B2,** where the nuclei were stained with DAPI (blue). **C, D** & **E** show measurement of the total (**C**, *p*=0.0056), longest (**D**, *p*<0.0001) and 2nd longest (**E**, *p*=0.0782) neurite lengths. Neurons growing on rHIgM12 bear fewer neurites (**F**, *p*<0.0001), and the majority are stage 3 neurons (**G**) as compared to neurons seeded on control PDL substrates (72% vs. 18%) which have multiple symmetric neurites. Statistical analyses show the mean ± SEM (unpaired *t*-test).

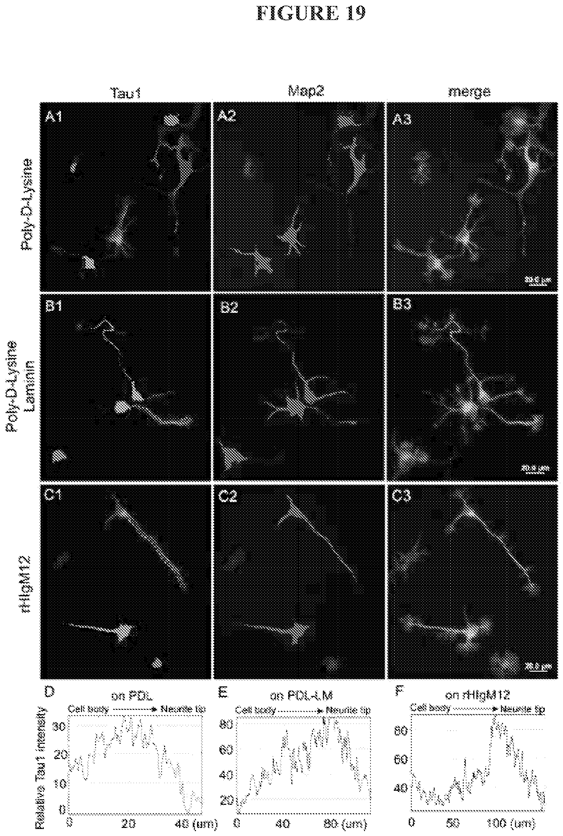

**FIGURE 19****. rHIgM12 drives axon formation** Hippocampal neurons 12 hours after plating on substrates of PDL (**A**), PDL+Laminin (**B**) or rHIgM12 (**C**) were stained with Tau1 (**A1-C1**, green) or MAP2 (**A2-C2**, blue). **A3-C3** are overlays of **A1-C1** and **A2-C2,** and F-actin (red) was labeled with Texas-red Phalloidin. **D-F** show the relative Tau1 intensity along the longest neurites from cell body to the growth cone in **A1-C1** labeled by dashed lines. Tau1 staining is asymmetrically enriched in the distal part of the longest neurite in stage 3 neurons grown on both rHIgM12 and laminin substrates.

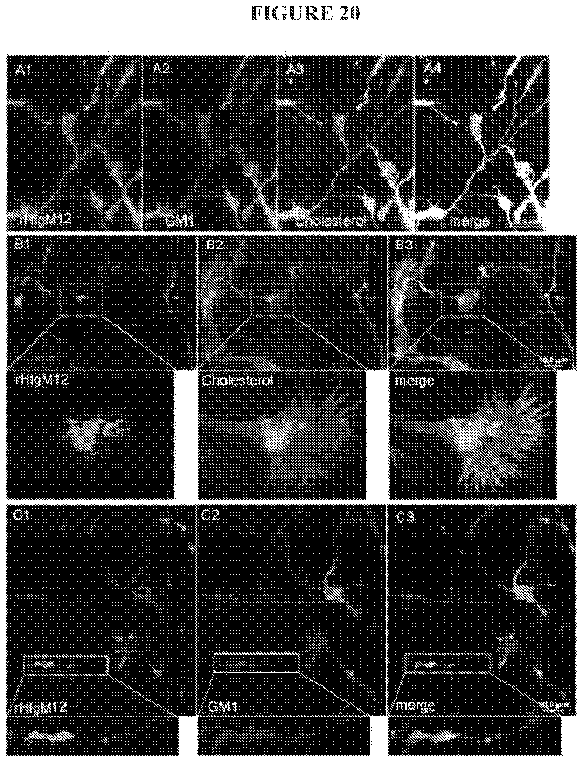

**FIGURE 20****. rHIgM12 binding reorganizes neuronal membranes** DIV3 hippocampal neurons were triple stained with rHIgM12, cholera toxin B (CTB, Alexa Fluor 594) and filipin after fixation (**A**). rHIgM12 (**A1**) evenly labels the neuron surface, with a pattern similar to that of GM1 (**A2**) or cholesterol (**A3**) labeled by CTB or filipin respectively. However, after treatment with rHIgM12 for 30 min at 37°C, membrane-bound rHIgM12 aggregates on the neuron surface (**B1-C1**). Treating with rHIgM12 results in clustering of both cholesterol (**B2**) and GM1 (**C2**), which co-localizes with the aggregated rHIgM12 (**B3-C3**). Lower panels in the bottom of **B** & **C** are higher magnification of the boxed regions, showing colocalization of aggregated rHIgM12 (green) with clustered cholesterol (blue) or GM1 (red) in the growth cone region.

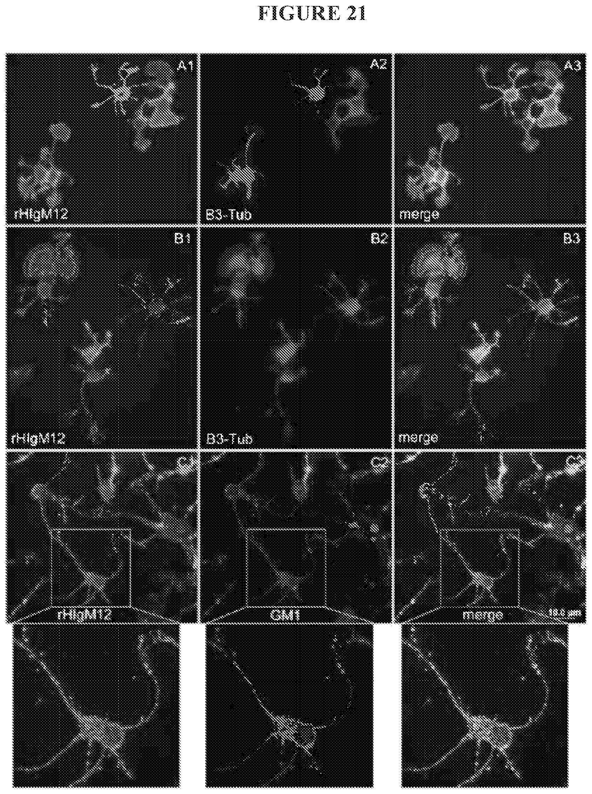

**FIGURE 21****. rHIgM12 co-localizes with GM1 on neuron membrane microdomains A.** DIV1 hippocampal neurons were fixed with 4% paraformaldehyde first and then stained by rHIgM12. rHIgM12 labels smaller punctum structures. **B.** Live DIV1 hippocampal neurons were cooled down to 4 °C for 30 min and then labeled with rHIgM12, followed by staining with anti β-III-tubulin antibody after fixation. rHIgM12 stains much larger punctate formations on neuron surface. **C.** Live DIV3 hippocampal neurons were treated with methyl-β-cyclodextrin for 30 min at 37°C to deplete cholesterol and then cooled down to 4°C. The fixed neurons were then stained with rHIgM12 (green) and cholera toxin B (red). rHIgM12 binds to the larger puncta which co-localizes GM1 labeled by cholera toxin B. The lower panel shows higher magnification of the boxed regions.

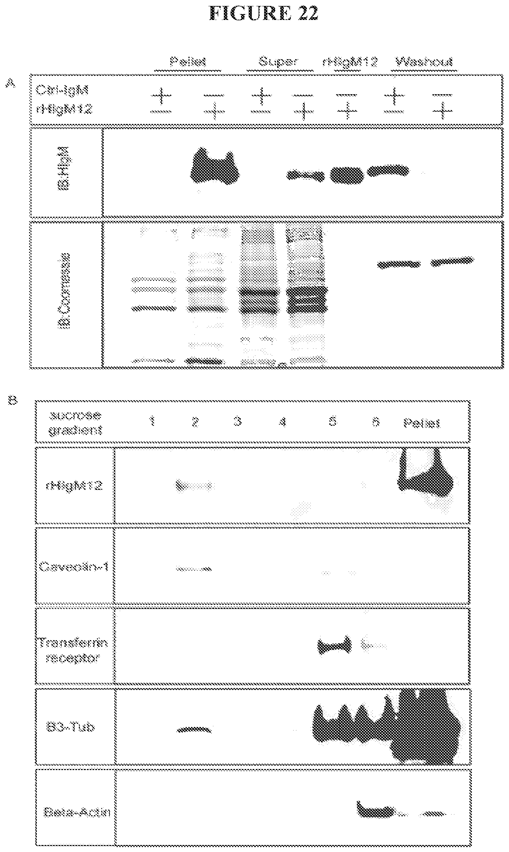

**FIGURE 22****. rHIgM12 binds to lipid rafts** Live DIV7 cortical neurons were allowed to bind rHIgM12 or control IgM antibody at 4 °C for 30 min. **A**. Neurons were washed three times after antibody binding and lysed in buffer containing 0.5% NP-40. The lysates were separated by micro-centrifugation at 4 °C, and both the supernatants and the pellets were blotted with anti-human IgM secondary antibody. rHIgM12 distributes into both the pellet and the supernatant compared with the control IgM antibody, which does not bind to neurons and goes to the washout. The lower panel shows a separate Coomassie-stained gel loaded with the same amount of protein. **B**. Neurons lysed in buffer containing 1% Triton X-100 were fractionated by ultracentrifugation in sucrose gradients at 4 °C. The resulting fractions were sequentially blotted for rHIgM12, caveolin-1, transferrin receptor, β3-tubulin (B3-Tub), and beta-actin with specific antibodies. rHIgM12 localizes to the low density fraction which contains caveolin-1 and β3-tubulin. The higher density fractions contain transferrin receptor, β3-tubulin and actin. Some rHIgM12 localizes to the detergent insoluble pellet, which is mainly composed of cytoskeletal proteins enriched in tubulin. The majority of actin goes to detergent soluble higher density fractions.

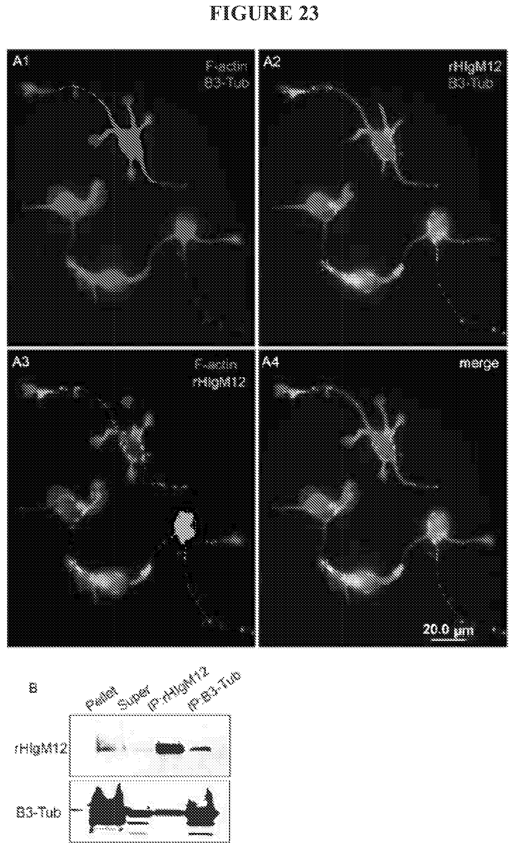

**FIGURE 23****. rHIgM12 associates with microtubules A.** DIV1 hippocampal neurons were first treated with rHIgM12 for 30 min at 37 °C, then simultaneously fixed and extracted with buffer containing 4% paraformaldehyde and 0.1% Triton X-100 and followed by staining for rHIgM12 (green), β3-tubulin (blue) and F-actin (red). rHIgM12 binds the detergent-insoluble molecules which co-localize with the fasciculated microtubules along the neurite shafts (**A 2** & **4**) and at the growth cone central domain, but not with F-actin localized in the growth cone periphery (**A 3** & **4**). **B**. DIV7 cortical neurons were allowed to bind rHIgM12 first, and then lysed in 0.5% NP-40. The supernatants were subjected to co-immunoprecipitation. Both rHIgM12 and β3-tubulin were co-immunoprecipitated with each other.

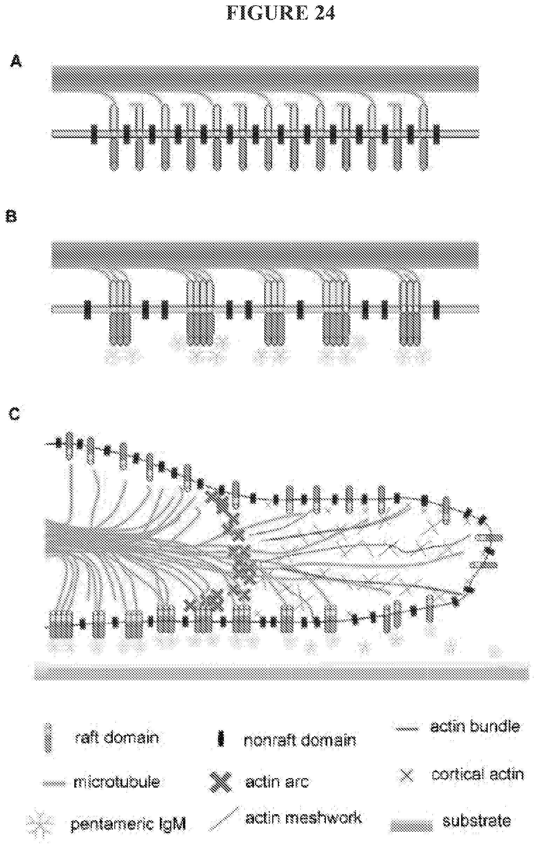

**FIGURE 24****. Proposed mechanisms by which rHIgM12 promotes axon formation A.** Neuron membranes contain both raft and nonraft microdomains. The raft microdomains, which are evenly distributed on neuronal membranes, segregate into two pools. One of them is associated with microtubules. **B**. Binding of rHIgM12 induces clustering of raft microdomains, which promotes microtubule stabilization and anchoring neuron membranes. **C**. During neurite outgrowth, the growth cones explore the environment and the raft microdomains cluster by interacting with rHIgM12. As a result, the microtubules are stabilized at the rHIgM12-contacting sites and anchor the neuron membranes, which enhances growth cone periphery to central domain transition as observed in Figure 3 & 6 where the bath-applied rHIgM12 is aggregated in the growth cone central domain.

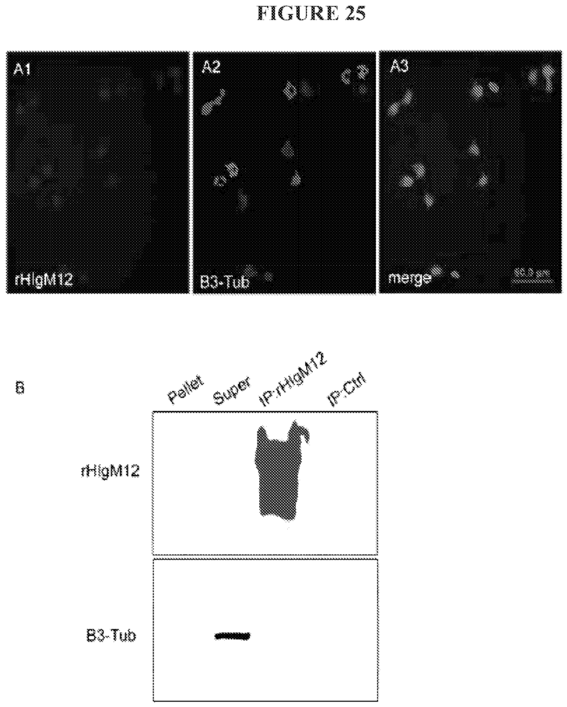

**FIGURE 25****. rHIgM12 does not associate with tubulin directly A.** N2A neuroblastoma cells were stained with rHIgM12 (**A1**, green) and β3-tubulin (**A2**, red). The nuclei were labeled with DAPI (**A3**, blue). N2A cells are negative for rHIgM12 staining but express β3-tubulin. **B**. Supernatants of N2A cell lysates were incubated with rHIgM12 and molecules associated with rHIgM12 were pulled down by protein-L agarose and subject to western blotting. rHIgM12 is not associated with the pellet. β3-tubulin was not pulled down by rHIgM12, though detected in the supernatants.

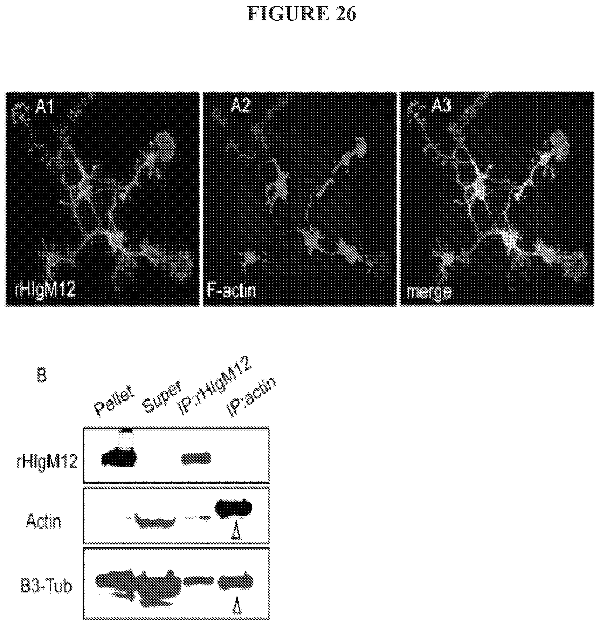

**FIGURE 26****. rHIgM12 pulls down actin, but does not colocalize with F-actin A.** DIV1 live hippocampal neurons were stained at 4 °C with rHIgM12 (**A1**, green) first, and F-actin (**A2**, red) was labeled with Texas-red phalloidin after fixation. In the growth cone region, F-actin shrunk and was enriched in the central domain, whereas rHIgM12 evenly stained the puncta structures on growth cone surface, which labeled the very edge of growth cone. **B**. A small amount of actin was pulled down by rHIgM12, and none of three anti-actin antibodies tested worked in immunoprecipitation. The band at the similar position as β3-tubulin is the IgG heavy chain indicated by the empty arrowhead.

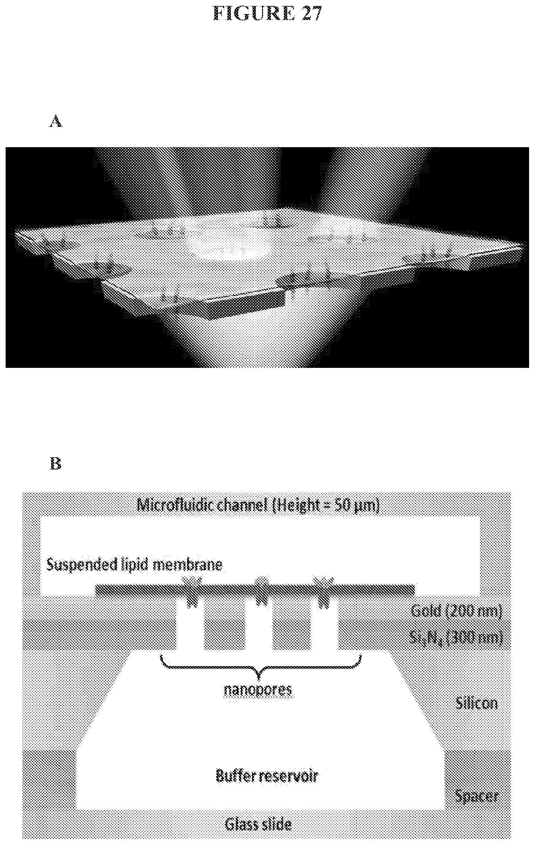

**FIGURE 27 (a)** Cell membranes will be reconstituted on a thin gold film perforated with thousands of nanopores (200 nm in size). **(b)** Suspended gold film is in contact with a buffer solution on both sides.

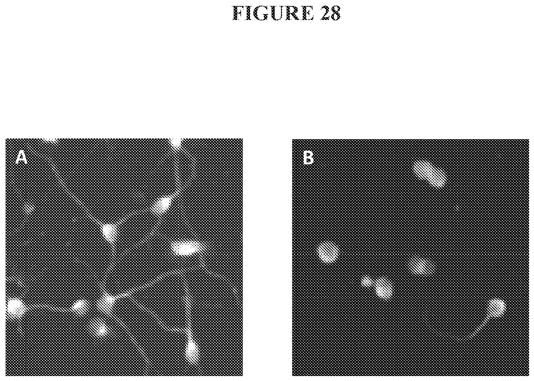

**FIGURE 28****. A human antibody that binds to neurons promotes neurite extension.** Neurons were seeded onto substrates coated with human IgM, allowed to grow for 24 hr and immunolabeled with anti-neurofilament. sHIgM42 induced outgrowth (**A**) compared to a non-permissive human IgM **(B)**

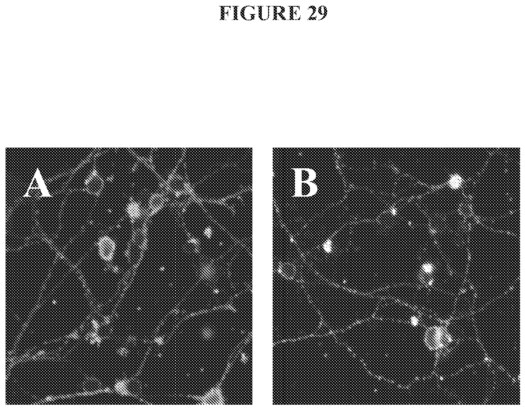

**FIGURE 29****. A human antibody that binds to neurons clusters membrane domains.** Uniform immunolabeling on soma and axons was performed at 0° C (**A**). When cells are warmed to 15°C for 15 min sHIgM42 is localized to more discrete patches on the cell surface of axons (**B**).

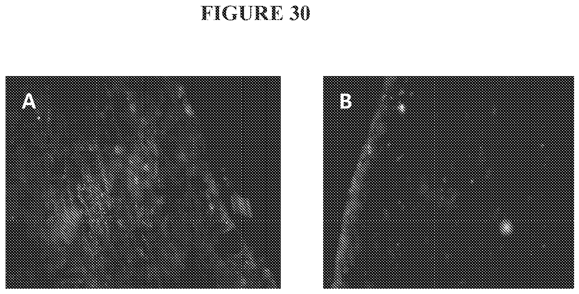

**FIGURE 30****. Neurite extension promoting human antibody targets in vivo to areas of injury.** Mice with chronic SCI were given antibody i.p., spinal cords removed 4 hr later and human IgM detected in spinal cord cross sections. Injured areas of spinal cord from a mouse given antibody demonstrate parallel fibers that bind a fluorescently tagged anti-human IgM (**A**). Injured areas of spinal cord from a mouse given a control human IgM do not contain human IgM (**B**).

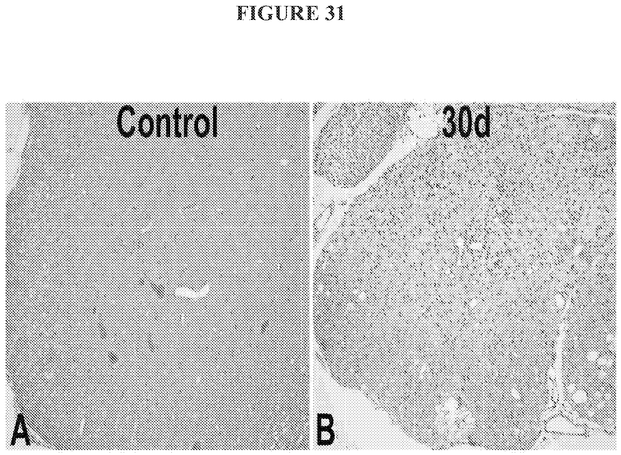

**FIGURE 31****. Spinal cord crush injury.** Photomicrographs show typical appearance of H&E stained spinal cord from control mice(**A**) or mice 30d post 8 g Fejota clip compression (**B**). Compression injury is associated with extensive inflammatory cell infiltration, loss of motoneurons.

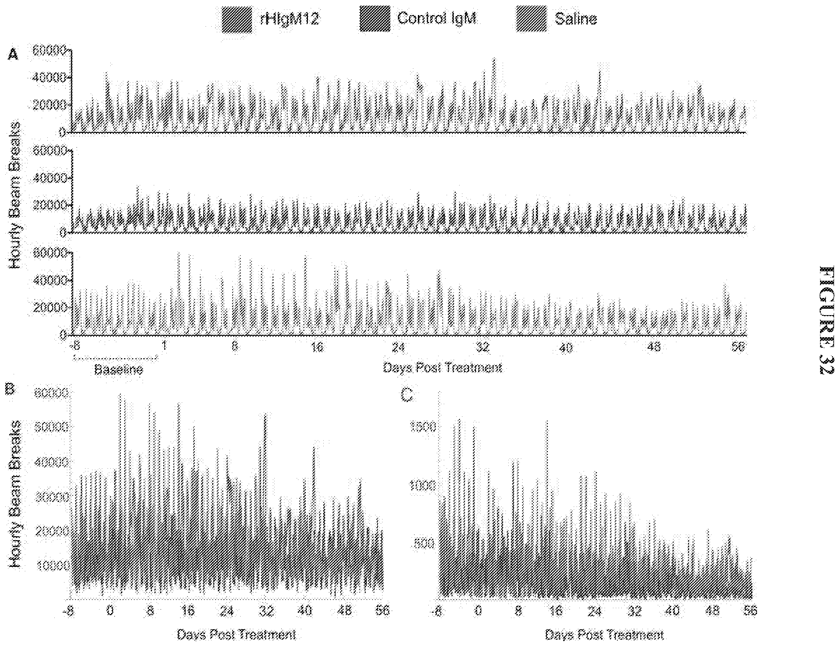

**FIGURE 32****. Example of spontaneous nocturnal activities of TMEV infected mice. A)** Original complete recordings of horizontal activity over the period of 64 days. High nocturnal and low diurnal activities are easy to appreciate. Because mice are normally active during the night time we selected nocturnal activities for analysis (6PM-6AM). However, by visual inspection of unfiltered compressed recordings in either horizontal (**B**) or vertical (**C**) activity it may be difficult to identify real long-term changes.

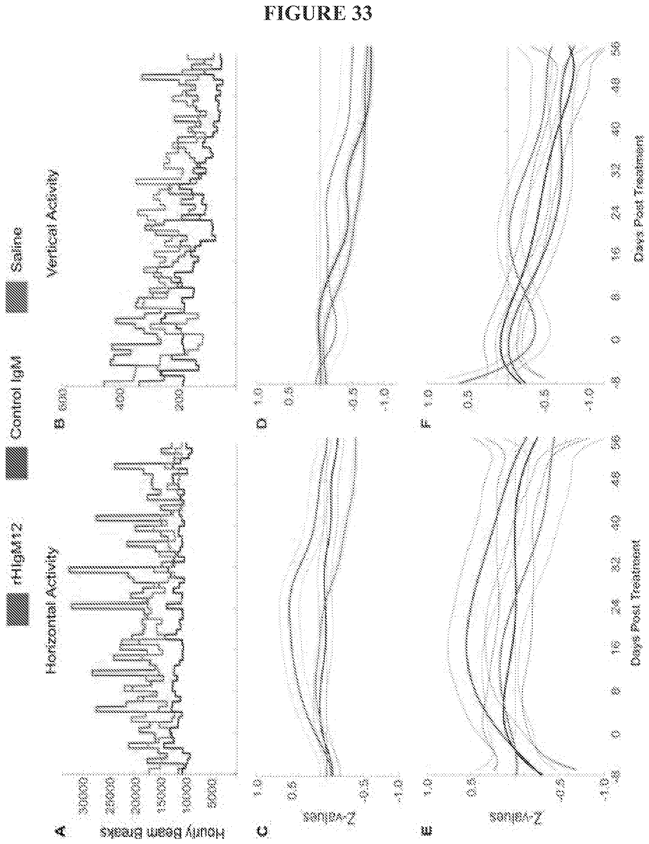

**Figure 33****. A single i.p. dose of neuron-binding antibody (rHIgM12) improves horizontal activity in chronically infected SJL mice.** Three groups of 5 SJL mice at 90dpi were placed in AccuScan activity monitoring boxes. Baseline measurements were collected over 8 days. After the treatment mice were monitored continuously over 8 weeks. Panels A, C and E correspond to horizontal activity and B, D and F correspond to vertical activity. **A, B**) Average nocturnal activities presented as bins for each day suggested the improvement in horizontal activity only in rHIgM12-treated group; **C**) Gaussian filtering (GB=2 days) and **E)** 6th order polynomial fitting of standardized Zvalues provided a clear and distinct improvement in rHIgM12-treated mice. **B, D and F)** Vertical activity was not affected by any treatment. Activity (vertical scale) is the number of beam interruptions per hour. Parameter GB can be interpreted as a value (in days) of the filter half width at the half height. With the increase of GB value the standard deviation decreases. The pointwise 95% confidence bands are given for each day.

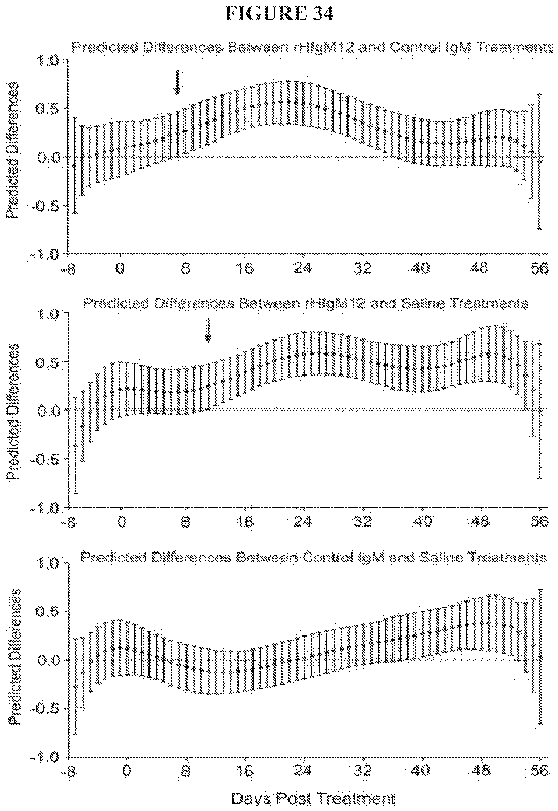

**FIGURE 34****. Comparison of three treatments at 90dpi by using predicted model values.** Statistical analysis was performed for each day before and after the treatment. Horizontal nocturnal activity of rHIgM12-treated mice as compared to control IgM- and saline-treated mice significantly diverged/improved on day 7 (p=0.045) and day 11 (p=0.042) post-treatment, respectively (denoted by black arrows). The pointwise 95% confidence bands are given for each day.

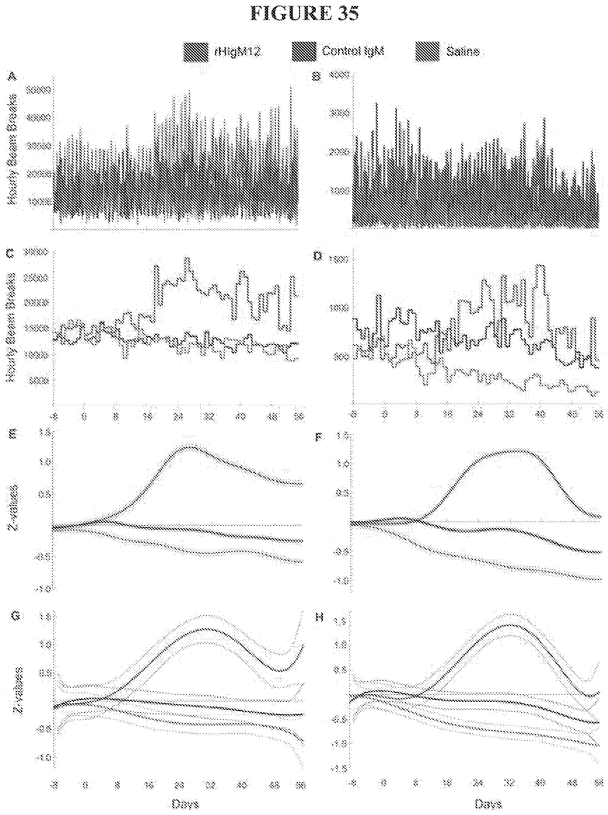

**FIGURE 35****. rHIgM12 improves both horizontal and vertical activity in SJL mice when given early in the disease.** Three groups of 5 SJL mice at 45dpi were placed in AccuScan activity monitoring boxes. Baseline measurements were collected over 8 days. After the treatment mice were monitored continuously over 8 weeks. Panels A, C, E and G correspond to horizontal activity and B, D, F and H correspond to vertical activity. **A, B)** Original, unfiltered recordings for horizontal and vertical activity; **C, D)** Average nocturnal activities presented as bins for each day, **E, F)** Gaussian filtering (GB=2 days) and **G, H)** 6th order polynomial fitting of standardized Z-values revealed improvement in both horizontal and vertical activity in rHIgM12-treated group. Control IgM- and salinetreated groups showed similar levels of motor activity throughout the study. The point wise 95% confidence bands are given for each day.

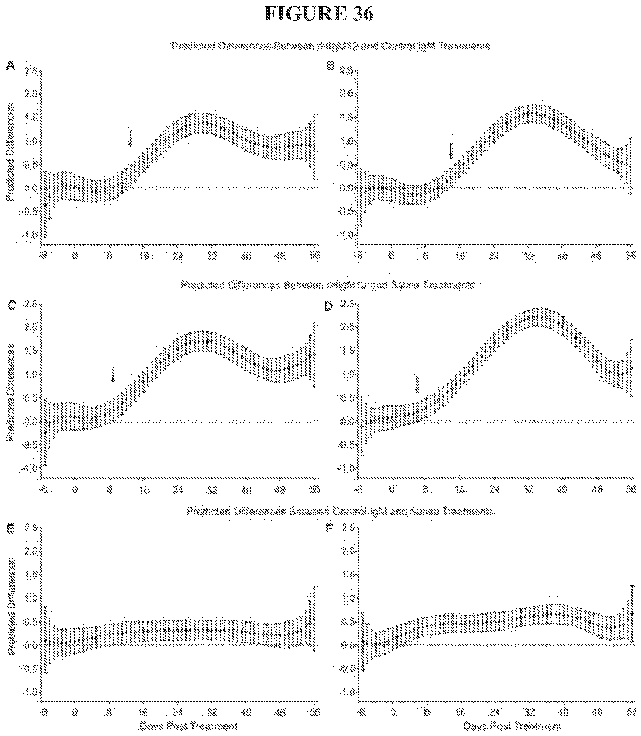

**FIGURE 36****. Comparison of three treatments at 45dpi by using predicted model values.** Statistical analysis was performed for each day before and after the treatment. **A, C**) Horizontal nocturnal activity of rHIgM12-treated mice as compared to control IgM- and saline-treated mice significantly diverged on day 13 (p=0.015) and day 9 (p=0.036) posttreatment, respectively. **B, D**) Vertical (rearing) activity of rHIgM12-treated mice diverged/improved on day 14 (p=0.019) and day 6 (p=0.037) post-treatment, respectively. Black arrows denote days with onset of significant improvement. The pointwise 95% confidence bands are given for each day.

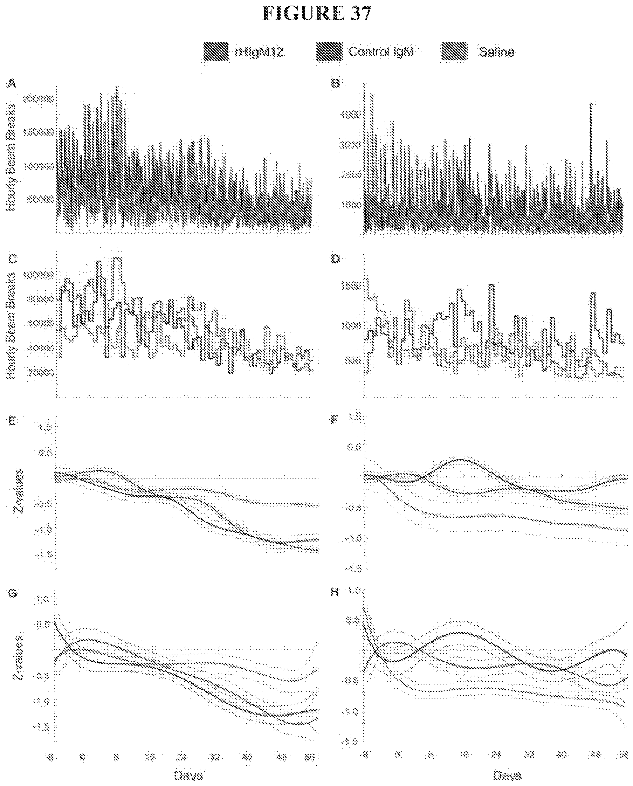

**FIGURE 37****. Normal, uninfected SJL mice have highly variable spontaneous activity which is not influenced by any treatment.** Three groups of 5 uninfected SJL mice were placed in AccuScan activity monitoring boxes. Baseline measurements were collected over 8 days. After the treatment mice were monitored continuously over 8 weeks. None of the treatments induced changes in either horizontal (**A, C, E** and **G**) or vertical (**B, D, F** and **H**) activity. The pointwise 95% confidence bands are given for each day.

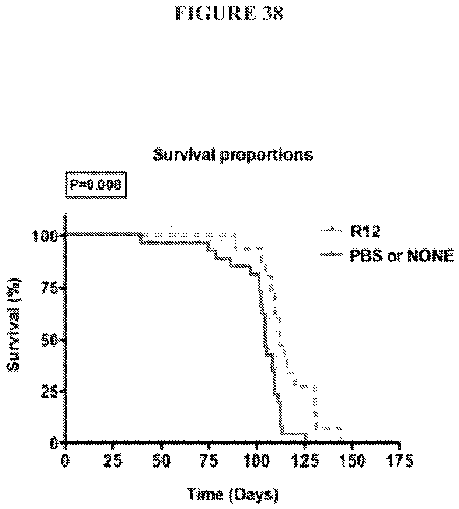

**FIGURE 38****. A single dose of human antibody increased survival in SOD1 G86R mutant transgenic mice.** Mice were treated with a single dose of rHIgM12 or PBS at 55 days of age. Some mice were left untreated. Mice were followed until moribund and then sacrificed for pathology. Some mice died from disease. All mice had profound weight loss prior to death. Survival was plotted using Kaplan-Meir curves and statistical significance determined using the non-parametric Mantel-Cox test. There was an increase in survival in mice treated with rHIgM12 compared to PBS-treated or untreated mice, P=0.008. All analyses and treatment were done in a blinded fashion such that the investigator making the decision to sacrifice the mice had no knowledge of treatment groups. A total of 45 mice were studied in this analysis. Littermate wild-type SOD +/+ mice had normal survival and no weight loss (data not shown).

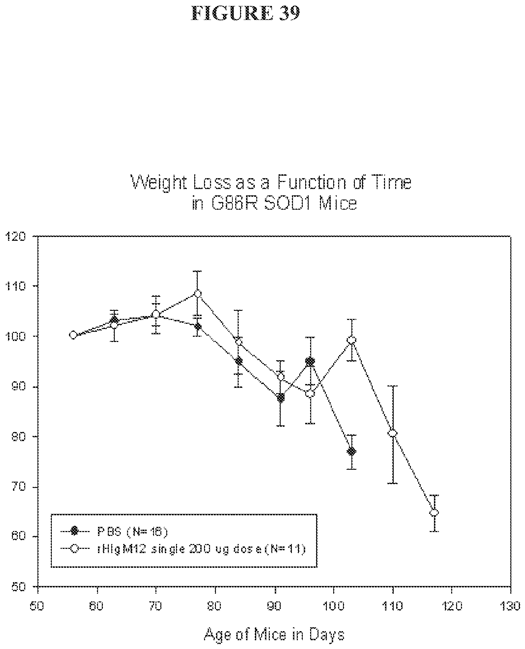

**FIGURE 39** depicts weight loss in G86R SOD1 mice as a function of time in mice administered a single 200 µg dose of rHIgM12 at 50 days of age versus PBS administered control mice. rHIgM12 administered mice showed less weight loss over a period of 60 days after the single injection of antibody.

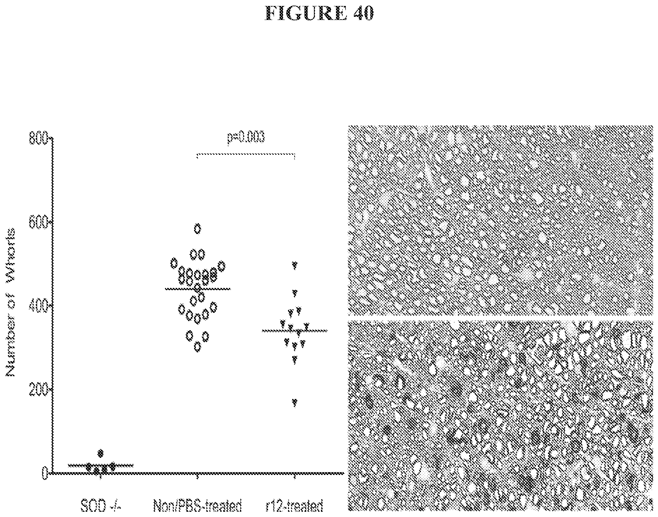

**FIGURE 40****. A single dose of human antibody decreased spinal cord axon degeneration in SOD1 G86R mutant transgenic mice.** Mice were treated with a single 250µg dose of rHIgM12 or PBS at 55 days of age. Some mice were left untreated. The nunber of myelin whorls, characteristic of degenerating axons, were counted in the spinal cord at the mid thoracic level. The number of degenerating axons was decreased in SOD1 mutant mice treated with rHIgM12 compared to PBS treated (P=0.003, T-test) and untreated mice. Note that the number of degenerating axons in the spinal cord of wildtype SOD-/- mice is minimal compared to SOD1+/+ mutant mice. At time of sacrifice, mice were perfused with Trump's fixative (4% formaldehyde, 0.1% gluataraldehyde) and the spinal cord cut into 1mm blocks. Blocks were embedded in Araldite plastic and 1 micron sections from the T6 level were stained with para phenylene diamine to visualize myelin wrapping. Spinal cord sections of wildtype SOD-/- (top-right) and SOD1 +/+ mutant (bottomright) are shown.

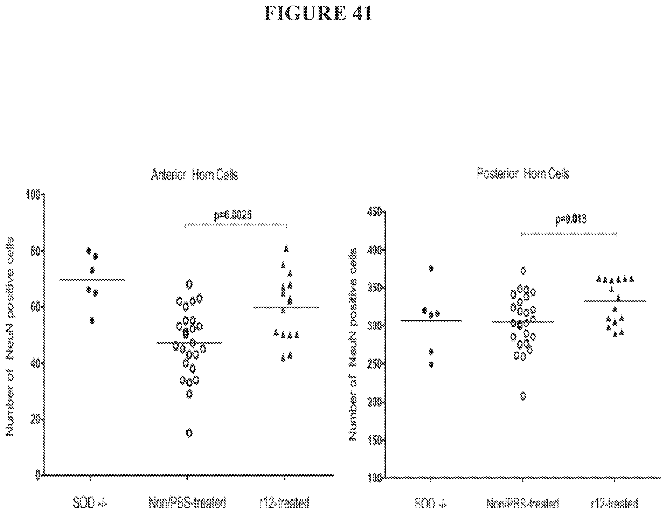

**FIGURE 41****. A single dose of human antibody preserved both anterior and posterior horn neurons in the spinal cord of SOD1 G86R mutant transgenic mice.** Mice were treated with rHIgM12 or PBS at 55 days of age. Some mice were left untreated. The nunber of anterior and posterior horn cells, stained with an antibody to NeuN which specifically labels neurons, were counted in paraffin sections of spinal cord at the a mid thoracic level. The number of anterior and posterior horn cells in rHIgM12 treated mice were significantly greater (P=0.0025 and P=0.018 by T-test) than in mice treated with PBS or those left untreated. The number of anterior and posterior horn cells in SOD mutant mice treated with rHgM12 were similar to those observed in wild type SOD-/- mice.

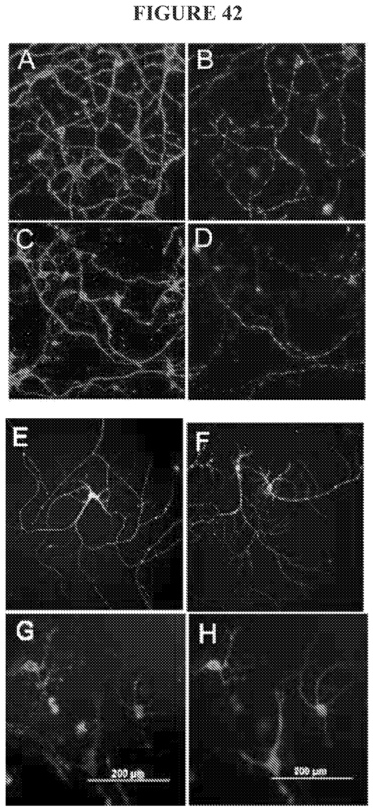

**FIGURE 42****. The recombinant human antibody, rHIgM12, binds to neurons across species.** Mouse cortical neurons (A,B,C,D), mouse hippocampal neurons (E, F) and human cortical neurons (G,H) were stained live with rHIgM12 (A,C,E and G) and the same cells co-labeled with antibodies to neurofilaments (B,D), or beta tubulin (F,G). rHIgM12 was detected with a fluorescently tagged secondary antibody.

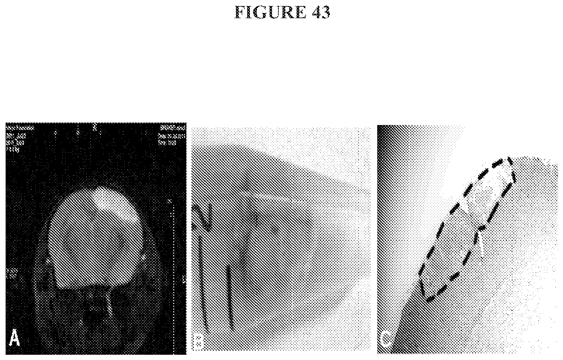

**Figure 43****. Cerebral ischemia in CD-1 mice. A.** DWI-MRI image scan showing the area of cerebral ischemia (hyperintense region) at 24H. **B.** Ischemic damage on the mouse brain following cerebral ischemia (pale circular region on the right hemisphere of the brain). **C**. H&E section at x4 showing the damaged tissue (highlighted in black).

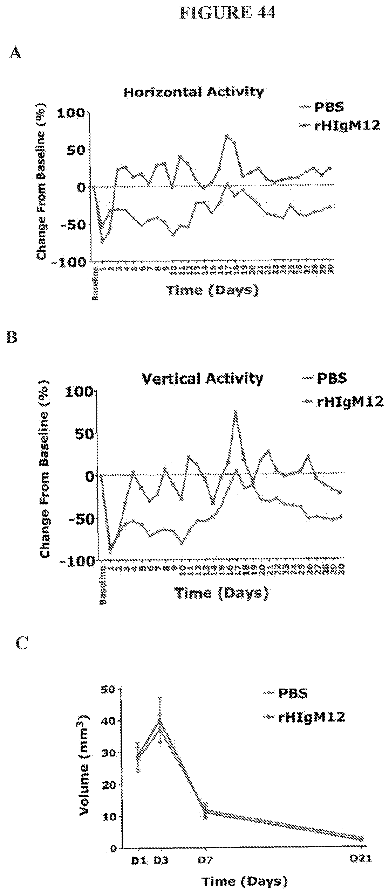

**Figure 44****. A single i.p. dose of neuron-binding antibody (rHIgM12) improves horizontal (A) and vertical (B) activities** in CD1 mice subjected to photothrombotic cerebral ischemia. Mice were treated with a single dose of 200 µg rHIgM12 (n=5) or PBS (n=5). Activity improved from day three in rHIgM12 treated group. **C**. Mice were scanned in a small animal MRI system to measure the total volume of cerebral ischemia that is expressed as mm³ in the graph.

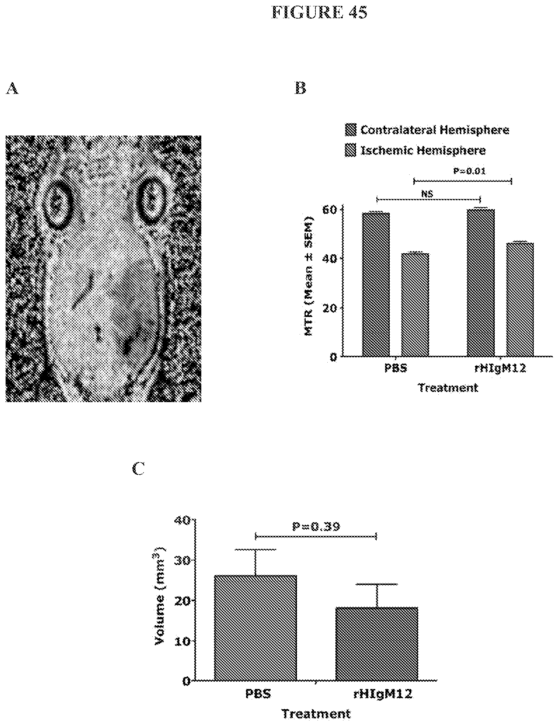

**Figure 45****. MT-MRI metrics demonstrate improved outcome.** Mice were subjected to cerebral ischemia and treated with rHIgM12 or PBS. Three days following the ischemic insult, MTR maps (panel A) of the ischemic (red outline) and contralateral (green outline) hemispheres of the brain were computed. Group treated with rHIgM12 demonstrated relative increase of MTR within the stroke ROI (panel B, blue bars represent values computed on the control side) and decreased stroke volume (panel C) as compared to group treated with PBS.

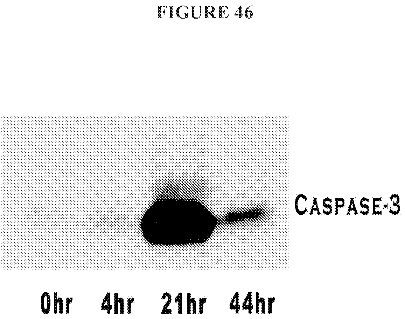

**Figure 46****. Western blot analysis of cortical neurons (CN)** grown on poly-D-Lysine for 5 days and incubated for 4-44 hrs under hypoxic conditions (2.7 % O₂, 5 % CO₂). Representative blot showing the levels of cleaved caspase-3 after hypoxic treatment for 4 hr, 21 hr and 44 hr compared to control (44 hr, 0 hr of incubation in the blot).

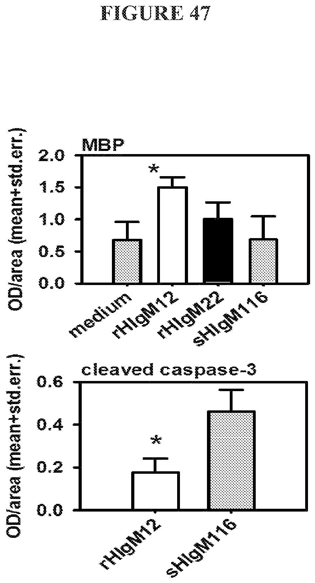

**Figure 47** depicts bar graphs showing quantitative analysis of Western blots from three independent experiments with myelin marker MBP and apoptosis marker cleaved capase-3. Mixed glial cultures were grown for 4 days in FBS (10 %) containing medium and treated for 7 days in chemically defined medium with remyelination-promoting IgM rHIgM22, regenerative mAb rHIgM12, isotype controls sHIgM116 (10 µg/ml each) or medium only. Medium with fresh antibodies was exchanged every second day. At the end of 4 days, cultures were evaluated for MBP or caspase-3 using Western blot, quantitated by OD/area, and plotted.

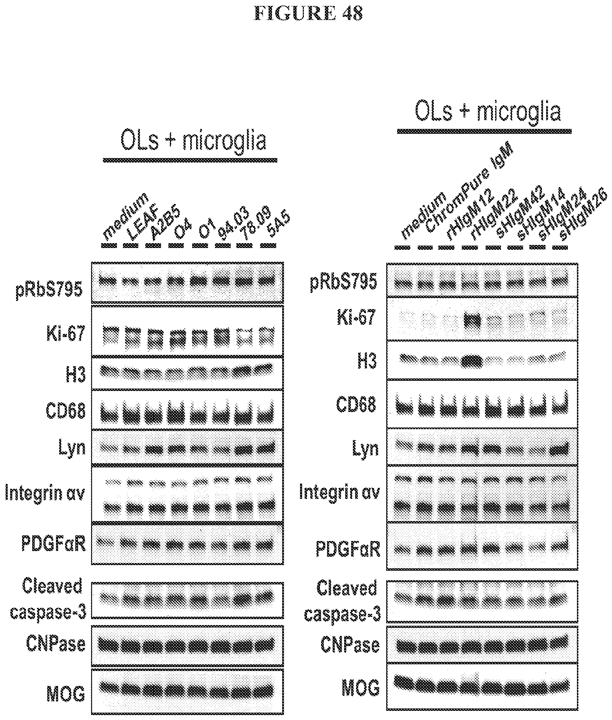

**Figure 48** provides representative Western blots showing levels of various proteins and markers: oligodendrocyte progenitor cell (OPC) markers PDGFaR, myelin markers CNPase and MOG, microglial markers CD68 and Lyn, proliferation marker Ki-67 and histone H3, apoptosis marker cleaved caspase-3 and cell cycle marker pRbS795. OPC-microglial co-cultures were cultured in astrocyte-conditioned medium with remyelination promoting mAbs A2B5, 04, O1, 78.09, 94.03, rHIgM22, regenerative mAb rHIgM12, isotype controls (LEAF, 5A5, ChromPure IgM, rHIgM42, sHIgM14, sHIgM24, sHIgM26) (10 µg/ml each) or medium as indicated.

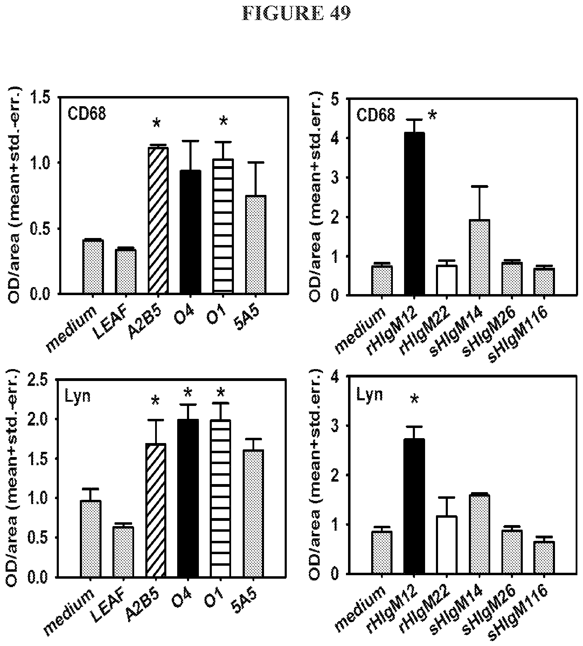

**Figure 49** depicts bar graphs showing quantitative analysis of Western blots from three independent experiments with marker CD68 for activated microglia and Lyn kinase, a highly abundant kinase in microglia and to a lower extent in OPCs but not in astrocytes. Mixed cultures (OPC-microglial co-cultures) were treated with antibodies as noted above for Figure 48 and indicated.

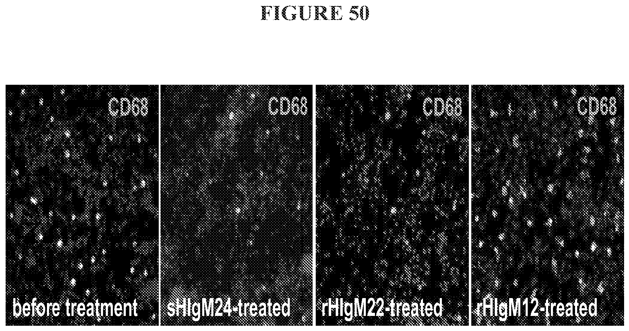

**Figure 50** provides representative Immunofluorescence images show levels of CD68 (green) and nuclear marker DAPI (blue) in mixed glial cultures before mAb treatment at day 4 in culture and at day 11 in culture after 7 days of treatment with rHIgM12, rHIgM22 and isotype control sHIgM24 (10 µg/ml each).

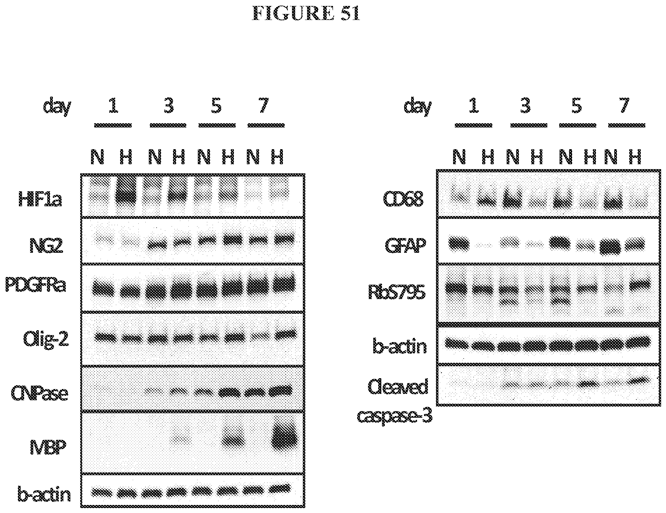

**Figure 51** provides representative Western blots showing levels of hypoxia-inducible factor 1 (HIF1α), oligodendrocyte markers NG2, PDGFα, Olig-2, myelin markers MBP and CNPase, astrocyte marker GFAP, microglial marker CD68, cell cycle marker pRbS795 and apoptosis marker cleaved caspase-3, with Beta-actin was used as a loading control. Mixed glial cultures were grown for 4 days in FBS (10 %) containing medium and treated for up to 7 additional days in chemically defined medium under hypoxia (3 % 02) compared to normoxic conditions (21%O2). Proteins were assessed by Western blot at day 1, 3, 5 and 7 as noted.

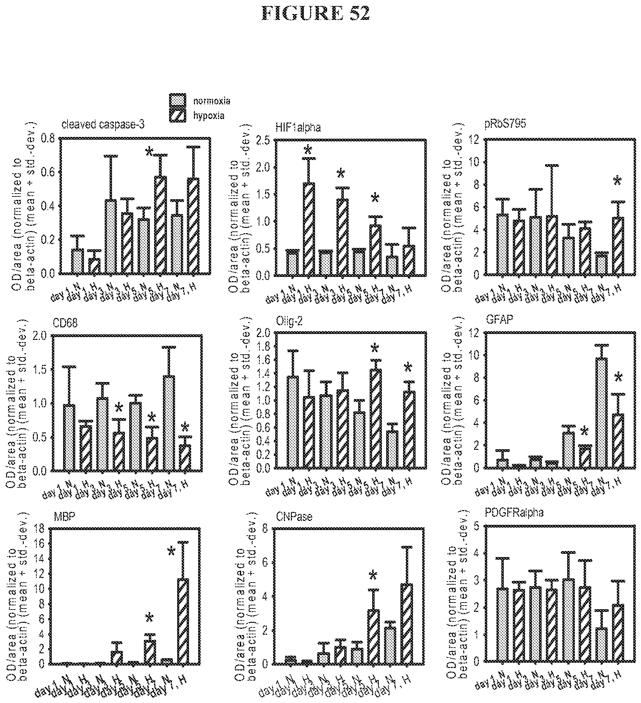

**Figure 52** depicts quantitative analysis of the Western blots of Figure 51, to provide quantitative values for expression and comparison of hypoxic and normoxic conditions for the protein markers.

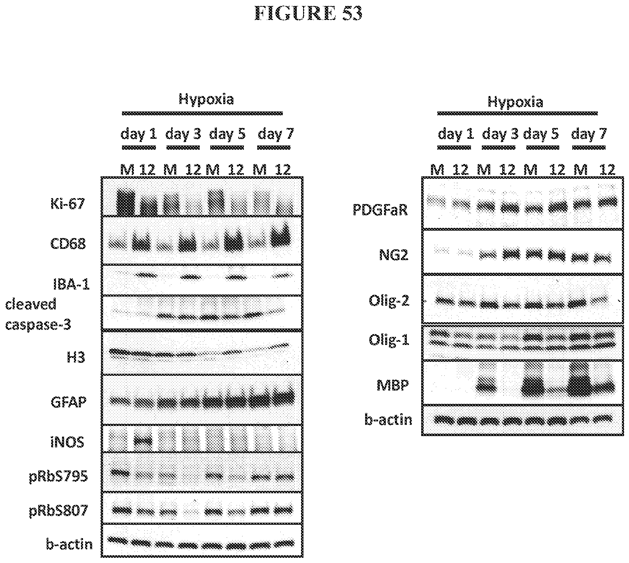

**Figure 53** provides representative Western blots of proliferation marker Ki-67, microglial markers IBA-1, CD68, and iNOS, apoptosis marker cleaved caspase-3, astrocytic marker GFAP, cell cycle markers pRbS795 and pRbS807, oligodendrocyte markers PDGFaR, NG2, Olig-1 and Olig-2, and myelin marker MBP. Mixed glial cultures were grown for 4 days under normoxic conditions in FBS (10 %) containing medium and subsequently treated for 1-7 days in chemically defined medium with human regenerative antibody rHIgM12 or medium only under hypoxia (3 % 02).

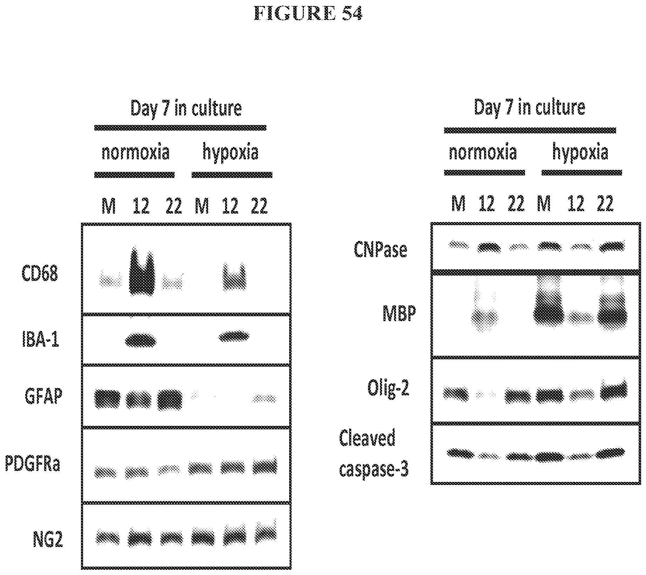

**Figure 54** provides representative Western blots showing levels microglial markers IBA-1 and CD68, apoptosis marker cleaved caspase-3, astrocytic marker GFAP, oligodendrocyte markers PDGFaR, NG2, , Olig-2 and myelin markers MBP and CNPase in day 7 mixed cultures grown under normoxic and hypoxic conditions and treated with rHIgM12 or rHIgM22 antibodies versus medium alone. Mixed glia was cultured as described before and treated in chemically defined medium for 7 days with rHIgM12, rHIgM22 or medium only under hypoxic or normoxic conditions.

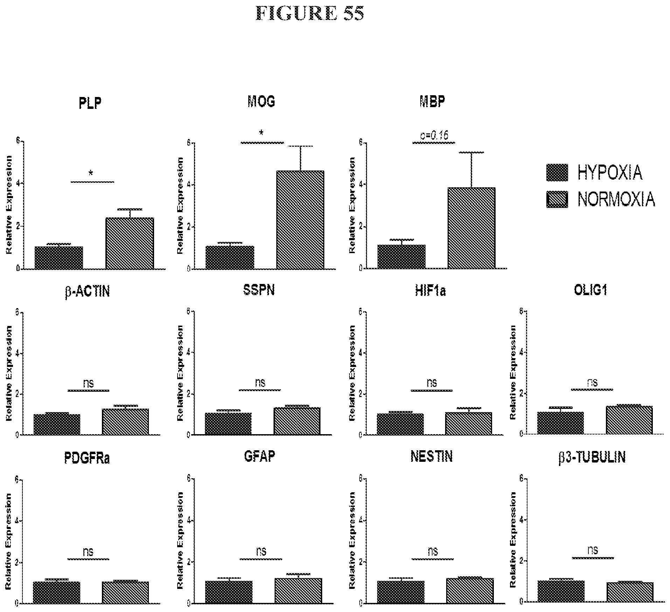

**Figure 55** provides RT-PCR analysis of various markers from cortices from hypoxic and normoxic C57/BI6 neonate mice at P13. C57/BI6 neonatal mice were cross-fostered by CD1 dams and exposed to hypoxia (10 % 02) for 4 days from P3 to P7. Mice were subsequently exposed to room air for additional 6 and sacrificed at P13. RNA was isolated from cortices in both treatment groups and probed for myelin markers PLP1, MOG, MBP1, oligodendrocyte progenitor markers PDGFaR and Olig-1, astrocytic marker GFAP, neuronal markers Nestin and β3-tubulin, hypoxia-inducible factor 1α and the sarcospan protein SSPN. Expression levels were analyzed by RT-PCR and results normalized to levels of β-actin.

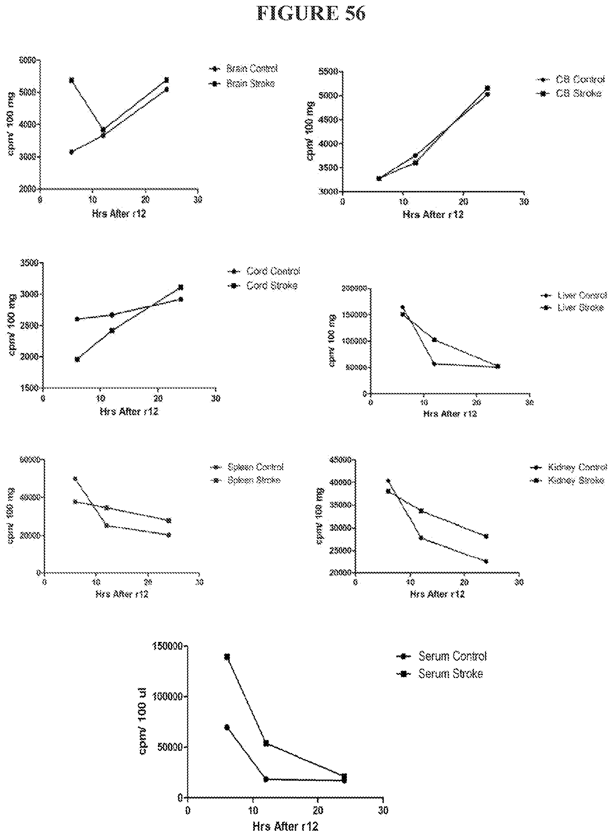

**Figure 56** provides graphs indicating amount of radiolabeled rIgM12 antibody - as indicated by radioactivity measured as cpm/100µl - in various tissues from control (sham manipulated) and stroke mice (Rose Bengal protocol) as denoted in each panel. At 30 to 40 min post stroke 10 million cpm of 35S labeled rHIgM12, about 60 ug of IgM, was administered intraperitoneally to each mouse in a volume of 500 ul saline. At 6, 12 and 24 hr post IgM treatment blood was collected from mice, the vasculature washed with 30 ml of saline and tissues acutely collected. For analysis of radiolabel in stroke tissue the right front cortex of the brain containing the lesion area was removed as a 75 mg tissue block (denoted Brain in panels). A portion of other tissues were removed, weighed and processed. CB = cerebellum, cord= spinal cord.

### DETAILED DESCRIPTION

In accordance with the present invention there may be employed conventional molecular biology, microbiology, and recombinant DNA techniques within the skill of the art. Such techniques are explained fully in the literature. See, e.g., Sambrook et al, "Molecular Cloning: A Laboratory Manual" (1989); "Current Protocols in Molecular Biology" Volumes I-III [Ausubel, R. M., ed. (1994)]; "Cell Biology: A Laboratory Handbook" Volumes I-III [J. E. Celis, ed. (1994))]; "Current Protocols in Immunology" Volumes I-III [Coligan, J. E., ed. (1994)]; "Oligonucleotide Synthesis" (M.J. Gait ed. 1984); "Nucleic Acid Hybridization" [B.D. Hames & S.J. Higgins eds. (1985)]; "Transcription And Translation" [B.D. Hames & S.J. Higgins, eds. (1984)]; "Animal Cell Culture" [R.I. Freshney, ed. (1986)]; "Immobilized Cells And Enzymes" [IRL Press, (1986)]; B. Perbal, "A Practical Guide To Molecular Cloning" (1984).

Therefore, if appearing herein, the following terms shall have the definitions set out below.

### A. TERMINOLOGY

The term "specific binding member" describes a member of a pair of molecules which have binding specificity for one another. The members of a specific binding pair may be naturally derived or wholly or partially synthetically produced. One member of the pair of molecules has an area on its surface, or a cavity, or a component on its surface, which specifically binds to and is therefore complementary to a particular spatial and polar organization of the other member of the pair of molecules. Thus the members of the pair have the property of binding specifically to each other. Examples of types of specific binding pairs are antigen-antibody, biotin-avidin, hormone-hormone receptor, receptor-ligand, enzyme-substrate. This application is concerned with antigen-antibody type reactions.

The term "antibody" describes an immunoglobulin whether natural or partly or wholly synthetically produced. The term also covers any polypeptide or protein having a binding domain which is, or is homologous to, an antibody binding domain. CDR grafted antibodies are also contemplated by this term. An "antibody" is any immunoglobulin, including antibodies and fragments thereof, that binds a specific epitope. The term encompasses polyclonal, monoclonal, and chimeric antibodies, the last mentioned described in further detail in U.S. Patent Nos. 4,816,397 and 4,816,567. The term "antibody(ies)" includes a wild type immunoglobulin (Ig) molecule, generally comprising four full length polypeptide chains, two heavy (H) chains and two light (L) chains, or an equivalent Ig homologue thereof (e.g., a camelid nanobody, which comprises only a heavy chain); including full length functional mutants, variants, or derivatives thereof, which retain the essential epitope binding features of an Ig molecule, and including dual specific, bispecific, multispecific, and dual variable domain antibodies; Immunoglobulin molecules can be of any class (e.g., IgG, IgE, IgM, IgD, IgA, and IgY), or subclass (e.g., IgG1, IgG2, IgG3, IgG4, IgA1, and IgA2). Also included within the meaning of the term "antibody" are any "antibody fragment".

An "antibody fragment" means a molecule comprising at least one polypeptide chain that is not full length, including (i) a Fab fragment, which is a monovalent fragment consisting of the variable light (VL), variable heavy (VH), constant light (CL) and constant heavy 1 (CH1) domains; (ii) a F(ab')2 fragment, which is a bivalent fragment comprising two Fab fragments linked by a disulfide bridge at the hinge region; (iii) a heavy chain portion of an Fab (Fd) fragment, which consists of the VH and CH1 domains; (iv) a variable fragment (Fv) fragment, which consists of the VL and VH domains of a single arm of an antibody, (v) a domain antibody (dAb) fragment, which comprises a single variable domain (Ward, E.S. et al., Nature 341, 544-546 (1989)); (vi) a camelid antibody; (vii) an isolated complementarity determining region (CDR); (viii) a Single Chain Fv Fragment wherein a VH domain and a VL domain are linked by a peptide linker which allows the two domains to associate to form an antigen binding site (Bird et al, Science, 242, 423-426, 1988; Huston et al, PNAS USA, 85, 5879-5883, 1988); (ix) a diabody, which is a bivalent, bispecific antibody in which VH and VL domains are expressed on a single polypeptide chain, but using a linker that is too short to allow for pairing between the two domains on the same chain, thereby forcing the domains to pair with the complementarity domains of another chain and creating two antigen binding sites (WO94/13804; P. Holliger et al Proc. Natl. Acad. Sci. USA 90 6444-6448, (1993)); and (x) a linear antibody, which comprises a pair of tandem Fv segments (VH-CH1-VH-CH1) which, together with complementarity light chain polypeptides, form a pair of antigen binding regions; (xi) multivalent antibody fragments (scFv dimers, trimers and/or tetramers (Power and Hudson, J Immunol. Methods 242: 193-204 9 (2000)); and (xii) other non-full length portions of heavy and/or light chains, or mutants, variants, or derivatives thereof, alone or in any combination.

As antibodies can be modified in a number of ways, the term "antibody" should be construed as covering any specific binding member or substance having a binding domain with the required specificity. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023 and U.S. Patent Nos. 4,816,397 and 4,816,567.

An "antibody combining site" is that structural portion of an antibody molecule comprised of light chain or heavy and light chain variable and hypervariable regions that specifically binds antigen.

The phrase "antibody molecule" in its various grammatical forms as used herein contemplates both an intact immunoglobulin molecule and an immunologically active portion of an immunoglobulin molecule.

Exemplary antibody molecules are intact immunoglobulin molecules, substantially intact immunoglobulin molecules and those portions of an immunoglobulin molecule that contains the paratope, including those portions known in the art as Fab, Fab', F(ab')₂ and F(v), which portions are preferred for use in the therapeutic methods described herein.

Antibodies may also be bispecific, wherein one binding domain of the antibody is a specific binding member of the invention, and the other binding domain has a different specificity, e.g. to recruit an effector function or the like. Bispecific antibodies of the present invention include wherein one binding domain of the antibody is a specific binding member of the present invention, including a fragment thereof, and the other binding domain is a distinct antibody or fragment thereof, including that of a distinct anti-cancer or anti-tumor specific antibody. The other binding domain may be an antibody that recognizes or targets a particular cell type, as in a neural or glial cell-specific antibody. In the bispecific antibodies of the present invention the one binding domain of the antibody of the invention may be combined with other binding domains or molecules which recognize particular cell receptors and/or modulate cells in a particular fashion, as for instance an immune modulator (e.g., interleukin(s)), a growth modulator or cytokine (*e.g*. tumor necrosis factor (TNF), and particularly, the TNF bispecific modality or a toxin (*e.g.*, ricin) or anti-mitotic or apoptotic agent or factor. Thus, the anti-FAP antibodies of the invention may be utilized to direct or target agents, labels, other molecules or compounds or antibodies to stromal sites, particular areas of wound healing, inflammation, cancer or tumors.

The phrase "monoclonal antibody" in its various grammatical forms refers to an antibody having only one species of antibody combining site capable of immunoreacting with a particular antigen. A monoclonal antibody thus typically displays a single binding affinity for any antigen with which it immunoreacts. A monoclonal antibody may also contain an antibody molecule having a plurality of antibody combining sites, each immunospecific for a different antigen; *e.g*., a bispecific (chimeric) monoclonal antibody.

The term "antigen binding domain" describes the part of an antibody which comprises the area which specifically binds to and is complementary to part or all of an antigen. Where an antigen is large, an antibody may bind to a particular part of the antigen only, which part is termed an epitope. An antigen binding domain may be provided by one or more antibody variable domains. Preferably, an antigen binding domain comprises an antibody light chain variable region (VL) and an antibody heavy chain variable region (VH).

Immunoconjugates or antibody fusion proteins of the present invention, wherein the antibodies, antibody molecules, or fragments thereof, of use in the present invention are conjugated or attached to other molecules or agents further include, but are not limited to such antibodies, molecules, or fragments conjugated to a chemical ablation agent, toxin, immunomodulator, cytokine, cytotoxic agent, chemotherapeutic agent, antimicrobial agent or peptide, cell wall and/or cell membrane disrupter, or drug.

The term "specific" may be used to refer to the situation in which one member of a specific binding pair will not show any significant binding to molecules other than its specific binding partner(s). The term is also applicable where e.g. an antigen binding domain is specific for a particular epitope which is carried by a number of antigens, in which case the specific binding member carrying the antigen binding domain will be able to bind to the various antigens carrying the epitope.

The term "comprise" generally used in the sense of include, that is to say permitting the presence of one or more features or components.

The term "consisting essentially of" refers to a product, particularly a peptide sequence, of a defined number of residues which is not covalently attached to a larger product. In the case of the peptide of the invention referred to above, those of skill in the art will appreciate that minor modifications to the N- or C- terminal of the peptide may however be contemplated, such as the chemical modification of the terminal to add a protecting group or the like, e.g. the amidation of the C-terminus.

The term "isolated" refers to the state in which specific binding members of the invention, or nucleic acid encoding such binding members will be, in accordance with the present invention. Members and nucleic acid will be free or substantially free of material with which they are naturally associated such as other polypeptides or nucleic acids with which they are found in their natural environment, or the environment in which they are prepared (e.g. cell culture) when such preparation is by recombinant DNA technology practiced *in vitro* or *in vivo.* Members and nucleic acid may be formulated with diluents or adjuvants and still for practical purposes be isolated - for example the members will normally be mixed with gelatin or other carriers if used to coat microtitre plates for use in immunoassays, or will be mixed with pharmaceutically acceptable carriers or diluents when used in diagnosis or therapy.

As used herein, "pg" means picogram, "ng" means nanogram, "ug" or "µg" mean microgram, "mg" means milligram, "ul" or "µl" mean microliter, "ml" means milliliter, "I" means liter.

The terms "antibody", "neuron binding antibody", "IgM12 antibody", "IgM42 antibody", "antibody 12", "antibody 42", "sHIgM12", "sHIgM42", "rHIgM12", "rHIgM42" and any variants not specifically listed, may be used herein, and as used throughout the present application and claims refer to proteinaceous material including single or multiple proteins, and extends to those proteins having the amino acid sequence data described herein and presented in Figures 5 and 6 and the profile of activities set forth herein and in the Claims. In particular antibody, antibody 12, rHIgM12 particularly refer to polypeptides or antibodies or fragments, particularly recombinant antibodies or fragments, comprising sequence presented in Figure 5. antibody, antibody 12, rHIgM12 particularly refer to polypeptides or antibodies or fragments, particularly recombinant antibodies or fragments, comprising heavy chain variable region CDR sequences set out in SEQ ID NOS: 31-33 and light chain variable region CDR sequences set out in SEQ ID NOS: 34-36. antibody, antibody 12, rHIgM12 includes antibody having the heavy chain variable region sequence of SEQ ID NO: 1 and the light chain variable region sequence of SEQ ID NO: 11. In particular IgM42 antibody, antibody 42, rHIgM42 particularly refer to polypeptides or antibodies or fragments, particularly recombinant antibodies or fragments, comprising sequence presented in Figure 6. IgM42 antibody, antibody 42, rHIgM42 particularly refer to polypeptides or antibodies or fragments, particularly recombinant antibodies or fragments, comprising heavy chain variable region CDR sequences set out in SEQ ID NOS: 37-39 and light chain variable region CDR sequences set out in SEQ ID NOS: 40-42. IgM42 antibody, antibody 42, rHIgM42 includes antibody having the heavy chain variable region sequence of SEQ ID NO: 17 and the light chain variable region sequence of SEQ ID NO: 27. Accordingly, proteins displaying substantially equivalent or altered activity are likewise contemplated. These modifications may be deliberate, for example, such as modifications obtained through site-directed mutagenesis, or may be accidental, such as those obtained through mutations in hosts that are producers of the complex or its named subunits. Also, the terms "antibody", "neuron binding antibody", "IgM12 antibody", "IgM42 antibody", "antibody 12", "antibody 42", "sHIgM12", "sHIgM42", "rHIgM12", "rHIgM42" are intended to include within their scope proteins specifically recited herein as well as all substantially homologous analogs and allelic variations.

The amino acid residues described herein are preferred to be in the "L" isomeric form. However, residues in the "D" isomeric form can be substituted for any L-amino acid residue, as long as the desired functional property of immunoglobulin-binding is retained by the polypeptide. NH₂ refers to the free amino group present at the amino terminus of a polypeptide. COOH refers to the free carboxy group present at the carboxy terminus of a polypeptide. In keeping with standard polypeptide nomenclature, J. Biol. Chem., 243:3552-59 (1969), abbreviations for amino acid residues are shown in the following

Table of Correspondence:

**TABLE OF CORRESPONDENCE**

| SYMBOL | SYMBOL | |
|---|---|---|
| 1-Letter | 3-Letter | AMINO ACID |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| W | Trp | tryptophan |
| R | Arg | arginine |
| D | Asp | aspartic acid |
| N | Asn | asparagine |
| C | Cys | cysteine |

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues. The above Table is presented to correlate the three-letter and one-letter notations which may appear alternately herein.

A "replicon" is any genetic element (*e.g*., plasmid, chromosome, virus) that functions as an autonomous unit of DNA replication *in vivo; i.e.,* capable of replication under its own control.

A "vector" is a replicon, such as plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia*, in linear DNA molecules (*e.g*., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (*i.e.*, the strand having a sequence homologous to the mRNA).

An "origin of replication" refers to those DNA sequences that participate in DNA synthesis.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence. This sequence encodes a signal peptide, N-terminal to the polypeptide, that communicates to the host cell to direct the polypeptide to the cell surface or to secrete the polypeptide into the media, and this signal peptide is clipped off by the host cell before the protein leaves the cell. Signal sequences can be found associated with a variety of proteins native to prokaryotes and eukaryotes.

The term "oligonucleotide," as used herein in referring to the probe of the present invention, is defined as a molecule comprised of two or more ribonucleotides, preferably more than three. Its exact size will depend upon many factors which, in turn, depend upon the ultimate function and use of the oligonucleotide.

The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and use of the method. For example, for diagnostic applications, depending on the complexity of the target sequence, the oligonucleotide primer typically contains 15-25 or more nucleotides, although it may contain fewer nucleotides.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

As used herein, the terms "restriction endonucleases" and "restriction enzymes" refer to bacterial enzymes, each of which cut double-stranded DNA at or near a specific nucleotide sequence.

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

Two DNA sequences are "substantially homologous" when at least about 75% (preferably at least about 80%, and most preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, *e.g.*, Maniatis et al., *supra*; DNA Cloning, Vols. I & II, *supra*; Nucleic Acid Hybridization, *supra.*

It should be appreciated that also within the scope of the present invention are DNA sequences encoding specific binding members (antibodies) of the invention which code for e.g. an antibody having the same amino acid sequence as provided in Figure 2 or 10, or comprising the CDR domain region sequences set out herein or in Figure 10 but which are degenerate thereto. By "degenerate to" is meant that a different three-letter codon is used to specify a particular amino acid. It is well known in the art that the following codons can be used interchangeably to code for each specific amino acid:

| | |
|---|---|
| Phenylalanine (Phe or F) | UUU or UUC |
| Leucine (Leu or L) | UUA or UUG or CUU or CUC or CUA or CUG |
| Isoleucine (Ile or I) | AUU or AUC or AUA |
| Methionine (Met or M) | AUG |
| Valine (Val or V) | GUU or GUC of GUA or GUG |
| Serine (Ser or S) | UCU or UCC or UCA or UCG or AGU or AGC |
| Proline (Pro or P) | CCU or CCC or CCA or CCG |
| Threonine (Thr or T) | ACU or ACC or ACA or ACG |
| Alanine (Ala or A) | GCU or GCG or GCA or GCG |
| Tyrosine (Tyr or Y) | UAU or UAC |
| Histidine (His or H) | CAU or CAC |
| Glutamine (Gln or Q) | CAA or CAG |
| Asparagine (Asn or N) | AAU or AAC |
| Lysine (Lys or K) | AAA or AAG |
| Aspartic Acid (Asp or D) | GAU or GAC |
| Glutamic Acid (Glu or E) | GAA or GAG |
| Cysteine (Cys or C) | UGU or UGC |
| Arginine (Arg or R) | CGU or CGC or CGA or CGG or AGA or AGG |
| Glycine (Gly or G) | GGU or GGC or GGA or GGG |
| Tryptophan (Trp or W) | UGG |
| Termination codon | UAA (ochre) or UAG (amber) or UGA (opal) |

It should be understood that the codons specified above are for RNA sequences. The corresponding codons for DNA have a T substituted for U.

Mutations can be made in the sequences encoding the amino acids, antibody fragments, CDR region sequences set out in Figures 5 or 6, or in the nucleic acid sequences set out in Figure 5 or 6, such that a particular codon is changed to a codon which codes for a different amino acid. Such a mutation is generally made by making the fewest nucleotide changes possible. A substitution mutation of this sort can be made to change an amino acid in the resulting protein in a non-conservative manner (for example, by changing the codon from an amino acid belonging to a grouping of amino acids having a particular size or characteristic to an amino acid belonging to another grouping) or in a conservative manner (for example, by changing the codon from an amino acid belonging to a grouping of amino acids having a particular size or characteristic to an amino acid belonging to the same grouping). Such a conservative change generally leads to less change in the structure and function of the resulting protein. A non-conservative change is more likely to alter the structure, activity or function of the resulting protein. The present invention should be considered to include sequences containing conservative changes which do not significantly alter the activity or binding characteristics of the resulting protein.

The following is one example of various groupings of amino acids:

### Amino acids with nonpolar R groups

Alanine, Valine, Leucine, Isoleucine, Proline, Phenylalanine, Tryptophan, Methionine

### Amino acids with uncharged polar R groups

Glycine, Serine, Threonine, Cysteine, Tyrosine, Asparagine, Glutamine

### Amino acids with charged polar R groups (negatively charged at Ph 6.0)

Aspartic acid, Glutamic acid

### Basic amino acids (positively charged at pH 6.0)

Lysine, Arginine, Histidine (at pH 6.0)

Another grouping may be those amino acids with phenyl groups:
Phenylalanine, Tryptophan, Tyrosine

Another grouping may be according to molecular weight (i.e., size of R groups):

| | | | |
|---|---|---|---|
| Glycine | 75 | Alanine | 89 |
| Serine | 105 | Proline | 115 |
| Valine | 117 | Threonine | 119 |
| Cysteine | 121 | Leucine | 131 |
| Isoleucine | 131 | Asparagine | 132 |
| Aspartic acid | 133 | Glutamine | 146 |
| Lysine | 146 | Glutamic acid | 147 |
| Methionine | 149 | Histidine (at pH 6.0) | 155 |
| Phenylalanine | 165 | Arginine | 174 |
| Tyrosine | 181 | Tryptophan | 204 |

Particularly preferred substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free NH₂ can be maintained.

Exemplary and preferred conservative amino acid substitutions include any of:
glutamine (Q) for glutamic acid (E) and vice versa; leucine (L) for valine (V) and vice versa; serine (S) for threonine (T) and vice versa; isoleucine (I) for valine (V) and vice versa; lysine (K) for glutamine (Q) and vice versa; isoleucine (I) for methionine (M) and vice versa; serine (S) for asparagine (N) and vice versa; leucine (L) for methionine (M) and vice versa; lysine (L) for glutamic acid (E) and vice versa; alanine (A) for serine (S) and vice versa; tyrosine (Y) for phenylalanine (F) and vice versa; glutamic acid (E) for aspartic acid (D) and vice versa; leucine (L) for isoleucine (I) and vice versa; lysine (K) for arginine (R) and vice versa.

Amino acid substitutions may also be introduced to substitute an amino acid with a particularly preferable property. For example, a Cys may be introduced a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces β-turns in the protein's structure.

Two amino acid sequences are "substantially homologous" when at least about 70% of the amino acid residues (preferably at least about 80%, and most preferably at least about 90 or 95%) are identical, or represent conservative substitutions. The CDR regions of two antibodies are substantially homologous when one or more amino acids are substituted with a similar or conservative amino acid substitution, and wherein the antibody/antibodies have the profile of binding and activities of one or more of or IgM42 disclosed herein.

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous coding sequence is a construct where the coding sequence itself is not found in nature (*e.g.*, a cDNA where the genomic coding sequence contains introns, or synthetic sequences having codons different than the native gene). Allelic variations or naturally-occurring mutational events do not give rise to a heterologous region of DNA as defined herein.

A DNA sequence is "operatively linked" to an expression control sequence when the expression control sequence controls and regulates the transcription and translation of that DNA sequence. The term "operatively linked" includes having an appropriate start signal (*e.g.*, ATG) in front of the DNA sequence to be expressed and maintaining the correct reading frame to permit expression of the DNA sequence under the control of the expression control sequence and production of the desired product encoded by the DNA sequence. If a gene that one desires to insert into a recombinant DNA molecule does not contain an appropriate start signal, such a start signal can be inserted in front of the gene.

The term "standard hybridization conditions" refers to salt and temperature conditions substantially equivalent to 5 x SSC and 65°C for both hybridization and wash. However, one skilled in the art will appreciate that such "standard hybridization conditions" are dependent on particular conditions including the concentration of sodium and magnesium in the buffer, nucleotide sequence length and concentration, percent mismatch, percent formamide, and the like. Also important in the determination of "standard hybridization conditions" is whether the two sequences hybridizing are RNA-RNA, DNA-DNA or RNA-DNA. Such standard hybridization conditions are easily determined by one skilled in the art according to well known formulae, wherein hybridization is typically 10-20°C below the predicted or determined Tₘ with washes of higher stringency, if desired.

The term 'agent' means any molecule, including polypeptides, antibodies, polynucleotides, chemical compounds and small molecules. In particular the term agent includes compounds such as test compounds or drug candidate compounds.

The term 'agonist' refers to a ligand that stimulates the receptor the ligand binds to in the broadest sense.

The term 'assay' means any process used to measure a specific property of a compound. A 'screening assay' means a process used to characterize or select compounds based upon their activity from a collection of compounds.

The term 'preventing' or 'prevention' refers to a reduction in risk of acquiring or developing a disease or disorder (i.e., causing at least one of the clinical symptoms of the disease not to develop) in a subject that may be exposed to a disease-causing agent, or predisposed to the disease in advance of disease onset.

The term 'prophylaxis' is related to and encompassed in the term 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Therapeutically effective amount' means that amount of a drug, compound, agent, antibody, or pharmaceutical agent that will elicit the biological or medical response of a subject that is being sought by a medical doctor or other clinician. In particular, with regard to neurological diseases or conditions, the term "effective amount" is intended to include an effective amount of a compound or agent, such as an antibody or fragment thereof, that will bring about a biologically meaningful change in a clinically relevant parameter of the disease. This may include a change in the amount of neurological injury or death observable or assessable upon imaging, a change in a functional parameter such as movement, speech or cognition, or the absence or reduction of such a change under conditions of risk or susceptibility. The phrase "therapeutically effective amount" is used herein to mean an amount sufficient to ameliorate or prevent, and preferably reduce by at least about 20 percent, at least about 30 percent, more preferably by at least 50 percent, more preferably by at least 70 percent, more preferably by at least 80 percent, most preferably by at least 90 percent, a clinically significant change in a measurable or assessable phenomenon, or feature of pathology, or assessment of function.

The term 'treating' or 'treatment' of any disease, condition, injury or infection refers, in one embodiment, to ameliorating the disease, condition, injury or infection (i.e., arresting the death of or injury to a cell or tissue, or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease, condition, injury or infection, either physically, (e.g., stabilization of a discernible symptom), physiologically, (e.g., stabilization of a physical parameter), or both. In a further embodiment, 'treating' or 'treatment' relates to slowing the progression of a disease or reducing the amount of injury, damage, death to cells or tissues.

The phrase "pharmaceutically acceptable" refers to molecular entities and compositions that are physiologically tolerable and do not typically produce an allergic or similar untoward reaction, such as gastric upset, dizziness and the like, when administered to a human.

As used herein, "pg" means picogram, "ng" means nanogram, "ug" or "µg" mean microgram, "mg" means milligram, "ul" or "µl" mean microliter, "ml" means milliliter, "I" means liter.

### B. DETAILED DISCLOSURE.

Neuroprotective agents are aimed at preventing and treating complications that result in CNS damage. Compounds or agents with capacity for neuroprotection have application in acute disorders such as stroke and injuries of the nervous system as well as in chronic diseases such as neurodegenerative disorders. Many of the underlying mechanisms of damage to neural tissues are similar in these conditions and a neuroprotective compound or agent could be used in more than one disorder. Diseases include cerebrovascular disorders, traumatic brain injury, spinal cord injury, Alzheimer's disease, Parkinson's disease, Huntington's disease, amyotrophic lateral sclerosis (ALS), multiple sclerosis, epilepsy, motor neuron disease(s) and ischemic neuropathy. Other applications include neuroprotection during anesthesia and surgery. Numerous products have been investigated for neuroprotective effects including those from the categories of free radical scavengers, anti-excitotoxic agents, apoptosis (programmed cell death) inhibitors, anti-inflammatory agents, neurotrophic factors, metal ion chelators, ion channel modulators and gene therapy. Several neuroprotective candidate products have reached clinical trials and failed to show significance, including NMDA antagonists and agents for reducing neurotransmitter release Clark WM et al (2000) Stroke 31(6):1234-9; Sarco RI et al (2001) JAMA 285 (13):1719-28; Albers, GW et al (2001) JAMA 286(21):2673-82). One challenge for neuroprotective agents is specificity and targeting so that an agent's effects are directed against injury or damaged/compromised neurons without significant activity against normal cells and thereby untoward, even potentially harmful side effects. The present invention now provides specific and tolerable agent(s) capable of crossing the blood brain barrier and targeting areas of injury, compromise or damage to effect protection, with minimal untoward effects on normal neurons.

The invention is defined by the claims. The disclosure generally provides specific binding members, particularly antibodies which demonstrate neuron binding, with application as neuroprotective agents for use in diagnosis, monitoring, amelioration and treatment of diseases and conditions where neurons or nerve cells are compromised, including neurodegenerative conditions, nerve cell death and injury. The antibodies have certain unique and remarkable qualities which make them particularly useful and applicable in neuroprotection and/or neuroregeneration and in diagnostics and therapeutics for neurological disease and conditions. The novel antibodies of the invention, particularly recombinant antibodies, are demonstrated herein to cross the blood brain barrier effectively and without any modification, to protect neurons from cell death, and to target neural lesions and sites of injury in the CNS. The antibodies improve nerve function and maintain axons in animal models of chronic axonal injury and demyelination. The antibodies have minimal toxicity in animals and do not exacerbate autoimmune conditions, as assessed in animal models.

The disclosure generally provides specific binding members, particularly antibodies which demonstrate neuron binding, with application as therapeutic agents, alone or in combination with other or alternative neuroactive agents, for the amelioration and treatment of diseases and conditions where neurons or nerve cells are compromised, including neurodegenerative conditions, nerve cell death and injury. The application provides evidence of the capability and therapeutically relevant activity of antibodies of the present invention, including IgM12, in animal models of neurological diseases or deficits. In particular, studies provided herein demonstrate capability and activity in a TMEV model, in a recognized animal model of motor neuron disease particularly ALS, and provides studies in spinal cord injury models. In particular, the present application provides evidence of the capability and therapeutically relevant activity of antibodies of the present invention, including IgM12, in animal models of stroke and cerebral thrombosis or cerebral ischemia. The studies provided herein demonstrate capability and activity in a in a recognized animal model of stroke and cerebral ischemia. Compositions for use in the amelioration or treatment of any of such diseases or in the prevention of nerve injury or protection of nerves in instances of compromise or risk of compromise are provided.

Prior studies with and IgM42 have shown that the serum-derived antibody binds to CNS neurons, supports neurite extension on antibody-coated substrate, and override the neurite extension inhibition of CNS myelin in in vitro studies (Warrington A et al (2004) J Neuropath Exp Neurol 63(5):461-473). Studies of sHIgM12 injected in TMEV animals showed increased spontaneous nocturnal activity, however, virus titers were not assessed in the animals and possible anti-viral effects could not be determined (Rodrigues M et al (2009) Neurology 72:1269-1276). The studies described herein provide fully human recombinant 12 and the sequence of the antibody 42 as well as recombinant rHIgM42. Studies demonstrating remarkable neuroprotective and therapeutically-relevant effects of these serum-derived and recombinant antibodies in animals and animal models of disease as well as their specific binding/targeting to sites of neural injury are now provided. In particular studies using animal models of cerebral ischemia and stroke provide demonstration of therapeutically-relevant effects of these serum-derived and recombinant antibodies, particularly recombinant IgM12, rHIgM12, in stroke, cerebral ischemia, and conditions of or resulting from loss of blood flow in or to the brain or cells in the CNS.

### Antibodies and Compositions

Auto reactive human mAbs fall under the classification of human natural autoantibodies (NatAbs) (Cohen I.R. (2007) J Autoimmun 29:246-249). NatAbs are part of our human immunoglobulin repertoire (Cohen, I.R., and M. Schwartz (1999) Journal of neuroimmunology 100:111-114), naturally made from our own genes, usually without somatic mutations and may function to stimulate cell processes or remove cell debris (Lutz, HU (2007) J Autoimmun 29:287-294). NatAbs react to self antigens, whereas conventional Abs react to exogenous antigens. NatAbs are of relatively low affinity, usually poly reactive, and are frequently IgMs. The mechanism of the action of these human IgMs appears to be similar, acting through membrane micro domains. NatAbs are by definition polyreactive, therefore identifying the relevant antigen for a specific in vivo function can be a challenge. Without being bound by theory, it is presently understood that these IgMs bind to membrane microdomains cross-linking molecules on the cell surface. Molecules not normally in context are brought together assembling a signaling complex (Rodriguez, M., A. E. Warrington, and L. R. Pease (2009) Neurology 72:1269-1276).

Accordingly, therapeutic molecules were identified by screening for auto reactive human monoclonal antibodies (mAbs) from the sera of individuals that carry the mAbs in high concentration without Ab-mediated disease. Such mAbs are tested for binding to the surface of nervous system cells without regard for antigen. The mAbs are then tested for the ability to modulate a disease model. This method of identifying mAbs with biologic efficacy is different than the methodology commonly used by the pharmaceutical industry (Rodriguez, M., A. E. Warrington, and L. R. Pease (2009) Neurology 72:1269-1276). It has been demonstrated that certain human IgMs can promote remyelination (Warrington AE et al (2000) Proc Natl Acad Sci U S A 97:6820-6825). For example, one such IgM is recombinant human monoclonal rHIgM22 that promotes remyelination (Mitsunaga YB et al (2002) Faseb J 16:1325-1327). Antibody rHIgM22 binds to oligodendrocytes and myelin and promotes CNS remyelination in virus and toxin induced models of MS (Warrington AE et al (2000) Proc Natl Acad Sci U S A 97:6820-6825; Bieber AJ et al (2002) Glia 37:241-249). Spinal cord remyelination is induced after a single low dose of rHIgM22 (Warrington AE et al (2007) J Neurosci Res 85:967-976).

It is remarkable that one intraperitoneal (i.p.) treatment with a short lived molecule (15 hr half life estimated in mice) promotes maximal tissue repair within 5 weeks in a model of MS with little inherent spontaneous repair. After peripheral injection, rHIgM22 crosses the blood brain barrier (BBB) and accumulates within brain and spinal cord lesions of mice with demyelination. Ferritin bead labeled rHIgM22 has been detected in lesions in vivo by MRI (Pirko I et al (2004) Faseb J 18:1577-1579). An additional serum human IgM antibody with remyelinating capability, sHIgM46, and its recombinant counterpart rHIgM46, has been also been described. The serum-derived antibodies sHIgM22 and sHIgM46, and recombinant versions thereof, and methods for remyelination are described, e.g., in WO 0185797. The rHIgM22 antibody and methods thereof is covered, e.g., by US Patent 7,473,423 and 7,807166.

The invention provides human antibodies, including monoclonal antibodies and particularly recombinant antibodies, which demonstrate activity in the promotion, stimulation, protection and/or regeneration of neurons in the central nervous system. The present antibodies, including fragments thereof, demonstrate activity in neuroprotection, neuronal and axon preservation and regeneration in the prevention or reduction of injury-mediated nerve cell death in the central nervous system. The antibodies are applicable in the treatment or amelioration of diseases or conditions, or in the prevention or reduction of nerve cell deficits and nerve cell death or apoptosis associated with diseases or conditions, particularly where nerves are damaged, injured or otherwise compromised. Conditions or diseases for use or application of the antibodies of the invention include scenarios where loss of structure, function or survival of neurons is involved or associated including brain injury or trauma, spinal cord injury (SCI), nerve injury, head injury, conditions where blood supply to the brain is reduced or compromised, infectious diseases of the brain, neurodegenerative diseases. Exemplary such diseases or conditions include spinal cord injury (SCI), Amyolotropic Lateral Sclerosis (ALS), multiple sclerosis (MS), Alzheimer's disease, stroke, Parkinson's disease, Huntington's disease, prenatal anoxia/perinatal ischemia and/or Cerebral Palsy, encephalopathy, myelopathy, and motor neuron diseases.

The neuromodulatory agents or antibodies of the invention have one or more of the following characteristics: they protect and/or stabilize neurons; they target sites in the CNS or nerve cell damage, compromise or injury; and/or block cell death, e.g. hydrogen-peroxide induced cell death. The invention provides neuron-binding monoclonal antibodies of the invention are capable of promoting neurite extension and protecting neurons from damage for diagnostic and therapeutic purposes in the central nervous system. In particular, recombinant antibodies are provided, wherein said antibodies recognize and are capable of binding neurons, including cortical neurons, hippocampal neurons, cerebellar granule cells and retinal ganglion cells.

The present invention provides exemplary antibody(ies) or fragment(s) thereof, antibody 12 and antibody 42, particularly serum derived or recombinant or IgM42. In a further particular aspect, the antibody of the invention comprises the amino acid sequence of antibody 12 or 42 including as set out in Figure 5 and/or Figure 6. Recombinant antibody of the present invention comprises the variable heavy chain (SEQ ID NO: 1) and light chain sequence (SEQ ID NO: 11) as set out in Figure 5. Recombinant antibody IgM42 of the present invention comprises the variable heavy chain (SEQ ID NO: 17) and light chain sequence (SEQ ID NO: 27) as set out in Figure 6. In an aspect of the invention a recombinant or synthetic neuron binding antibody is provided comprising the variable region CDR sequences set out in Figures 5 and 6. Antibody 12 comprises heavy chain CDR sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5. Antibody 42 comprises heavy chain CDR sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6.

Panels of recombinant antibodies or fragments thereof, including Fab fragments or phage display libraries, which are capable of recognizing neurons, particularly human neurons, can be screened for various properties; i.e., affinity, isotype, epitope, stability, etc. Of particular interest are antibodies that mimic the activity of exemplary antibodies and IgM42, and have the ability to bind neurons and to protect neurons from cell death or injury, such as for example peroxide mediated cell death. Such antibodies can be readily identified and/or screened in specific binding and activity assays. Recombinant antibodies comprising the antigen binding region or the heavy and/or light chain CDR regions of the present antibodies and/or IgM42, may be generated and screened for activity.

In general, the CDR regions, comprising amino acid sequences substantially as set out as the CDR regions of Figure 5 and 6 will be carried in a structure which allows for binding of the CDR regions to the surface or at the surface of neurons, and particularly to mammalian neurons, particularly human, monkey, baboon, rat, and/or mouse neurons. By "substantially as set out" it is meant that that variable region sequences, and/or particularly the CDR sequences, of the invention will be either identical or highly homologous to the specified regions of Figure 5 and 6. By "highly homologous" it is contemplated that only a few substitutions, preferably from 1 to 8, preferably from 1 to 5, preferably from 1 to 4, or from 1 to 3, or 1 or 2 substitutions may be made in the variable region sequence and/or in the CDR sequences. The term "substantially as set out" includes particularly conservative amino acid substitutions which do not materially or significantly affect the specificity and/or activity of the instant antibodies.

Substitutions may be made in the variable region sequence outside of the CDRs so as to retain the CDR sequences. Thus, changes in the variable region sequence or alternative non-homologous or veneered variable region sequences may be introduced or utilized, such that the CDR sequences are maintained and the remainder of the variable region sequence may be substituted. Alternatively, substitutions may be made particularly in the CDRs. CDR sequences for the antibodies of the present invention are set out and described herein including in Figure 5 and 6. Antibody 12 comprises heavy chain CDR sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5. Antibody 42 comprises heavy chain CDR sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6.

The structure for carrying the CDRs will generally be of an antibody heavy or light chain sequence or substantial portion thereof in which the CDR regions are located at locations corresponding to the CDR region of naturally occurring VH and VL antibody variable domains encoded by rearranged immunoglobulin genes. The structures and locations of immunoglobulin variable domains may be determined by reference to Kabat, E.A. et al, Sequences of Proteins of Immunological Interest. 4th Edition. US Department of Health and Human Services. 1987, and updates thereof, now available on the Internet (immuno.bme.nwu.edu). The variable domains may be derived from any germline or rearranged human variable domain, or may be a synthetic variable domain based on consensus sequences of known human variable domains. The CDR-derived sequences of the invention, as defined in the preceding paragraph, may be introduced into a repertoire of variable domains lacking CDR regions, using recombinant DNA technology.

For example, Marks et al (Bio/Technology, 1992, 10:779-783) describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR/CDRs. Marks et al further describe how this repertoire may be combined with a CDR of a particular antibody. The repertoire may then be displayed in a suitable host system such as the phage display system of WO92/01047 so that suitable specific binding members may be selected. A repertoire may consist of from anything from 10⁴ individual members upwards, for example from 10⁶ to 10⁸ or 10¹⁰ members. Analogous shuffling or combinatorial techniques are also disclosed by Stemmer (Nature, 1994, 370:389-391), who describes the technique in relation to a β-lactamase gene but observes that the approach may be used for the generation of antibodies.

A further alternative is to generate novel VH or VL regions carrying the CDR-derived sequences using random mutagenesis of, for example, the Ab VH or VL genes to generate mutations within the entire variable domain. Such a technique is described by Gram et al (1992, Proc. Natl. Acad. Sci., USA, 89:3576-3580), who used error-prone PCR. Another method which may be used is to direct mutagenesis to CDR regions of VH or VL genes. Such techniques are disclosed by Barbas et al, (1994, Proc. Natl. Acad. Sci., USA, 91:3809-3813) and Schier et al (1996, J. Mol. Biol. 263:551-567). All the above described techniques are known as such in the art. The skilled person will be able to use such techniques to provide specific binding members of the invention using routine methodology in the art.

A substantial portion of an immunoglobulin variable domain will comprise at least the three CDR regions, together with their intervening framework regions. Preferably, the portion will also include at least about 50% of either or both of the first and fourth framework regions, the 50% being the C-terminal 50% of the first framework region and the N-terminal 50% of the fourth framework region. Additional residues at the N-terminal or C-terminal end of the substantial part of the variable domain may be those not normally associated with naturally occurring variable domain regions. For example, construction of specific binding members of the present invention made by recombinant DNA techniques may result in the introduction of N- or C-terminal residues encoded by linkers introduced to facilitate cloning or other manipulation steps. Other manipulation steps include the introduction of linkers to join variable domains of the invention to further protein sequences including immunoglobulin heavy chains, other variable domains (for example in the production of diabodies) or protein labels as provided herein and/or known to those of skill in the art.

Although in a preferred aspect of the invention recombinant antibodies comprising a pair of binding domains based on sequences substantially set out in Figures 5 and/or 6 are preferred, single binding domains based on either of these sequences form further aspects of the invention. In the case of the binding domains based on the sequence substantially set out in Figure 5 and/or 6, such binding domains may be used as targeting agents for CNS neurons, particularly sites of nerve damage or injury, since it is known that immunoglobulin VH domains are capable of binding target antigens in a specific manner.

Specific binding members of the present invention may further comprise antibody constant regions or parts thereof. For example, recombinant antibodies based on the VH and VL sequences of Figures 5 and 6 may be attached at their C-terminal end to antibody light chain constant domains including human C_{K} or CA chains, preferably CA chains, including constant domains which are distinct or variant from the respective constant domains set out in Figure 5 or 6. Similarly, recombinant antibodies based on the sequences of Figures 5 or 6 may be attached at their C-terminal end to all or part of an immunoglobulin heavy chain derived from any antibody isotype, e.g. IgG, IgA, IgE, IgD and IgM and any of the isotype sub-classes, particularly IgG1, IgG2b, and IgG4, and then tested to affirm or determine comparable and/or suitable activity and capability. IgM is preferred.

The antibodies, or any fragments thereof, may be conjugated or recombinantly fused to any cellular toxin, bacterial or other, e.g. pseudomonas exotoxin, ricin, or diphtheria toxin. The part of the toxin used can be the whole toxin, or any particular domain of the toxin. Such antibody-toxin molecules have successfully been used for targeting and therapy of different kinds of cancers, see e.g. Pastan, Biochem Biophys Acta. 1997 Oct 24;1333(2):C1-6; Kreitman et al., N Engl J Med. 2001 Jul 26;345(4):241-7; Schnell et al., Leukemia. 2000 Jan;14(1):129-35; Ghetie et al., Mol Biotechnol. 2001 Jul;18(3):251-68. Bi- and tri-specific multimers can be formed by association of different scFv molecules and have been designed as cross-linking reagents for T-cell recruitment into tumors (immunotherapy), viral retargeting (gene therapy) and as red blood cell agglutination reagents (immunodiagnostics), see e.g. Todorovska et al., J Immunol Methods. 2001 Feb 1;248(1-2):47-66; Tomlinson et al., Methods Enzymol. 2000;326:461-79; McCall et al., J Immunol. 2001 May 15;166(10):6112-7.

Antibodies of the invention may be labeled with a detectable or functional label. Detectable labels include, but are not limited to, radiolabels such as the isotopes ³H, ¹⁴C, ³²P, ³⁵S, ³⁶Cl, ⁵¹Cr, ⁵⁷Co, ⁵⁸Co, ⁵⁹Fe, ⁹⁰Y, ¹²¹I, ¹²⁴I, ¹²⁵I, ¹³¹I, ¹¹¹In, ¹¹⁷Lu, ²¹¹At, ¹⁹⁸Au, ⁶⁷Cu, ²²⁵Ac, ²¹³Bi, ⁹⁹Tc and ¹⁸⁶Re, which may be attached to antibodies of the invention using conventional chemistry known in the art of antibody imaging. Labels also include fluorescent labels (for example fluorescein, rhodamine, Texas Red) and labels used conventionally in the art for MRI-CT imaging. They also include enzyme labels such as horseradish peroxidase, β-glucoronidase, β-galactosidase, urease. Labels further include chemical moieties such as biotin which may be detected via binding to a specific cognate detectable moiety, e.g. labeled avidin. Functional labels include substances which are designed to be targeted to the site of compromise, damage or injury to provide protection of or cause destruction of neural tissue. Such functional labels include cytotoxic drugs such as 5-fluorouracil or ricin and enzymes such as bacterial carboxypeptidase or nitroreductase, which are capable of converting prodrugs into active drugs at the site. Immunoconjugates or antibody fusion proteins of the present invention are contemplated, wherein the antibodies and fragments thereof are conjugated or attached to other molecules or agents further include, but are not limited to binding members conjugated to a chemical ablation agent, toxin, immunomodulator, cytokine, cytotoxic agent, chemotherapeutic agent or drug. When radioactive labels are used, known currently available counting procedures may be utilized to identify and quantitate the specific binding members. In the instance where the label is an enzyme, detection may be accomplished by any of the presently utilized colorimetric, spectrophotometric, fluorospectrophotometric, amperometric or gasometric techniques known in the art.

In vivo animal models of complications that result in CNS damage, including nerve cell injury or damage or of neurodegenerative conditions may be utilized by the skilled artisan to further or additionally screen, assess, and/or verify the antibodies or fragments thereof of the present invention, variants thereof, or of combinations thereof, or of combinations with other CNS reactive antibodies. Such animal models include, but are not limited to models of conditions or diseases associated with nerve cell damage, compromise, alteration, death, injury or degeneration. Models include those of or mimicking aspects of stroke, brain injury, ischemia, MS, Alzheimer's disease, Huntington's disease, Parkinson's disease, dementia, encephalitis, meningitis, ALS or motor neuron disease. Stroke models utilizing middle cerebral artery occlusion in primates, such as baboons, monkeys or macaques are known in the art and may be suitable (Young A et al (1997) Stroke 28:1471-1476; delZoppo GJ et al (1986) Stroke 17:1254-1265; Marshall and Ridley 91996) Neurodegeneration 5:275-286). MS models such as TMEV are known and described and utilized herein. SOD mouse models for ALS and motor neuron disease are known and described and utilized herein (Gurney ME et al (1994) Science 264:1772-1775).

### Stroke and Therapeutic Methods

The Examples and studies provided herein demonstrate efficacy and application of the antibodies provided, described and contemplated herein in models of stroke, ischemia and instances of hypoxia to the brain or neuronal cells. Thus, in a particular aspect, the present invention relates to use of the antibodies in intervention relating to stroke, cerebral ischemia, suspected or presumed stroke, transient ischemic attacks (TIAs) or minor strokes, or in individuals at high risk of stroke or a cerebral ischemic event or brain hypoxic event.

In the event of a stroke, or suspected stroke, fast treatment is important as the worst damage from a stroke usually occurs within the first few hours. Faster treatment results in less initial and permanent or long-term damage. Treatment depends on the nature or cause of stroke, whether restoring blood flow in ischemic stroke due to a blood clot (ischemic) or by controlling bleeding for a hemorrhagic stroke. CT scans and MRIs are utilized to assess or diagnose the type or extent of stroke. Current treatments for acute ischemic stroke include IV thrombolytic therapy, particularly with tissue-type plasminogen activator (tPA), and endovascular therapies, including intra-arterial thrombolytic therapy and the use of clot retrieval devices. However, tPA has a limited therapeutic window and is typically administered within 3 hr of stroke onset. Protecting damaged neurons following cerebral ischemia is a promising strategy to limit permanent disability. While therapies that modulate the inflammatory response can have neuro-protective properties, there are currently very few drugs that act directly on neurons to protect them.

A number of medications that can help prevent stroke in high-risk patients, particularly those who have had previous transient ischemic attacks (TIAs) or minor strokes, are available. These drugs fall into two main categories - anticoagulants and antiplatelet agents. Examples of anticoagulants include heparin, Coumadin, warfarin, ximelagatran and Exanta. These drugs work by thinning the blood to help prevent clotting, including in instances of deep vein thromboses and pulmonary emboli, and are very effective in helping to prevent stroke in patients with atrial fibrillation. Antiplatelet agents include aspirin, Persantine, dipyridamole, Plavix and clopidogrel. These agents prevent or reduce platelet aggregation and thereby reduce clots or clot formation and can reduce the risk of stroke in patients who have had TIAs or prior ischemic strokes. Despite the availability of thrombolytic therapy, stroke remains the third leading cause of death in the United States and the most common cause of adult disability. Scientists and physicians continue to seek ways to achieve better functional recovery in stroke patients. Neuroprotective agents have generated intense interest and have the potential to make the brain less susceptible to the damaging effects of stroke.

In assessing and evaluating the extent and effects of a stroke, clinicians and medical personnel review and consider various aspects of the patient's capabilities and functions. In addition to imaging techniques to identify and localize the area of a stroke or clot and ischemia, patients are evaluated using stroke assessment scales and functional assessment scales. Numerous scales are accepted or utilized and will be recognized and known to clinicians and those of skill in the art. Functional assessment scales include for example the Berg Balance Scale, Lawton Instrumental Activities of Daily Living (IADL) Scale, Rankin or modified Rankin Scale, and NIH Stroke Scale (NIHHS). These scales assess and determine several or a number of various parameters, including upper and lower extremity motor function, limb ataxia, sensory function, language, articulation, attention, gaze and visual function, arm, hand and leg power and movement. Patients can be evaluated on one or more scale just after the stroke or ischemic event, following the event, before and after treatment, for evaluation of possible or candidate treatments of agents, for comparison of treatments or agents and over time, including days, weeks, months, years.

The antibodies, fragments thereof and recombinant antibodies comprising the variable region sequences may be used in methods of treatment, prevention or diagnosis of the human or animal body, such as a method of neuroprotection in a mammal, particularly in instances of established, suspected or possible stroke or cerebral ischemia, which comprises administering to said mammal an effective amount of the antibodies, fragments thereof and recombinant antibodies of the invention. Recombinant antibodies or fragments thereof comprising the CDR domain region sequences according to the invention may be used in such methods. The agents, particularly recombinant antibodies or fragments thereof may be used in instances of established, suspected or possible stroke or cerebral ischemia, including as neuroprotective agents in methods for prevention, treatment or amelioration of nerve injury, damage or compromise and complications that can, may or do result in CNS damage. The methods of the invention are applicable where loss of structure, function or survival of neurons is involved or associated including brain injury or trauma, spinal cord injury (SCI), nerve injury, head injury, conditions where blood supply or oxygen to the brain is reduced or compromised. Exemplary such diseases or conditions for treatment, prevention or amelioration in accordance with the methods of the invention include stroke, TIAs, cerebral ischemia, prenatal anoxia/perinatal ischemia and/or Cerebral Palsy.

The agents, particularly recombinant antibodies or fragments thereof may be used as agents in methods for prevention, treatment or amelioration of stroke or cerebral ischemia. The present invention provides methods of treating or ameliorating stroke or cerebral ischemia comprising administering an antibody of the present invention, particularly recombinant antibody, particularly antibody IgM12 or recombinant IgM12, including an antibody or fragment thereof comprising heavy chain CDR sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ IDNO: 34), CDR2 AAS (SEQ ID NO:35) and CDR3 QQSYHTPW (SEQ ID NO:36), as set out in Figure 5.

The present disclosure provides methods of treating or ameliorating diseases or conditions in mammals where nerves are compromised, injured or damaged or scenarios where nerves or neurons are susceptible or at risk of compromise, injury or damage comprising administering a recombinant antibody or fully human antibody or fragment thereof selected from and IgM42. Methods may comprise administration of more than one antibody or fragment, including combinations of antibody and 42. In a further such method, one or more of antibody and/or of antibody IgM42 may be combined with another CNS active antibody, particularly including one or more of antibodies rHIgM22 and/or rHIgM46. Combinations of antibodies may be administered collectively or in series, and at various times and various amounts or concentrations. Thus, antibody 12 and/or 42 may be administered in combination with antibody 22 and/or 46, by combined administration or in series, separated by a short length of time or longer length of time, including by hours, days or weeks. Antibody 12 and/or 42 may particularly be administered in combination with antibody 22 and/or 46 (sHIgM22, rHIgM22, sHIgM46 or rHIgM46), by combined administration or in series for the treatment of amelioration of a disease or condition involving neurodegeneration, and particularly including demyelination. In one such method antibody12 and/or 42 is administered in combination with antibody 22 and/or 46, by combined administration or in series for the treatment of amelioration of a demyelinating disease or condition, particularly including multiple sclerosis (MS). The variable heavy and light chain sequences of antibody IgM22 are set out in SEQ ID NO: 43 and SEQ ID NO: 44 respectively. The variable heavy and light chain sequences of antibody IgM46 are set out in SEQ ID NO: 45 and SEQ ID NO: 46 respectively. One or more of antibody and/or of antibody IgM42 may be combined with one or more remyelinating antibody comprising (a) heavy chain variable region CDRs comprising CDR1 sequence SSGMH CDR2 sequence V(I)ISYDGSRKYYADSVKG and CDR3 sequence GVTGSPTLDY, and light chain variable region CDRs comprising CDR1 sequence SGSSSNIGNNFVS CDR2 sequence DITKRPS and CDR3 sequence G(E)TWDSSLSAV V; or (b) heavy chain variable region CDRs comprising CDR1 sequence SGFTFSSYW CDR2 sequence IKKDGSEK and CDR3 sequence ARPNCGGDCYLPWYFD, and light chain variable region CDRs comprising CDR1 sequence QSVLYSSNNKNY CDR2 sequence YWAS and CDR3 sequence QQYYNTPQA.

Methods of the invention may include administration of the antibodies of the invention alone or in combination with, in series with, or subsequent to administration of stroke or ischemic agents including one or more of thrombolytic agents, anti-platelet agents, blood pressure modulators, anti-hypertensives, antiinflammatories, agents that alter excitatory amino acids or their receptors, intracellular calcium modulators, channel modulators free-radical scavengers. In a particular aspect, the antibodies or active fragments thereof, particularly antibody and/or IgM42, are administered with thrombolytic agents and/or anti-platelet agents. In an aspect, the antibodies may be administered with or after a thrombolytic agent, such as TPA.

Administered with may include at about the same time, shortly after, minutes after, up to an hour after, hours after, a day after. Administration of the antibody(ies), either alone particularly as antibodies or IgM42, or in combination, or with other agents, may be within or after the 3 hour, 4 hour, 5 hour, 6 hour, up to 9 hour therapeutic window for TPA or thrombolytic agent administration following a documented or suspected stroke or cerebral ischemic event. Antibody(ies) may be administered in a single dose or multiple dose. By way of example and not limitation, antibody(ies) may be administered within 3 hours, after 3 hours, up to 4 hours, up to 5 hours, up to 6 hours, up to 8 hours, up to 12 hours, up to 24 hours, up to a day, up to 2 days, up to 3 days, up to several days, up to a week, or longer following a documented or suspected stroke or cerebral ischemic event. Antibody(ies) may, by way of example and not limitation, be initially administered within 3-6 hours following a documented or suspected stroke or cerebral ischemic event, followed by one or more additional administration of antibody(ies) up to one day, 2 days, 3 days, 4 days, 5 days, 6 days, a week, more than a week, 2 weeks, more than 2 weeks, or later following the initial or any subsequent administration. Dosing may be modulated based on the response or function of the patient. For example, the patient may be evaluated, for example using a stroke scale such as NIHSS before or after, before and after, or following initial administration of antibody, and antibody administration may be modified or continued or altered based on such a scale or other neurological or motor evaluation of the patient. In a particular aspect of the invention and the method, the antibody(ies) are effective in improving stroke scale measures or other neurological or functional scales, particularly when compared to no antibody administration, administration of TPA or other agent alone, or versus an alternative agent, including an alternative antibody or neuroprotective agent.

Thus, as demonstrated herein in animal models of stroke, animals administered the antibody(ies) of the invention demonstrate improved functional activity. The antibody(ies) of the present invention are effective in improving locomotor activity, including improvement in horizontal motor function, by administration thereof after a stroke or cerebral ischemia. Following ischemic injury or stroke, the disability associated with clinical deficits is primarily due to dysfunction of neurons. The lack of functional recovery is in part attributed to the restriction of regeneration and neuroplasticity (Walmsley, A.R., and Mir, A.K. (2007) Current Pharmaceutical Design 13:2470-2484). Stroke may result in a altered motor function such as an inability to move one or more limbs on one side of the body, cognitive effects such as an inability to understand or formulate speech, or other deficits including visual effects such as an inability to see one side of the visual field. In an aspect of the invention and its methods, the antibody(ies) of the invention are effective to prevent or reverse stroke-related neurologic deficits. Thus, the antibody(ies) of the invention can ameliorate one or more neurological symptom or deficit in instances of stroke or cerebral ischemia. The antibody(ies) of the invention are effective in mitigating the neurological effects or damage, particularly in the CNS, in the event or instance of a stroke. The antibody(ies) are of use in methods of reducing neurological damage or neuronal cell death, and effective to preserve or protect neurons in the event of a stroke or cerebral ischemia.

In an aspect of the invention, the antibodies, fragments thereof and recombinant antibodies comprising the variable region sequences according to the invention, may be utilized in methods or administered in compositions for improvement or stabilization of neurological function or of motor function in instances, particularly during, following, or upon a stroke or cerebral ischemic event, wherein nerves are compromised, injured or damaged or scenarios where nerves or neurons are susceptible or at risk of compromise, injury or damage. In a particular aspect, the antibodies or fragments thereof are utilized or administered in early or initial stages of a disease, trauma, or condition, such as a stroke or suspected stroke, or repeated over the course or duration of a disease, trauma, or condition, such as repeated following a stroke, so as to facilitate or maintain the improvement or stabilization of neurological function or motor function, including or selected from movement, such as walking, or cognitive function, such as recall or recognition.

Antibodies of the present invention may be administered to a patient in need of treatment via any suitable route, including by injection intraperitoneally, into the bloodstream or CSF, or directly into the site of injury or compromise. A particular advantage of the exemplary antibodies of the present invention is that they cross the BBB and can therefore target the CNS even on i.p. administration. The precise dose will depend upon a number of factors, including whether the antibody is for diagnosis or for treatment, the size or extent and location of the injury, the precise nature of the antibody (whether whole antibody, fragment, diabody, etc), and the nature of any detectable or functional label attached to the antibody. Where a radionuclide is used for therapy, a suitable maximum single dose may be about 45 mCi/m², to a maximum of about 250 mCi/m². Preferable dosage is in the range of 15 to 40 mCi, with a further preferred dosage range of 20 to 30 mCi, or 10 to 30 mCi. Such therapy may require bone marrow or stem cell replacement. Clinically-approved naked antibodies are generally administered in mg quantities, with adult doses of 20 to 2000 mg protein per dose, 20 to 1500 mg protein per dose, or 20 to 1000 mg protein per dose, or 20 to 500 mg protein per dose, or 20 to 100 mg protein per dose. Clinically-approved injectable monoclonal antibodies are administered in mg amounts, 3-5 mg/kg, 5-10mg/kg per dose, 300-400mg/dose, 300-500mg/dose (Newsome BW and Ernstoff MS (2008) Br J Clin Pharmacol 66(1):6-19; herceptin.net, tysabri.net, avastin.net, remicade.com). It is notable that the remyelinating antibody IgM22 has been shown to be effective in comparatively significantly lower doses, in the µg range, and is capable of crossing the BBB to be active in the CNS with even a single dose of antibody (WO 2004/110355; Warrington AE et al (2007) J Neurosci Res 85(5):967-976). Recombinant antibody is shown herein to have therapeutically relevant activity in animal models upon a single i.p. dose in the µg range. Thus dosing of the antibodies of the present invention, in either single dose, or multiple and/or periodic doses, in the range of µgs per dose or µg/kg doses, or in low mg per dose (100µg-1mg, less than 1 mg, 1mg-5mg, 1mg-10mg, 5mg-15mg, 10mg-20mg per dose), may be applicable and effective. A dose for a single treatment of an adult patient, may be proportionally adjusted for children and infants, and also adjusted for other antibody formats, in proportion for example to molecular weight. Treatments may be repeated at daily, twice-weekly, weekly or monthly intervals, at the discretion of the physician. One advantage of the exemplary antibodies is that they cross the BBB and target sites of damage or injury, thus facilitating the use of lower and potentially fewer doses to achieve suitable effects.

### Pharmaceutical and Therapeutic Compositions

Antibodies or fragments of the present invention will usually be administered in the form of a pharmaceutical composition, which may comprise at least one component in addition to the antibody(ies) or fragments of the invention. Thus pharmaceutical compositions according to the present invention, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be oral, or by injection, e.g. intravenous, or by deposition at a tumor site.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. For intravenous, injection, or injection at the site of affliction, the active ingredient may be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required.

A composition may be administered alone or in combination with other treatments, therapeutics or agents, either simultaneously or sequentially dependent upon the condition to be treated. Compositions comprising combinations of one or more recombinant antibody or fragment thereof as described herein are contemplated. In addition, the present invention contemplates and includes compositions comprising the antibody or fragment thereof, herein described and other agents or therapeutics such as neuroactive agents or therapeutics, anti-inflammatory agents, neurotransmitter release modulating agents, neuroreceptor ligands or agonists or antagonists, calcium channel agents, immune modulators, or other CNS reactive antibodies. Compositions comprising combinations of one or more recombinant antibody or fragment thereof as described herein are contemplated. Other treatments or therapeutics may include the administration of suitable doses of pain relief drugs such as non-steroidal anti-inflammatory drugs (*e.g*. aspirin, paracetamol, ibuprofen or ketoprofen) or opiates such as morphine, or anti-emetics. In addition, the composition may be administered with immune modulators, such as interleukins, tumor necrosis factor (TNF) or other growth factors, colony stimulating factors, cytokines or hormones such as dexamethasone which stimulate the immune response and reduction or elimination of cancer cells or tumors. The composition may also be administered with, or may include combinations along with other CNS reactive antibodies, particularly including remyelinating antibodies, including rHIgM22 and/or rHIgM46.

The present disclosure further contemplates therapeutic compositions useful in practicing the therapeutic methods of this invention. A subject therapeutic composition includes, in admixture, a pharmaceutically acceptable excipient (carrier) and one or more of an antibody, polypeptide analog thereof or fragment thereof, as described herein as an active ingredient. In a preferred embodiment, the composition comprises an antigen capable of modulating the specific binding of the present binding member/antibody with a target cell. The preparation of therapeutic or pharmaceutical compositions which contain antibodies, polypeptides, or active fragments as active ingredients is well understood in the art. Typically, such compositions are prepared as injectables, either as liquid solutions or suspensions. However, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH buffering agents which enhance the effectiveness of the active ingredient. An antibody or active fragment can be formulated into the therapeutic composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The therapeutic antibody- or active fragment-containing compositions are conventionally administered intraperitonneally or intravenously, as by injection of a unit dose, for example. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; i.e., carrier, or vehicle.

The compositions are administered in a manner compatible with the dosage formulation, and in a therapeutically effective amount. The quantity to be administered depends on the subject to be treated, capacity of the subject's system to utilize the active ingredient, and degree of neuron binding capacity desired or extent of neural injury. Precise amounts of active ingredient required to be administered depend on the judgment of the practitioner and are peculiar to each individual. Suitable regimes for initial administration and follow on administration are also variable, and may include an initial administration followed by repeated doses at one or more hour, day, week or month intervals by a subsequent injection or other administration. Alternatively, continuous infusion or administration sufficient to maintain appropriate and sufficient concentrations in the blood, CNS or at the site of desired therapy are contemplated.

The timing of administration may vary and may be determined by the skilled artisan or medical practitioner, based on the teaching of the specification, the clinical parameters of the patient or subject, the status or severity of the condition or disease, or the degree or nature of neural injury, involvement or compromise. Thus, improvement in neural function or enhanced protection of neurons, e.g. from death or compromise, may be enhanced by administration early in the onset or clinical demonstration of a disease, so as to minimize the extent of neurological damage or compromise. In an aspect, timing of administration is coordinated with neurological function assessments, status determination and/or other clinical evaluations so as to minimize or alleviate progression of disease of neurological impairment or damage.

### Diagnostic Assays

The present disclosure also relates to a variety of diagnostic applications, including methods for detecting or determining nerve cell damage, injury or compromise. The antibodies and fragments of the invention may be utilized to assess, quantitate, target and/or image neurons, *in vitro* or *in vivo.* The present antibodies including fragments thereof, and drugs that modulate the production or activity of the specific binding members, antibodies and/or their subunits may possess certain diagnostic applications and may for example, be utilized for the purpose of detecting and/or measuring conditions or diseases involving neuron compromise, degeneration, injury, damage or death. The present antibodies including fragments thereof possess certain diagnostic applications and may for example, be utilized for the purpose of detecting and/or measuring conditions or diseases involving cerebral ischemia including stroke or brain injury including traumatic brain injury. The labeled, including radiolabelled, antibodies and fragments thereof, are useful in *in vitro* diagnostics techniques and in *in vivo* radioimaging techniques and in radioimmunotherapy. In the instance of *in vivo* imaging, the antibodies or fragments of the present invention may be conjugated to an imaging agent rather than a radioisotope(s), including but not limited to a magnetic resonance image enhancing agent, wherein for instance an antibody molecule is loaded with a large number of paramagnetic ions through chelating groups. Examples of chelating groups include EDTA, porphyrins, polyamines crown ethers and polyoximes. Examples of paramagnetic ions include gadolinium, iron, manganese, rhenium, europium, lanthanium, holmium and ferbium. In a further aspect of the invention, radiolabelled antibodies and fragments thereof, particularly radioimmunoconjugates, are useful in radioimmunotherapy, particularly as radiolabelled antibodies for cellular therapy. In a still further aspect, the radiolabelled specific binding members, particularly antibodies and fragments thereof, are useful in radioimmuno-guided surgery techniques, wherein they can identify and indicate the presence and/or location of compromised or damaged neurons or the sites of nerve injury, during or following surgery to target or remove such cells or to transplant or administer cells to those specific sites.

Radioimmunotherapy (RAIT) has entered the clinic and demonstrated efficacy using various antibody immunoconjugates. ¹³¹I labeled humanized anti-carcinoembryonic antigen (anti-CEA) antibody hMN-14 has been evaluated in colorectal cancer (Behr TM et al (2002) Cancer 94(4Suppl): 1373-81) and the same antibody with ⁹⁰Y label has been assessed in medullary thyroid carcinoma (Stein R et al (2002) Cancer 94(1):51-61). Radioimmunotherapy using monoclonal antibodies has also been assessed and reported for non-Hodgkin's lymphoma and pancreatic cancer (Goldenberg DM (2001) Crit Rev Oncol Hematol 39(1-2):195-201; Gold DV et al (2001) Crit Rev Oncol Hematol 39 (1-2) 147-54). Radioimmunotherapy methods with particular antibodies are also described in U.S. Patent 6,306,393 and 6,331,175. Radioimmunoguided surgery (RIGS) has also entered the clinic and demonstrated efficacy and usefulness, including using anti-CEA antibodies and antibodies directed against tumor-associated antigens (Kim JC et al (2002) Int J Cancer 97(4):542-7; Schneebaum S et al (2001) World J Surg 25(12):1495-8; Avital S et al (2000) Cancer 89(8):1692-8; Mcintosh DG et al (1997) Cancer Biother Radiopharm 12 (4):287-94).

The radiolabelled antibodies and fragments thereof, are useful in *in vitro* diagnostics techniques and in *in vivo* radioimaging techniques. The antibodies and fragments thereof, particularly radioimmunoconjugates, are useful in radioimmunotherapy, particularly as radiolabelled antibodies for nerve injury repair, neurodegenerative recovery, cancer or CNS tumor therapy, or alternatively for ablation of damaged nerve tissue or neurons in certain instances. In an *in vivo* aspect, the antibody or neuron binding fragment thereof is labeled and administered to the animal prior to, during or after surgery or a surgical technique, including a stereotactic or minimally invasive technique, for the purpose of locating nerve injury or for assessing remaining damaged or injured neural tissue. In one such aspect, the radiolabelled specific binding members, particularly antibodies and fragments thereof, are useful in radioimmuno-guided surgery techniques, wherein they can identify and indicate the presence and/or location of compromised, damaged, injured or dying nerve cells or neurons or nervous tissue, prior to, during or following surgery to target, identify or remove such cells.

Diagnostic applications of the antibodies and fragments thereof of the disclosure include *in vitro* and *in vivo* applications well known and standard to the skilled artisan and based on the present description. Diagnostic assays and kits for *in vitro* assessment and evaluation of neurons or nervous tissue, may be utilized to diagnose, evaluate and monitor patient samples including those known to have or suspected of having neurological conditions or diseases where nerve cells are compromised, damaged or injured or determining the extent of cell death or injury or of a CNS tumor or cancer, including in a sample from a patient or subject. The assessment and evaluation of neurological disease status is also useful in determining the suitability of a patient for a clinical trial of a drug or for the administration of a particular neurotherapeutic or chemotherapeutic agent or an antibody of the present invention, including combinations thereof, versus a different agent or antibody.

The present invention includes an assay system which may be prepared in the form of a test kit for the quantitative analysis of the extent of the presence of, for instance, damaged, compromised or injured neurons or to quantitate neurons in a sample. The system or test kit may comprise a labeled component prepared by one of the radioactive and/or enzymatic techniques discussed herein, coupling a label to the antibody, and one or more additional immunochemical reagents, at least one of which is a free or immobilized components to be determined or their binding partner(s).

In a further embodiment of this invention, commercial test kits suitable for use by a medical specialist may be prepared based on the above to determine the status of neurons in a sample. In accordance with the testing techniques discussed above, one class of such kits will contain at least the labeled antibody or its binding partner, for instance an antibody specific thereto, and directions, of course, depending upon the method selected, e.g., "competitive," "sandwich," "DASP" and the like. The kits may also contain peripheral reagents such as buffers, stabilizers, etc.

Accordingly, a test kit may be prepared for the demonstration of the presence or determination of the status of neurons, comprising:
(a) a predetermined amount of at least one labeled immunochemically reactive component obtained by the direct or indirect attachment of the present antibody or fragment or a specific binding partner thereto, to a detectable label;
(b) other reagents; and
(c) directions for use of said kit.

A test kit may be prepared for the demonstration of the presence of nerve cell injury, damage or compromise comprising:
(a) a predetermined amount of at least one labeled immunochemically reactive component obtained by the direct or indirect attachment of the present antibody or a specific binding partner thereto, to a detectable label;
(b) other reagents; and
(c) directions for use of said kit.

### Nucleic Acids

The present disclosure further provides an isolated nucleic acid encoding an antibody, particularly a recombinant antibody, particularly a fully human antibody, of the present invention. Nucleic acid includes DNA and RNA. In a preferred aspect, the present disclosure provides a nucleic acid which codes for a polypeptide of the invention as defined above, including a polypeptide as set out in Figures 5 or 6 or capable of encoding the CDR regions thereof.

Antibodies and encoding nucleic acid molecules and vectors according to the present invention may be provided isolated and/or purified, e.g. from their natural environment, in substantially pure or homogeneous form, or, in the case of nucleic acid, free or substantially free of nucleic acid or genes origin other than the sequence encoding a polypeptide with the required function. Nucleic acid according to the present invention may comprise DNA or RNA and may be wholly or partially synthetic.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells, cancer cells, ovarian cancer cells and many others. A common, preferred bacterial host is *E.coli.* The expression of antibodies and antibody fragments in prokaryotic cells such as *E.coli* is well established in the art. Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Short Protocols in Molecular Biology, Second Edition, Ausubel et al. eds., John Wiley & Sons, 1992.

Thus, a further aspect provides a host cell containing nucleic acid as disclosed herein. A still further aspect provides a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. The introduction may be followed by causing or allowing expression from the nucleic acid, e.g. by culturing host cells under conditions for expression of the gene. The present disclosure also provides a method which comprises using a construct as stated above in an expression system in order to express a specific binding member or polypeptide as above.

Another feature of this disclosure is the expression of the DNA sequences disclosed herein. As is well known in the art, DNA sequences may be expressed by operatively linking them to an expression control sequence in an appropriate expression vector and employing that expression vector to transform an appropriate unicellular host. A wide variety of host/expression vector combinations may be employed in expressing the DNA sequences. Useful expression vectors, for example, may consist of segments of chromosomal, non-chromosomal and synthetic DNA sequences. Suitable vectors include derivatives of SV40 and known bacterial plasmids, e.g., *E. coli* plasmids col EI, pCR1, pBR322, pMB9 and their derivatives, plasmids such as RP4; phage DNAs, e.g., the numerous derivatives of phage λ, *e.g.*, NM989, and other phage DNA, *e.g.*, M13 and filamentous single stranded phage DNA; yeast plasmids such as the 2u plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Any of a wide variety of expression control sequences -- sequences that control the expression of a DNA sequence operatively linked to it -- may be used in these vectors to express the DNA sequences of this invention. Such useful expression control sequences include, for example, the early or late promoters of SV40, CMV, vaccinia, polyoma or adenovirus, the *lac* system, the *trp* system, the *TAC* system, the *TRC* system, the *LTR* system, the major operator and promoter regions of phage λ, the control regions of fd coat protein, the promoter for 3-phosphoglycerate kinase or other glycolytic enzymes, the promoters of acid phosphatase (*e.g*., Pho5), the promoters of the yeast 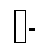 -mating factors, and other sequences known to control the expression of genes of prokaryotic or eukaryotic cells or their viruses, and various combinations thereof.

A wide variety of unicellular host cells are also useful in expressing the DNA sequences of this invention. These hosts may include well known eukaryotic and prokaryotic hosts, such as strains of *E. coli, Pseudomonas, Bacillus, Streptomyces,* fungi such as yeasts, and animal cells, such as CHO, YB/20, NSO, SP2/0, RI.I, B-W and L-M cells, African Green Monkey kidney cells (*e.g*., COS 1, COS 7, BSC1, BSC40, and BMT10), insect cells (*e.g*., Sf9), and human cells and plant cells in tissue culture.

It will be understood that not all vectors, expression control sequences and hosts will function equally well to express the DNA sequences of this invention. Neither will all hosts function equally well with the same expression system. However, one skilled in the art will be able to select the proper vectors, expression control sequences, and hosts without undue experimentation to accomplish the desired expression without departing from the scope of this invention. In selecting an expression control sequence, a variety of factors will normally be considered. These include, for example, the relative strength of the system, its controllability, and its compatibility with the particular DNA sequence or gene to be expressed, particularly as regards potential secondary structures. Suitable unicellular hosts will be selected by consideration of, *e.g*., their compatibility with the chosen vector, their secretion characteristics, their ability to fold proteins correctly, and their fermentation requirements, as well as the toxicity to the host of the product encoded by the DNA sequences to be expressed, and the ease of purification of the expression products. Considering these and other factors a person skilled in the art will be able to construct a variety of vector/expression control sequence/host combinations that will express the DNA sequences of this invention on fermentation or in large scale animal culture.

A DNA sequence encoding an antibody or fragment thereof can be prepared synthetically rather than cloned. The DNA sequence can be designed with the appropriate codons for the specific binding member amino acid sequence. In general, one will select preferred codons for the intended host if the sequence will be used for expression. The complete sequence is assembled from overlapping oligonucleotides prepared by standard methods and assembled into a complete coding sequence. See, e.g., Edge, Nature, 292:756 (1981); Nambair et al., Science, 223:1299 (1984); Jay et al., J. Biol. Chem., 259:6311 (1984). Synthetic DNA sequences allow convenient construction of genes which will express specific binding member analogs or "muteins". Alternatively, DNA encoding muteins can be made by site-directed mutagenesis of native specific binding member genes or cDNAs, and muteins can be made directly using conventional polypeptide synthesis.

### EXAMPLES

The invention may be better understood by reference to the following non-limiting Examples, which are provided as exemplary of the invention. The following examples are presented in order to more fully illustrate the preferred embodiments of the invention and should in no way be construed, however, as limiting the broad scope of the invention.

### EXAMPLE 1: Identification of Natural Autoantibodies (NatAbs) that Bind to Self Nervous System Cells

Natural serum human IgMs that bind to neurons were identified by screening the Mayo Clinic serum bank, which contains over 140,000 samples collected over 45 years, for candidates with a high monoclonal spike of IgG or IgM (greater than 10 mg/ml in the blood) and then testing serum for antibody binding to slices of live brain cortex and cerebellum (31). Such IgMs were then purified from positive samples and further tested for 1) binding to the surface of isolated primary neurons 2) as substrates for the support of neurite extension 3) the ability to protect neurons from apoptosis in the face of stressor molecules. This screening protocol is based on that used to identify human IgMs that bind to oligodendrocytes and promote remyelination in models of MS (22, and as described in WO 0185797). The recognition of the surface of appropriate tissues or cells appears to be an important defining characteristic of therapeutic IgMs.

Two novel, distinct serum-derived human neuron binding IgMs have been identified (sHIgM12 and sHIgM42). Certain characteristics of these serum-derived antibodies are listed in TABLE 1. Serum derived sHIgM12 and sHIgM42 have been shown initially in vitro to support neurite extension as well as the potent substrate laminin and to override the neurite outgrowth inhibition of CNS myelin (23).

Further studies have shown that serum-derived sHIgM12 improves spontaneous function in TMEV infected mice, as assessed by mean hourly spontaneous nocturnal activity in mice (21). In contrast to previously identified IgM NatAbs (sHIgM22 and sHIgM46), neither sHIgM12 nor sHIgM42 promote spinal cord remyelination.

**Table 1 - Therapeutic Human IgMs**

| **Human IgM** | **Promotes Remyelination** | **Supports Neurite Extension** | **Binds to Oligodendrocyt es** | **Binds to Neurons** |
|---|---|---|---|---|
| sHIgM12 | no | yes | No | yes |
| sHIgM42 | no | yes | No | yes |
| sHIgM22 | yes | no | yes | no |
| sHIgM46 | yes | no | yes | no |
| sHIgM39 | no | no | No | No |

Often, autoantibodies are thought of as pathogenic. In contrast, as demonstrated herein, the autoantibodies to neurons (sHIgM12 and sHIgM42 and recombinant antibodies based thereon) do not kill neurons. Instead these IgMs protect neurons from death, promote neurite extension, increase NAA in vivo, protect axons in vivo in the TMEV model, and improve nocturnal spontaneous function of TMEV diseased mice.

The neuron binding antibodies and IgM42 target neurons in CNS lesions and applicable to reverse neuronal loss and/or ameliorate the effects of neuronal injury or disease.. Neuron-binding human IgMs represent a new class of therapeutics with unique application for various diseases and conditions involving neurodegeneration, neuron injury or neuron death. Such diseases include without limitation, MS, spinal cord injury, ALS, Alzheimer's Disease, traumatic brain injury, cerebro-vascular events or stroke. These human IgMs have been minimally antigenic when administered systemically in animals.

### Identification and Characterization of Human IgMs That Bind to the Surface of Multiple Types of Living Neurons:

Serum samples with a high concentrations of IgG or IgM (>10 mg/ml) were screened for Ab binding to neuronal layers in live CNS tissue slices (cortex and cerebellum). Of 152 sera tested, 17 human sera were positive on tissue slices (23).

Antibodies sHIgM12 and sHIgM42 bind to the surface of a wide variety of neurons that co-label with neuronal markers neurofilament or β III tubulin. These include cerebellar granule cells (23), cortical neurons, hippocampal neurons, neurons from human temporal lobe biopsy (FIGURE 1), and retinal ganglion cells (data not shown). rHIgM12 stains the soma, neurites and growth cones of hippocampal neurons (data not shown). This cross reactivity suggests the human IgMs may act on CNS cells affected in a number of neurologic conditions/diseases, such as MS, amyotrophic lateral sclerosis, or stroke.

The present data indicate that the binding of sHIgM12 or sHIgM42 to the surface of neurons is carbohydrate-dependent. Sialidase treatment of neurons in culture eliminated the binding of both human IgMs to the cell surface, whereas blocking sphingolipid synthesis with Fumonisin B1 or removing GPI-linked proteins with PIPLC did not eliminate IgM binding (23). Gangliosides are candidates for antigens of these human IgMs. In co-labeling experiments, rHIgM12 co-localizes with GM1 on the neuronal membrane (see Example 11 and FIGURE 21C).

Although they share certain functional characteristics such as eliciting neurite outgrowth, sHIgM12 and sHIgM42 do demonstrate differences and are each unique antibodies. This is evident in binding and immunofluorescence studies; they label the surface of cerebellar granule cells in distinct patterns. In immunofluorescence studies of rat cerebellar granule cells in culture, the patterns of neuronal membrane labeling by use of these two antibodies are distinct (FIGURE 2). Short areas of neurite membrane are bound by sHIgM12 resulting in a punctate pattern. Larger areas of neurite membrane are bound by sHIgM42 resulting in a more segmented pattern.

### Example 2: Human IgMs Protect Cortical Neurons from Peroxide Induced Death

Remyelination-promoting human IgMs have been shown to protect oligodendrocytes in culture from peroxide-induced activation of caspase 3 (33), a marker of active apoptosis. As set forth herein, sHIgM12 or sHIgM42, were evaluated in analogous protocols. The sHIgM12 or sHIgM42, respectively, and peroxide were added together to cultures of primary mouse cortical neurons and the degree of caspase 3 activation assayed 24 hr later (FIGURE 3).

Treatment of cultured neurons with rHIgM12 resulted in protection of 80% of caspase 3 activation. Treatment of neurons with sHIgM42 was protective also with approximately of 40% of caspase 3 activation. These results were significantly different (P<0.01) compared to a control human IgM, which resulted in less than 10% protection from caspase 3 activation.

Thus, neuron-binding sHIgM12 or sHIgM42 protected cortical neurons from cell death induced by peroxide. Thus, when an inducer or agent of nerve cell damage (or death) was provided, IgM12 and IgM42 independently and significantly prevented the damage (or death) from occurring.

### Example 3: Recombinant Antibodies Derived from sHIgM12

Two recombinant forms of sHIgM12 have been constructed. Each used the same expression vector as was previously utilized for recombinant IgM22 antibody (rHIgM22) for the heavy and light chains (22, 28, WO0185797). The vector includes a selectable dHfR gene expressed under the control of the SV40 promoter. A partially human recombinant IgM12 antibody form with a mouse J chain was initially constructed as follows, thereafter antibody produced in human/mouse hybridoma line F3B6 cells.

The recombinant IgM12 antibody (PAD12) vector construction was performed by inserting the heavy chain variable region cDNA with a leader sequence from the nucleotide database and complete light chain cDNA with attached leader sequence from the nucleotide database in a manner similar to that described previously (6), using the primers described below. The vector is depicted in FIGURE 4 The sequence of the heavy and light chain utilized for recombinant human IgM12 antibody, with variable and constant regions noted, is shown in FIGURE 5.

Primers used to make rHIgM12VH and splice it by overlap extension (Horton RM et al (1989) Gene 77:61-68) to a leader sequence with an intron (lower case letters) from data base (M29812) are as follows:
(1) 5' primer with BspEI site: TCC GGA CGG TCC GGG A
(2) TCC GGA CGG TCC G ggacctcct gtgcaagaac atgaaacatc tgtggttctt ccttctcctg gtggcagctc ccagatgtga gtatctcagg gatccagaca
(3) cagg gatccagaca tggggatatg ggaggtgcct ctgatcccag ggctcactgt gggtctctct gttcacaggg gtcctgtccc aggtgcagct gcaggagtcg ggcccaggac
(4) 3' primer with PAC I site: CCTTAATTAAGACCTGG AGAGGCCATTCTTACCTGAG GAGACGGTGACCAGGGTTC

Primers used to make rHIgM12Vk and splice it by overlap extension (soe) to the leader sequence from the data base (lower case letters, accession X59312) are as follows:
(1) 5'-Ck primer to soe lym 12 Vk to Ck: CGA ACT GTGGCT GCA C
(2) 3' Ck primer with Xho 1: CCGCTCGAGTATCTAACACTCTCCCCTGTT
(3) 5' Lym 12 Vk with Nhe I: AGCATTACTAGCTAGCTC AAGACTCAGCCTGGAC atggaca tgagggtccc cgctcagctc ctggggctcc tgctactctggctccgag gtgccaga tgt GAC ATC CAG ATG ACC CA

The vector with the synthetic antibody genes was introduced into F3B6 hybridoma cells by electroporation and methotrexate (MTX) amplification was performed as described previously (6).

Briefly, 8 million F3B6 mouse/human heterohybridoma cells (American Type Culture Collection: ATCC) were incubated with 10µg PAD12 vector linearized with Bgl II for 10 min in 800µl serum free media at which time they were electroporated at 0·2 V in a Biorad Gene Pulser™ (Biorad, Hercules, CA, USA). Following a 10-min incubation on ice, the cells were diluted to 24 ml in RPMI-1640 containing 10% fetal calf serum (FC) (Gibco, Carlsbad, CA, USA), plated in a 24-well plate and incubated at 37°C; 48 h later, the cells containing vector were selected using 1µM methotrexate (Calbiochem, La Jolla, CA, USA). Following a 2-week incubation period, colonies were picked to a new plate and allowed to grow until confluent. At this point, the supernatant was harvested and assayed by enzyme linked immunosorbent assay (ELISA) for the presence of human IgM. Positive colonies were selected further over the course of 2 months with increasing doses of methotrexate ranging from 1µM to 200 µM. In this manner, cells were generated that produced greater than 10µg/ml IgM.

The first recombinant antibody constructed as above is not fully human. To generate a completely human form, CHO cells (GibcoBRL cat # 11619) were co-transfected with vector encoding the recombinant heavy and light chain under control of an E1A promoter and a construct that expresses the human J chain in pCI (Promega). The cells were selected with increasing doses of methotrexate in a 50/50 mixture of PowerCho1 and IMDM with 10% cosmic clone and the two clones that produced the most antibody as measured by ELISA were sub-cloned. The sub-clones were expanded and vials were frozen down. Both recombinant IgM12 forms maintain the neuron binding and in vivo efficacy character of the serum isolated IgM, but have a shorter half life in mice (which may be due to differences in glycosylation).

A similar and comparable procedure was utilized to construct recombinant HIgM42 antibody in the comparable vector. The sequence of the heavy and light chain of human IgM42 antibody, with variable and constant regions noted, is shown in FIGURE 6.

### Example 4: Single Peripheral Dose of rHIgM12 Improved Neurologic Function in TMEV Model of MS

In view of the fact that rHIgM12 protected primary neurons in culture, and that disability in the TMEV model of multiple sclerosis (MS) correlates with axon loss (2), treatment of TMEV infected mice with rHIgM12 was used to evaluate the ability to slow the progression of neurologic deficits.

Groups of 5 mice at 90 days post TMEV infection, the point at which axons begin to be lost, were treated with a single 100 µg dose of rHIgM12 or a control human IgM. Five infected mice were randomly selected for each group and pretreatment functional recordings used to be certain that the baseline activity was not different between groups. Mice were followed as groups for 3 consecutive days over several weeks using activity boxes (34, 35). Changes in nocturnal behavior are sensitive measures of neurologic deficit in TMEV-mediated disease. Activity boxes are clear acrylic boxes with opposed infrared beams creating a grid across the enclosure that records all horizontal and vertical movements. The sensitivity of the assay reflects the ability to measure rearing and walking. In TMEV-infected mice the hind limbs become stiff and rearing is reduced. However, spontaneous walking about the cage is not as severely affected. It is easier for mice with stiff hind limbs to walk than to rear, and even mice with advanced disease can be extremely active during the night.

Each treatment group was randomly assembled and housed in activity boxes for 72 hr prior to treatment, and then for 72 hr each week after treatment. The mean hourly beam breaks were calculated for horizontal and vertical activity over the 12 hr from 6 pm to 6 am over the 72 hr analysis period. There were no differences between treatment groups in daytime horizontal or vertical activity, which was typically below 600 breaks/hr as mice sleep. However, an increase in spontaneous nocturnal horizontal activity compared to their pre-treatment baseline was recorded in the rHIgM12 treated group (P<0.01, weeks 3 through 7) (FIGURE 7). A control human IgM that does not bind to cells did not improve activity.

In contrast to the effect seen with serum-derived sHIgM12, and now also recombinant antibody rIgM12, in a similar study the human antibody sHIgM42 did not alter nocturnal activity of TMEV infected mice under the same conditions. Alternative dosing parameters for sHIgM42 in the same assay format produce different and more positive results on nocturnal activity.

One possible explanation for the improvement in function is that the effective IgMs interfere with virus loads which results in less disease. This does not appear to be the explanation, however. Mice with chronic TMEV disease were treated with a single dose of rHIgM12, sHIgM42 or control IgMs, the brain and spinal cord harvested 5 wk later and then the level of TMEV RNA genomic transcripts were measured by PCR with a probe to ViralProtein2. Viral transcripts were not different across groups (P<0.01) (data not shown).

### Example 5: NAA Levels within the Brain Stem of Mice with Spinal Cord Disease - A Noninvasive Surrogate Marker of Axon Preservation Throughout the Spinal Cord

NAA is a metabolite associated with neuron function (36, 37). NAA is the second most abundant amino acid in brain and is almost exclusively restricted to neurons. The preservation of NAA levels at the brain stem is a measure of overall spinal cord axon health validated by our group using the TMEV mouse model (8). When axons at lower levels of the spinal cord are damaged, cells in the brain stem die, reducing NAA. NAA levels measured by MRS reflects primarily neuron density. NAA is expressed in other neural cells, but the primary expression of NAA is in neurons. Studies of the MRS profile of purified CNS cells indicate that NAA signal amplitude is predominate in neurons, whereas the NAA signal amplitude of oligodendrocytes or astrocytes were 5% and 10% of the neuron signal, respectively (38).

To further evaluate the ability of sHIgM12 and IgM42 to improve activity in mice by preserving axon function, we used a non-invasive imaging assay and traditional morphology to assess spinal cord axons. Retrograde tracing was used to demonstrate spinal cord axon dysfunction following demyelination in TMEV-mediated disease (5). A dramatic reduction of retrograde label from the thoracic level axons to brain stem nucleic was measured. The brainstem is where many of the cell bodies reside that project long axon tracts along the length of the spinal cord. Thereafter N-acetyl aspartate (NAA) levels at the brain stem were assessed by magnetic resonance spectroscopy (MRS) in TMEV infected mice (FIGURE 8) using a previously reported protocol (8).

We observed a reduction in brainstem NAA over time in mice with TMEV-induced disease (FIGURE 9). NAA levels fell over the first 45 days of infection and remained depressed out to 270 days post infection. In SJL mice infected with TMEV, the extent of spinal cord demyelination plateaus by 90 days following infection (39). It is at this time point that axon loss is notable by histology in this model (2). Therefore NAA is a sensitive measure of axon dysfunction.

After the last MRS collection, axons were systematically sampled from 6 areas of normal-appearing white matter within a cross section of spinal cord at the T6 level. This level was chosen because this provided a global representation of the axon loss from multiple, randomly distributed demyelinated lesions throughout the spinal cord (39). We found there were 30.5% fewer axons in 270 day TMEV infected SJL mice compared to uninfected controls (p<0.001). A positive correlation was found to exist between brain stem NAA levels and axon counts at the T6 level of the spinal cord (r=0.823) (8).

### Single Dose of Neuron Binding Human IgM Preserves NAA Levels at Brain Stem and Axons in the Spinal Cord:

The ability of human neuron-binding IgMs to alter NAA levels or axon counts in TMEV infected mice was evaluated. It was found that when TMEV-infected mice at the onset of spinal cord axon drop out (90 days post infection) were treated with a single 100 µg dose of sHIgM12 or of sHIgM42 neuron binding IgM, myelinated axon density in the normal white matter of the thoracic spinal cord was greater when measured 10 wk later.

Groups of 10-15 mice at 90 days post-infection were treated with a single 100 µg dose of sHIgM12, sHIgM42, control human IgM or saline (FIGURE 10). Before treatment and at 5 and 10 wk later, each mouse was placed in a small bore magnet and MRS collected at the brain stem. At 10 wk mice were killed, spinal cords collected, plastic embedded and cross sections cut at the T6 level stained with paraphenylamindiamine to visualize myelin sheaths, 6 images per section were collected that encompass 400,000 µm of normal appearing white matter and the axons auto counted. In the groups treated with either of the two neuron-binding IgMs (sHIgM12, sHIgM42), NAA levels were increased at both 5 and 10 week later compared to pretreatment levels. Control IgM-treated mice trended lower and saline treated mice remained constant.

NAA levels of the sHIgM12 and sHIgM42 treated groups increased to 9.13 and 9.3 mM, respectively, each of which was well below the 12.0 mM levels of uninfected mice. When axons at the T6 level were compared across treatment groups (TABLE 2), mice treated with sHIgM12 or sHIgM42 contained more axons than the saline treated group (17,303 and 17,771 axons compared to 15,198 axons, P=0.008 and P<0.001), but fewer than the number of axons counted in uninfected mice (21,284 axons).

These results indicated that each of the sHIgM12 and rHIgM12 antibodies improved function by preservation of axons, as evidenced in the TMEV model. Although the sHIgM42 antibody preserved axons in the TMEV model, demonstrable changes in the nocturnal activity assessment were not observed in the initial test. Following dose-ranging studies it is found that sHIgM42 also achieves heightened activity in the nocturnal activity test.

The use of brainstem MRS to assess axon status in mouse models of spinal cord disease further validates the present invention; accordingly NAA in the brain stem serves as an excellent endpoint for clinical trial use of these human antibodies.

sHIgM42 and sHIgM12-treated mice with improved NAA levels also contained more axons in the mid-thoracic spinal cord. Groups of 10-15 TMEV-infected SJL mice were treated with a single 100 µg dose of rHIgM22, sHIgM42, sHIgM12, control sHIgM39 and saline at 90 days post infection. Ten weeks after treatment, spinal cords were removed and a mid-thoracic section stained with PPD to visualize myelin. Six areas encompassing 400,000 µm² of white matter were sampled from each mouse and the number of myelinated axons counted (1). The average of absolute number of myelinated axons per T6 cross section ± SEM is listed in Table 2.

**TABLE 2 Treatment with Neuron Binding IgMs Preserve Axon Counts in the Spinal Cord**

| **Treatment** | **Average Number of T6 Axons** | **Compare** |
|---|---|---|
| sHIgM42 | 17,771 ± 436 | p<0.001 |
| sHIgM12 | 17,303 ± 505 | p=0008 |
| Control IgM | 15,508 ± 627 | |
| Saline | 15,198 ± 484 | p=0.701 |
| Uninfected Mice | 21,284 ± 829 | |

### Example 6: Neuron-Binding IgMs Preserve Axons without Promoting Remyelination

Several human IgMs (e.g., sHIgM22 and sHIgM46) that bind to oligodendrocytyes and promote remyelination (22, 40, 41) have been identified. As shown below, neuron binding IgMs improve neuron numbers and function yet without obvious remyelination. Without being bound by theory, the mechanism of action is understood to be due to direct activation of axons (protection, neurite extension) and/or by activating the innate or adaptive immune system to secrete factors that protect neurons. The results presented above clearly demonstrate that the antibodies have a direct effect on axons/neurons. Within the same spinal cords in which sHIgM12 and sHIgM42 mediated axon preservation and/or regrowth was measured, overall demyelination, remyelination and inflammation were not different across treatment groups (FIGURE 11).

These attributes were quantitated by grading the quadrants of 10 spinal cord cross sections (6) representing samples along the length of the spinal cord. The rHIgM22 treated group (positive control) showed the expected increase in remyelination, whereas sHIgM12 and sHIgM42 treated mice contained little spinal cord remyelination. Accordingly, neurologic deficits in the TMEV model were improved (e.g., IgM12) without the requirement of significant remyelination; moreover, remyelination is not necessary for axon preservation and/or regrowth within the time frame examined.

### Example 7: Serum Half Life of rHIgM12 (with human J chain) and sHIgM42

To determine the half life of human IgMs, rHIgM12 (with human J chain) and sHIgM42, 100 µg of rHIgM12 that contains the human J chain or 100 µg sHIgM42 in 200 µl of saline was injected into the tail veins of normal CD-1 mice (FIGURE 12). At defined intervals (15 min, 1, 4, 8, 24, 48 hr) blood was collected from groups of 3 mice by cardiac puncture. The serum was collected and assayed for the presence of human IgM mu chain using a sandwich ELISA.

For rHIgM12 the half life between the first collection at 15 min and the collection at 8 hr was 3.8 hr. For sHIgM42 the half life between the first collection at 15 min and the collection at 24 hr was 20.5 hr. These values bracket the half life of the remyelination-promoting rHIgM22 which has a half life in mice of 15 hr (29) and in rabbits of 90 hr. Half life was calculated using the formulas kₑₗᵢₘ = (In(Cₚₑₐₖ)-In(c_{low}))/tᵢₙₜₑᵣᵥₐₗ and t_{1/2} = 0.693/kₑₗᵢₘ.

### Example 8: Radiolabeled Monoclonal Human IgMs Cross the Blood Brain Barrier

It has been often accepted that IgMs with a molecular weight of close to 1 million may be too large to cross the blood brain barrier (BBB) from the circulation and thereby enter the CNS (42, 43). There is certain evidence, however, that some IgMs do cross the BBB.

Tissue distribution of ³⁵S labeled rHIgM12 in normal and TMEV-infected SJL mice was measured (FIGURE 13). 50 µg of rHIgM12 (1x10⁷ cpm) was administered i.p. Mice were perfused with saline 4 or 24 hr later tissues acutely harvested, minced and dissolved in scintillation fluid. The brain and spinal cord of uninfected mice contained radiolabel at both time points. The CNS of TMEV-infected mice contained twice as much radiolabel at the 4 hr time point as uninfected mice-this increased to 4x by 24 hr.

It is also found that rHIgM12 (both with and without human J chain) and sHIgM42 are able to cross the BBB in normal and SAMP8 mice, which model Alzheimer's disease. Accordingly, ¹²⁵I labeled human IgMs are injected i.v. and brains collected two hr later. Each of these antibodies accumulates in the brains of normal and diseased mice. These antibodies also reverse cognitive impairment in the mouse model of Alzheimer's disease whether the IgM is delivered by intracerebral injection or i.v.

### Example 9: Human Neuron Binding IgMs Delivered Intraperitoneally Enter Demyelinated Spinal Cord Lesions and Localize to Neurofilament Positive Axons

The study of the isotopically labeled remyelination promoting mouse IgM, SCH94.03, by Hunter (44) used autoradiography to demonstrate radiolabel localized in vivo in the spinal cord of TMEV infected mice, specifically to cells that were ultrastructurally identified as oligodendrocytes. Similar autoradiography studies with ³⁵S rHIgM12 have been performed. Using traditional immunocytochemistry, we have detected neuron binding human IgMs within spinal cord lesions (FIGURE 14).

Accordingly, 1.0 mg of rHIgM12 (with human J chain), sHIgM42 or a commercial control human IgM (Jackson Immuno Research) were administered intraperitoneally to TMEV infected mice with chronic demyelination. Four hours later mice were perfused with paraformaldehyde, the spinal cords frozen sectioned longitudinally and immunostained for the presence of human IgM mu chain. In mice receiving rHIgM12 or sHIgM42 human mu chain was localized in demyelinated lesions in parallel tracts, suggesting axon fibers. The control human IgM was not found within lesions or in the non-lesioned cord. Thus, it was clear that neuron-binding human IgMs rHIgM12 or sHIgM42 cross the BBB in TMEV infected mice.

Adjacent spinal cord sections were then immunolabeled with anti-neurofilament (NF) Abs (SMI-32 and 34, Sternberger) followed by fluorescent secondary Abs anti-human mu chain-FITC and anti-mouse-TRITC. Confocal microscopy demonstrated that rHIgM12 and sHIgM42 co-localized to NF+ axons within lesions, in parallel tracks of fibers and as fiber bundles of axons cut on end (FIGURE 15).

### Example 10: rHIgM12 or sHIgM42 do not Exacerbate MOG Peptide Induced EAE When Given at the Onset of Disease

To address concerns that administering autoreactive CNS binding IgMs to animals with active autoimmunity may exacerbate disease, the effects of rHIgM12 and sHIgM42 were tested in mice with EAE. Groups of 10 C57BL6 mice with MOG peptide-induced (200 µg) EAE were administered a single 100 µg dose of rHIgM12, sHIgM42, control human IgM or saline intravenously. Individual mice were treated at the time that their clinical score reached 1 (limp tail). Mice were then followed by an investigator blinded to the treatment groups who recorded weights and graded clinical scores every other day until mice reached 28 days post immunization or were moribund.

There were no differences between treatment groups in weights, or mean clinical scores (FIGURE 16, P=0.14). In addition 10 spinal cord cross sections encompassing the entire spinal cord of each mouse was graded blindly for the presence of meningeal inflammation and demyelination in each spinal cord quadrant (6). There were no differences in the percent of quadrants with meningeal inflammation (P=0.825) or demyelination (P=0.766) across treatment groups (FIGURE 17). Thus it was found that a single dose of neuron binding human IgMs that are efficacious in protecting axons in the TMEV model does not worsen EAE clinical deficits, accelerate deficit progression or increase spinal cord pathology.

### REFERENCES

1. Howe, C. L., J. D. Adelson, and M. Rodriguez. 2007. Absence of perforin expression confers axonal protection despite demyelination. Neurobiol Dis 25:354-359.
2. McGavern, D. B., P. D. Murray, C. Rivera-Quinones, J. D. Schmelzer, P. A. Low, and M. Rodriguez. 2000. Axonal loss results in spinal cord atrophy, electrophysiological abnormalities and neurological deficits following demyelination in a chronic inflammatory model of multiple sclerosis. Brain 123 Pt 3:519-531.
3. Jones, M. V., T. T. Nguyen, C. A. Deboy, J. W. Griffin, K. A. Whartenby, D. A. Kerr, and P. A. Calabresi. 2008. Behavioral and pathological outcomes in MOG 35-55 experimental autoimmune encephalomyelitis. Journal of neuroimmunology*.*
4. Rodriguez, M., E. Oleszak, and J. Leibowitz. 1987. Theiler's murine encephalomyelitis: a model of demyelination and persistence of virus. Crit Rev Immunol 7:325-365.
5. Ure, D. R., and M. Rodriguez. 2002. Preservation of neurologic function during inflammatory demyelination correlates with axon sparing in a mouse model of multiple sclerosis. Neuroscience 111:399-411.
6. Mitsunaga, Y., B. Ciric, V. Van Keulen, A. E. Warrington, M. Paz Soldan, A. J. Bieber, M. Rodriguez, and L. R. Pease. 2002. Direct evidence that a human antibody derived from patient serum can promote myelin repair in a mouse model of chronic-progressive demyelinating disease. Faseb J 16:1325-1327.
7. Van Keulen, V. P., B. Ciric, S. Radhakrishnan, K. L. Heckman, Y. Mitsunaga, K. lijima, H. Kita, M. Rodriguez, and L. R. Pease. 2006. Immunomodulation using the recombinant monoclonal human B7-DC cross-linking antibody rHIgM12. Clin Exp Immunol 143:314-321.
8. Denic, A., A. Bieber, A. Warrington, P. K. Mishra, S. Macura, and M. Rodriguez. 2009. Brainstem (1)H nuclear magnetic resonance (NMR) spectroscopy: Marker of demyelination and repair in spinal cord. Annals of neurology 66:559-564.
9. Duncan, I. D. 1996. Glial cell transplantation and remyelination of the central nervous system. Neuropathol Appl Neurobiol 22:87-100.
10. Fernandez, M., A. Giuliani, S. Pirondi, G. D'Intino, L. Giardino, L. Aloe, R. Levi-Montalcini, and L. Calza. 2004. Thyroid hormone administration enhances remyelination in chronic demyelinating inflammatory disease. Proc Natl Acad Sci U S A 101:16363-16368.
11. Craig, C. G., V. Tropepe, C. M. Morshead, B. A. Reynolds, S. Weiss, and D. van der Kooy. 1996. In vivo growth factor expansion of endogenous subependymal neural precursor cell populations in the adult mouse brain. J Neurosci 16:2649-2658.
12. Rodriguez, M. 2007. Effectors of Demyelination and Remyelination in the CNS: Implications for Multiple Sclerosis. Brain Pathol 17:219-229.
13. Pitt, D., P. Werner, and C. S. Raine. 2000. Glutamate excitotoxicity in a model of multiple sclerosis. Nat Med 6:67-70.
14. Okuda, Y., S. Sakoda, H. Fujimura, and T. Yanagihara. 1997. Nitric oxide via an inducible isoform of nitric oxide synthase is a possible factor to eliminate inflammatory cells from the central nervous system of mice with experimental allergic encephalomyelitis. Journal of neuroimmunology 73:107-116.
15. Waxman, S. G. 2002. Sodium channels as molecular targets in multiple sclerosis. J Rehabil Res Dev 39:233-242.
16. Irvine, K. A., and W. F. Blakemore. 2008. Remyelination protects axons from demyelination-associated axon degeneration. Brain 131:1464-1477.
17. Linker, R. A., M. Maurer, S. Gaupp, R. Martini, B. Holtmann, R. Giess, P. Rieckmann, H. Lassmann, K. V. Toyka, M. Sendtner, and R. Gold. 2002. CNTF is a major protective factor in demyelinating CNS disease: a neurotrophic cytokine as modulator in neuroinflammation. Nat Med 8:620-624.
18. Chang, A., W. W. Tourtellotte, R. Rudick, and B. D. Trapp. 2002. Premyelinating oligodendrocytes in chronic lesions of multiple sclerosis. N Engl J Med 346:165-173.
19. McTigue, D. M., R. Tripathi, and P. Wei. 2006. NG2 colocalizes with axons and is expressed by a mixed cell population in spinal cord lesions. J Neuropathol Exp Neurol 65:406-420.
20. Franklin, R. J., G. L. Hinks, R. H. Woodruff, and M. T. O'Leary. 2001. What roles do growth factors play in CNS remyelination? Prog Brain Res 132:185-193.
21. Rodriguez, M., A. E. Warrington, and L. R. Pease. 2009. Invited Article: Human natural autoantibodies in the treatment of neurologic disease. Neurology 72:1269-1276.
22. Warrington, A. E., K. Asakura, A. J. Bieber, B. Ciric, V. Van Keulen, S. V. Kaveri, R. A. Kyle, L. R. Pease, and M. Rodriguez. 2000. Human monoclonal antibodies reactive to oligodendrocytes promote remyelination in a model of multiple sclerosis. Proc Natl Acad Sci U S A 97:6820-6825.
23. Warrington, A. E., A. J. Bieber, V. Van Keulen, B. Ciric, L. R. Pease, and M. Rodriguez. 2004. Neuron-binding human monoclonal antibodies support central nervous system neurite extension. J Neuropathol Exp Neurol 63:461-473.
24. Radhakrishnan, S., L. T. Nguyen, B. Ciric, D. Flies, V. P. Van Keulen, K. Tamada, L. Chen, M. Rodriguez, and L. R. Pease. 2004. Immunotherapeutic potential of B7-DC (PD-L2) cross-linking antibody in conferring antitumor immunity. Cancer Res 64:4965-4972.
25. Cohen, I. R. 2007. Biomarkers, self-antigens and the immunological homunculus. J Autoimmun 29:246-249.
26. Cohen, I. R., and M. Schwartz. 1999. Autoimmune maintenance and neuroprotection of the central nervous system. Journal of neuroimmunology 100:111-114.
27. Lutz, H. U. 2007. Homeostatic roles of naturally occurring antibodies: an overview. J Autoimmun 29:287-294.
28. Bieber, A. J., A. Warrington, K. Asakura, B. Ciric, S. V. Kaveri, L. R. Pease, and M. Rodriguez. 2002. Human antibodies accelerate the rate of remyelination following lysolecithin-induced demyelination in mice. Glia 37:241-249.
29. Warrington, A. E., A. J. Bieber, B. Ciric, L. R. Pease, V. Van Keulen, and M. Rodriguez. 2007. A recombinant human IgM promotes myelin repair after a single, very low dose. J Neurosci Res 85:967-976.
30. Pirko, I., B. Ciric, J. Gamez, A. J. Bieber, A. E. Warrington, A. J. Johnson, D. P. Hanson, L. R. Pease, S. I. Macura, and M. Rodriguez. 2004. A human antibody that promotes remyelination enters the CNS and decreases lesion load as detected by T2-weighted spinal cord MRI in a virus-induced murine model of MS. Faseb J 18:1577-1579.
31. Warrington, A. E., and S. E. Pfeiffer. 1992. Proliferation and differentiation of 04+ oligodendrocytes in postnatal rat cerebellum: analysis in unfixed tissue slices using anti-glycolipid antibodies. J Neurosci Res 33:338-353.
32. Ciric, B., V. Van Keulen, M. Paz Soldan, M. Rodriguez, and L. R. Pease. 2004. Antibody-mediated remyelination operates through mechanism independent of immunomodulation. Journal of neuroimmunology 146:153-161.
33. Howe, C. L., A. J. Bieber, A. E. Warrington, L. R. Pease, and M. Rodriguez. 2004. Antiapoptotic signaling by a remyelination-promoting human antimyelin antibody. Neurobiol Dis 15:120-131.
34. Rivera-Quinones, C., D. McGavern, J. D. Schmelzer, S. F. Hunter, P. A. Low, and M. Rodriguez. 1998. Absence of neurological deficits following extensive demyelination in a class I-deficient murine model of multiple sclerosis. Nature medicine 4:187-193.
35. McGavern, D. B., L. Zoecklein, K. M. Drescher, and M. Rodriguez. 1999. Quantitative assessment of neurologic deficits in a chronic progressive murine model of CNS demyelination. Exp Neurol 158:171-181.
36. De Stefano, N., P. M. Matthews, L. Fu, S. Narayanan, J. Stanley, G. S. Francis, J. P. Antel, and D. L. Arnold. 1998. Axonal damage correlates with disability in patients with relapsing-remitting multiple sclerosis. Results of a longitudinal magnetic resonance spectroscopy study. Brain 121 (Pt 8):1469-1477.
37. Tourbah, A., C. Linnington, C. Bachelin, V. Avellana-Adalid, H. Wekerle, and A. Baron-Van Evercooren. 1997. Inflammation promotes survival and migration of the CG4 oligodendrocyte progenitors transplanted in the spinal cord of both inflammatory and demyelinated EAE rats. J Neurosci Res 50:853-861.
38. Manganas, L. N., X. Zhang, Y. Li, R. D. Hazel, S. D. Smith, M. E. Wagshul, F. Henn, H. Benveniste, P. M. Djuric, G. Enikolopov, and M. Maletic-Savatic. 2007. Magnetic resonance spectroscopy identifies neural progenitor cells in the live human brain. Science 318:980-985.
39. McGavern, D. B., P. D. Murray, and M. Rodriguez. 1999. Quantitation of spinal cord demyelination, remyelination, atrophy, and axonal loss in a model of progressive neurologic injury. J Neurosci Res 58:492-504.
40. Asakura, K., D. J. Miller, L. R. Pease, and M. Rodriguez. 1998. Targeting of IgMkappa antibodies to oligodendrocytes promotes CNS remyelination. J Neurosci 18:7700-7708.
41. Asakura, K., D. J. Miller, K. Murray, R. Bansal, S. E. Pfeiffer, and M. Rodriguez. 1996. Monoclonal autoantibody SCH94.03, which promotes central nervous system remyelination, recognizes an antigen on the surface of oligodendrocytes. J Neurosci Res 43:273-281.
42. Kozlowski, G. P., I. Sterzl, and G. Nilaver. 1992. Localization patterns for immunoglobulins and albumins in the brain suggest diverse mechanisms for their transport across the blood-brain barrier (BBB). Prog Brain Res 91:149-154.
43. Banks, W. A. 2008. Developing drugs that can cross the blood-brain barrier: applications to Alzheimer's disease. BMC Neurosci 9 Suppl 3:S2.
44. Hunter, S. F., D. J. Miller, and M. Rodriguez. 1997. Monoclonal remyelination-promoting natural autoantibody SCH 94.03: pharmacokinetics and in vivo targets within demyelinated spinal cord in a mouse model of multiple sclerosis. J Neurol Sci 150:103-113.
45. Banks, W. A., S. A. Farr, J. E. Morley, K. M. Wolf, V. Geylis, and M. Steinitz. 2007. Anti-amyloid beta protein antibody passage across the blood-brain barrier in the SAMP8 mouse model of Alzheimer's disease: an age-related selective uptake with reversal of learning impairment. Exp Neurol 206:248-256.
46. Schenk, D., R. Barbour, W. Dunn, G. Gordon, H. Grajeda, T. Guido, K. Hu, J. Huang, K. Johnson-Wood, K. Khan, D. Kholodenko, M. Lee, Z. Liao, I. Lieberburg, R. Motter, L. Mutter, F. Soriano, G. Shopp, N. Vasquez, C. Vandevert, S. Walker, M. Wogulis, T. Yednock, D. Games, and P. Seubert. 1999. Immunization with amyloid-beta attenuates Alzheimer-disease-like pathology in the PDAPP mouse. Nature 400:173-177.
47. Bard, F., C. Cannon, R. Barbour, R. L. Burke, D. Games, H. Grajeda, T. Guido, K. Hu, J. Huang, K. Johnson-Wood, K. Khan, D. Kholodenko, M. Lee, I. Lieberburg, R. Motter, M. Nguyen, F. Soriano, N. Vasquez, K. Weiss, B. Welch, P. Seubert, D. Schenk, and T. Yednock. 2000. Peripherally administered antibodies against amyloid beta-peptide enter the central nervous system and reduce pathology in a mouse model of Alzheimer disease. Nat Med 6:916-919.
48. Mikami, Y., M. Toda, M. Watanabe, M. Nakamura, Y. Toyama, and Y. Kawakami. 2002. A simple and reliable behavioral analysis of locomotor function after spinal cord injury in mice. Technical note. J Neurosurg 97:142-147.
49. Mikami, Y., H. Okano, M. Sakaguchi, M. Nakamura, T. Shimazaki, H. J. Okano, Y. Kawakami, Y. Toyama, and M. Toda. 2004. Implantation of dendritic cells in injured adult spinal cord results in activation of endogenous neural stem/progenitor cells leading to de novo neurogenesis and functional recovery. J Neurosci Res 76:453-465.
50. Gilgun-Sherki, Y., H. Panet, V. Holdengreber, R. Mosberg-Galili, and D. Offen. 2003. Axonal damage is reduced following glatiramer acetate treatment in C57/bl mice with chronic-induced experimental autoimmune encephalomyelitis. Neurosci Res 47:201-207.
51. Genain, C. P., B. Cannella, S. L. Hauser, and C. S. Raine. 1999. Identification of autoantibodies associated with myelin damage in multiple sclerosis. Nat Med 5:170-175.
52. Genain, C. P., M. H. Nguyen, N. L. Letvin, R. Pearl, R. L. Davis, M. Adelman, M. B. Lees, C. Linington, and S. L. Hauser. 1995. Antibody facilitation of multiple sclerosis-like lesions in a nonhuman primate. J Clin Invest 96:2966-2974.
53. Lennon, V. A., D. M. Wingerchuk, T. J. Kryzer, S. J. Pittock, C. F. Lucchinetti, K. Fujihara, I. Nakashima, and B. G. Weinshenker. 2004. A serum autoantibody marker of neuromyelitis optica: distinction from multiple sclerosis. Lancet 364:2106-2112.
54. Rodriguez, M., W. E. Karnes, J. D. Bartleson, and A. A. Pineda. 1993. Plasmapheresis in acute episodes of fulminant CNS inflammatory demyelination. Neurology 43:1100-1104.
55. Herrero-Herranz, E., L. A. Pardo, R. Gold, and R. A. Linker. 2008. Pattern of axonal injury in murine myelin oligodendrocyte glycoprotein induced experimental autoimmune encephalomyelitis: implications for multiple sclerosis. Neurobiol Dis 30:162-173.
56. Yednock, T. A., C. Cannon, L. C. Fritz, F. Sanchez-Madrid, L. Steinman, and N. Karin. 1992. Prevention of experimental autoimmune encephalomyelitis by antibodies against alpha 4 beta 1 integrin. Nature 356:63-66.
57. Kanwar, J. R., J. E. Harrison, D. Wang, E. Leung, W. Mueller, N. Wagner, and G. W. Krissansen. 2000. Beta7 integrins contribute to demyelinating disease of the central nervous system. Journal of neuroimmunology 103:146-152.
58. Warrington, A. E., and M. Rodriguez. 2008. Remyelination-promoting human IgMs: developing a therapeutic reagent for demyelinating disease. Curr Top Microbiol Immunol 318:213-239.
59. Jaffe, J. D., H. Keshishian, B. Chang, T. A. Addona, M. A. Gillette, and S. A. Carr. 2008. Accurate inclusion mass screening: a bridge from unbiased discovery to targeted assay development for biomarker verification. Mol Cell Proteomics 7:1952-1962.
60. Keshishian, H., T. Addona, M. Burgess, E. Kuhn, and S. A. Carr. 2007. Quantitative, multiplexed assays for low abundance proteins in plasma by targeted mass spectrometry and stable isotope dilution. Mol Cell Proteomics 6:2212-2229.

### Example 11: Human IgM Antibody rHlgM12 Promotes Axon Formation and Interacts with Lipid Rafts to Target Microtubules

Antibody sHIgM12, promoted neurite outgrowth in primary cultured cerebellar granule neurons. In the present example, using primary hippocampal neurons fully human (rHIgM12 having a human J chain) was found to promote axon formation. rHIgM12 binds to neuron membranes and induces clustering of cholesterol and ganglioside GM1.

Moreover, the membrane-bound rHIgM12 is distributed into two pools, one associated with lipid raft domains, and the other with the detergent insoluble pellet enriched in cytoskeleton. rHIgM12 aggregates colocalize over microtubules, but not over filamentous actin after detergent extraction. Coimmunoprecipitation studies demonstrated that rHIgM12 and β3-tubulin exist in a complex. These results indicate that rHIgM12 specifies axon formation by clustering membrane domains to signal the microtubule cytoskeleton.

Neurons develop axons though regulation of neurite outgrowth (Barnes and Polleux, 2009). The neuron cytoskeleton, which includes filamentous actin (F-actin) and microtubules, plays a crucial role in neurite growth and growth cone pathfinding.

Serum-derived antibody may not be suitable for larger scale studies, particularly if it cannot be produced in quantity and must be isolated for each use from the patient that produces the antibody, the generation of a recombinant form which demonstrates comparable activity and capability is advantageous. The present study demonstrated that recombinant and fully human IgM12 antibody (rHIgM12) promoted axon formation and thus drove neuronal polarization in cultured hippocampal neurons. The rHIgM12 clusters the neuronal membrane domains containing cholesterol and ganglioside GM1.

Sucrose density gradient fractionation indicated that neuronal membrane-bound rHIgM12 segregates into two pools, one associated with the detergent-resistant lighter fraction which contains caveolin-1, and the other with the cytoskeleton-enriched pellet. Detergent extraction of live neurons demonstrated that rHIgM12 is associated with microtubules. rHIgM12 also co-immunoprecipitated with β3-tubulin. Taken together, it is understood that rHIgM12 binds membrane domains associated with microtubules. When present as a substrate on a surface, rHIgM12 promotes microtubule anchoring on neuron membranes, facilitating neurite outgrowth and axon formation.

*Recombinant human IgM 12 (rHIgM12):* rHIgM12 was expressed in CHO cells (GibcoBRL, cat# 11619). Plasmids expressing the heavy and light chain coding sequences for the predominant antibody expressed in the serum of Waldenstrom's macroglobulinemia patient 12 were transfected along with a human J chain transgene into CHO-S cells. The resulting CHO cells were selected with increasing doses of methotrexate and a stable clone that produced the antibody as measured by ELISA was subcloned and expanded. rHIgM12 antibody from culture supernatant was purified by chromatography to 97% as measured by HPLC analysis.

*Cell culture and neurite outgrowth assay.* Primary hippocampal neurons were prepared from FVB mice. Embryonic day 15 hippocampal neurons were dissociated in trypsin-EDTA, plated on poly-D-lysine (PDL), PDL with laminin or rHIgM12 substrates coated on a thin layer of nitrocellulose membrane attached to glass coverslips and grown in Neurobasal Media containing 2% (v/v) of B27. Neurite outgrowth was assayed 12 hr after neurons were seeded. The neurons were fixed with 4% paraformaldehyde and stained with anti β3-tubulin antibody. Filamentous actin (F-actin) was labeled with Texas-Red phalloidin, and nuclei with DAPI. Neurite length was measured using Neuron J software, processed with Excel (Microsoft) and analyzed statistically with Prism (GraphPad). A Stage-3 neuron was defined as a neuron with multiple neurites in which the longest neurite (Dotti et al., 1988), determined as an axon by Tau1 staining, was at least two times the length of the second longest neurite. Tau1 was asymmetrically enriched in the distal part of the axon. In contrast, Stage-2 neurons had multiple symmetric neurites.

*Immunostaining, immunoprecipitation and sucrose density gradient fractionation of primary cultured neurons:* One to three days in vitro (DIV) cultured hippocampal neurons were permeabilized with 0.2% Triton X-100 after fixation with 4% paraformaldehyde followed by immunostaining. Images were collected using an Olympus upright microscope and processed using Photoshop (Adobe). rHIgM12 distribution was determined by ultracentrifugation in non-continuous sucrose gradient. Briefly, rHIgM12 was allowed to bind to live DIV7 cortical neurons at 4°C for 30 min then lysed in ice-cold lysis buffer (50 mM Tris.HCl, pH 7.4, 150 mM NaCl, 1 mM EDTA, 1% Triton X-100 and protease inhibitor cocktail) for 30 min. The neuronal lysates were mixed with an equal volume of 100% (w/v) sucrose. The mixture was transferred to a centrifuge tube, and 8 ml of 35% sucrose and 3.5 ml of 5% sucrose were overlaid sequentially. After centrifugation at 2x10⁵ g for 20 h at 4°C, six fractions (2ml of each) were collected from the top of the gradient. Each fraction and the pellet were dissolved in SDS-sample buffer and subjected to western blotting.

For co-immunoprecipitation of rHIgM12 with cytoskeleton protein, DIV7 live cortical neurons were treated with rHIgM12 at 4°C for 30 min and lysed in lysis buffer containing 0.5% NP-40. rHIgM12 was captured by protein L-agarose beads, and β3-tubulin by protein G-resin (Thermo). For double-labeling, live neurons were stained with rHIgM12 on ice and permeabilized with 0.2% Triton X-100 after fixation. Live neuron extraction and fixation was performed in buffer containing 60 mM Pipes, 25 mM Hepes, 5 mM EGTA, 1 mM MgCl₂, 4% paraformaldehyde and 0.1% Triton X-100.

*Antibodies and other reagents in this example:* Anti-β3-tubulin (Promega); anti-actin, EDTA, poly-D-lysine, methyl-β-cyclodextrin and filipin (Sigma); anti-Tau1, anti-caveolin-1 and anti-transferrin receptor (Millipore); Texas Red-phalloidin, cholera toxin B, Neurobasal Medium and B27 (Invitrogen); protease inhibitor tablet (Roche).

### Recombinant Human IgM, rHIgM12, Promoted Axon Formation:

To further understand the effect of the IgM on neuron differentiation and the underlying molecular mechanisms, neurite outgrowth assays were carried out with cultured hippocampal neurons using a recombinant rHIgM12. Hippocampal neurons undergo three stages of differentiation to form an axon (Dotti et al., 1988). Multiple processes initiate from the cell body. One of the fast growing neurites differentiates into the axon, the others into dendrites later, which can be identified by the differential distribution of Tau1 and MAP2 respectively (Lewis et al., 1989; (Kanai and Hirokawa, 1995). We found that rHIgM12, when presented as a substrate, substantially promoted neuron differentiation. Within 12 hours after plating, the hippocampal neurons growing on rHIgM12 developed multiple neurites, and one of them was much longer compared to the neighboring neurites. In contrast, neurons plated on poly-D-lysine (PDL) had multiple symmetric neurites (FIGURE 18 A, B).

After measuring neurite length, we found that neurons plated on rHIgM12 (n=86) compared to PDL (n=74) had significantly longer total neurite length (195.8 µm vs. 150.7 µm, *p*=0.0056) (FIGURE 18C). The longest neurite length more than doubled (117.1 µm vs. 51.8 µm, *p*<0.0001) (FIGURE 18D), but no significant difference in the second longest neurite length (38.7 µm vs. 33.3 µm, *p*=0.0782) (FIGURE 18E) was found. Neurons growing on rHIgM12 bore fewer primary neurites (2.9 vs. 4.1, *p*<0.0001) (FIGURE 18F), and the majority of them were of stage 3 neuron phenotype (72% on rHIgM12 vs. 18% on PDL) (FIGURE 18G). These results indicated that rHIgM12 promoted neuron differentiation when presented as a substrate.

The characteristic feature of neuron differentiation is polarized axon outgrowth and segregation from dendrites. We observed that rHIgM12 promoted neurite extension, with one significantly longer neurite. To further verify that these longest neurites from the stage 3 neurons developed into axons, the hippocampal neurons plated on different substrates were stained with anti-tau1 and MAP2 antibodies (FIGURE 19). Tau1 staining was weak in neurons grown on PDL (FIGURE 19A, D), but much stronger in neurons plated on PDL-laminin (FIGURE 19B, E) or rHIgM12 (FIGURE 19C, F). Laminin is a classic substrate for neuron differentiation and axon formation (Chen et al., 2009) and was used as positive control.

Comparing the tau1 intensity from neurons grown on PDL (FIGURE 19D), the distribution of tau1 was asymmetrically enriched in the distal part of the longest neurite and much greater in neurons plated on either PDL-laminin (FIGURE 19E) or rHIgM12 (FIGURE 19F). In contrast, MAP2 stained the proximal part of all neurites (FIGURE 19A2-C2). This result indicated that the longest neurites from the stage 3 neurons grown on both rHIgM12 and laminin were axons. These studies demonstrate that rHIgM12 supports neurite outgrowth from multiple neuron types, and that rHIgM12 drives axon formation.

### rHIgM12 Induced Neuronal Membrane Microdomain Clustering

Hippocampal neurons growing on rHIgM12 as a substrate showed enhanced differentiation (FIGURE 18 & 19). However, we did not observe that rHIgM12 induced neurite outgrowth when rHIgM12 was applied to the bath media (data not shown). This observation suggested that rHIgM12, to exert its function, is required to be presented as an extracellular matrix molecule. To further understand rHIgM12-neuron membrane interactions, live neurons were first treated with rHIgM12, then fixed and double stained with filipin or cholera toxin B (CTB), which bind cholesterol or ganglioside, GM1 respectively (Shogomori and Futerman, 2001). rHIgM12, CTB and filipin evenly labeled cell surfaces of the untreated control neurons (FIGURE 20A). In contrast, treatment with rHIgM12 at 37°C induced reorganization of neuronal membranes 30 min (FIGURE 20B, C). First, rHIgM12 aggregated into "patch"-like structures (FIGURE 20B1, C1) at the membrane, which were not observed in untreated neurons (FIGURE 20A) or neurons treated with a control IgM antibody which does not bind neuron membrane (data not shown). Second, both cholesterol (FIGURE 20B2) and GM1 (FIGURE 20C2) clusters were formed on neuronal membranes at the cell body, neurite shaft and growth cone. Third, the aggregated rHIgM12 colocalized with clustered cholesterol (FIGURE 20B3) or GM1 (FIGURE 20C3), especially in the growth cone central domain (FIGURE 20B-C, high magnification), but not in the growth cone periphery. These results indicated that the bath-applied rHIgM12 induced neuronal membrane rearrangement, even though it does not support neurite/axon extension. It is possible that rHIgM12, when presented as a substrate, may recruit membrane molecules to rHIgM12-neuronal membrane contact sites and result in directional signaling.

### rHIgM12 Bound Lipid Rafts

Biochemical studies based on nonionic detergent extraction in cold conditions have been used to demonstrate the existence of lipid rafts which contain cholesterol and glycosphingolipid (Chichili and Rodgers, 2009). However, there is limited morphological information about lipid rafts because the nanoscale size is beyond the resolution of the light microscope (Lingwood and Simons, 2010). The observation that rHIgM12 induces membrane clustering and colocalizes with cholesterol- and GM1-containing membrane domains (FIGURE 20) raised the possibility that rHIgM12 associates with lipid raft domains.

Since neuronal membranes are enriched in cholesterol and glycosphingolipid, which have higher melting temperatures (Tm) (Samsonov et al., 2001), decreasing the membrane temperature below Tm may facilitate visualization of lipid raft and its associated molecules which are more dynamic at 37 °C. To test this hypothesis, live neurons were cooled down to 4°C, followed by rHIgM12 staining. In contrast to neurons fixed at 37°C where rHIgM12 bound evenly (FIGURE 21A), at 4°C rHIgM12 labeled much larger punctate structures. In addition, the neuronal growth cones shrunk (FIGURE 21B).

We took advantage of the fact that lipid rafts are cholesterol dependent membrane domains and that cholesterol depletion disrupts their structure (Chichili and Rodgers, 2009). Cultured hippocampal neurons were first treated with methyl-β-cyclodextrin (MβCD) to deplete cholesterol (Ko et al., 2005) and then cooled down to 4°C, followed by rHIgM12 and CTB staining after fixation. rHIgM12 bound to punctate structures in neuronal membranes after cholesterol depletion, and colocalized with CTB-labeled GM1 (FIGURE 21C). Because GM1 resides within lipid raft microdomains and rHIgM12 induces GM1 clustering (FIGURE 20), we concluded that rHIgM12 binds GM1-containing microdomains, which are independent of neuronal membrane cholesterol. These experiments confirmed that lipid rafts exist as distinct morphological structures, which are evenly inserted into membranes (FIGURE 20A & FIGURE 21A) or aggregate to form larger domains (FIGURE 20B & C, FIGURE 21B & C). These results indicate that rHIgM12 binds to molecule(s) which traffic between raft and non-raft domains depending on the biophysical interactions with other molecules.

We further tested the hypothesis that rHIgM12 binds to lipid rafts. Because lipid rafts localize within the nonionic detergent-resistant membranes (DRM) at cold temperature (Samsonov et al., 2001), the localization of rHIgM12 was analyzed in cortical neuron lysates prepared at 4°C. Blotting the neuronal lysates with anti-human secondary IgM antibody revealed that membrane-bound rHIgM12 was found in both the pellet and the supernatant, while the non-neuron-binding control IgM antibody was found in only the wash eluates (FIGURE 22A). These observations indicated that rHIgM12 segregated into two pools, one localized in the "detergent soluble" fraction in the supernatant and the other associated with the "detergent-insoluble" pellet.

In a second experiment, cortical neurons were first labeled with rHIgM12, then fractionated by sucrose density gradient at 4°C. Blotting of different fractions indicated that rHIgM12 localized to the lighter fraction which also contained caveolin-1, a lipid raft marker, but not to the transferrin receptor-enriched non-raft fraction (FIGURE 22B). However, even in this strict fractionating process (1% Triton X-100 and ultracentrifugation at 2x105 g), some rHIgM12 was detected in the pellet, which mainly contains detergent-insoluble cytoskeleton (FIGURE 22B). Although both tubulin (Sorice et al., 2009) and actin (Levitan and Gooch, 2007) are present in lipid rafts, only β3-tubulin was detected in the raft and the majority of actin went to the non-raft fraction (FIGURE 22B). These data indicated the existence of two pools of membrane-bound rHIgM12, one present in the lipid raft, and the other in the pellet.

### rHIgM12 Associated with Microtubules

Our data showed that aggregated rHIgM12 localized to the regions such as the cell body, neurite shaft and growth cone central domain (FIGURE 20), that are dominated by microtubules (Forscher and Smith, 1988). Together, with the fact that membrane-bound rHIgM12 segregated into the lipid raft and the pellet fractions, both of which contained β3-tubulin (FIGURE 22B), these findings suggested that rHIgM12 may be associated with cytoskeleton component(s). To test this hypothesis, hippocampal neurons were treated with rHIgM12 to induce membrane rearrangement, then simultaneously fixed and extracted at 37oC with 4% paraformaldehyde containing 0.1% Triton X-100. After extraction, rHIgM12-labeled detergent insoluble membrane puncta aligned with bundled microtubule fascicles in the neurite shaft (FIGURE 23A2). In the growth cone, rHIgM12 mainly localized to the central domain occupied by defasciculated microtubules, but not in the F-actin enriched growth cone periphery (FIGURE 23A3 & A4). These results indicated that rHIgM12 may associate with microtubules

We found that rHIgM12 colocalized with GM1 (FIGURE 21C). GM1 was shown to be anchored by membrane tubulin in cerebellar granule neurons, and was pulled down by an anti-tubulin antibody after crosslinking (Palestini et al., 2000). Thus, it is likely that rHIgM12 associates with membrane microdomains containing tubulin. To further test this hypothesis, the modified pull-down experiments were carried out with cell lysates from rHIgM12-treated live cortical neurons at 4°C. We found that both rHIgM12 and β3-tubulin coimmunoprecipitated with each other (FIGURE 23B), suggesting that rHIgM12 and β3-tubulin exist as a complex. IgM molecule has a pentamer structure with a molecular weight of about 900 kDa. To rule out the possibility that rHIgM12 associates with tubulin or microtubules directly in neuronal lysates, pull-down assays were carried out with N2A neuroblastoma cells which do not bind rHIgM12, but do express β3-tubulin (FIGURE 25A). rHIgM12 neither associated with β3-tubulin nor presented in the pellet. It was only detected in the supernatants of N2A neuroblastoma cell lysates (FIGURE 25B). Together, these results confirmed that lipid rafts mediate the association between rHIgM12, its antigen and microtubules.

The data herein demonstrated that a fully human recombinant IgM, rHIgM12, drove neuron polarization by promoting axon outgrowth in primary cultured hippocampal neurons when presented as a substrate (FIGURE 18 & 19). The rHIgM12 aggregated on neuron surfaces and induced the clustering of cholesterol and ganglioside, GM1 (FIGURE 20). rHIgM12 colocalized with GM1 (FIGURE 21). Sucrose gradient fractionation of neuron lysates indicated that membrane-bound rHIgM12 segregated into two pools: one associated with lipid rafts, the other with the pellet (FIGURE 22). By extracting live neurons with nonionic detergent, we further showed that membrane-bound rHIgM12 co-localized with microtubules and was coimmunoprecipitated with β3-tubulin (FIGURE 23).

The foregoing indicates that rHIgM12 binds to and interacts with lipid raft microdomains, and further indicates that rHIgM12-associated raft domains convey signaling to microtubules. As a result, rHIgM12 mediates microtubule stabilization, which drives axon outgrowth. This example demonstrated that rHIgM12 selectively promotes axon outgrowth of primary hippocampal neurons (FIGURE 18 & 19).

Recently, we showed that the human sera-derived IgM, sHIgM12, improved neurological function of a multiple sclerosis animal model which develops extensive axonal degeneration and loss (Rodriguez et al., 2009).These studies support the concept that HIgM12 exerts its function by encouraging axon growth.

Neurons specify axons through a controlled intrinsic program which regulates neurite extension. A developing neuron initiates multiple neurites. One of the neurites differentiates into the axon, the others dendrites. The neighboring neurites compete with each other. The longest neurite, which also has the fastest extension rate, first breaks the symmetric neurite extension to develop into an axon, whereas the other neurites extend much slower and develop into dendrites later (Dotti et al., 1988; Goslin and Banker, 1989). F-actin and microtubules are the two major cytoskeletons which are involved in neurite extension and axon formation. Our understanding of axon specification has been evolving. F-actin, which mainly localizes to the growth cone periphery, was regarded to play a major role. Microtubules, which dominate the neurite shaft and are confined to the growth cone central domain, were thought to be secondary to F-actin. Recently, microtubules were found to also play a critical role in axon growth. Microtubules are capable of dynamically exploring the growth cone actin meshwork. Microtubule stabilization is sufficient to induce axon formation (Witte and Bradke, 2008).

Our results, that rHIgM12 and β3-tubulin co-immunoprecipitated, that rHIgM12 colocalized with microtubules and that rHIgM12 was present in the pellet after detergent extraction, not only support microtubules as a central player. In addition, this data provided the first evidence in the art that microtubules directly interact with neuron membranes . These findings support the concept that rHIgM12 interacts with or binds to a transmembrane cascade which conveys extracellular signal to microtubules. The dynamic property of microtubules to grow and retract, for example in the growth cones, enables them to drive neuron membrane movement (Dent and Kalil, 2001). The stabilized microtubules anchor the neuron membranes. The existence of dynamic and stabilized microtubules and/or the transition between these two states orchestrate the process of neurite outgrowth to specify an axon. Accordingly, IgM12 enhances axon extension by effecting microtubule stability.

Lipids and proteins are continually incorporated into cell membranes which are then partitioned into microdomains, the so-called lipid rafts. Membrane rafts are generally defined as nano scale (10-200 nm), heterogeneous, and dynamic membrane compartments enriched with sterol and sphingolipid (Lingwood and Simons).

We propose that rHIgM12 binds to lipid rafts. First, we observed that rHIgM12 aggregated on neuron membranes and induced the clustering of cholesterol or ganglioside, GM1 (FIGURE 20). These results indicate that rHIgM12 binds to lipid raft domains containing cholesterol and GM1. Small rafts can interact with lipids and/or proteins. The individual tiny rafts can stabilize and fuse into larger platforms which integrate signals and regulate the intensity and amplitude of signaling pathways. IgM antibodies, which possess a pentameric structure, may amplify signals by crosslinking adjacent antigens (receptors) and/or bringing the antigens close enough to enhance crosslinking or interaction. Thus, the crosslinked antigens and the associated signaling molecules may cluster. Secondly, we showed that rHIgM12 bound to much bigger puncta on neuron membranes when the hippocampal neurons were cooled down to 4°C (FIGURE 21). Cholesterol and sphingolipid have high melting temperature (Tm). Treatment of neurons at the temperature below Tm may decrease membrane dynamics, which facilitates visualization of aggregated lipid rafts. Together with the finding that rHIgM12 colocalized with GM1 even after cholesterol depletion (FIGURE 21), these results suggest that rHIgM12 binds to and interacts with neuron membrane molecules which colocalize with GM1. The precise identity is not clear, though our previous finding, that sialidase treatment of cultured rat cerebellar granule neurons abolished sHIgM12 binding, suggested that the sHIgM12-bound epitope is a carbohydrate (Warrington et al., 2004). Thirdly, after sucrose fractionation of neuron lysates, the membrane-bound rHIgM12 localized into the lighter fraction, which also contained the lipid raft marker, caveolin-1. Taken together, our results support the hypothesis that rHIgM12 binds to lipid rafts.

Signal cascades, from the outer leaflet of neuron membranes to the intracellular cytoskeleton, are central to axon specification. rHIgM12 aggregates were distributed to the neurite shaft and the growth cone central domain (FIGURE 20), which are dominated by microtubules, but not filamentous actin (FIGURE 23). Some of the rHIgM12 aggregates were detergent insoluble and localized in fasciculated microtubule bundles after detergent extraction (FIGURE 23A). These observations indicate the existence of another pool of rHIgM12-bound molecules, which may be different from the one that associated lipid rafts. The sucrose fractionation study further confirmed that there exists a pool of rHIgM12-associated molecules localizing in the detergent-insoluble pellet (FIGURE 22B), which may be the pool that localized with microtubules as detected in FIGURE 23A.

These results demonstrated that some of rHIgM12 associates with cytoskeletal component(s), possibly microtubules. The finding that rHIgM12, which associated with "detergent-soluble" molecules in the supernatant (FIGURE 22A & FIGURE 23B), was co-immunoprecipitated with β3-tubulin (FIGURE 23B), indicates that rHIgM12-bound molecule(s) and β3-tubulin may exist as a complex in lipid rafts. However, it is not likely that rHIgM12 interacts with β3-tubulin directly because rHIgM12 did not pull down β3-tubulin in N2A neuroblastoma cells which rHIgM12 does not bind (FIGURE 25). Thus both the cytoskeleton and lipid raft-associated rHIgM12 binds to molecules which localize in the vicinity of tubulin. This concept is further strengthened by observations that rHIgM12 colocalized with GM1 (FIGURE 21C), and that GM1 was pulled down by an anti-tubulin antibody after a crosslinking reaction (Palestini et al., 2000).

The rHIgM12-bound molecules in the lipid raft domains may be incorporated constantly into the cytoskeletons during the process of neurite outgrowth and axon extension. This hypothesis is supported by the findings that, at the growth cone region, rHIgM12 aggregated in the central domain (Fig. 3 & 6A), and was detergent insoluble at 37°C (FIGURE 23A). Taken together, it is disclosed that HIgM12 binds to and affects the dynamics of lipid rafts which mediate the signaling from HIgM12 to microtubules.

Whether F-actin is involved in rHIgM12-induced signaling is not clear. A number of lines of evidences indicate that both actin and actin-binding proteins are associated with lipid rafts (Levitan and Gooch, 2007). Actin filaments and microtubules actively interact in coordinated cell movement (Rodriguez et al., 2003) which also include neurons. Thus it is likely that actin also plays a role in rHIgM12-induced signaling. Along with β3-tubulin, a small amount of actin was pulled down by rHIgM12 (FIGURE 26B). Thus rHIgM12 binds lipid rafts which contain both actin and β3-tubulin.

However, rHIgM12-labeled puncta remained in the very edge of the growth cone, whereas F-actin networks shrunk from the growth cone periphery when the neurons were treated at 4°C (FIGURE 26A). After sucrose gradient fractionation, actin mainly localized in the nonraft fraction, and only a small amount of actin was detected in the detergent-insoluble pellet (FIGURE 22B). These observations indicate that F-actin is very dynamic and the majority is depolymerized at cold conditions and/or by detergent extraction. Thus, actin may be involved in rHIgM12 mediated signaling.

We conclude that the human IgM rHIgM12 binds to and reorganizes lipid rafts, and that microtubules are one of the downstream targets. rHIgM12 promotes axon extension only when presented as substrate. As a substrate, rHIgM12 was immobilized and constrained in its interaction with the neuronal membrane. The immobilized rHIgM12 may have created a gradient of signals across the neuronal membranes as frequently observed in morphogen-induced signaling (Schmitt et al., 2006). In contrast, bath application of rHIgM12 may only facilitate random lipid raft clustering (FIGURE 20) and may not be sufficient to break symmetric neurite outgrowth and enhance axon extension. Briefly, our results support the model proposed in Figure 24. Neuron membranes contain both raft and nonraft microdomains. There are two pools of raft domains with one of them associated with microtubules (FIGURE 24A). 2) rHIgM12 binds to and clusters raft domains, which promotes microtubule stabilization and anchoring the membrane (FIGURE 24B). 3) At the growth cone, rHIgM12-induced raft clustering may enhance the growth cone periphery to central domain transition, breaking symmetric neurite outgrowth to specify axon formation (FIGURE 24C).

### REFERENCES

Allen JA, Halverson-Tamboli RA, Rasenick MM (2007) Lipid raft microdomains and neurotransmitter signalling. Nat Rev Neurosci 8:128-140.
Barnes AP, Polleux F (2009) Establishment of axon-dendrite polarity in developing neurons. Annu Rev Neurosci 32:347-381.
Chen ZL, Haegeli V, Yu H, Strickland S (2009) Cortical deficiency of laminin gamma1 impairs the AKT/GSK-3beta signaling pathway and leads to defects in neurite outgrowth and neuronal migration. Dev Biol 327: 158-168.
Chichili GR, Rodgers W (2009) Cytoskeleton-membrane interactions in membrane raft structure. Cell Mol Life Sci 66:2319-2328.
Conde C, Caceres A (2009) Microtubule assembly, organization and dynamics in axons and dendrites. Nat Rev Neurosci 10:319-332.
de Anda FC, Pollarolo G, Da Silva JS, Camoletto PG, Feiguin F, Dotti CG (2005) Centrosome localization determines neuronal polarity. Nature 436:704-708.
Dent EW, Kalil K (2001) Axon branching requires interactions between dynamic microtubules and actin filaments. J Neurosci 21:9757-9769.
Dent EW, Callaway JL, Szebenyi G, Baas PW, Kalil K (1999) Reorganization and movement of microtubules in axonal growth cones and developing interstitial branches. J Neurosci 19:8894-8908.
Dotti CG, Sullivan CA, Banker GA (1988) The establishment of polarity by hippocampal neurons in culture. J Neurosci 8:1454-1468.
Forscher P, Smith SJ (1988) Actions of cytochalasins on the organization of actin filaments and microtubules in a neuronal growth cone. J Cell Biol 107:1505-1516.
Goslin K, Banker G (1989) Experimental observations on the development of polarity by hippocampal neurons in culture. J Cell Biol 108:1507-1516.
Herdegen T, Skene P, Bahr M (1997) The c-Jun transcription factor--bipotential mediator of neuronal death, survival and regeneration. Trends Neurosci 20:227-231.
Higginbotham HR, Gleeson JG (2007) The centrosome in neuronal development. Trends Neurosci 30:276-283.
Kanai Y, Hirokawa N (1995) Sorting mechanisms of tau and MAP2 in neurons: suppressed axonal transit of MAP2 and locally regulated microtubule binding. Neuron 14:421-432.
Ko M, Zou K, Minagawa H, Yu W, Gong JS, Yanagisawa K, Michikawa M (2005) Cholesterol-mediated neurite outgrowth is differently regulated between cortical and hippocampal neurons. J Biol Chem 280:42759-42765.
Levitan I, Gooch KJ (2007) Lipid rafts in membrane-cytoskeleton interactions and control of cellular biomechanics: actions of oxLDL. Antioxid Redox Signal 9:1519-1534.
Lewis SA, Ivanov IE, Lee GH, Cowan NJ (1989) Organization of microtubules in dendrites and axons is determined by a short hydrophobic zipper in microtubule-associated proteins MAP2 and tau. Nature 342:498-505.
Lingwood D, Simons K Lipid rafts as a membrane-organizing principle. Science 327:46-50.
Niethammer P, Delling M, Sytnyk V, Dityatev A, Fukami K, Schachner M (2002) Cosignaling of NCAM via lipid rafts and the FGF receptor is required for neuritogenesis. J Cell Biol 157:521-532.
Palestini P, Pitto M, Tedeschi G, Ferraretto A, Parenti M, Brunner J, Masserini M (2000) Tubulin anchoring to glycolipid-enriched, detergent-resistant domains of the neuronal plasma membrane. J Biol Chem 275:9978-9985.
Rodriguez M, Warrington AE, Pease LR (2009) Invited Article: Human natural autoantibodies in the treatment of neurologic disease. Neurology 72:1269-1276.
Rodriguez OC, Schaefer AW, Mandato CA, Forscher P, Bement WM, Waterman-Storer CM (2003) Conserved microtubule-actin interactions in cell movement and morphogenesis. Nat Cell Biol 5:599-609.
Samsonov AV, Mihalyov I, Cohen FS (2001) Characterization of cholesterol-sphingomyelin domains and their dynamics in bilayer membranes. Biophys J 81:1486-1500.
Schmid RS, Maness PF (2008) L1 and NCAM adhesion molecules as signaling coreceptors in neuronal migration and process outgrowth. Curr Opin Neurobiol 18:245-250.
Schmitt AM, Shi J, Wolf AM, Lu CC, King LA, Zou Y (2006) Wnt-Ryk signalling mediates medial-lateral retinotectal topographic mapping. Nature 439:31-37.
Segal RA (2003) Selectivity in neurotrophin signaling: theme and variations. Annu Rev Neurosci 26:299-330.
Shogomori H, Futerman AH (2001) Cholera toxin is found in detergent-insoluble rafts/domains at the cell surface of hippocampal neurons but is internalized via a raft-independent mechanism. J Biol Chem 276:9182-9188.
Sorice M, Matarrese P, Tinari A, Giammarioli AM, Garofalo T, Manganelli V, Ciarlo L, Gambardella L, Maccari G, Botta M, Misasi R, Malorni W (2009) Raft component GD3 associates with tubulin following CD95/Fas ligation. Faseb J 23:3298-3308.
Warrington AE, Bieber AJ, Van Keulen V, Ciric B, Pease LR, Rodriguez M (2004) Neuron-binding human monoclonal antibodies support central nervous system neurite extension. J Neuropathol Exp Neurol 63:461-473.
Witte H, Bradke F (2008) The role of the cytoskeleton during neuronal polarization. Curr Opin Neurobiol 18:479-487.
Witte H, Neukirchen D, Bradke F (2008) Microtubule stabilization specifies initial neuronal polarization. J Cell Biol 180:619-632.

### EXAMPLE 12: Nanopore Optical Biosensor Assays for Human MAbs Beneficial for Spinal Cord Injury

Axon protection and repair following spinal cord injury (SCI) holds great potential as an effective strategy to prevent motor neuron loss and permanent disability. Neuron protection has been achieved using targeted trophic factors for preventing damage and promoting repair of axons following injury. These molecules were predominantly identified using selection strategies based upon in vitro systems that focused upon targeting specific small molecule neurotrophic factors.

Natural autoreactive monoclonal antibodies have demonstrated beneficial biological functions in CNS cells using multiple models of injury and disease. Antibody-mediated promotion of neuron survival, axon regeneration and functional recovery has been demonstrated *in vivo* using the mouse monoclonal IgM, IN-1 (Bregman 1995; Caroni and Schwab 1988). Similar results were obtained using immunization of spinal cord homogenate (SCH) prior to CNS damage (Ellezam 2003; Huang 1999).

Based on our data from neuron binding human antibodies including IgM12 and the remyelinating antibody IgM22, it is disclosed that human monoclonal antibody interactions that protect axons from damage by promoting neuron survival and function, and beneficial for treating CNS injury and/or disease such as SCI, are dependent upon surface plasma membrane protein-lipid bilayer interactions under pathological/physiological conditions.

Human antibodies that promote neuron survival following nerve injury or damage (e.g. in SCI) or that protect neurons from injury or death, in each instance resulting in preservation of neurological function, are assessed, characterized and/or identified using technologies that maintain the pathological/physiological surface plasma membrane protein-lipid bilayers.

In this study, we determine surface binding interaction kinetics of the human monoclonal antibodies using protein-lipid bilayer surface plasmon resonance (SPR) sensors. Human monoclonal antibodies are characterized or identified using binding in spinal cord lesions of *ex vivo* tissue sections from rodents following spinal cord injury. Next, a rodent spinal cord crush injury model is used to further assess the ability of human antibodies to promote neuron survival, neurite extension or remyelination *in vivo* resulting in reduced pathology and improved neurologic function.

A novel SPR lipid bilayer sensor provides a rapid, label-free technology allowing characterization of surface protein-lipid binding kinetics and affinity of beneficial human IgM antibodies. This surface plasmon resonance (SPR) sensor technology was developed based on periodic nanopore arrays in metallic films combined with physiological planar lipid bilayer. The SPR technology has allowed rapid, label-free characterization of IgM antibody binding kinetics and affinity with relevant antigens.

Binding kinetics important to quantify small differences provide a rationale for selecting lead molecules during development and impact both the dosing and potency of a therapeutic molecule in vivo. SPR has become an accepted standard in industrial and academic settings, in which typically the molecular interaction between a pair of soluble binding partners are characterized. However, the technology is only beginning to be adapted to the needs of membrane-bound antigens. The gold substrate used in SPR sensing is not amenable to the formation of supported lipid membrane. Furthermore, membrane proteins in direct contact with the gold surface often lose their functionality. However, the development of novel nanopore-sensing architecture technology utilizing a thin gold film perforated with periodic arrays of nanopores has overcome these challenges (see, e.g., FIGURE 27). Each nanopore sits on a glass substrate and forms a tiny well to confine the supported lipid membranes, while the surrounding gold film provides SPR effects to dynamically monitor binding of molecules onto the membrane (FIGURE 27B). Nanopore arrays milled in a gold film provide a unique geometry, since a thin lipid bilayer can be suspended over the nanopores while maintaining mechanical stability and being surrounded by a buffer on both sides (FIGURE 27B).

Membrane proteins can thereby be seamlessly integrated with the SPR sensing capability of gold nanopore arrays, maintaining their functionality in an environment that more closely mimics their natural state. Furthermore, membrane proteins integrated in the free-standing lipid bilayer can be easily accessed from both sides, making this approach more attractive for studying the mechanism by which antigen-antibody binding on the cell surface results in cell signaling (FIGURE 27B). This SPR lipid bilayer sensor provides a rapid, label-free technology that is used for the assessment, identification and characterization of surface protein-lipid binding kinetics and affinity of therapeutic human IgM antibodies.

This sensor platform characterizes surface protein-lipid binding kinetics and affinity of therapeutic IgM antibodies for testing in an animal model of spinal cord injury. We have extensively characterized certain IgM antibodies identified by binding to the "live" unfixed surface of cerebella tissue and cells in the CNS. These IgM antibodies (exemplified by IgM22 and IgM46) stimulated calcium flux *in vitro* and promoted remyelination *in vivo.* Based upon the same criteria, human antibodies have been identified by screening dysproteinemia serum bank samples at the Mayo Clinic. Additional human IgM antibodies (exemplified herein by IgM12 and IgM42) bind to the surface of neurons and promoted neurite outgrowth and prevented neuron death *in vitro.*

The neuron-binding antibodies exhibited specific immunoreactivity to specific CNS cells, structures, of injury and disease pathology using "live" unfixed cortical sections from the multiple mouse models of CNS injury and disease.

Natural human monoclonal antibodies have been shown to potentiate beneficial biological functions in CNS cells from multiple models of injury and disease. Human monoclonal antibodies bind to the surface of a wide variety of neurons in culture including cerebella, cortical, retinal ganglion and spinal cord neurons (for instance human antibodies IgM12 and IgM42). These antibodies induce neurite extension from CNS neurons (FIGURE 28) and override the inhibition of CNS myelin on neurite outgrowth (Warrington et al. 2004).

The *in vitro* studies demonstrate binding of human monoclonal antibodies to surface plasma membrane of neurons. The characteristic binding is uniform at 0° C while surface rearrangements form punctate structures at 15° C (FIGURE 29). The cell surface binding are characteristic of membrane microdomain clustering associated with signaling molecules initiating signal cascades (Howe et al. 2004).

Human monoclonal antibodies enter the spinal cord tissue and accumulate at sites of damage 4 hours after intravenous administration (FIGURE 30). Immunocytochemistry performed on vibratome sections of spinal cord detects human IgM within lesions after administration of neuron binding antibodies, but not after the administration of a control human IgM. Mice with chronic spinal cord disease (TMEV infected mice) were given 0.5 mg of sHIgM42 intraperitoneally, the spinal cords removed 4 hr later, and the presence of human IgM detected in spinal cord cross sections (FIGURE 30). Injured areas of spinal cord from a mouse given sHIgM42 demonstrate parallel fibers that bind a fluorescently tagged anti-human IgM (FIGURE 30A). Injured areas of spinal cord from a mouse given a control human IgM do not contain human IgM (FIGURE 30B).

Human monoclonal antibodies have been identified using biological function assays to screen samples from the Mayo Clinic dysproteinemia serum bank. The serum samples, 115,000 collected over 40 years, contain high concentrations of monoclonal antibody from patients with monoclonal gammopathy. We have synthesized and tested recombinant human IgM 22, which has remyelination promoting activity in animal models (Mitsunaga 2002; Warrington 2000).

In one embodiment, the present Example generates GMP-quality monoclonal antibody useful for therapeutic, prognostic, diagnostic and/or prophylactic applications in conditions of nerve degeneration, nerve injury and/or damage, such as in human SCI or in demyelinating conditions that expose CNS neurons.

These studies further characterize and assess human IgM antibodies that promote neuron survival following traumatic SCI resulting in preservation of neurological function. Behavioral tests and morphological measurements of axon numbers following SCI are evaluated and differences between endogenous and antibody-mediated preservation of neurological function are determined. Treatments with the axon-protecting and axon-extending antibodies compared to antibody controls following SCI are utilized to characterize differences of neurological function preservation for specific antibody-mediated activities.

Membrane vesicles containing the plasma membrane protein-lipid bilayer are isolated from cells under physiological conditions. Nanopores of the SPR sensors are coated using microvesicle preparations of isolated neurons, glia, synaptosome and myelin membranes from normal CNS and spinal cord-injured tissues. Human monoclonal antibody serum samples and recombinant antibody samples are screened to determine binding kinetics for specific membrane types.

Human monoclonal antibodies that bind specifically to the surface plasma membrane of unfrozen unfixed "live" cells and tissues under physiological conditions as confirmed using SPR sensors are tested for specific binding using unfrozen, unfixed, "live" pathological tissues following SCI. Human monoclonal antibodies demonstrating binding to spinal cord lesions are furtherer characterized for applicability and activity in treatment following SCI in the rodent.

**Protocol Design.** Compression injury is generated at the level of T9 in ten-week old female (18-22 g) C57BL/6J mice (Jackson Laboratories) using a modified aneurysm clip (FEJOTA mouse clip, 3g or 8g closing force). This model of SCI includes not only an initial impact, but also a persistent compression phase that promotes microcystic cavitation, axon degeneration, and robust astrogliosis, all hallmarks of human traumatic SCI (Joshi and Fehlings 2002). Behavioral tests using the Basso, Beattie, Bresnahan (BBB) Locomotor Rating Scale for rats and the Basso Mouse Scale (BMS) for Locomotion are determined following SCI to evaluate neurological deficits. The technique is depicted in FIGURE 31.

Live unfrozen, unfixed *ex vivo* tissue sections are then prepared from pathological lesions following spinal cord compression injury in rodents. Immunoreactivity of human IgM antibodies binding is screened using immunofluorescent (IF) staining of the sections maintained under physiological conditions.

Mice are treated acutely after a spinal cord compression injury with human antibodies, including rHIgM12 and sHIgM42 or rHIgM42, administered intraperitoneally. The ability of the human antibodies to promote axon preservation or tissue repair is measured. Behavioral tests using BBB and BMS scale are performed at regular intervals during the 5 week period following SCI. A single 0.5 mg dose of human antibody is administered i.p. to mice 30 min after SCI. Groups of mice (15 mice) receive human antibodies that promote neuron protection, neurite outgrowth, control human antibodies that do not bind to cells or control. Individual mice are housed overnight once a week in automated infrared activity boxes (Mikami et al. 2002) and spontaneous rearing, a measure of hind limb disability, and horizontal activity recorded (Accuscan Inc, Ohio). Traditional BBB scores are also collected weekly. Functional assessments are performed blinded.

Fluoro-gold and fast blue, 4 weeks after treatment, is injected into spinal cords of four mice at the lower thoracic level. One week later mice are perfused and the brains and spinal cords removed. Retrograde labeled cell bodies in the reticular, vestibular and red nuclei of the brain stem are counted across groups by fluorescent microscopy (Ure and Rodriguez 2002). The number of labeled cell bodies in the brain stem (vibratome section) reflects the level of functional axons throughout the spinal cord. Immunocytochemistry for β amyloid protein accumulation is performed on tissue cross sections taken every 1 mm along the spinal cord. The number of β amyloid aggregates in a section reflects impaired axon transport and axon dysfunction at that level.

Five weeks after injury, the remaining mice are perfused, with formaldehyde/gluteraldehyde and the spinal cords removed. Every other 1 mm block comprising the spinal cord lesion site is embedded in araldite plastic and sectioned (1 micron) and stained with paraphenylene to visualize preserved axons encircled by myelin. Area of lesion cross section, the preserved axon number and lesion immune cell infiltration are measured using Microsuite software (Olympus). Axon frequency is measured and compared across groups (McGavern et al. 2000; Rodriguez et al. 1987). All analyses are done on coded and blinded samples without knowledge of the experimental group. Preserved axons are expressed as the number of preserved axons/mm² of lesion. Pair-wise comparison of data between experimental and control groups uses the Mann-Whitney Rank Sum test.

### References

Rodriguez M, Warrington AE, Pease LR. Human Natural Autoantibodies in the Treatment of Neurologic Disease. Neurology 2009; 72: 1269-1276.
Im H, Lesuffleur A, Lindquist NC, Oh SH. Plasmonic nanoholes in a multichannel microarray format for parallel kinetic assays and differential sensing. Analytical Chemistry. 2009;81:2854-2859
Maynard JA, Lindquist NC, Sutherland JN, Lesuffleur A, Warrington AE, Rodriguez M, and Oh S-H. Next generation SPR technology of membrane-bound proteins for ligand screening and biomarker discovery. J Biotech 2009; 4(11):1542-1558
Nagpal P, Lindquist NC, Oh SH, Norris DJ. Ultrasmooth patterned metals for plasmonics and metamaterials. Science. 2009;325:594-597.
Bieber AJ, Warrington A, Asakura K, Ciric B, Kaveri SV, Pease LR, Rodriguez M. 2002. Human antibodies accelerate the rate of remyelination following lysolecithin-induced demyelination in mice. Glia 37(3):241-249.
Bregman BS, Kunkel-Bagden E, Schnell L, Dai HN, Gao D, Schwab ME. 1995. Recovery from spinal cord injury mediated by antibodies to neurite growth inhibitors. Nature 378(6556):498-501.
Caroni P, Schwab ME. 1988. Antibody against myelin-associated inhibitor of neurite growth neutralizes nonpermissive substrate properties of CNS white matter. Neuron 1(1):85-96.
Corse AM, Bilak MM, Bilak SR, Lehar M, Rothstein JD, Kuncl RW. 1999. Preclinical testing of neuroprotective neurotrophic factors in a model of chronic motor neuron degeneration. Neurobiol Dis 6(5):335-346.
Ellezam B, Bertrand J, Dergham P, McKerracher L. 2003. Vaccination stimulates retinal ganglion cell regeneration in the adult optic nerve. Neurobiol Dis 12(1):1-10.
Estevez AG, Spear N, Manuel SM, Radi R, Henderson CE, Barbeito L, Beckman JS. 1998. Nitric oxide and superoxide contribute to motor neuron apoptosis induced by trophic factor deprivation. J Neurosci 18(3):923-931.
Festoff BW, Ameenuddin S, Arnold PM, Wong A, Santacruz KS, Citron BA. 2006. Minocycline neuroprotects, reduces microgliosis, and inhibits caspase protease expression early after spinal cord injury. J Neurochem 97(5): 1314-1326.
Hemendinger RA, Armstrong EJ, 3rd, Persinski R, Todd J, Mougeot JL, Volvovitz F, Rosenfeld J. 2008. Huperzine A provides neuroprotection against several cell death inducers using in vitro model systems of motor neuron cell death. Neurotox Res 13(1):49-61.
   Howe CL, Bieber AJ, Warrington AE, Pease LR, Rodriguez M. 2004. Antiapoptotic signaling by a remyelination-promoting human antimyelin antibody. Neurobiol Dis 15(1):120-131.
Huang DW, McKerracher L, Braun PE, David S. 1999. A therapeutic vaccine approach to stimulate axon regeneration in the adult mammalian spinal cord. Neuron 24(3):639-647.
Joshi M, Fehlings MG. 2002. Development and characterization of a novel, graded model of clip compressive spinal cord injury in the mouse: Part 1. Clip design, behavioral outcomes, and histopathology. J Neurotrauma 19(2): 175-190.
McGavern DB, Murray PD, Rivera-Quinones C, Schmelzer JD, Low PA, Rodriguez M. 2000. Axonal loss results in spinal cord atrophy, electrophysiological abnormalities and neurological deficits following demyelination in a chronic inflammatory model of multiple sclerosis. Brain 123 Pt 3:519-531.
McTigue DM, Tripathi R, Wei P. 2006. NG2 colocalizes with axons and is expressed by a mixed cell population in spinal cord lesions. J Neuropathol Exp Neurol 65(4):406-420.
Mikami Y, Toda M, Watanabe M, Nakamura M, Toyama Y, Kawakami Y. 2002. A simple and reliable behavioral analysis of locomotor function after spinal cord injury in mice. Technical note. J Neurosurg 97(1 Suppl): 142-147.
Mitsunaga Y, Ciric B, Van Keulen V, Warrington AE, Paz Soldan M, Bieber AJ, Rodriguez M, Pease LR. 2002. Direct evidence that a human antibody derived from patient serum can promote myelin repair in a mouse model of chronic-progressive demyelinating disease. Faseb J 16(10):1325-1327.
Pannu R, Christie DK, Barbosa E, Singh I, Singh AK. 2007. Post-trauma Lipitor treatment prevents endothelial dysfunction, facilitates neuroprotection, and promotes locomotor recovery following spinal cord injury. J Neurochem 101(1):182-200.
Pirko I, Ciric B, Gamez J, Bieber AJ, Warrington AE, Johnson AJ, Hanson DP, Pease LR, Macura SI, Rodriguez M. 2004. A human antibody that promotes remyelination enters the CNS and decreases lesion load as detected by T2-weighted spinal cord MRI in a virus-induced murine model of MS. Faseb J 18(13):1577-1579.
Rodriguez M, Lennon VA, Benveniste EN, Merrill JE. 1987. Remyelination by oligodendrocytes stimulated by antiserum to spinal cord. J Neuropathol Exp Neurol 46(1):84-95.
Warrington AE, Asakura K, Bieber AJ, Ciric B, Van Keulen V, Kaveri SV, Kyle RA, Pease LR, Rodriguez M. 2000. Human monoclonal antibodies reactive to oligodendrocytes promote remyelination in a model of multiple sclerosis. Proc Natl Acad Sci U S A 97(12):6820-6825.
Warrington AE, Bieber AJ, Ciric B, Pease LR, Van Keulen V, Rodriguez M. 2007. A recombinant human IgM promotes myelin repair after a single, very low dose. J Neurosci Res 85(5):967-976.
Warrington AE, Bieber AJ, Van Keulen V, Ciric B, Pease LR, Rodriguez M. 2004. Neuron-binding human monoclonal antibodies support central nervous system neurite extension. J Neuropathol Exp Neurol 63(5):461-473.

### Example 13: Multiple Sclerosis Model - Dose:Response Evaluation

IgM antibodies are evaluated for their ability to promote functional improvement and augment the processes of remyelination, neurite outgrowth or both in a multiple sclerosis model in mice. The model involves the use of the picornavirus Theiler's Murine Encephalomyelitis Virus (TMEV) to induce persistent chronic, progressive demyelinating lesions of the CNS with characteristics similar to those found in human MS using a method similar to Warrington et al., 2007.

Female and male mice (approximately 8 w, SJL/J strain) are injected with Theiler's Murine Encephalomyelitis Virus (2 x 10⁵ plaque forming units of Daniel's strain, 10 µL intracerebroventricularly). Mice are allowed to recover for a period of 6 months prior to randomization for treatment. Mice are then examined for coat condition, gait and righting reflex and randomly assigned to treatment groups (as indicated in treatment tables). Mice are then treated with a single intravenous injection of vehicle (normal saline) or recombinant human IgM antibody (0.025 to 2.5 mg/kg). Neurological function is monitored at 2 weeks, 1 month and 2 months after treatment.

Functional endpoints included scoring based on coat condition, gait, and Rotarod performance. For coat appearance, scoring is as follows: no disease (0); minimal coat changes (1); scruffy coat (2); incontinence and stained coat (3). Activity changes are quantitated in an automated activity box. Further, gait is analyzed using digital gait acquisition (DigiGait) using walking speeds > 90 cm/s. Quantitative gait analysis endpoints include stance width and duration, stride length and frequency, paw angle as well as swing, braking and propulsion duration. Gait changes from baseline are quantitated. Rotarod performance (a measure of sensory and balance coordination is assessed by the ability of animal to walk on a rotating shaft measuring the motor in terms of speed and duration on the rotating shaft) is quantitated as the amount of time animals remain on the rotating apparatus over a series of trials.

At the completion of the experiment, animals are euthanized by overexposure to CO₂. Brains and spinal cords are removed for evaluation of 1) degree of myelination (electron microscopic analysis of plastic-embedded paraformaldehyde/glutaraldehyde fixed osmicated tissue) and 2) neurite growth (assessment of the density of neurites by stereological assessment).

In the first embodiment, recombinant human antibodies are administered alone at three dose levels as indicated in Table 3A and Table 3B.

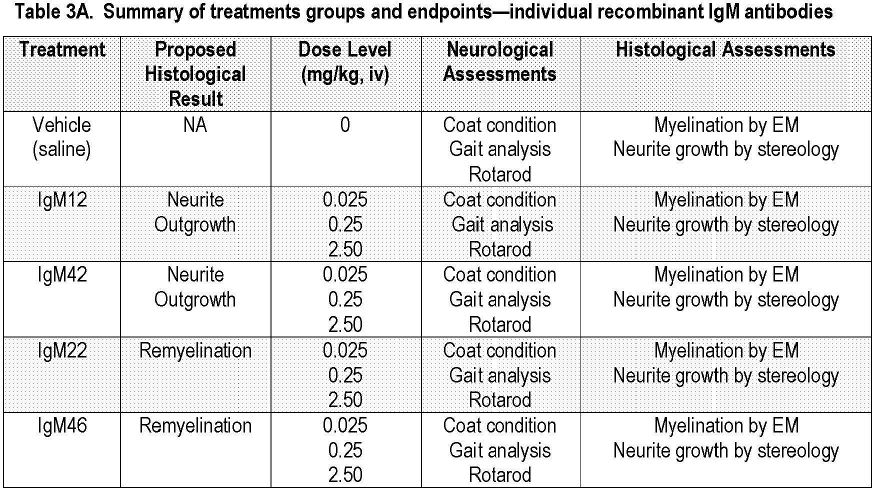

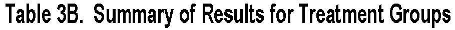

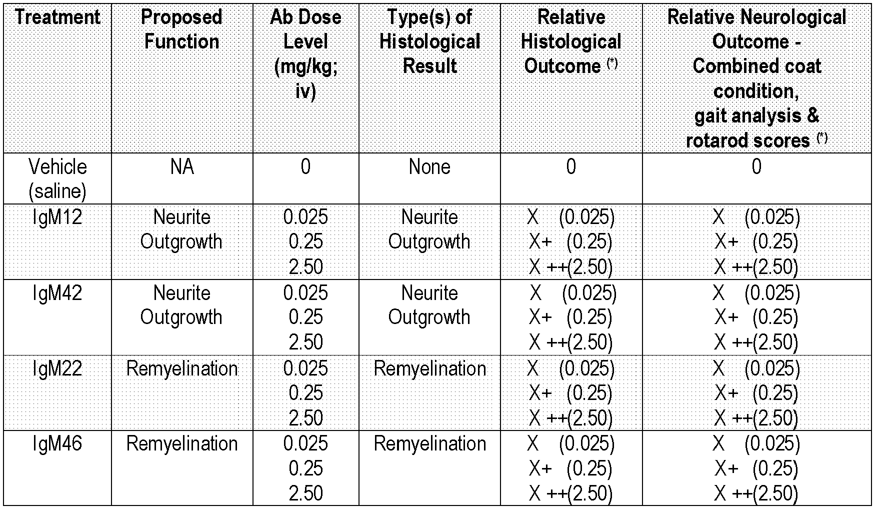

(*) X indicates improvement, X+ indicates more improvement, and X++ indicates even more improvement. The improvement score values are relative to the dosing of a given antibody. Thus an X+ is a greater improvement than X for that same Ab. The improvement X for one Ab is not necessarily the same as the X value for another Ab; the X+ value of one Ab is not necessarily the same as the X+ value of another Ab, and the X+++ value of one Ab is not necessarily the same as the X+++ value of another Ab.

In this example, the recombinant antibodies IgM12, IgM 42, IgM22 and IgM46 are each found to produce statistically significant, dose-dependent improvements in various sensorimotor endpoints including coat condition, gait parameters and Rotarod score (p < 0.05) for each endpoint compared to vehicle treatment). Animals treated with vehicle yield a stable deficits in various sensorimotor endpoints including coat condition, gait parameters and Rotarod score within 6 months of TMEV injury. A significant, dose-dependent change in sensorimotor endpoints over the dose range from 0.025-2.5 mg/kg, where X is an improvement, are found for each Ab, with an improvement X increase in sensorimotor endpoints at the low dose, a greater improvement X+ increase in sensorimotor endpoints with 0.25 mg/kg and an even greater improvement X+++ in sensorimotor endpoints at the 2.5 mg/kg dose. This trend is observed for group sizes of 12 animals powered at a level of 0.8 and an alpha level of 0.05. Thus, each of antibodies IgM12, IgM 42, IgM22 and IgM46 produce statistically significant, dose-dependent improvements in various sensorimotor endpoints including coat condition, gait parameters and Rotarod score (p < 0.05 for each endpoint) compared to vehicle treatment. No distinctions in improvement are found based on gender.

In this Example, IgM12 and IgM42 are found to produce statistically significant, dose-dependent increases in neurite outgrowth in brain and spinal cord sections from TMEV infected mice as assessed by stereology compared to vehicle-treated controls. Significant, dose-dependent changes in neurite outgrowth over the dose range from 0.025-2.5 mg/kg, where X is an improvement, are found for each Ab, with an improvement X increase in neurite outgrowth at the low dose, a greater improvement X+ increase in neurite outgrowth with 0.25 mg/kg and an even greater increase in neurite outgrowth X+++ at the 2.5 mg/kg dose. This trend is observed for group sizes of 12 animals powered at a level of 0.8 and an alpha level of 0.05.

IgM22 and IgM46 are found to produce statistically significant, dose-dependent increases in remyelination in brain and spinal cord sections from TMEV infected mice as assessed by stereology compared to vehicle-treated controls. Significant, dose-dependent changes in remyelination over the dose range from 0.025-2.5 mg/kg, where X is an improvement, are found for each Ab, with an improvement X increase in remyelination at the low dose, a greater improvement X+ increase in remyelination with 0.25 mg/kg and an even greater increase in remyelination X+++ at the 2.5 mg/kg dose. This trend is observed for group sizes of 12 animals powered at a level of 0.8 and an alpha level of 0.05.

### Example 14: Multiple Sclerosis Model - Ab combinations

In this experiment, various combinations of fixed dose ratios of recombinant IgM antibodies are examined for improvement of neurological outcome in the TMEV model of MS as described in the previous example. Six months after exposure to TMEV, treatment with recombinant IgM combinations is initiated in mice. In this series of studies, mice are given either vehicle alone, or various combinations of IgMs, in a single intravenous administration (admixture). Sensorimotor endpoints are monitored as above. Myelination and neurite growth are evaluated to examine the changes produced by antibody combinations. The combinations under evaluation are shown in Table 4A and Table 4B.

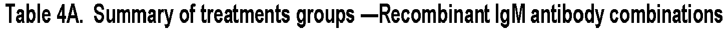

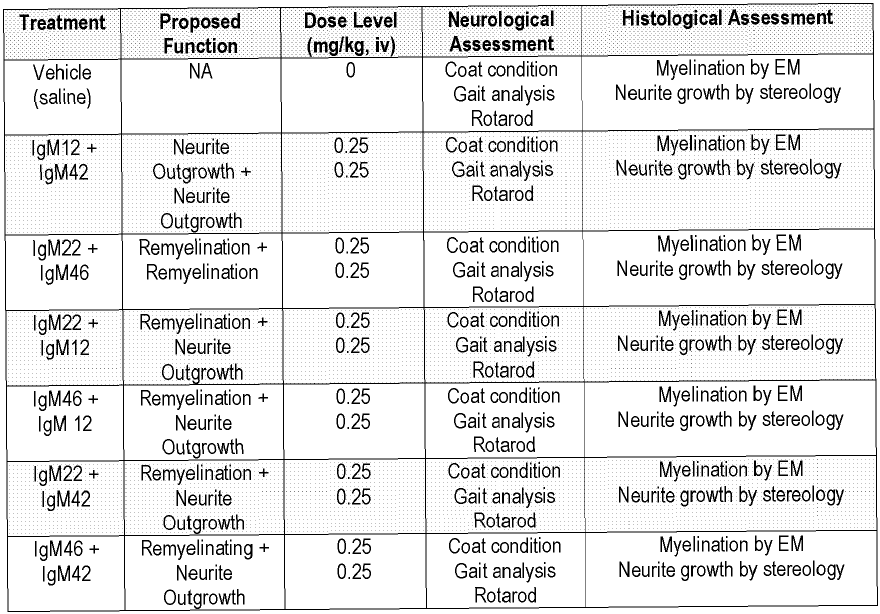

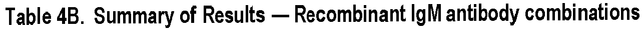

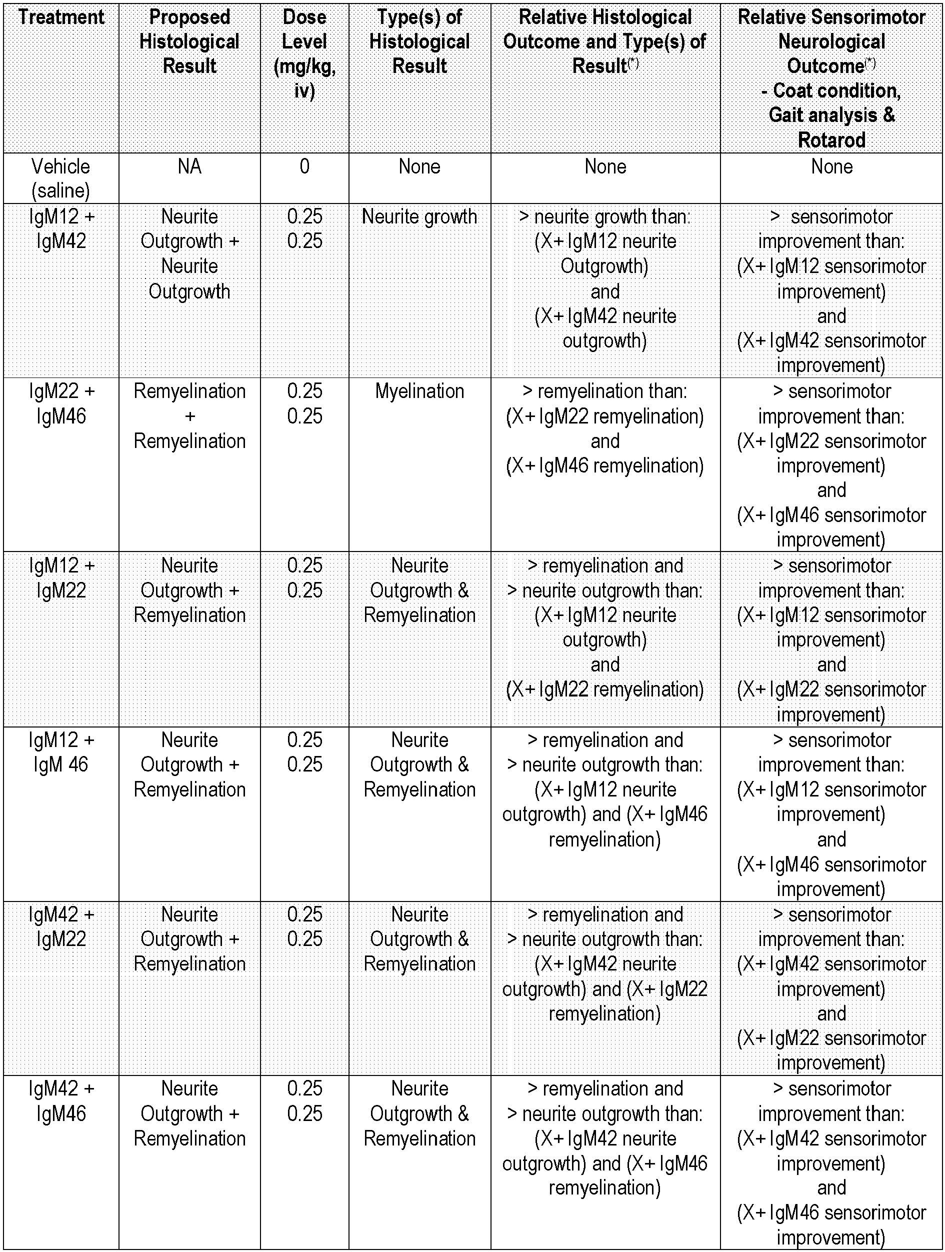

(*) X indicates improvement, X+ indicates more improvement, and X++ indicates even more improvement. The improvement score values are relative to the dosing of a given antibody. Thus an X+ is a greater improvement than X for that same Ab. The improvement X for one Ab is not necessarily the same as the X value for another Ab; the X+ value of one Ab is not necessarily the same as the X+ value of another Ab, and the X+++ value of one Ab is not necessarily the same as the X+++ value of another Ab.

In this Example, the recombinant antibody combinations of IgM12 + IgM42, IgM22 + IgM46, IgM12 + IgM22, IgM12 + IgM46, IgM42 + IgM22, and IgM42 + IgM46 produce statistically significant, dose-dependent improvements in various sensorimotor endpoints including coat condition, gait parameters and Rotarod score compared to vehicle treatment (p < 0.05 for each endpoint compared to vehicle treatment). No distinctions in improvement are found based on gender.

Further, the degree of sensorimotor endpoint improvement with each of these combinations is supra-additive (exhibited synergism) in a statistically significant, dose-dependent manner compared to the predicted additive improvements of each of the antibodies alone. Again, no distinctions in improvement are found based on gender.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12+IgM42 significantly enhanced neurite outgrowth compared to vehicle controls (p < 0.05). The degree of neurite outgrowth is supraadditive compared to the additive effect of each antibody administered alone. Thus, it is understood that these antibodies elicit outgrowth by distinct mechanisms of action; this is consistent with the respective distinct binding patterns of these antibodies to neural tissue.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM22 + IgM 46 produced a significant increase in myelination compared to vehicle-treated controls (p < 0.05). Further, the degree of remyelination is supraadditive compared to the predicted additive effect of IgM22 or IgM46 administered alone. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12 + IgM 22 produce a significant increase in myelination as well as neuron growth compared to vehicle-treated controls in the injured spinal cord (p < 0.05). Further, the degree of neuron growth and remyelination is supraadditive compared to the effect of IgM12 or IgM22, respectively, administered alone at the doses used here. Without being bound by theory, this is understood to be a result of paracrine activity. Accordingly, when growing neurons and growing oligodendrocytes are each present, these cells respectively elicit paracrine production that positively affects the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12 + IgM 46 produce a significant increase in myelination as well as neuron growth compared to vehicle-treated controls (p < 0.05). Further, the degree of neuron growth and remyelination is supraadditive compared to the effect of IgM12 or IgM46, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM22 + IgM 42 produce a significant increase in myelination as well as neuron growth compared to vehicle-treated controls (p < 0.05). Further, the degree of neuron growth and remyelination is supraadditive compared to the effect of IgM22 or IgM42, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM46 + IgM 42 produce a significant increase in myelination as well as neuron growth compared to vehicle-treated controls (p < 0.05). Further, the degree of neuron growth and remyelination is supraadditive compared to the effect of IgM46 or IgM42, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

### Example 15: Spinal Cord Injury Model

IgM antibodies are evaluated for their ability to promote functional improvement and augment the processes of remyelination, neurite outgrowth or both in a spinal cord injury model in rats. The model involves laterally compressing the spinal cord between the blades of modified cover slip forceps, where the width of the blade of forceps is 2.5mm, to a pre-set separation of the blades to 0.9mm for a period of 15 seconds. The resulting lesion has characteristics similar to those found in human SCI (Gruner et al., 1995)

Male and female rats (approximately 200-225 g, Long Evans) undergo surgery to produce a spinal cord injury as described above. Recombinant IgM antibodies or vehicle treatment are given intravenously 10 min after surgery. Hind limb locomotor function and gait are evaluated for a period of 12 weeks using the BBB locomotor rating scale (see, e.g., Basso DM, Beattie MS, Bresnahan JC, Anderson DK, Faden Al, Gruner JA, Holford TR, Hsu CY, Noble LJ, Nockels R, Perot PL, Salzman SK, Young W. (1996) MASCIS evaluation of open field locomotor scores: Effects of experience and teamwork on reliability. Journal of Neurotrauma, 13:343-359; Basso DM, Beattie MS, Bresnahan JC. (1995) A sensitive and reliable locomotor rating scale for open field testing in rats. Journal of Neurotrauma, 12:1-21) Rats are tested at 1, 3, 5, 7 and 10 days, and then weekly following SCI for 9-12 weeks on the BBB Changes from baseline are quantitated.

At the completion of the experiment, animals are euthanized by overexposure to CO₂. Brains and spinal cords are removed for evaluation of 1) degree of myelination (electron microscopic analysis of plastic-embedded paraformaldehyde/glutaraldehyde fixed osmicated tissue) and 2) neurite growth (assessment of the density of neurites by stereological assessment).

### Example 16: Use of Individual Antibodies in a Spinal Cord Injury Model

In this experiment, recombinant human antibodies are administered alone at three dose levels as indicated in Table 5A and Table 5B.

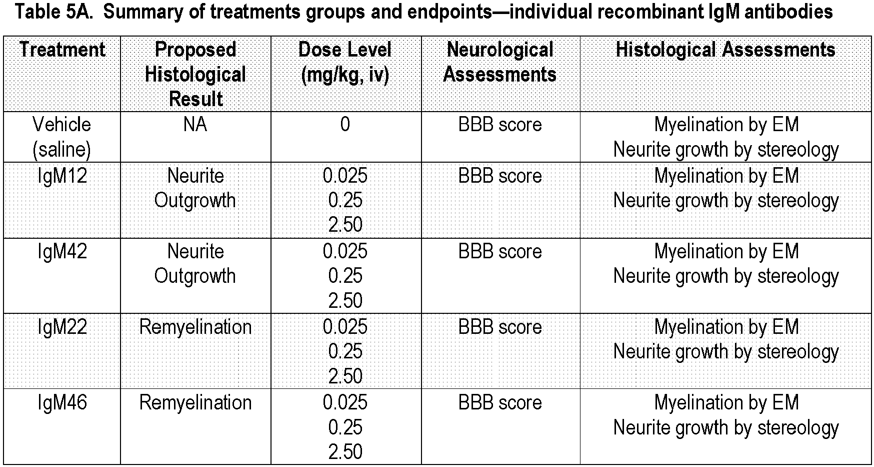

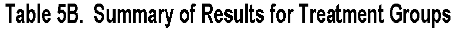

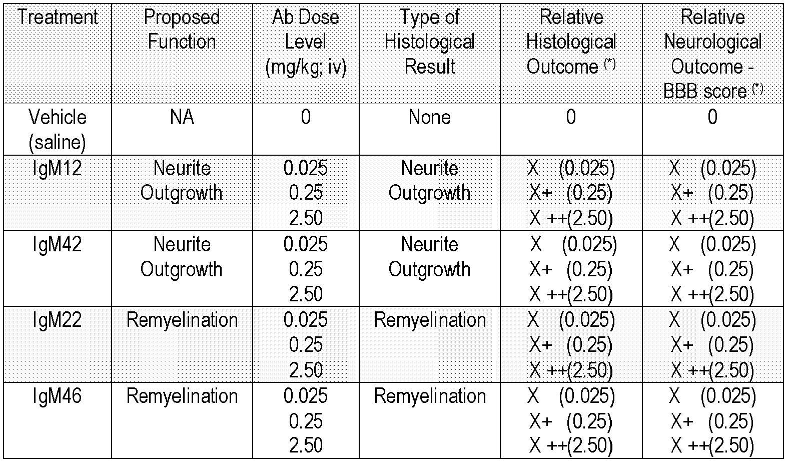

(*) X indicates improvement, X+ indicates more improvement, and X++ indicates even more improvement. The improvement score values are relative to the dosing of a given antibody. Thus an X+ is a greater improvement than X for that same Ab. The improvement X for one Ab is not necessarily the same as the X value for another Ab; the X+ value of one Ab is not necessarily the same as the X+ value of another Ab, and the X+++ value of one Ab is not necessarily the same as the X+++ value of another Ab.

In this example, the recombinant antibodies IgM12, IgM 42, IgM22 and IgM46 are each found to produce statistically significant, dose-dependent improvements in hind limb locomotor function with improved BBB parameters (p < 0.05 for each endpoint compared to vehicle treatment). Animals treated with vehicle yield a stable BBB score within 6 weeks of a moderate level of spinal cord injury. A significant, dose-dependent change in BBB score over the dose range from 0.025-2.5 mg/kg, where X is an improvement, are found for each Ab, with an improvement X increase in BBB level at the low dose, a greater improvement X+ BBB increase with 0.25 mg/kg and an even greater BBB improvement X+++ at the 2.5 mg/kg dose. This trend is observed for group sizes of 12 animals powered at a level of 0.8 and an alpha level of 0.05. Thus, each of antibodies IgM12, IgM 42, IgM22 and IgM46 No distinctions in improvement are found based on gender. produce statistically significant, dose-dependent improvements in hind limb locomotor function with improved BBB (p < 0.05 for each endpoint) compared to vehicle treatment.

IgM12 and IgM42 are found to produce statistically significant, dose-dependent increases in neurite outgrowth in brain and spinal cord sections from TMEV infected mice as assessed by stereology compared to vehicle-treated controls. Significant, dose-dependent changes in neurite outgrowth over the dose range from 0.025-2.5 mg/kg, where X is an improvement, are found for each Ab, with an improvement X increase in neurite outgrowth at the low dose, a greater improvement X+ increase in neurite outgrowth with 0.25 mg/kg and an even greater increase in neurite outgrowth X+++ at the 2.5 mg/kg dose. This trend is observed for group sizes of 12 animals powered at a level of 0.8 and an alpha level of 0.05.

IgM22 and IgM46 are found to produce statistically significant, dose-dependent increases in remyelination in brain and spinal cord sections from TMEV infected mice as assessed by stereology compared to vehicle-treated controls. Significant, dose-dependent changes in remyelination over the dose range from 0.025-2.5 mg/kg, where X is an improvement, are found for each Ab, with an improvement X increase in remyelination at the low dose, a greater improvement X+ increase in remyelination with 0.25 mg/kg and an even greater increase in remyelination X+++ at the 2.5 mg/kg dose. This trend is observed for group sizes of 12 animals powered at a level of 0.8 and an alpha level of 0.05.

### Example 17: Spinal Cord Injury-Antibody Combinations

In this Example, various combinations of fixed dose ratios of recombinant IgM antibodies are examined for improvement of neurological outcome in the spinal cord injury model as described in Example 14 herein. Accordingly, intravenous treatment with IgM antibody combinations (as admixtures) or vehicle control is given 10 min after injury. Locomotor endpoints are monitored as above. Further, myelination and neurite growth are evaluated to examine the changes produced by antibody combinations. The combinations evaluated are shown in Table 6A and Table 6B.

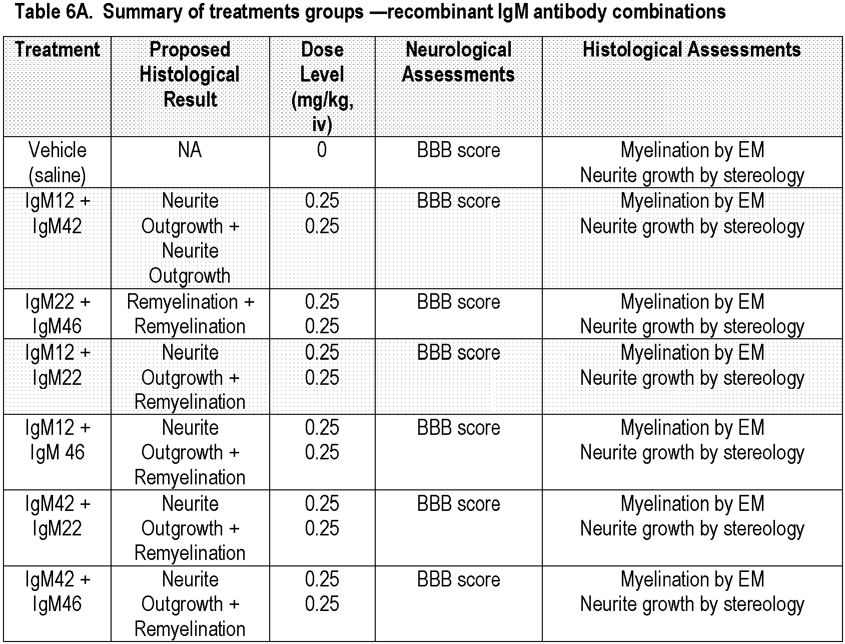

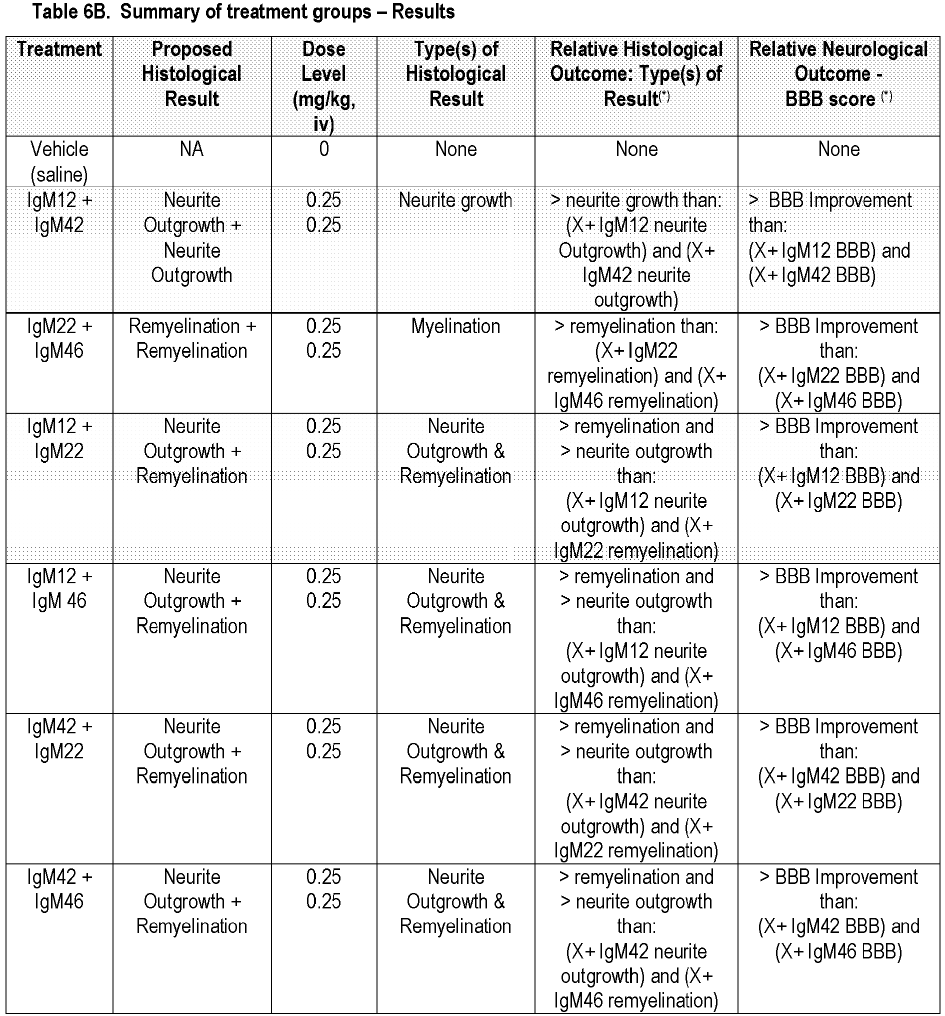

(*) X indicates improvement, X+ indicates more improvement, and X++ indicates even more improvement. The improvement score values are relative to the dosing of a given antibody. Thus an X+ is a greater improvement than X for that same Ab. The improvement X for one Ab is not necessarily the same as the X value for another Ab; the X+ value of one Ab is not necessarily the same as the X+ value of another Ab, and the X+++ value of one Ab is not necessarily the same as the X+++ value of another Ab.

In this experiment, each of recombinant antibody combinations IgM12 + IgM42, IgM22 + IgM46, IgM12 + IgM22, IgM12 + IgM46, IgM42 + IgM22, and IgM42 + IgM46 are found to produce statistically significant, dose-dependent improvements in hindlimb function (locomotor endpoints) as found by BBB parameters. No distinctions in improvement are found based on gender.

Furthermore, each of the combinations are found to improve locomotor function in a supra-additive (i.e. synergistic) manner compared to the improvements of each of the antibodies alone at the same dose. Again, no distinctions in improvement are found based on gender.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12+IgM42 significantly enhanced neurite outgrowth compared to vehicle controls (p < 0.05). The degree of neurite outgrowth is supraadditive compared to the additive effect of each antibody administered alone. Thus, it is understood that these antibodies elicit outgrowth by distinct mechanisms of action; this is consistent with the respective distinct binding patterns of these antibodies to neural tissue.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM22 + IgM 46 produced a significant increase in myelination compared to vehicle-treated controls (p < 0.05). Further, the degree of remyelination is supraadditive compared to the predicted additive effect of IgM22 or IgM46 administered alone. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12 + IgM 22 produce a significant increase in myelination as well as neuron growth compared to vehicle-treated controls in the injured spinal cord (p < 0.05). Further, the degree of neuron growth and remyelination is supraadditive compared to the effect of IgM12 or IgM22, respectively, administered alone at the doses used here. Without being bound by theory, this is understood to be a result of paracrine activity. Accordingly, when growing neurons and growing oligodendrocytes are each present, these cells respectively elicit paracrine production that positively affects the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM12 + IgM 46 produce a significant increase in myelination as well as neuron growth compared to vehicle-treated controls (p < 0.05). Further, the degree of neuron growth and remyelination is supraadditive compared to the effect of IgM12 or IgM46, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM22 + IgM 42 produce a significant increase in myelination as well as neuron growth compared to vehicle-treated controls (p < 0.05). Further, the degree of neuron growth and remyelination is supraadditive compared to the effect of IgM22 or IgM42, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

In addition to producing statistically significant, dose-dependent improvements in various sensorimotor endpoints including coat condition, gait parameters and Rotarod score compared to vehicle treatment (p < 0.05 for each endpoint compared to vehicle treatment), the combination of IgM46 + IgM 42 produce a significant increase in myelination as well as neuron growth compared to vehicle-treated controls (p < 0.05). Further, the degree of neuron growth and remyelination is supraadditive compared to the effect of IgM46 or IgM42, respectively, administered alone at the doses used here. Without being bound by theory, this is understood to be a result of paracrine activity. Accordingly, when growing neurons and growing oligodendrocytes are each present, the cells respectively elicit paracrine production that positively affect the other cell type under evaluation.

### Example 18: Recombinant rHlgM12 Antibody, a Human Neuron Binding IgM. Protects Spinal Cord Axons

We have demonstrated that a natural human serum antibody, sHIgM12, binds to neurons *in vitro* and promotes neurite outgrowth. We generated a recombinant form, rHIgM12, with identical properties. Intracerebral infection with Theiler's Murine Encephalomyelitis Virus (TMEV) of susceptible mouse strains causes chronic demyelinating disease with progressive axonal loss and neurologic dysfunction similar to progressive forms of multiple sclerosis. Treating this model with IgM class antibodies that bind to oligodendrocytes improves CNS remyelination (Warrington et al (2007) J Neurosci Res 85:967-976). In contrast, the serum-derived human monoclonal antibody (sHIgM12) that binds to neurons, promotes robust neurite outgrowth to the same degree as laminin, and reduces the inhibitory effects of CNS myelin on neurite outgrowth (Warrington et al (2004) J Neuropathol Exp Neurol 63(5):461-473). More recently a recombinant form of human sHIgM12 was generated (rHIgM12) with identical biological properties, as described above. To study the effects of rHIgM12 on the spinal cord axonal integrity we performed retrograde- labeling, a technique that relies on anatomically continuous and functionally preserved axons.

### METHODS

Retrograde Labeling: After anesthetizing mice, dorsal laminectomy was performed at the lower thoracic vertebrae (T10-11). The spinal cord was hemi-sected on the right side and the hemisection site was packed with a sterile solution of 4% Fluorogold. One week after surgery, mice were sacrificed, and the brains removed. Serial sections (40mm thick) of the brainstem were made and every fourth section was analyzed. Large, brightly fluorescent neurons were counted under 200x magnifications from 16 brainstem slices.

### RESULTS

The TMEV model offers a platform to develop strategies to promote neuroprotection and prevent axon loss. Early stages of disease include inflammation and demyelination; later stages present with axon loss and functional deficits. As detailed in the prior examples, rHIgM12 enters the spinal cord and localizes to neurofilament+ (NF) axons after a single 1mg i.p. injection, as confirmed by confocal images (FIGURE 15). rHIgM12 also colocalized as cross cut NF+fiber bundles (FIGURE 15D). In animal studies, rHIgM12 improves the number of retrograde-labeled brainstem neurons. rHIgM12 or saline were administered to SJL mice at 90 days post infection (dpi). Spinal cord surgery for retrograde labeling was performed 9 weeks following the treatment. One week after surgery, mice were sacrificed and fluorescently-labeled neurons were quantified in serial brainstem sections. rHIgM12 improves the number of retrograde-labeled brainstem neurons as compared to saline control (data not shown). Thus, rHIgM12-treated mice had more retrograde-labeled brainstem neurons as compared to saline-treated controls.

### Example 19: A Single Dose of Neuron-Binding Human Monoclonal Antibody Improves Spontaneous Activity in a Murine Model of Demyelination

Our laboratory demonstrated that a natural human serum antibody, sHIgM12, binds to neurons *in vitro* and promotes neurite outgrowth. We generated a recombinant form, rHIgM12, with identical properties. Intracerebral infection with Theiler's Murine Encephalomyelitis Virus (TMEV) of susceptible mouse strains results in chronic demyelinating disease with progressive axonal loss and neurologic dysfunction similar to progressive forms of multiple sclerosis. To study the effects of rHIgM12 on the motor function of TMEV-infected mice, we monitored spontaneous nocturnal activity over many weeks. Nocturnal behavior is a sensitive measure of rodent neurologic function because maximal activity changes are expected to occur during the normally active night time monitoring period. Mice were placed in activity boxes eight days prior to treatment to collect baseline spontaneous activity. After treatment, activity in each group was continuously recorded over 8 weeks. We chose a long 8-week monitoring period for two reasons: (1) we previously demonstrated that IgM induced remyelination is present by 5 weeks post treatment, and (2) TMEV-induced demyelinating disease in this strain progresses very slowly. Due to the long observation periods and large data sets, differences among treatment groups may be difficult to appreciate studying the original unfiltered recordings. To clearly delineate changes in the highly fluctuating original data we applied three different methods: (1) binning, (2) application of Gaussian low-pass filters (GF) and (3) polynomial fitting. Using each of the three methods we showed that compared to control IgM and saline, early treatment with rHIgM12 induced improvement in both horizontal and vertical motor function, whereas later treatment improved only horizontal activity. rHIgM12 did not alter activity of normal, uninfected mice. This study supports the hypothesis that treatment with a neuron-binding IgM not only protects neurons *in vitro,* but also influences functional motor improvement.

### Introduction

Long-term monitoring and analysis of neurological function in rodent disease models remains a challenge. Nocturnal behavior is a sensitive measure of rodent neurologic function because maximal activity changes are expected to occur during the normally active night time monitoring period [1]. However, in animal models of slowly progressive diseases performance monitoring often extends over several weeks. We previously reported that oligodendrocyte-binding antibody (rHIgM22) enhanced remyelination by 5 weeks after the treatment

| | |
|---|---|
| **Publication** | **Length of recorded locomotor activity session** |

[2]. Taken together that both the evolution of disease and repair is a slow process and to assure that any fluctuation in activity is taken into account, we monitored prolonged activity with the same sampling density used in short-term monitoring. This created large and highly fluctuating data sets (Figure 32 A,C). To clearly delineate post-treatment changes and recover general trends in long-term activity, we compared the use of data binning, Gaussian filtering, and polynomial fitting by using Mathematica (Wolfram Research, Inc.).

Intracerebral infection with Theiler's Murine Encephalomyelitis Virus (TMEV) of susceptible mouse strains results in chronic demyelinating disease with progressive neurologic dysfunction similar to progressive forms of multiple sclerosis [3]. Treating this model with IgM class antibodies that bind to oligodendrocytes improves CNS remyelination [4]. In contrast, a serum-derived human monoclonal antibody (sHIgM12) that binds to neurons, promotes robust neurite outgrowth to the same degree as laminin, and reduces the inhibitory effects of CNS myelin on neurite outgrowth [5]. More recently a recombinant form of human sHIgM12 was generated (rHIgM12) with identical biological properties. We showed previously that rHIgM12 has a half-life of 3.6 hours, but still crosses the blood brain barrier and binds to nervous tissues (unpublished observation). To study the effects of rHIgM12 on the activity of TMEV-infected mice we monitored spontaneous activity over several weeks using AccuScan activity boxes (Accuscan Instruments, Inc., Columbus, OH). We chose a relatively long 8-week monitoring period for two reasons: (1) IgM induced remyelination is present by 5 weeks post treatment, and (2) TMEV-induced demyelinating disease in this strain progresses very slowly compared to the purely autoimmune EAE models of MS [6]. Due to the long observation periods compared to previously published studies (TABLE 7), identifying changes by visual inspection of raw data proved difficult.

**TABLE 7 -Length of Spontaneous Activity Monitoring in Published Studies Compared to Current Study**

| | |
|---|---|
| Mikami et al. 2002, 2004 [14, 15] | 10 minutes (10 minutes of habituation) |
| Melnick and Dow-Edwards 2001 [16] | 60 minutes (no habituation) |
| Chen et al. 2005 [17] | 30 minutes (no habituation) |
| Torres-Reveron and Dow-Edwards 2006 [18] | 60 minutes (20 minutes of habituation) |
| Zhu et al. 2007 [19] | 60 minutes (no habituation) |
| Li et al. 2009 [20] | 3 hours (1 hour of habituation) |
| (Rivera-Quinones et al. 1998; McGavern et al. 1999 [1, 21] | 3 days (no habituation) |
| Current Study | 64 days (8 days of habituation) |

### Materials and Methods:

Mice: SJL/J mice (Jackson Laboratories, Bar Harbor, ME) were housed and bred in_Mayo Clinic's animal care facility. Animal protocols were approved by the Mayo Clinic-Institutional Animal Care and Use Committee.

Theiler's virus model of demyelination: Demyelinating disease was induced in 6- to 8-week-old female mice by_intracerebral injection of TMEV. A 27-gauge needle delivered 10 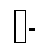 l containing 2.0 x 105_plaque-forming units of Daniel's strain TMEV. This resulted in >98% incidence of infection with rare fatalities. All animals developed mild encephalitis, which resolved within 2 weeks. Animals worsen over the next 6-8 months with chronic spinal cord demyelinating disease. Axon injury and loss begin three months after infection correlating with neurologic dysfunction [3].

Antibody Treatment: SJL mice (uninfected, 45 and 90 days post infection) were treated with a single 200 µg intraperitoneal dose of antibodies (rHIgM12 or isotype IgM control) dissolved in 0.5ml of PBS. Third group was treated with 0.5ml PBS only.

Spontaneous activity monitoring: Spontaneous locomotor activity was recorded with Digiscan open field (OF) apparatus (Omnitech Electronics; Columbus, OH) and Versamax software, v.4.12-1AFE (Accuscan Instruments, Inc., Columbus, OH). The apparatus consists of six acrylic cages (40x40x30.5cm) supported by a metal frame that holds two sets of photo cells. The device measures the number of discrete horizontal and vertical movements by tabulating the number of projected infrared beam interruptions. In all cages mice were exposed to identical environmental conditions: (a) freely accessible food and water, (b) a normal 12h light/dark cycle, (c) 70°F ambient temperature. In each experiment hyperactive and, in rare occasion, overweight animals were excluded and the remainder of mice were randomized. Several groups of 5 SJL mice were placed in the center of the each cage and the baseline spontaneous activity was collected over the period of 8 consecutive days. After this period, three groups of mice with the most similar baseline activity were treated with rHIgM12, control IgM antibody or saline and then monitored over the period of 8 weeks. Data was collected as number of beam breaks per 1-hour blocks. The total horizontal and vertical activities were recorded using Versadat program, v.3.02-1AFE (Accuscan Instruments). We could not put more than 5 animals per activity box because this is the maximal number of animals allowed by Institutional Animal Care and Use Committee (IACUC).

Data Analysis: Differences among treatment groups may be difficult to appreciate studying original unfiltered recordings (Figure 32 A-C). To recover slow trends in the highly fluctuating original data we applied three different methods: 1) binning, 2) application of Gaussian low-pass filter (GF), and 3) polynomial fitting.

Data binning is the simplest method and replaces a group of data points within a preselected bin by their average value. In our case, we selected a nocturnal bin given that murine activity peaks at night. Therefore, we replaced all nocturnal readings (12 h/day) by their average value as shown in Figures 33A, 33B, 35C, 35D, 37C and 37D. Comparison of groups can be performed by a simple t-test for a difference of means, with an effective sample size of 12 hours per treatment. Whereas these comparisons are simple, the small sample size at each time point results in large standard errors and statistical comparisons are of limited use. Overall, data binning is an effective method for visualizing noisy data, but is of limited use for statistical testing.

Gaussian filtering (GF) (also known as low-pass filtering, data smoothing, or sensitivity enhancement) is a noise-reduction procedure commonly used in Fourier transform spectroscopy and image processing [7]. GF, performed with an appropriate selection of Gaussian broadening (GB, in days) filtered out high-frequency fluctuations at a desired level. The filter choice is arbitrary and may be guided by the rate of expected activity changes. GF is a smoothing method using information from points taken from both sides of a value and weighting the influence of these points such that influence fades according to the Gaussian function. Computationally, GF can be implemented in two equivalent ways: 1) Fourier transformation (FT) of the data, followed by multiplication with a Gaussian function and inverse FT of the product; and 2) direct convolution of the data with a Gaussian kernel. In high-level software packages (Matlab (Mathworks) or Mathematica (Wolfram)) the Gaussian filter function is available with minimal or no programming by the user. With appropriate selection of GB the above detailed GF method enables simplified visualization of highly complex and heterogeneous data. One limitation of GF is that whereas it allows for easy visualization of trends, statistical comparisons are complicated by the choice of the GB.

For comparison and to demonstrate a method that allows for simplified statistical comparison, we fit polynomials to the data. These models allowed for polynomial terms up through the any degree (xn), and estimated shape parameters separately for each treatment group (interaction terms). Our choice of a 6th degree polynomial was arbitrary after exploring several options, but allowed sufficient flexibility to model non-linear effects over time. Because the different treatment groups were optimally fit using varying order polynomials, we chose to allow for the higher order flexibility. Akaike Information Criteria (AIC) was used to determine the "best fit" of polynomial lines across several of the treatment groups [8]. AIC is a method of model comparison which balances the gain in R² by adding additional terms, but penalizes for overcomplexity (i.e., use of degrees of freedom). In general, the 3rd order fit was sufficient to capture the trend of the data, and for some cases the 2nd order or even the linear fit was "best" according to AIC. However, our primary goal was to compare treatment groups at observed time points. Because of the large number of data points, and because we are not using the polynomial fit to predict (or extrapolate) treatment values outside of our observed data, the impact of "overfitting" on results is minimal.

One advantage of polynomial fitting is that the statistical comparison of treatment groups is straightforward: treatments can be compared at specified time points using the predicted model values and respective standard errors. Direct pairwise comparisons of treatments can be performed at regular intervals across the entire time frame to determine when treatment groups have significantly diverged. Finally, the polynomial fit removes additional medium and low frequency noise which focuses visual attention on the general trend over the entire time frame.

However, within each of the experiments some groups of mice had somewhat different baseline horizontal and vertical activity (8 days). Therefore, we first used Z-values to standardize baseline activity independently for each group and then performed Gaussian filtering or fitted polynomials to these values.

Statistical analysis: Binning data and Gaussian low-pass filter smoothing of data were performed by using a macro written in Mathematica (Wolfram). Macro and instructions are available on mayoresearch.mayo.edu/mayo/research/rodriguez_lab/software.cfm. Polynomial regression models and statistical comparisons of treatment groups were performed using the predicted model values and respective standard errors based on the z-statistic (SAS Institute, Inc.). Direct pairwise comparisons of treatments were performed for each day across the entire time frame, and statistical significance was determined at the typical α = 0.05 threshold. No adjustments were made for multiple comparisons.

### RESULTS

### rHIgM12 improves horizontal activity in SJL mice when given at 90dpi

Three groups of 5 SJL mice at 90 days post infection (dpi) were placed in activity boxes and baseline activity was measured for 8 consecutive days. Two groups of mice were treated with a single 200µg dose of rHIgM12 or an isotype-control IgM. The third group was treated with saline. After the treatment, spontaneous activity was continuously recorded over 8 weeks. Because data was collected in 1-hour blocks, we obtained approximately 900 data points for each group. This original, raw data (Figure 32A-C) is highly fluctuating, so differences among treatment groups may be difficult to appreciate. All three methods (binning, gaussian filtering and polynomial fitting) revealed that mice treated with rHIgM12 showed improvement in horizontal activity, whereas control IgM- and saline-treated mice had no changes in the activity over the period of 8 weeks (Figure 33A, C and E). Using direct pairwise comparisons of three treatments after polynomial fitting, we determined that improvement in horizontal motor function in rHIgM12-treated mice became statistically significant at day 7 post treatment (as compared to control IgM) and at day 11 (as compared to saline) (Figure 34). Observed improvement in horizontal nocturnal activity of rHIgM12-treated animals persisted for approximately 30 days and then returned to baseline levels. Pairwise comparison of saline- versus control IgM- treatment groups showed statistical significance for days 38-52. However, when compared to major differences observed between rHIgM12-treatment group and the controls, we believe that these differences between the control groups are not biologically significant. On the other hand, vertical activity did not show major differences and was similar in all three groups (Figure 33B, D, and F).

### rHIgM12 improves horizontal and vertical activity in SJL mice when given at 45dpi

The previous study used vertical activity (rearing behavior) as a primary readout [1]. Due to axonal dropout in chronically TMEV-infected mice, hind limbs became progressively weak and stiff so rearing was reduced. In the first experiment of this study, when rHIgM12 was administered at the time of maximal demyelination (90dpi) and onset of progressive axonal loss, only horizontal activity was improved. The rearing behavior was not affected and was equivalent in all three treatment groups. Therefore we asked whether treatment at an earlier time point may be more beneficial. In a second experiment, groups of 5 mice were treated at 45 dpi with a single 200µg dose of rHIgM12, an isotype-control IgM or saline. An identical experimental design was used; baseline activity was collected for 8 days, and post-treatment activity for 8 weeks. In this experiment, beginning around 2 weeks post-treatment, rHIgM12-treated mice showed clear improvements in both horizontal and vertical activity. This was evident after using all three methods: averaging data, applying Gaussian filter or polynomial fitting (Figure 35C-H). Control IgM- and saline-treated mice showed similar activity levels throughout the end of study. In rHIgM12-treated mice, improvement in horizontal activity was evident until the experiment ended. On the other hand, improvement in vertical activity lasted for about 4 weeks and then dropped to baseline values. Direct pairwise comparisons of three treatments showed that improvement in horizontal motor function in rHIgM12-treated mice significantly diverged at day 13 post treatment (as compared to control IgM) and at day 9 (as compared to saline) (Figure 36A and C). Likewise, vertical motor function in rHIgM12-treated mice significantly diverged at day 14 post treatment (as compared to control IgM) and at day 6 (as compared to saline) (Figure 36B and D). Comparison of control IgM- versus saline-treated groups did not reveal major biological differences for either horizontal or vertical activity (Figure 36E and F).

### rHIgM12 does not alter the spontaneous activity in normal uninfected mice

Treatment with rHIgM12 in previous two experiments showed clear beneficial effect in neurologically disabled infected mice. To exclude the possibility that this antibody may have irritable properties and therefore elicits increased motor function, we performed similar experiment with uninfected mice. Three groups of age matched uninfected mice were treated with rHIgM12, control IgM or saline. Compared to the activity enhancement in infected mice, rHIgM12 as well as the other two treatments did not induce any increase in motor function of normal mice (Figure 37). All three groups showed a tendency to decline in spontaneous activity. This result indicates that none of the antibodies has an influence on increasing activity in normal mice.

### DISCUSSION

There is a critical need for the development of neuroprotective therapies for multiple sclerosis as well as for other demyelinating and neurodegenerative diseases. Even though there are some anti-inflammatory drugs that may indirectly lead to a decrease in axonal damage during inflammatory CNS disease, there are no drugs that act directly at the level of neurons/axons. The main goal of neuroprotection is to limit neuronal dysfunction and to attempt to maintain the functional integrity of neurons and axons. For many years demyelination, the pathological hallmark of MS, was considered as a cause of permanent neurological deficits. It is clear now that demyelination is necessary, but not sufficient for permanent axonal loss [9]. Demyelination only predisposes denuded axons to the secondary injury caused by either T-cell cytotoxicity or loss of local neurotrophic support from dead oligodendrocytes [10].

Previous observation that neuron-binding antibody sHIgM12 promoted robust neurite extension [5] represents a clear beneficial *in vitro* response. Because recombinant version of this antibody showed similar in vitro properties we asked whether it would affect motor activity of mice with TMEV-induced demyelinating disease. Analysis of motor function was performed by monitoring the spontaneous nocturnal activity. First, we treated mice at the time of maximal demyelination and onset of axonal loss (90dpi). Eight weeks after the treatment, rHIgM12 improved only horizontal motor activity, whereas vertical activity was not affected. However, when mice were treated earlier in the disease (at 45dpi), rHIgM12 improved both horizontal and vertical activity. In the chronic TMEV-induced disease, rearing behavior (vertical activity) is most severely affected and it appears that degeneration and loss of axons responsible for this activity is irreversible. Conversely, the early phase of the disease, when these axons are not irreversibly injured, appears to be ideal time for the treatment. Jones et al. utilized EAE model and by studying axonal dropout and motor function they proposed an identical paradigm; treatment with neuroprotective drugs should begin early in the disease, even before the onset of motor deficits [11]. Second, because the functional improvement occurs about two weeks after the treatment and starts to fade approximately 25-30 days later, it may be that repeated treatments will be necessary to sustain motor function. Unfortunately in our studies, it was not possible to test multiple doses of human IgMs because of anti-human antibody immune response in mice which results in anaphylaxis. A2B5 is a mouse monoclonal antibody that also promotes neurite outgrowth [5] and represents a likely candidate to test single versus multiple dosing on functional outcome and its duration of action. Finally, none of the treatments had an effect on motor function of uninfected, normal animals. Irrespective of the treatment all groups of normal mice showed a gradual decline in spontaneous activity, which may be explained by habituation to the environment. This decline of activity in normal animals makes rHIgM12-induced increase in activity of diseased mice even more impressive.

We have demonstrated improvement in motor function in a chronic progressive model of inflammatory demyelinating disease in which has been generally very difficult to prevent the development of neurologic deficits. Therefore, neuron-binding monoclonal antibody rHIgM12 represents very promising therapeutic agent for the treatment not only of human MS, but also other demyelinating or neurodegenerative disorders. In addition, because there are examples of clinically silent MS attacks [12, 13], we and others have proposed that neuroprotective compounds should be complemented with immunomodulatory agents [11]. We propose that the combined treatment of immunomodulatory drugs and rHIgM12 may result in a significant enhancement of CNS preservation and repair following axonal injury.

Taken together, results from this study provide three important conclusions: 1) the stage of the disease when the treatment is given is critical (early treatment is more beneficial); 2) to further maintain improved motor function, there may be a need for repeated treatments and 3) rHIgM12 is not toxic and does not affect motor function in normal, uninfected animals. The findings are consistent with the hypothesis that a recombinant antibody that targets neurons improve neurologic function in a chronic axonal model of demyelination.

### REFERENCES

1. McGavern, D.B., et al., Quantitative assessment of neurologic deficits in a chronic progressive murine model of CNS demyelination. Exp Neurol, 1999.158(1): p. 171-81.
2. Warrington, A.E., et al., Human monoclonal antibodies reactive to oligodendrocytes promote remyelination in a model of multiple sclerosis. Proc Natl Acad Sci USA, 2000. 97(12): p. 6820-5.
3. McGavern, D.B., et al., Axonal loss results in spinal cord atrophy, electrophysiological abnormalities and neurological deficits following demyelination in a chronic inflammatory model of multiple sclerosis. Brain, 2000. 123 Pt 3: p. 519-31.
4. Warrington, A.E., et al., A recombinant human IgM promotes myelin repair after a single, very low dose. J Neurosci Res, 2007. 85(5): p. 967-76.
5. Warrington, A.E., et al., Neuron-binding human monoclonal antibodies support central nervous system neurite extension. J Neuropathol Exp Neurol, 2004. 63(5): p. 461-73.
6. Denic, A., et al., The relevance of animal models in multiple sclerosis research. Pathophysiology, 2010. 18(1): p. 21-9.
7. Hoch, J.C. and A.S. Stern, NMR Data Processing. 1 ed1996, New York: Wiley- Liss. 230.
8. Akaike, H., A new look at the statistical model identification. IEEE Transactions on Automatic Control, 1974. 19(6): p. 716-723.
9. Howe, C.L., J.D. Adelson, and M. Rodriguez, Absence of perforin expression confers axonal protection despite demyelination. Neurobiol Dis, 2007. 25(2): p. 354-9.
10. Rodriguez, M., A function of myelin is to protect axons from subsequent injury: implications for deficits in multiple sclerosis. Brain, 2003. 126(Pt 4): p. 751-2.
11. Jones, M.V., et al., Behavioral and pathological outcomes in MOG 35-55 experimental autoimmune encephalomyelitis. J Neuroimmunol, 2008. 199(1-2): p. 83-93.
12. Jacobs, L., P.R. Kinkel, and W.R. Kinkel, Silent brain lesions in patients with isolated idiopathic optic neuritis. A clinical and nuclear magnetic resonance imaging study. Arch Neurol, 1986. 43(5): p. 452-5.
13. Pohl, D., et al., Pediatric multiple sclerosis: detection of clinically silent lesions by multimodal evoked potentials. J Pediatr, 2006.149(1):p. 125-7.
14. Mikami, Y., et al., A simple and reliable behavioral analysis of locomotor function after spinal cord injury in mice. Technical note. J Neurosurg, 2002. 97(1 Suppl): p. 142-7.
15. Mikami, Y., et al., Implantation of dendritic cells in injured adult spinal cord results in activation of endogenous neural stem/progenitor cells leading to de novo neurogenesis and functional recovery. J Neurosci Res, 2004. 76(4): p. 453-65.
16. Melnick, S.M. and D.L. Dow-Edwards, Differential behavioral responses to chronic amphetamine in adult male and female rats exposed to postnatal cocaine treatment. Pharmacol Biochem Behav, 2001. 69(1-2): p. 219-24.
17. Shen, H., et al., Inosine reduces ischemic brain injury in rats. Stroke, 2005. 36(3): p. 654-9.
18. Torres-Reveron, A. and D.L. Dow-Edwards, Prenatal cocaine dampened behavioral responses to methylphenidate in male and female adolescent rats. Neurotoxicol Teratol, 2006. 28(2): p. 165-72.
19. Zhu, N., J. Weedon, and D.L. Dow-Edwards, Oral methylphenidate improves spatial learning and memory in pre- and periadolescent rats. Behav Neurosci, 2007. 121(6): p. 1272-9.
20. Li, X., et al., Attenuation of basal and cocaine-enhanced locomotion and nucleus accumbens dopamine in cannabinoid CB1-receptor-knockout mice. Psychopharmacology (Berl), 2009. 204(1): p. 1-11.
21. Rivera-Quinones, C., et al., Absence of neurological deficits following extensive demyelination in a class I-deficient murine model of multiple sclerosis. Nat Med, 1998. 4(2): p. 187-93.

### EXAMPLE 20: Neuron-Protective Human mAbs for Treatment of the Motor Neuron Disease ALS

Amyotrophic lateral sclerosis (ALS) is a devastating neurological illness that primarily affects anterior horn cells and corticospinal tract neurons. ALS is a motor neuron disease characterized by progressive degeneration of the motor cells in the brain and spinal cord. The motor cells (neurons) control the muscles that enable an individual to move around, speak, breathe and swallow. With no nerves to activate them, muscles gradually weaken and waste. Despite extensive research, the etiology of this disorder is largely unknown, and there are no effective treatments. Genes implicated in a minority of patients with a rare genetic form of ALS provide a potential clue to this disorder.[1] One identified gene mutation has been the Cu/Zn SOD (copper/zinc superoxide dismutase) mutation, which is present in a small percentage of patients with the genetic form of ALS.[2] It is apparent that patients carrying the SOD mutations have very similar neurological outcomes compared to patients who develop the disease spontaneously without an associated genetic mutation. [3] This enzyme catalyzes superoxide to oxygen and hydrogen peroxide. SOD1 is the form of the enzyme associated with ALS. There appears to be a gain of function of SOD1, which affects fast axonal transport.[4] The frequency of the SOD mutation in familial ALS varies from 12 to 23% and the gene is usually inherited as an autosomal dominant.

Identifying the gene allowed the development of animal models with disease features of ALS for designing and testing new drugs. Because inherited and non-inherited forms of ALS are similar diseases, the underlying cause may be related. Therefore, drugs that are effective in the mouse models of inherited ALS may also work in people with the more common random forms of ALS. The availability of transgenic mouse models with human SOD1 genes and the pathologic features of ALS have advanced the development of drugs for the disease.[5] These transgenic mice develop progressive motor neuron loss and neurologic deficits. Because inherited and noninherited forms of ALS are similar clinically, the underlying defects in motor neuron function may be related. Reagents efficacious in SOD1-linked ALS may also aid the more prevalent sporadic forms of ALS. At present there is only one drug on the market for ALS, the glutamate blocking drug Rilutek® (Riluzole tablets). However, studies have shown that this drug does not improve patient quality of life and may only extend life an average of 2 months. Clearly more effective drugs are critically needed.

The laboratory has developed novel therapies for the treatment of demyelinating and degenerative disorders of the central nervous system (CNS)[6] and have identified a series of human monoclonal antibodies (mAbs) that bind either to oligodendrocytes[7] or neurons,[8] which have been shown to induce repair in the CNS. These antibodies have been cloned, sequenced[9] and produced in large quantities in a GMP facility for future clinical trials.[10] Recombinant human antibody IgM12 (rHIgM12) is derived from an antibody isolated from a patient with Waldenstrom's macroglobulinemia.[11] This antibody specifically labels neurons and axons of the CNS.[8] Despite being an IgM antibody, IgM12, as shown in the above Examples, crosses the blood-brain barrier and binds specifically to axons and neurons within the CNS. *In vitro* rHIgM12 binds to a wide array of neurons, including cerebellar granular neurons, cortical neurons, hippocampal neurons and retinal ganglion cell neurons.[12] Experiments *in vitro* demonstrate that rHIgM12 protects neurons from cell death. Monoclonal antibody rHIgM12, administered to patients in early stages of ALS (both the genetic and spontaneous forms of disease), may act to protect anterior horn cells and prevent axon injury and thereby delay the onset of disability and improve survival.

### RESULTS

We have performed experiments treating mice with the G86R hSOD1 mutation [13] (FVBTg SOD1 G86R M1Jwg, Jackson Labs) with human antibody rHIgM12. G86R mice appear normal at birth. However, beginning at approximately 90-100 days of age, they undergo pronounced weight loss, develop significant muscle atrophy, and die of respiratory failure within weeks of the precipitous weight loss. LUDOLPH? In a randomized, "blinded" trial, these mice received GMP-purified rHIgM12 at the age of 55 days as a single intraperitoneal dose (200 µg). In contrast, the placebo group received phosphate buffered saline (PBS). A few mice were left untreated to determine the natural course of the disease without human handling. Because the data for PBS-treated and non-treated mice were similar, the groups were combined for statistical analyses. Mice were randomized for treatment by one investigator in our laboratory and were observed for neurological deficits by another "blinded" investigator until they became moribund. The treating investigators and other investigators performing the pathologic analyses were unaware of the randomization protocol until the code was broken.

Animals were weighed on a weekly basis to determine whether treatment with the human mAbs would not only increase survival but also delay the onset of weight loss. The results were striking and showed a statistically significant increase in survival in animals receiving rHIgM12 in comparison to animals receiving PBS. On average, investigators recorded an increased survival of 25 to 30 days, which, by Kaplan-Meier curves (FIGURE 38), showed statistical significance (P = 0.008). In addition, mice treated with rHIgM12 had less pronounced weight loss (FIGURE 39), a commonly assessed parameter in assessment of disease progression in animals. [18] To our knowledge, this is the first demonstration of a fully recombinant human monoclonal antibody directed against neurons being efficacious in increasing survival of animals with an ALS phenotype.

When animals reached the moribund phase, they were sacrificed and perfused with Trump's fixative. The spinal cords were removed and cut into 1mm blocks and embedded in Araldite plastic for 1µm thick sections. These sections were examined histologically. Animals that developed ALS from the SOD mutation showed significant axonal degeneration in the white-matter tracts, such that when the axon collapses, relatively easily identified myelin whorls develop in the spinal cord white matter. When the number of spinal cord whorls (degenerating axons) was quantified in thoracic sections in animals receiving rHIgM12 versus PBS, there was a statistically significant decrease in the number of myelin whorls in animals treated with monoclonal antibody therapy (FIGURE 40).

Spinal cord sections were also stained with an antibody for NeuN, a marker that specifically labels neurons in the CNS and very nicely defines the anterior horn cells as well as neurons in the posterior horn. A number of anterior horn cells were quantitated at the thoracic spinal cord level in animals receiving rHIgM12 compared to PBS (FIGURE 41, left). There was a highly statistical difference with an increase in the number of preserved anterior horn cells in animals receiving rHIgM12 versus PBS. A similar analysis on neurons in the posterior horns also revealed significant increase in the neurons of mice treated with rHIgM12 (FIGURE 41, right).

The human antibody, rHIgM12, binds to the surface of many types of CNS neurons obtained from many species including mice, humans (FIGURE 42), rabbits and primates. This provides evidence that rHIgM12 neuron signaling is preserved in mammals including from mice to humans. Our studies in the SOD mice suggest that protecting anterior and posterior horn cells from death may lead to fewer degenerating spinal cord axons and increased animal survival. Experiments described above in the prior Examples with cortical neurons in culture demonstrate that treating neurons with rHIgM12 can protect them from cell death (see FIGURE 3). Cortical neurons grown from neonatal mice were exposed to very high concentrations of hydrogen peroxide to determine a concentration where 90% of the neurons would die. Neurons exposed to hydrogen peroxide were treated at the same time with rHIgM12 or another human IgM that does not bind to neurons. Treatment with rHIgM12 resulted in the preservation of 80% of the neurons. In contrast, treatment with a control IgM resulted in the death of 90% of neurons after exposure to hydrogen peroxide. We hypothesize that rHIgM12 extends lifespan in animals with ALS by protecting neurons from cell death.

Human antibodies were also tested as substrates on tissue culture plates, and compared for the ability to promote normal cell process extension from cerebellar or cortical neurons. rHIgM12 and sHIgM42, antibodies that bind to the surface of neurons, and rHIgM22 and sHIgM39, antibodies that do not bind to neurons, were compared to the potent process supporting extracellular matrix molecule, laminin. Growing neurons on a substrate of human antibodies rHIgM12 or sHIgM42 resulted in a dramatic extension of neuron outgrowth, similar to that observed with laminin [8]. This study also showed that neurons behave normally in the presence of rHIgM12.

It has been generally accepted dogma that IgMs with a molecular weight of close to 1 million are too large to cross the blood brain barrier (BBB) from the circulation to enter the CNS [15] [16]. However, there is accumulating evidence some antibodies do cross the BBB. We have above described detecting rHIgM12 within the spinal cord after peripheral injection. 1.0 mg of rHIgM12 or a control human IgM were administered intraperitoneally to mice with demyelinated spinal cord lesions. Four hours later mice were killed and sections of the spinal cord were immunostained for the presence of human IgM mu chain and anti-neurofilament. In mice receiving rHIgM12 confocal microscopy demonstrated human mu chain within demyelinated lesions co-localized with antineurofilament, a marker of axons, in parallel tracts and as bundles cut on end (see FIGURE 15). Human IgM was not found within spinal cord lesions of mice that received a control IgM.

While the studies provided are in animal models, there are a number of tantalizing aspects of these research results that further indicate that this novel approach will be effective in human patients. Importantly, the IgM12 antibody described and used herein is a fully human, monoclonal antibody. As a result, only a single dose of this antibody can be used in mice. Subsequent doses cause animals to develop an immune response to the antibody resulting in either neutralization of rHIgM12 or the death of the animal due to anaphylaxis. However, since these are "true" human antibodies, human patients treated with rHIgM12 are unlikely to develop an immune response to them. Also, rHIgM12 can potentially be given on a continuing intermittent basis to patients without deleterious effect or development of an antibody-blocking reaction. Because rHIgM12 is a natural human autoantibody, adverse side effects are unlikely, and therefore, we expect the toxicity of this agent to be minimal. There is much evidence that rHIgM12 will be safe in humans. Before these positive results in the ALS model the serum form of the antibody, sHIgM12, was tested in several models of neurologic disease without toxicity, as described above herein. In these studies we found, 1) no increase in CNS pathology in mice with virus mediated demyelination after a 1 mg dose, 2) no increase in severity of clinical scores in mice with active EAE after a 200 µg dose and 3) clean blood chemistry and tissue pathology in normal CD-1 mice after a 300 µg dose. Even so, it is notable that a single dose to a mouse in a recognized animal model of ALS gave remarkable results.

rHIgM12 has already been grown in a GMP facility according to FDA guidelines, and, per FDA guidelines, stable, transfected cell lines have been generated and stored without any adventitial infections. These cell lines have been tested against a panel of >50 infectious organisms, all with negative results. In addition, the cell lines make large amounts of antibody in tissue culture (150µg/ml). A methodology for purifying rHIgM12 to >97% purity without adventitial viruses, DNA, RNA or other extraneous material according to FDA guidelines has been established, providing a strong preclinical basis for rHIgM12 in future initial phase clinical trial in patients with early stages of ALS.

### ONGOING STUDIES

Having shown above that the recombinant human monoclonal antibody (rHIgM12) can prolong survival in a transgenic model of human ALS by preventing the loss of anterior horn cells and axons, studies are ongoing to generate pre-clinical data through pharmacokinetics and toxicology to move toward a phase I clinical trial in ALS patients.

*Blinded Studies Versus Placebo Ab:* A definitive "blinded," placebo-controlled study is performed in two strains of SOD mice with the ALS phenotype (SOD1 G86R and G93A), testing recombinant human antibody rHIgM12 versus an isotype control human antibody, sHIgM39. The efficacy of a single 200µg dose of recombinant human antibody, rHIgM12, is tested to reduce disease in both the G86R and G93A SOD1 [14] (B6 Cg-Tg SOD1 G93A 1Gur, Jackson Labs) mutant transgenic mouse models, compared to a 200µg dose of an isotype control human antibody, sHIgM39. Antibody treatment is at 55 days of age following ENMC recommendation of pre-onset treatment [18], with results to mirror the pilot studies comparing rHIgM12 to PBS (Figures 38-41). Primary endpoints are survival and prevention of weight loss. Each experimental group consists of 24 mice, which is sufficient to detect a difference based on the above data. In addition, the CNS is examined in all mice after sacrifice to determine the number of anterior horn cells in the mid-thoracic spinal cord using a label for NeuN. The number of degenerating axons indicated by abnormal myelin whorls is counted. The G93A SOD1 mutant model (B6.Cg-Tg)SOD1G93A)1Gur/J #004435, Jackson Laboratory) is included because this is the first genetically based, most widely used and best characterized model of ALS and allows results with rHIgM12 to be compared to a broad data base of treatment paradigms. G86R mice have a late onset of disease (7 months) and a fast progression; while G93A mice have a faster onset (3-4 months) with a slower progression. This study controls for the introduction of a foreign protein into SOD1 mice and also addresses concepts of mechanism of action. The neuron binding character of rHIgM12 is critical and an IgM that does not bind to neurons should not improve disease. The primary endpoint in this study is increased survival (10% or greater P<0.05) and decreased weight loss (10% or greater P<0.05). Improvement in at least one strain of SOD1 mice is considered a success. All mice are sacrificed at the time they are unable to right themselves within 15-30 seconds if laid on either side. Secondary endpoints measure the number of degenerating axons, as indicated by abnormal myelin whorls, and the density of NeuN positive anterior horn cells at the thoracic level (T6) of the spinal cord. Adverse events are considered if mice treated with rHIgM12, 1) lose 20% more body weight compared to controls, 2) develop seizures, 3) have a death rate of 20% greater than controls.

*Dose Titration Study:* Following the positive results in the above study, a dose-titration study (a single dose of 0, 5, 50, 100 and 250 µg per mouse given at 55 days of age) is undertaken to determine the minimal dose required to protect SOD1 mice from death. MR spectroscopy of the mouse brainstem is used to measure N-acetyl aspartate (NAA). We have shown in other models of neurologic disease that NAA in the brainstem is an excellent surrogate indicator of axon health throughout the spinal cord.[17] The MR spectroscopy data helps validate NAA as a surrogate marker of antibody efficacy in potential human trials using rHIgM12. A single dose of 0, 5, 50, 100 and 250 µg is given intraperitoneally at 55 days of age to groups of 20-24 SOD1 mice. Primary and secondary endpoints and adverse events are the same as described above with the addition of MR spectroscopy at the brainstem to measure Nacetyl aspartate (NAA). MR spectroscopy is collected for each mouse on the day of antibody treatment, at 100 days of age and immediately prior to sacrifice. A 10% (P<0.05) increase in brain stem NAA concentration in any rHIgM12 treated group at the 100 day or final scan compared saline treated mice is considered an improvement.

*Pharmacokinetic Studies and BBB:* Studies are performed in SOD1 G86R and G93A mice at 50-70 days of age [18] after a single 200µg intravenous dose, using an established ELISA detection system that specifically detects human IgM within the background of normal mouse blood immunoglobulin. Human IgM is measured in the blood at various time points after dosing (0, 15 min, 30 min, 1 hr, 4hr, 8hr, 12 hr, 18 hr, 24 hr, 2 days, 3 days, 5 days, and 7 days). Because the blood volume of mice is small (total <1.5ml), three individual mice are used for each collection point.

rHIgM12 is proposed to act directly on the nervous system, crossing the blood-brain barrier after peripheral injection to interact with neurons to protect them from death. It is possible, but unlikely, that the human antibody does not cross the blood-brain barrier, but stimulates aspects of an immune response, which then provides axon protection. *In vivo* tracking of 35S labeled rHIgM12 is used to fully address this question. Two dose levels of 35S labeled rHIgM12, 250µg and the minimal effective dose established above, are followed in SOD1 mice at 50-70 days of age. After an intravenous dose of 35S labeled rHIgM12, the percentage of 35S that crosses the blood-brain barrier and is found in brain/spinal cord parenchyma at 4, 8, 24, 48 and 72 hr post injection is determined. In addition, the site of 35S localization in the brain/spinal cord is determined using autoradiography techniques already published. [19]

*Serum Half Life Studies:* Blood kinetics for rHIgM12 is performed using a dose of 200µg in the SOD1 strain(s) in which rHIgM12 demonstrated efficacy. The study is performed in groups of 3 SOD1 mice per time point; 13 collection points over 7 days after intravenous injection. SOD1 mice are treated at 50-70 days of age to understand antibody kinetics at the time of treatment. rHIgM12 serum half life, saturation of exposure and the presence of neutralizing Abs IgM will be determined. Studies tracking isotopically (35S) labeled rHIgM12 require the determination of the minimal effective dose described above. SOD1 mice are given a single intravenous dose of 250µg and the minimum effective dose of rHIgM12 (containing at least 1x107 cpm) [19] at 50-70 days of age to address whether rHIgM12 enters the CNS at the time of therapeutic treatment. At 4, 8, 24, 48 and 72 hr post injection major tissues are acutely removed, including brain, spinal cord, liver, heart, lung, stomach, intestine, muscle, spleen, liver, pancreas. A 150 mg portion of tissue is removed, weighed, solubilized in Solvable (Perkin Elmer) and cpm determined in scintillation fluid (Ultima Gold, Perkin Elmer). Isotopically labeled rHIgM12 is localized in spinal cord sections using autoradiography [19]. In this experiment, the primary endpoint is an accumulation of 35S isotope in the brain or spinal cord of mice at 4, 8, 24, 48 and 72 hr after rHIgM12 injection compared to the zero time point controls. A 50% (P<0.05) increase in 35S counts/mg in whole brain or spinal cord any time point is considered significant and evidence that rHIgM12 can enter the CNS. For autoradiography studies mice are dosed with labeled rHIgM12 and spinal cords removed at a time in which rHIgM12 was increased in the CNS. A 50% increase in silver grains/mm2 over neurons in spinal cord sections is considered significant evidence of antibody targeting *in vivo.*

*Initial Toxicity Studies:* Experiments are conducted with rHIgM12 to determine whether the antibody is toxic in normal CD-1 mice and rabbits. Groups of 10 normal CD-1 mice of both genders are administered 1 time (1X), 10 times (10X) and 20 times (20X) the minimal efficacious dose of rHIgM12 determined above or saline intravenously once or daily for 7 days. Two weeks later blood and tissues are collected by a "total" necropsy and analyzed. Tissues sections of all major organs are examined "blinded" by a veterinary pathologist with expertise in toxicology assessments. Blood is characterized in chemistry and hematology for the usual panel of toxicology screens including blood studies for liver enzymes, heart enzymes, electrolytes and effects on hematology group. Identical exposure studies are performed in a non-rodent species; two rabbits of each sex are tested at each dose. In addition, tissue cross reactivity studies using rHIgM12 are used to determine whether the antibody binds to any other tissues or organs. A panel of paraffin embedded tissue sections from mouse, rabbit, primate and human (Zymed) is immunolabeled with rHIgM12 or a control human IgM. The intensity and structures bound within each tissue are imaged and compared to rHIgM12 and control IgM labeling of live tissue slices cut with a tissue chopper [7] since this more closely mimics the situation *in vivo.* In addition, the binding of rHIgM12 and a control antibody is tested on frozen human and primate tissue as consistent with the preliminary toxicology section of an Investigational New Drug application to the FDA. Tissue cross-reactivity studies provide clues as to which organs should be particularly monitored during the exposure studies in mice and rabbits.

*Tissue Binding:* Following efficacy determination, these studies address binding to target and off target tissues. Commercially available tissue arrays (Zymed) and sections of human and cynomolgus monkey (Charles River) are immunolabeled with 10 µg/ml of rHIgM12 and control human IgM, sHIgM39. Label intensity is compared using digital images. rHIgM12 binding to fixed paraffin-embedded tissue arrays and frozen human and primate brain and spinal cord is compared to antibody binding observed on live mouse cerebellar slices. Antibody binding to live cerebellar slices a standard potency assay we have utilized and the first screen in which the serum form of rHIgM12 was identified.

### REFERENCES CITED

1. Gurney, M.E., et al., Mutant CuZn superoxide dismutase in motor neuron disease. J Inherit Metab Dis, 1998. 21(5): p. 587-97.
2. Morrison, B.M. and J. H. Morrison, Amyotrophic lateral sclerosis associated with mutations in superoxide dismutase: a putative mechanism of degeneration. Brain Res Rev, 1999. 29(1): p. 121-35.
3. Siddique, T., D. Nijhawan, and A. Hentati, Familial amyotrophic lateral sclerosis. J Neural Transm Suppl, 1997. 49: p. 219-33.
4. Cluskey, S. and D.B. Ramsden, Mechanisms of neurodegeneration in amyotrophic lateral sclerosis. Mol Pathol, 2001. 54(6): p. 386-92.
5. Turner, B.J. and K. Talbot, Transgenics, toxicity and therapeutics in rodent models of mutant SOD1-mediated familial ALS. Prog Neurobiol, 2008. 85(1): p. 94-134.
6. Bieber, A.J., et al., Humoral autoimmunity as a mediator of CNS repair. Trends Neurosci, 2001. 24(11 Suppl): p. S39-44.
7. Warrington, A.E., et al., Human monoclonal antibodies reactive to oligodendrocytes promote remyelination in a model of multiple sclerosis. Proc Natl Acad Sci USA, 2000. 97(12): p. 6820-5.
8. Warrington, A.E., et al., Neuron-binding human monoclonal antibodies support central nervous system neurite extension. J Neuropathol Exp Neurol, 2004. 63(5): p. 461-73.
9. Ciric, B., et al., Clonal evolution in Waldenstrom macroglobulinemia highlights functional role of B-cell receptor. Blood, 2001. 97(1): p. 321-3.
10. Mitsunaga, Y., et al., Direct evidence that a human antibody derived from patient serum can promote myelin repair in a mouse model of chronic-progressive demyelinating disease. Faseb J, 2002. 16(10): p. 1325-7.
11. Rodriguez, M., A.E. Warrington, and L.R. Pease, Invited article: human natural autoantibodies in the treatment of neurologic disease. Neurology, 2009. 72(14): p. 1269-76.
12. Wright, B.R., et al., Cellular mechanisms of central nervous system repair by natural autoreactive monoclonal antibodies. Arch Neurol, 2009. 66(12): p. 1456-9.
13. Bruijn, L.I., et al., ALS-linked SOD1 mutant G85R mediates damage to astrocytes and promotes rapidly progressive disease with SOD1-containing inclusions. Neuron, 1997.18(2): p. 327-38.
14. Gurney, M.E., et al., Motor neuron degeneration in mice that express a human Cu,Zn superoxide dismutase mutation. Science, 1994. 264(5166): p. 1772-5.
15. Banks, W.A., Developing drugs that can cross the blood-brain barrier: applications to Alzheimer's disease. BMC Neurosci, 2008. 9 Suppl 3: p. S2.
16. Kozlowski, G.P., I. Sterzl, and G. Nilaver, Localization patterns for immunoglobulins and albumins in the brain suggest diverse mechanisms for their transport across the blood-brain barrier (BBB). Prog Brain Res, 1992. 91: p. 149-54.
17. Denic, A., et al., Brainstem 1H nuclear magnetic resonance (NMR) spectroscopy: marker of demyelination and repair in spinal cord. Ann Neurol, 2009. 66(4): p. 559-64.
18. Ludolph, A.C., et al., Guidelines for the preclinical in vivo evaluation of pharmacological active drugs for ALS/MND: report on the 142nd ENMC international workshop. Amyotroph Lateral Scler, 2007. 8(4): p. 217-23.
19. Hunter, S.F., D.J. Miller, and M. Rodriguez, Monoclonal remyelination-promoting natural autoantibody SCH 94.03: pharmacokinetics and in vivo targets within demyelinated spinal cord in a mouse model of multiple sclerosis. J Neurol Sci, 1997. 150(2): p. 103-13.

### EXAMPLE 21: Treatment Of Cerebral Ishemia and Stroke With Neuron-Protective Human mAbs

The incidence of ischemic stroke increases with age. The aging of our population results in an increasing number of strokes per annum, placing a cumulative burden on our health care infrastructure and families. Stroke is the leading cause of long-term disability in the United States. Each year, close to 800,000 people suffer a stroke. Following ischemic injury, the disability associated with clinical deficits is primarily due to dysfunction of neurons. The lack of functional recovery is in part attributed to the restriction of regeneration and neuroplasticity (1). There are currently no effective treatments to prevent or reverse stroke-related neurologic deficits.

Naturally occurring human monoclonal IgM class antibodies have been identified and utilized to promote CNS protection and repair, including myelin repair and axonal/neuronal recovery (2). We identified a serum-derived human monoclonal IgM that binds to the surface of neurons. The IgM binds to neurons across species, including human, and promotes neurite extension. This IgM protects neurons from apoptosis *in vitro* and preserves spinal cord axons *in vivo* as measured by magnetic resonance spectroscopy and histology of the spinal cord. A recombinant form, rHIgM12 has been synthesized that preserves the characteristics of the serum-derived form. The neuroprotective properties of rHIgM12 are similar to those observed with ganglioside GM1. Gangliosides may be a/the antigen of antibody IgM12 and rHIgM12. rHIgM12 co-localizes on the neuronal membrane with GM1, cholesterol and integrin β1. GM1 is enriched in membrane microdomains, which function to cluster cell receptors and signaling molecules. rHIgM12 appears to mimic the pleiotropic effects of GM1 by clustering neuron membrane domains.

The present studies were undertaken to assess the efficacy of rHIgM12 to reverse neurological deficits following cerebral ischemic injury. These studies are aimed at applications of the antibody in patients with early stages of cerebral ischemic injury or suffering from stroke. The central hypothesis is that the antibody IGM12 and rHIgM12-mediated neuroprotective effects are applicable in therapy to enhance cellular and tissue restoration and to promote overall recovery from ischemic stroke.

Therapeutic management of cerebral ischemia relies on prevention and reduction of risk factors, such as elevated blood pressure, thrombosis and carotid artery atherosclerosis. Interventional therapy of cerebral ischemia remains limited to enzymatic thrombolysis by use of recombinant tissue plasminogen activator rtPA (3-5), and to interventional removal of the blood clot via direct catheterization (6). However, rtPA has a short 3 hr therapeutic window and increased incidence of hemorrhagic cerebral ischemia if infused beyond the 3 hr time frame (7, 8). Protecting damaged neurons following cerebral ischemia is a promising strategy to limit permanent disability. While therapies that modulate the inflammatory response can have neuro-protective properties, there are currently very few drugs that act directly on neurons to protect them. Neuro-protection may be achieved by modulating neurotoxic inflammatory environment in the ischemic region. Previous studies using antibodies to treat cerebral ischemia have not yielded a potential benefit: antagonists to intercellular adhesion molecules were employed in a large multi-center trial, treated subjects had higher mortality rates and poorer outcomes when compared to placebo group (9). The use of anti-platelet antibodies in a phase III clinical trial in cerebral ischemia led to fatal hemorrhage in a small cohort of patients (10). The use of polyclonal human IgG (IVIg) resulted in improvement of an animal model of cerebral ischemia (11), however clinical trials reported thromboembolic complications following IVIg administration, leading to a mixed opinion on the use of IVIg to treat cerebral ischemia (12, 13). A goal has been to identify therapeutic reagents that directly target the nervous system. The recombinant form of a completely human antibody rHIgM12 promotes neuronal survival and may have applicable use as a restorative/neuroprotective therapeutic. The experiments in this example focus on the therapeutic effects of antibody IgM12, rHIgM12, in a mouse model of cerebral ischemia. The model utilized is a non-invasive photo-thrombotic model using photo-sensitive dye and hence more physiological than invasive models.

Our approach to protect neurons and improve behavior in animals following cerebral ischemia using a fully human monoclonal antibody is both original and innovative and the antibody has the potential as the first drug designed to target and protect neurons in cerebral ischemia and stroke. Relevant aspects of the molecule are listed below:
**1)** rHIgM12 binds to surface of neurons (see Figure 1), and protects neurons from apoptotic cell death (see Figure 3).
**2)** rHIgM12 is a natural human autoantibody and adverse effects are unlikely.
**3)** rHIgM12, despite being an IgM crosses the blood-brain barrier (BBB) in normal SJL mice and in animals with inflammation (see Figure 13).
**4)** The present studies now demonstrate that when rHIgM12 is administered to animals following induction of ischemic stroke, it results in a clear improvement of function of the animals (Figure 44) and protects tissue integrity (Figure 45).

### RESULTS AND DISCUSSION

A unique approach was utilized to develop novel therapies for the treatment of injury to the central nervous system (CNS) (14, 15). A series of human monoclonal antibodies have been identified that bind either to oligodendrocytes (16) or neurons (17) and which have application in the CNS. Recombinant human antibody IgM12 (rHIgM12) is derived from an antibody isolated from a patient with Waldenstrom's macroglobulinemia. This antibody specifically labels neurons and axons of the CNS (17). Despite being an IgM antibody, it crosses the blood-brain barrier (BBB) (see Figure 13) and binds specifically to axons and neurons within the CNS. *In vitro* rHIgM12 binds to a wide array of neurons, including cerebellar granular neurons, cortical neurons, hippocampal neurons and retinal ganglion cell neurons. Our current hypothesis about the mechanism of action of reparative IgM autoantibodies is that they cross-link molecules in microdomains to stimulate signaling events that are involved in protection and repair.

### Standardization of a mouse model for cerebral ischemia

Photosensitive dye (Rose Bengal, RB) was utilized in a dye-mediated photo-thrombotic model of ischemic stroke (photo-thrombotic cerebral ischemia) in our laboratory. In 1985, Watson et al. introduced brain PT as a simple model of focal cerebral ischemia (Watson BD, et al (1985) Ann Neurol 17: 497-504). To achieve thrombosis, a photosensitive dye is injected systemically. Subsequent illumination through the intact skull leads to local activation of the dye with free radical formation and photoperoxidation of the endothelium. Clot formation is thereby mediated in illuminated vessels and occlusion of the vessels mimicks cerebral ischemia (Dietrich WD, et al (1987) Acta Neuropathol 72: 315-325). The PT lesions elicit a strong, but delayed inflammatory reaction (Schroeter M, et al (1997) Stroke 28: 382-386). This model has the advantage that the region of ischemia can be predefined and highly circumscribed, opening the possibility to coagulate distinct cortical areas with stereotactical precision.

RB, dissolved in PBS at 10 mg/mL was injected through the tail vein (0.02 mg/g body weight). The aperture (5mm) of the articulating fiber-optic attached to a light source (150W halogen lamp) was placed at a standard location on the mouse head. A minute after the injection of RB, brains were exposed to constant light (to activate RB) through the intact skull for 30 seconds. Upon activation, RB generates free radicals that disturb the endothelial cells within the microvasculature, which results in platelet aggregation and triggers coagulation that leads to occlusion of small blood vessels and causes thrombotic cerebral ischemia. We performed all experiments in 6 week old female CD-1® mice.

In a randomized, "blinded" trial, mice received GMP-purified rHIgM12 thirty minutes following induction of cerebral ischemia (an attempt to mimic the human clinical condition), as a single intraperitoneal dose (i.p. 200 µg in 500 µl). In contrast, the placebo group received phosphate-buffered saline (PBS). We standardized techniques to visualize and quantify ischemic region following cerebral ischemia (Figures 43 and 45)

### rHlqM12 improves functional activity in animals following cerebral ischemia without significant differences in diffusion-weighted imaging (DWI)-defined infarct volume

Functional deficits were computed using an Accuscan open-field apparatus. The apparatus consists of individual acrylic cages with photocells that measure discrete horizontal and vertical movements. Groups of 5 CD-1 mice were placed in each cage and baseline values were recorded for 1 week prior to the induction of cerebral ischemia. Data was collected as number of beam breaks per 1-hour blocks and expressed as % of horizontal (Figure 44A) or vertical change (Figure 44B) from baseline. At day one, following the induction of cerebral ischemia, there were similar deficits in locomotor activity of both groups of mice. Direct pairwise comparisons of two treatments showed that improvement in horizontal motor function in rHIgM12-treated mice significantly diverged (p<0.05) at day 3 post-treatment as compared to PBS (Figure 44A). Likewise, vertical motor function in rHIgM12-treated mice significantly diverged at day 3 post-treatment as compared to PBS (Figure 44B). Improvement persisted for up to 30 days, when the experiment was ended.

DWI-MRI was utilized to define ischemic volume and to correlate functional improvement to reduction in infarct size. Mice were scanned on days 1, 3, 7 and 21 in a Bruker Avance 300MHz (7 Tesla) small animal MRI system and obtained images in three-dimension (Figure 43A) were quantified using NIH Image J software. DWI did not show statistical significance between the two groups of mice at any of the days scanned (Figure 44C), an indication that the cerebral ischemic volumes in both groups of mice were similar. To our knowledge, this provides the first demonstration that a fully recombinant natural human monoclonal IgM antibody directed against neurons is efficacious in improving behavioral outcome in animals after experimental cerebral ischemia.

### rHIgM12 treatment improves MT-MRI metrics and demonstrates significant differences in MT ratios

DWI imaging estimates the presence and extent of cytotoxic edema on water diffusion following ischemic stroke. In order to assess MRI measures more sensitive to tissue integrity and recovery after injury, we utilized magnetization transfer MRI (MT-MRI) in a subsequent experiment, in 10 mice on day 3 after stroke induction and following treatment with rHIgM12 or PBS sham treatment. The obtained images were imported into FSL (23, 24) and a 3D parametric MTR map was calculated in FSL MATHS. We used the standard formula of 100*((Mo-Mt)/Mo), where Mo is the 3D image without, and Mt is the 3D image with the MT pulse. Images were appropriately scaled to provide a gray scale percentile mapping output with values ranging from 0 to 100 (Figure 45A). Lesional MT values were analyzed using the standard 3D ROI tools in Analyze 11.0, which allowed for analysis of MT sensitive tissue damage in areas of ischemia. A comparative ROI in the contralateral hemisphere was used to provide control values for comparison. MTR values on the control side were (59.9±0.9) and (58.3±0.8) in the rHIgM12 and PBS treated groups, respectively: as expected, statistical analysis did not reveal a significant difference between the two groups in the control hemispheres. Interestingly, rHIgM12 treatment resulted in a relative increase in MTR values in the stroke sub-volume compared to sham treatment with PBS (46.0±0.9 vs 41.9+08, p=0.01). This showed relative normalization of MT-MRI detectable tissue architecture in the antibody treated group. Furthermore, while statistical significance was not reached (p=0.39), a decreased stroke volume was seen in rHIgM12 treated mice vs PBS treated controls (18.1±5.9 mm³ vs 26.1±6.6 mm³). The highly significant difference on MT-MRI supports that the antibody induced changes result in enhanced tissue restoration as opposed to a direct reduction in stroke volumes.

### rHIgM12 binds to mouse and human neurons and protects mouse cortical neurons from oxidative damage-induced death

It is hypothesized that rHIgM12 protects neurons in the ischemic region by binding to neurons. The human antibody, rHIgM12, binds to the surface of many types of CNS neurons obtained from mice and humans (see Figure 1). This supports that rHIgM12-mediated neuro-protection and functional improvement will be preserved from mice to humans. Whether rHIgM12 would protect neurons *in vitro* from peroxide-induced activation of caspase-3, a marker of active apoptosis, has been previously assessed. Cultures of primary mouse cortical neurons were treated simultaneously with rHIgM12 or control human IgM and hydrogen peroxide. The degree of caspase-3 activation was determined 24 hrs later, using a FLICA caspase-3 assay kit (Immunochemistry Technologies) (see Figure 3). Treatment of injured neurons with rHIgM12 reduced caspase-3 activation by 80%. Injured neurons treated with control IgM showed less than a 10% reduction in caspase-3 activation.

In a different neurotoxic assay, cultured cortical neurons were directly subjected to hypoxic conditions (2.7% O₂, 92.3% N₂, 5% CO₂ atmosphere for 4, 21 and 44 hrs (44 hrs, non-hypoxia chamber cells were considered as 0hr control). The levels of caspase-3 were determined by Western blot (Figure 46) and caspase-3 levels found to be up-regulated by 4 hr and peaked out at 21 hr. By 44 hr most of the cells were dead and caspase-3 levels were down. These assays will be used to demonstrate that rHIgM12 is neuro-protective in ischemia-like conditions *in vitro.*

A mouse model of cerebral ischemic stroke was used in the present studies to demonstrate the capacity of rIgM12 to reverse neurological deficits following and provide protection from cerebral ischemic injury. This model is additionally be applied to optimize dose formulation, to determine the percentage of antibody that crosses blood-brain-barrier, and to better understand protection of cultured neurons by rHIgM12 *in vitro* from ischemia-like conditions.

### Dose and biomarkers for neurological improvement

A placebo controlled randomized trial in mice using rHIgM12 is used to determine the minimum dose required for improvement of spontaneous activity and identify metabolic biomarkers for neurologic improvement. The above data indicates that 200 µg of rHIgM12 improved neurologic function in a mouse model of ischemic stroke. Determining the minimum effective dose is relevant to the design of safety studies. A dose-titration study of rHIgM12 is performed in mice following cerebral ischemia.

Cerebral ischemia in female CD-1 mice using the photo-thrombotic model with dye RB is used in dosing studies. Groups of 5 mice are treated *i.p.* with 5, 50, 100 or 500 µg of rHIgM12, or with a commercial control human IgM (Jackson ImmunoResearch cat 009-000-012) that does not bind to neurons, or with saline, within 30 minutes following the induction of cerebral ischemia. Neurologic function is measured before induction of cerebral ischemia or treatment and weekly in activity boxes. Groups of 5 mice are housed in activity boxes and spontaneous vertical and horizontal activity is recorded. Nocturnal horizontal activity is a sensitive measure of an animal's functional ability (25). In addition, all mice undergo DWI-MRI (Fig 2C) to monitor infarct size in all groups.

The primary endpoint is motor improvement assessed by monitoring spontaneous activity. A 20% increase in mean nocturnal activity (2000 beam breaks/hr) recorded over 72 hr compared to control groups over 5 days of a rHIgM12 treated group (P<0.05) is considered a biological significant improvement (25). Established methodology from our laboratory (25) is used to calculate statistical differences between groups. DWI-MRI analysis is performed in a blinded fashion using 3D ROI analysis tools in Analyze 11.0, a biomedical image analysis software package developed at Mayo Clinic (26, 27).

It is determined whether improvement of neurological function following treatment with rHIgM12 correlates with preserved NAA metabolite (a metabolite associated with neuronal function (28)), and with relative normalization of the magnetization transfer ratio on MT-MRI in the brain. MT is an overall marker of tissue integrity and has been utilized as a sensitive marker for penumbra integrity in human ischemic stroke (29). These assays are useful as a future end-point of clinical trials in humans of rHIgM12 in cerebral ischemia.

Based on power calculations obtained for MT-MRI data, 12 mice per group are used in this experiment. Techniques were previously standardized to measure N-acetyl-asparate (NAA) levels in the CNS (30). In order to investigate whether NAA can be used as metabolite imaging biomarker for rHIgM12-mediated functional improvement following cerebral ischemia, we have standardized a voxel to perform MRS in the ischemic region. NAA metabolite is measured in animals treated with minimum effective dose, or isotype control IgM or saline. MRS is performed using the 7T vertical bore NMR spectrometer and MRS data obtained from a 3x2x1.5 mm voxel placed inside the ischemic area as identified on DWI and T2 weighted MRI. The contralateral hemisphere serves as an appropriate control for each mouse. As additional controls, mice in other groups are treated with control IgM or PBS. MT-MRI data is obtained by acquiring a standard PD-weighted RARE sequence with 256x96x96 matrix size, 6.4 x 1.92 x 1.92 cm FOV, TR:1500, TE:30, RARE factor of 16, with and without a MT pulse of a total MT time of 270 ms with excitation pulse bandwidth of 500 Hz and offset of 1500 Hz, before and after ischemic stroke followed by treatment with PBS or rHIgM12. Each experiment is repeated three times to confirm the reproducibility of using imaging biomarkers as endpoints of functional improvement, for a total of 108 mice (12 mice per group for a total of three groups, repeated three times) in this experiment.

Spectra for MRS are quantified using Bruker Biospin's TopSpin software and via LCModel (31-34). Statistical differences between groups is calculated by ANOVA (30). MTR data are computed using FSL MATHS and Analyze 11.0 software. Relative normalization of the MTR and preservation or relative increase of NAA levels is determined in the rHIgM12 treated groups as compared to the control groups. A 10 % increase in preserved NAA in any rHIgM12 treated group compared to control groups is considered an improvement. Excitatory neurotransmitters such as glutamate and glycine (35) have been reported to be present in high levels following cerebral ischemia. Since MRS spectroscopy is a versatile technique, we also quantify other metabolites such as lactate, myo-inositol, choline, creatine, glycine and glutamate.

### Blood Brain Barrier Assessment

The proportion of rHIgM12 that crosses blood-brain barrier (BBB) is determined in the photo-thrombotic RB stroke model. There has been a debate as to whether large IgM molecules can enter the CNS. A generally accepted dogma is that IgMs with a molecular weight of close to 1 million are too large to cross the BBB from the circulation to enter the CNS (36, 37). However, there is accumulating evidence some IgMs can cross the BBB. It has been shown that rHIgM12 can enter the CNS of normal uninfected mice as well as TMEV infected mice (see Figure 13). ³⁵S-labeled antibody is used in determining the percentage of antibody that crosses the BBB and further demonstrating that the IgM acts directly on the neurons in the ischemic region. We assess for neuron binding in CNS following i.v. injection in the cerebral ischemia model.

Isotopically labeled (³⁵S) rHIgM12 is produced and validated for binding to the surface of neurons. rHIgM12 at effective dose or an isotype control IgM or a control non-specific IgG are administered i.v., to groups of 3 mice per time point following induction of cerebral ischemia. Normal CD-1 mice without ischemia serve as control. The experiment is carried out three times using a total of 144 mice (3 mice per group for a total of 5 time points and 3 groups plus a group of 3 non-treated mice, repeated 3 times).

The percentage of ³⁵S that crosses the BBB and is found in brain/spinal cord parenchyma at 4, 8, 24, 48 and 72 hr post injection is determined. In addition, we determine the site of ³⁵S localization in the brain/spinal cord using autoradiography techniques published by our laboratory (38). We have proposed that rHIgM12 acts directly on the nervous system, crossing the BBB after peripheral injection to interact with neurons and protects them from death.

### Neuron Cultures

rHIgM12 neuro-protection is also assessed in *in vitro* 'ischemia-like' neuron culture assay. Demonstrating efficacy of rHIgM12 in protecting neurons in an *in vitro* environment will address concerns that a neuron-binding autoantibody may be toxic to neurons. We address this by analyzing caspase levels following treatment of normal, oxygen- and/or glucose-deprived cortical neurons with rHIgM12 or with control antibodies. In addition the development of an *in vitro* assay will provide necessary tools for future signaling studies.

Damage following cerebral ischemia might occur by several different mechanisms. Reagents that are neuro-protective for one type of damage are not necessarily protective for all types (39). We establish neurotoxicity assays using glucose deprivation (GD), oxygen deprivation (OD) and combined OD and GD (OGD), insults similar to those observed following cerebral ischemia. We have carried out analogous assays using hydrogen peroxide (Figure 3) and hypoxic chamber (Figure 46) to assess the protective effects of rHIgM12 on mouse cortical neurons. GM1 is used as a positive control, since the neuroprotective properties of rHIgM12 are reminiscent of those observed for GM1.

Dissociated cell cultures of neocortical fragments are established from 16-day CD-1 mouse embryos. Approximately 95% of the cells in such cultures are neurons, and the remaining cells are astrocytes. GD is induced by incubating neurons in glucose-free Locke's medium for 24h. An oxygen-free chamber with 95% N₂, 5% CO₂ atmosphere is used to induce OD (neurons are incubated in regular medium for 12 hr) and OGD (neurons are incubated in glucose-free Locke's medium for 12 hr). Neurons are incubated in the presence of culture medium alone, or culture medium supplemented with rHIgM12 (at effective dose), or control IgM or GM1. The experiment is performed three times independently in triplicates using coded treatments on different days to avoid bias.

The primary end point is neuronal cell viability, which is determined by a mitochondrial activity assay using Alamar blue solution. Neuronal cultures are analyzed for activated caspases using the FLICA Apoptosis Detection Kit. Two different fluorescence detection methods are used: fluorescence microscopy for qualitative analysis; and 96-well micro-titer plate fluorometry for quantitation. Cell survival is evaluated by incubating the cells for 30 min with Hoechst stain and read using a UV-filter with excitation at 365 nm and emission at 480 nm. When cells are viewed through a fluorescence microscope, apoptotic cells fluoresce red, while non-apoptotic cells appear mostly unstained. Cells in more advanced stages of apoptosis appear as brighter red than cells in earlier stages. Using a fluorescence plate reader (with a black micro-titer plate), apoptosis is quantitated as the amount of red fluorescence emitted from FLICA probes bound to caspases. ANOVA is used to compare significant differences between groups. The results from this experiment will help determine downstream signaling events that are involved in neuro-protection.

### REFERENCES CITED

1. Walmsley, A.R., and Mir, A.K. 2007. Targeting the Nogo-A signalling pathway to promote recovery following acute CNS injury. Current pharmaceutical design 13:2470-2484.
2. Rodriguez, M., Warrington, A.E., and Pease, L.R. 2009. Invited article: human natural autoantibodies in the treatment of neurologic disease. Neurology 72:1269-1276.
3. Brott, T.G., Haley, E.C., Jr., Levy, D.E., Barsan, W., Broderick, J., Sheppard, G.L., Spilker, J., Kongable, G.L., Massey, S., Reed, R., et al. 1992. Urgent therapy for stroke. Part I. Pilot study of tissue plasminogen activator administered within 90 minutes. Stroke; a journal of cerebral circulation 23:632-640.
4. Haley, E.C., Jr., Levy, D.E., Brott, T.G., Sheppard, G.L., Wong, M.C., Kongable, G.L., Torner, J.C., and Marler, J.R. 1992. Urgent therapy for stroke. Part II. Pilot study of tissue plasminogen activator administered 91-180 minutes from onset. Stroke; a journal of cerebral circulation 23:641-645.
5. Group, T.N.I.o.N.D.a.S.r.-P.S.S. 1995. Tissue plasminogen activator for acute ischemic stroke. The New England journal of medicine 333:1581-1587.
6. Gobin, Y.P., Starkman, S., Duckwiler, G.R., Grobelny, T., Kidwell, C.S., Jahan, R., Pile-Spellman, J., Segal, A., Vinuela, F., and Saver, J.L. 2004. MERCI 1: a phase 1 study of Mechanical Embolus Removal in Cerebral Ischemia. Stroke; a journal of cerebral circulation 35:2848-2854.
7. Clark, W.M., Wissman, S., Albers, G.W., Jhamandas, J.H., Madden, K.P., and Hamilton, S. 1999. Recombinant tissue-type plasminogen activator (Alteplase) for ischemic stroke 3 to 5 hours after symptom onset. The ATLANTIS Study: a randomized controlled trial. Alteplase Thrombolysis for Acute Noninterventional Therapy in Ischemic Stroke. JAMA : the journal of the American Medical Association 282:2019-2026.
8. Hacke, W., Kaste, M., Bluhmki, E., Brozman, M., Davalos, A., Guidetti, D., Larrue, V., Lees, K.R., Medeghri, Z., Machnig, T., et al. 2008. Thrombolysis with alteplase 3 to 4.5 hours after acute ischemic stroke. The New England journal of medicine 359:1317-1329.
9. Investigators, E.A.S.T. 2001. Use of anti-ICAM-1 therapy in ischemic stroke: results of the Enlimomab Acute Stroke Trial. Neurology 57:1428-1434.
10. Adams, H.P., Jr., Effron, M.B., Torner, J., Davalos, A., Frayne, J., Teal, P., Leclerc, J., Oemar, B., Padgett, L., Barnathan, E.S., et al. 2008. Emergency administration of abciximab for treatment of patients with acute ischemic stroke: results of an international phase III trial: Abciximab in Emergency Treatment of Stroke Trial (AbESTT-II). Stroke; a journal of cerebral circulation 39:87-99.
11. Arumugam, T.V., Tang, S.C., Lathia, J.D., Cheng, A., Mughal, M.R., Chigurupati, S., Magnus, T., Chan, S.L., Jo, D.G., Ouyang, X., et al. 2007. Intravenous immunoglobulin (IVIG) protects the brain against experimental stroke by preventing complement-mediated neuronal cell death. Proceedings of the National Academy of Sciences of the United States of America 104:14104-14109.
12. Vucic, S., Chong, P.S., Dawson, K.T., Cudkowicz, M., and Cros, D. 2004. Thromboembolic complications of intravenous immunoglobulin treatment. European neurology 52:141-144.
13. Rajabally, Y.A., and Kearney, D.A. 2011. Thromboembolic complications of intravenous immunoglobulin therapy in patients with neuropathy: a two-year study. J Neurol Sci 308:124-127.
14. Bieber, A.J., Warrington, A., Pease, L.R., and Rodriguez, M. 2001. Humoral autoimmunity as a mediator of CNS repair. Trends in neurosciences 24:S39-44.
15. Warrington, A.E., Bieber, A.J., Ciric, B., Van Keulen, V., Pease, L.R., Mitsunaga, Y., Paz Soldan, M.M., and Rodriguez, M. 2001. Immunoglobulin-mediated CNS repair. JAllergy Clin Immunol 108:S121-125.
16. Warrington, A.E., Asakura, K., Bieber, A.J., Ciric, B., Van Keulen, V., Kaveri, S.V., Kyle, R.A., Pease, L.R., and Rodriguez, M. 2000. Human monoclonal antibodies reactive to oligodendrocytes promote remyelination in a model of multiple sclerosis. Proc Natl Acad Sci USA 97:6820-6825.
17. Warrington, A.E., Bieber, A.J., Van Keulen, V., Ciric, B., Pease, L.R., and Rodriguez, M. 2004. Neuron-binding human monoclonal antibodies support central nervous system neurite extension. Journal of neuropathology and experimental neurology 63:461-473.
18. Ciric, B., VanKeulen, V., Rodriguez, M., Kyle, R.A., Gertz, M.A., and Pease, L.R. 2001. Clonal evolution in Waldenstrom macroglobulinemia highlights functional role of B-cell receptor. Blood 97:321-323.
19. Mitsunaga, Y., Ciric, B., Van Keulen, V., Warrington, A.E., Paz Soldan, M., Bieber, A.J., Rodriguez, M., and Pease, L.R. 2002. Direct evidence that a human antibody derived from patient serum can promote myelin repair in a mouse model of chronic-progressive demyelinating disease. The FASEB journal: official publication of the Federation of American Societies for Experimental Biology 16:1325-1327.
20. Wright, B.R., Warrington, A.E., Edberg, D.D., and Rodriguez, M. 2009. Cellular mechanisms of central nervous system repair by natural autoreactive monoclonal antibodies. Archives of neurology 66:1456-1459.
21. Howe, C.L., Bieber, A.J., Warrington, A.E., Pease, L.R., and Rodriguez, M. 2004. Antiapoptotic signaling by a remyelination-promoting human antimyelin antibody. Neurobiol Dis 15:120-131.
22. Watzlawik, J., Holicky, E., Edberg, D.D., Marks, D.L., Warrington, A.E., Wright, B.R., Pagano, R.E., and Rodriguez, M. 2010. Human remyelination promoting antibody inhibits apoptotic signaling and differentiation through Lyn kinase in primary rat oligodendrocytes. Glia 58:1782-1793.
23. Woolrich, M.W., Jbabdi, S., Patenaude, B., Chappell, M., Makni, S., Behrens, T., Beckmann, C., Jenkinson, M., and Smith, S.M. 2009. Bayesian analysis of neuroimaging data in FSL. Neurolmage 45:S173-186.
24. Smith, S.M., Jenkinson, M., Woolrich, M.W., Beckmann, C.F., Behrens, T.E., Johansen-Berg, H., Bannister, P.R., De Luca, M., Drobnjak, I., Flitney, D.E., et al. 2004. Advances in functional and structural MR image analysis and implementation as FSL. Neurolmage 23 Suppl 1:S208-219.
25. Denic, A., Macura, S.I., Warrington, A.E., Pirko, I., Grossardt, B.R., Pease, L.R., and Rodriguez, M. 2011. A single dose of neuron-binding human monoclonal antibody improves spontaneous activity in a murine model of demyelination. PloS one 6:e26001.
26. Robb, R.A. 1999. 3-D visualization in biomedical applications. Annual review of biomedical engineering 1:377-399.
27. Robb, R.A. 2001. The biomedical imaging resource at Mayo Clinic. IEEE transactions on medical imaging 20:854-867.
28. De Stefano, N., Matthews, P.M., Fu, L., Narayanan, S., Stanley, J., Francis, G.S., Antel, J.P., and Arnold, D.L. 1998. Axonal damage correlates with disability in patients with relapsing-remitting multiple sclerosis. Results of a longitudinal magnetic resonance spectroscopy study. Brain 121 (Pt 8):1469-1477.
29. Tourdias, T., Dousset, V., Sibon, I., Pele, E., Menegon, P., Asselineau, J., Pachai, C., Rouanet, F., Robinson, P., Chene, G., et al. 2007. Magnetization transfer imaging shows tissue abnormalities in the reversible penumbra. Stroke; a journal of cerebral circulation 38:3165-3171.
30. Denic, A., Bieber, A., Warrington, A., Mishra, P.K., Macura, S., and Rodriguez, M. 2009. Brainstem 1H nuclear magnetic resonance (NMR) spectroscopy: marker of demyelination and repair in spinal cord. Ann Neurol 66:559-564.
31. Henry, P.G., Oz, G., Provencher, S., and Gruetter, R. 2003. Toward dynamic isotopomer analysis in the rat brain in vivo: automatic quantitation of 13C NMR spectra using LCModel. NMR in biomedicine 16:400-412.
32. Opstad, K.S., Provencher, S.W., Bell, B.A., Griffiths, J.R., and Howe, F.A. 2003. Detection of elevated glutathione in meningiomas by quantitative in vivo 1H MRS. Magnetic resonance in medicine : official journal of the Society of Magnetic Resonance in Medicine/Society of Magnetic Resonance in Medicine 49:632-637.
33. Provencher, S.W. 2001. Automatic quantitation of localized in vivo 1H spectra with LCModel. NMR in biomedicine 14:260-264.
34. Pfeuffer, J., Tkac, I., Provencher, S.W., and Gruetter, R. 1999. Toward an in vivo neurochemical profile: quantification of 18 metabolites in short-echo-time (1)H NMR spectra of the rat brain. Journal of magnetic resonance 141:104-120.
35. Castillo, J., Davalos, A., and Noya, M. 1997. Progression of ischaemic stroke and excitotoxic aminoacids. Lancet 349:79-83.
36. Banks, W.A. 2008. Developing drugs that can cross the blood-brain barrier: applications to Alzheimer's disease. BMC Neurosci 9 Suppl 3:S2.
37. Kozlowski, G.P., Sterzl, I., and Nilaver, G. 1992. Localization patterns for immunoglobulins and albumins in the brain suggest diverse mechanisms for their transport across the blood-brain barrier (BBB). Prog Brain Res 91:149-154.
38. Hunter, S.F., Miller, D.J., and Rodriguez, M. 1997. Monoclonal remyelination-promoting natural autoantibody SCH 94.03: pharmacokinetics and in vivo targets within demyelinated spinal cord in a mouse model of multiple sclerosis. J Neurol Sci 150:103-113.
39. Ryu, B.R., Choi, D.W., Hartley, D.M., Costa, E., Jou, I., and Gwag, B.J. 1999. Attenuation of cortical neuronal apoptosis by gangliosides. The Journal of pharmacology and experimental therapeutics 290:811-816.

### EXAMPLE 22: Antibody Activity and Effects Under Normal (Normoxic) and Hypoxic Conditions

To further assess the effects and capability of neuroactive antibody, particularly antibody IgM12, in cells and under conditions relevant and mimicking stroke, myocardial infarction or ischemia, studies were undertaken to evaluate antibodies under normoxic and hypoxic conditions. Hypoxic conditions in culture serve to imitate aspects of oxygen deprivation which results from stroke and ischemia in clinical scenarios. Normoxic cultures were first evaluated for various markers in mixed glial cultures. Relevant markers were then evaluated under hypoxic conditions, with and without neuroactive and control antibodies to evaluate effects and capabilities.

### Normoxic Conditions

To evaluate normal or normoxic conditions, mixed glial cultures were grown for 4 days in FBS (10 %) containing medium and treated for 7 days in chemically defined medium with remyelination-promoting IgM rHIgM22, regenerative mAb rHIgM12, isotype controls sHIgM116 (10 µg/ml each) or medium only. Medium with fresh antibodies was exchanged every second day. The results are depicted in FIGURE 47. Bar graphs show the quantitative analysis of Western blots from three independent experiments with myelin marker MBP, apoptosis marker cleaved caspase-3 and CD68 as a marker for activated microglia. These results show that regenerative antibody rHIgM12 stimulates oligodendrocyte progenitor cell (OPC) differentiation and survival in mixed glial cultures. These data show that regenerative antibody rHIgM12, but not remyelination-promoting antibody rHIgM22 or isotype control sHIgM116, stimulates OPC differentiation, reduces levels of glial apoptosis and activates microglia. The specific cell type(s) rescued from undergoing apoptosis have not been determined.

OPC-microglial co-cultures were then cultured in astrocyte-conditioned medium with remyelination promoting mAbs A2B5, O4, O1, 78.09, 94.03, rHIgM22, regenerative mAb rHIgM12, isotype controls (LEAF, 5A5, ChromPure IgM, rHIgM42, sHIgM14, sHIgM24, sHIgM26) (10 µg/ml each) or medium. Medium with fresh antibodies was exchanged every second day. Representative Western blots in FIGURE 48 show levels of OPC markers PDGFaR, myelin markers CNPase and MOG, microglial markers CD68 and Lyn, proliferation marker Ki-67 and histone H3, apoptosis marker cleaved caspase-3 and cell cycle marker pRbS795. Astrocytes and microglia are required for GSL-binding regenerative mAbs to stimulate OPC differentiation and survival in astrocyte-conditioned medium.

The above normoxic data demonstrate that (i) remyelination promoting mAbs (A2B5, O4, O1, 78.09, 94.03) or regenerative mAb rHIgM12 do not induce OPC differentiation or survival compared to isotype controls in OPC/OL-microglia co-cultures ; (ii) soluble factors in astrocyte-conditioned medium were not sufficient to stimulate differences between treatment groups; (iii) remyelination promoting and regenerative mAbs did not activate microglia (CD68, Lyn) in the absence of astrocytes; (iv) soluble astrocytic factors seem to be sufficient to mediate OPC proliferation by rHIgM22 (Ki-67, histone H3) after 6 days of IgM-treatment.

In the next set of normoxic studies, mixed glial cultures were grown and treated as described previously (FIG 47 above). FIGURE 49 provides bar graphs of the quantitative analysis of Western blots from three independent experiments with marker CD68 for activated microglia and Lyn kinase, a highly abundant kinase in microglia and to a lower extent in OPCs but not in astrocytes. Representative Immunofluorescence images are provided in FIGURE 50 and show levels of CD68 (green) and nuclear marker DAPI (blue) in mixed glial before mAb treatment at day 4 in culture and at day 11 in culture after 7 days of treatment with rHIgM12, rHIgM22 and isotype control sHIgM24 (10 µg/ml each). These data demonstrate regenerative mAb rHIgM12 increases expression of CD68 and Lyn kinase in mixed glial cultures. Higher expression levels of CD68 in Western blots (FIG 49) can be correlated to higher numbers of activated microglia by immunocytochemistry (FIG 50). Thus, as shown in these studies, remyelination promoting antibodies (exemplary antibodies A2B5 and O1) and regenerative mAb IgM12 activate microglia (CD68) in mixed glial cultures.

### Hypoxic Conditions

The molecular effects of hypoxia on mixed glia cultures were then compared to normoxic cultures. Mixed glial cultures were grown for 4 days in FBS (10%) containing medium and treated for 7 additional days in chemically defined medium under hypoxia (3% O2) compared to normoxic conditions (21% O2). Representative Western blots (FIGURE 51) show levels of hypoxia-inducible factor 1 (HIF1α), oligodendrocyte markers NG2, PDGFα, Olig-2, myelin markers MBP and CNPase, astrocyte marker GFAP, microglial marker CD68, cell cycle marker pRbS795 and apoptosis marker cleaved caspase-3. Beta-actin was used as a loading control. Quantitative analysis of the Western blots from mixed glial cultures exposed to hypoxia and normoxia as shown in FIG 51 was completed from three independent experiments and is shown in FIGURE 52. These data demonstrate reduced expression levels of microglial and astrocytic markers and increased levels of myelin markers in mixed glial cultures under hypoxia compared to normoxic conditions. Verification of the hypoxic model is shown by increased HIF1α expression under hypoxia.

Having verified the hypoxic model, treatment of hypoxic cultures with regenerative antibody IgM12 was then undertaken to assess antibody effects in conditions relevant to and mimicking stroke, infarction and ischemia effects. Mixed glial cultures were grown for 4 days under normoxic conditions in FBS (10%) containing medium and subsequently treated for 1-7 days in chemically defined medium with human regenerative antibody rHIgM12 or medium only under hypoxia (3% O2). Representative Western blots (FIGURE 53) show levels of proliferation marker Ki-67, microglial markers IBA-1, CD68, and iNOS, apoptosis marker cleaved caspase-3, astrocytic marker GFAP, cell cycle markers pRbS795 and pRbS807 and oligodendrocyte markers PDGFaR, NG2, Olig-1, Olig-2 and myelin marker MBP. Results from these data indicate efficacy of rHIgM12 treatment under hypoxia. rHIgM12 activates microglia, stimulates cell cycle exit of glial cells (pRbs), reduces levels of glial apoptosis, and reduces increased levels of myelin production under hypoxia. Thus, regenerative antibody rHIgM12 stimulates glial survival and reduces OPC differentiation in hypoxic mixed glial cultures.

The molecular outcome of rHIgM12 under hypoxia versus normoxia was assessed by direct comparison of rHIgM12-mediated effects on mixed glia under hypoxia compared to normoxia. Mixed glia was cultured as described before and treated in chemically defined medium for 7 days with rHIgM12, rHIgM22 or medium only under hypoxic or normoxic conditions. Representative Western blots FIGURE 54) show levels of microglial markers IBA-1 and CD68, apoptosis marker cleaved caspase-3, astrocytic marker GFAP, oligodendrocyte markers PDGFaR, NG2 and Olig-2 and myelin markers MBP and CNPase. rHIgM12 shows efficacy under both normoxic and hypoxic conditions to stimulate microglial expression markers CD68 and IBA-1. Most significantly, rHIgM12 balances the impact of hypoxia on myelin marker expression MBP and CNPase compared to normoxic conditions.

Hypoxic conditions were evaluated in animals to check validity and consistency of the culture markers. A molecular analysis of hypoxic and normoxic cortices from P13 old neonatal B16 mice was performed. C57/B16 neonatal mice were cross-fostered by CD1 dams and exposed to hypoxia (10% O2) for 4 days from P3 to P7. Mice were subsequently exposed to room air for an additional 6 days and sacrificed at P13. RNA was isolated from cortices in both treatment groups (normoxia and hypoxia mice) and probed for myelin markers PLP1, MOG, MBP1, oligodendrocyte progenitor markers PDGFαR and Olig-1, astrocytic marker GFAP, neuronal markers Nestin and β3-tubulin, hypoxia-inducible factor 1α and the sarcospan protein SSPN. Expression levels were analyzed by RT-PCR and results normalized to levels of β-actin. RT-PCR analysis of the markers in cortices from hypoxic and normoxic C57/B16 neonates at P13 was conducted and the results are shown in FIGURE 55. Results from these data indicate that hypoxic treatment of neonatal mice selectively reduces expression levels of myelin markers but not neuronal, OPC or astrocytic markers in cortical tissue.

### EXAMPLE 23 - Localization of IgM Antibody rHIgM12 in a Mouse Model of Stroke

To evaluate antibody access and localization for intervention after a stroke or ischemic event, a stroke animal model was utilized with IP administration of monoclonal antibody. A stroke was induced in mice, radio-labeled antibody administered, and then animal tissues evaluated for antibody by determining radio-labeling at periods up to 24 hours post administration. Brain (cortex) containing the stroke lesion was evaluated, as well as cerebellum, spinal cord, liver, spleen, kidney and blood serum.

Stroke was induced in the right cerebral cortex in three adult CD-1 mice using the photo activated Rose Bengal protocol described above and three other mice received a sham stroke procedure, Rose Bengal but no light activation. At 30 to 40 min post stroke, 10 million cpm of ³⁵S labeled rHIgM12, corresponding to about 60 ug of IgM, was administered intraperitoneally to each mouse in a volume of 500 ul saline. At 6, 12 and 24 hr post IgM administration or treatment, blood was collected from mice, the vasculature washed with 30 ml of saline and tissues acutely collected. For analysis of radiolabel in tissue, the right front cortex of the brain containing the lesion area was removed as a 75 mg tissue block. Tissue was dissolved in Solvable (PerkinElmer) and cpm determined in UltraGold liquid scintillation cocktail following the manufacturer's protocols. A portion of other tissues were removed, weighed and processed according to the ratios provided by Perkin Elmer. The results are depicted in FIGURE 56.

Although this study utilized a single mouse at each tine point, it is notable that rHIgM12 accumulated in CNS tissue over 24 hr, while in contrast rHIgM12 levels fell in tissues responsible for blood metabolism, and in serum itself. The high level of rHIgM12 in the cortex (first panel denoted Brain in FIGURE 56) of the stroked mouse at 6 hr suggests the IgM has improved access to the stroke area during this time. There is significantly more label in the stroked animal in the brain/cortex at this time point versus the normal/sham procedure animal. Also of interest, rHIgM12 enters CNS tissue of mice with stroke and control mice equally suggesting that an intact BBB will not completely block the ability of this therapeutic IgM to reach areas of CNS damage. The rIgM12 antibody reached the area of stroke during the critical first 12 hours after stroke, when the drug is expected to have its maximal effect.

## Claims

1. An isolated human IgM antibody or fragment thereof which specifically binds neurons and protects neurons from cell death and which does not promote remyelination, wherein the isolated human IgM antibody or fragment comprises the following sequence:
(a) the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ ID NO: 34), CDR2 AAS (SEQ ID NO:35) and CDR3 QQSYHTPW (SEQ ID NO:36), as set out in Figure 5; or
(b) the variable heavy chain amino acid CDR domain sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6,
for use in a method of treating or ameliorating cerebral ischemia or stroke, improving neural function in an animal after stroke or cerebral ischemia, or in protecting neurons or neural cells from damage or cell death after stroke or cerebral ischemia,
wherein the method comprises administering the isolated human IgM antibody or fragment thereof in combination with a remyelinating antibody, and wherein the remyelinating antibody comprises:
(i) heavy chain variable region CDRs comprising CDR1 sequence SSGMH (SEQ ID NO: 47), CDR2 sequence V(I)ISYDGSRKYYADSVKG (SEQ ID NO: 48) and CDR3 sequence GVTGSPTLDY (SEQ ID NO: 49), and light chain variable region CDRs comprising CDR1 sequence SGSSSNIGNNFVS (SEQ ID NO: 50), CDR2 sequence DITKRPS (SEQ ID NO: 51) and CDR3 sequence G(E)TWDSSLSAW (SEQ ID NO: 52); or
(ii) heavy chain variable region CDRs comprising CDR1 sequence SGFTFSSYW (SEQ ID NO: 53), CDR2 sequence IKKDGSEK (SEQ ID NO: 54) and CDR3 sequence ARPNCGGDCYLPWYFD (SEQ ID NO: 55), and light chain variable region CDRs comprising CDR1 sequence QSVLYSSNNKNY (SEQ ID NO: 56), CDR2 sequence YWAS (SEQ ID NO: 57) and CDR3 sequence QQYYNTPQA (SEQ ID NO: 58).

2. A remyelinating antibody comprising:
(i) heavy chain variable region CDRs comprising CDR1 sequence SSGMH (SEQ ID NO: 47), CDR2 sequence V(I)ISYDGSRKYYADSVKG (SEQ ID NO: 48) and CDR3 sequence GVTGSPTLDY (SEQ ID NO: 49), and light chain variable region CDRs comprising CDR1 sequence SGSSSNIGNNFVS (SEQ ID NO: 50), CDR2 sequence DITKRPS (SEQ ID NO: 51) and CDR3 sequence G(E)TWDSSLSAW (SEQ ID NO: 52); or
(ii) heavy chain variable region CDRs comprising CDR1 sequence SGFTFSSYW (SEQ ID NO: 53), CDR2 sequence IKKDGSEK (SEQ ID NO: 54) and CDR3 sequence ARPNCGGDCYLPWYFD (SEQ ID NO: 55), and light chain variable region CDRs comprising CDR1 sequence QSVLYSSNNKNY (SEQ ID NO: 56), CDR2 sequence YWAS (SEQ ID NO: 57) and CDR3 sequence QQYYNTPQA (SEQ ID NO: 58),
for use in a method of treating or ameliorating cerebral ischemia or stroke, improving neural function in an animal after stroke or cerebral ischemia, or in protecting neurons or neural cells from damage or cell death after stroke or cerebral ischemia,
wherein the method comprises administering the remyelinating antibody in combination with an isolated human IgM antibody or fragment thereof which specifically binds neurons and protects neurons from cell death and which does not promote remyelination, and wherein the isolated human IgM antibody or fragment comprises the following sequence:
(a) the variable heavy chain amino acid CDR domain sequences CDR1 GGSVSLYY (SEQ ID NO:31), CDR2 GYIYSSGST (SEQ ID NO:32) and CDR3 ARSASIRGWFD (SEQ ID NO:33), and light chain CDR sequences CDR1 QSISSY (SEQ ID NO: 34), CDR2 AAS (SEQ ID NO:35) and CDR3 QQSYHTPW (SEQ ID NO:36), as set out in Figure 5; or
(b) the variable heavy chain amino acid CDR domain sequences CDR1 GFTFSTYA (SEQ ID NO: 37), CDR2 INVGGVTT (SEQ ID NO:38) and CDR3 VRRSGPDRNSSPADF (SEQ ID NO:39), and light chain CDR sequences CDR1 QGIG (SEQ ID NO: 40), CDR2 TTS (SEQ ID NO:41) and CDR3 QKYNSAPRT (SEQ ID NO: 42), as set out in Figure 6.

3. The isolated human IgM antibody or fragment thereof for use of claim 1, or the remyelinating antibody for use of claim 2, wherein the isolated human IgM antibody or fragment thereof comprises the variable heavy chain amino acid sequence set out in SEQ ID NO: 1 and the variable light chain amino acid sequence set out in SEQ ID NO: 11, or the variable heavy chain amino acid sequence set out in SEQ ID NO: 17 and the variable light chain amino acid sequence set out in SEQ ID NO: 27, or highly homologous variants thereof, wherein said variants retain neuron binding and neuroprotective or neuroregenerative activity.

4. The isolated human IgM antibody or fragment thereof for use of claim 1, or the remyelinating antibody for use of claim 2, wherein the isolated human IgM antibody or fragment thereof further comprises a human J chain sequence.

5. The isolated human IgM antibody or fragment thereof for use or the remyelinating antibody for use of claim 4, wherein the J chain comprises the amino acid sequence set out in SEQ ID NO: 15.

6. The isolated human IgM antibody or fragment thereof for use or the remyelinating antibody for use of claim 5, wherein the isolated human IgM antibody or fragment thereof is a fully human antibody or an antibody fragment thereof comprising a heavy chain and a light chain variable region comprising an amino acid sequence selected from the amino acid sequences SEQ ID NO: 1 and 11, or highly homologous variants thereof, wherein said variants retain neuron binding and neuroprotective or neuroregenerative activity.

7. The isolated human IgM antibody or fragment thereof for use or the remyelinating antibody for use of any one of claims 1 to 6, wherein the remyelinating antibody comprises:
(i) the amino acid sequence set forth in SEQ ID NO: 43 and 44, or
(ii) the amino acid sequence set forth in SEQ ID NO: 45 and 46.

8. The isolated human IgM antibody or fragment thereof for use or the remyelinating antibody for use of any one of claims 1 to 7, wherein the combination of antibodies is administered:
(i) collectively, or
(ii) in series.

9. The isolated human IgM antibody or fragment thereof for use or the remyelinating antibody for use of any one of claims 1 to 8, wherein the method comprises administering the isolated human IgM antibody or fragment thereof and the remyelinating antibody in combination with one or more agent which is a thrombolytic, an antiplatelet drug, an anti-hypertensive drug or a statin.

10. The isolated human IgM antibody or fragment thereof for use or the remyelinating antibody for use of any of claims 1 to 9, wherein the isolated human IgM antibody or fragment thereof and/or the remyelinating antibody is labeled with a detectable or functional label.

11. The isolated human IgM antibody or fragment thereof for use or the remyelinating antibody for use of claim 10 wherein the label is an enzyme, a specific binding partner, a ligand, a dye, a fluorescent tag and/or a radioactive element.

12. A pharmaceutical composition for the use as defined in any one of claims 1 to 11, wherein the pharmaceutical composition comprises an isolated human IgM antibody or fragment as defined in any one of claims 1 to 11 and a pharmaceutically acceptable vehicle, carrier or diluent.

13. A pharmaceutical composition for the use as defined in any one of claims 1 to 11, wherein the pharmaceutical composition comprises a remyelinating antibody as defined in any one of claims 1 to 11 and a pharmaceutically acceptable vehicle, carrier or diluent.

14. The pharmaceutical composition for use according to claim 12 or claim 13, wherein the method comprises administering the composition as a single dose to said animal suspected of or determined to be suffering from stroke or cerebral ischemia.

15. A kit for use in treating or ameliorating stroke or cerebral ischemia in an animal, comprising a pharmaceutical dosage form of the pharmaceutical composition of claim 12, and a separate pharmaceutical dosage form of the pharmaceutical composition of claim 13, and optionally further comprising one or more additional neuroactive agent or therapeutic, thrombolytic, anti-hypertensive agent, anti-platelet agent, antiinflammatory agent, neurotransmitter release modulating agent, neuroreceptor ligand or agonist or antagonist, calcium channel agent, immune modulator, or other CNS reactive antibody.

## Patentansprüche

1. Isolierter menschlicher IgM-Antikörper oder Fragment davon, der bzw. das Neuronen spezifisch bindet und Neuronen vor Zelltod schützt und keine Remyelinisierung fördert, wobei der isolierte menschliche IgM-Antikörper bzw. das Fragment die folgende Sequenz umfasst:
(a) die Variable-schwere-Kette-Aminosäure-CDR-Domäne-Sequenzen CDR1 GGSVSLYY (SEQ ID NO:31), CDR2 GYIYSSGST (SEQ ID NO:32) und CDR3 ARSASIRGWFD (SEQ ID NO:33) und Leichte-Kette-CDR-Sequenzen CDR1 QSISSY (SEQ ID NO:34), CDR2 AAS (SEQ ID NO:35) und CDR3 QQSYHTPW (SEQ ID NO:36), gemäß Figur 5; oder
(b) die Variable-schwere-Kette-Aminosäure-CDR-Domäne-Sequenzen CDR1 GFTFSTYA (SEQ ID NO:37), CDR2 INVGGVTT (SEQ ID NO:38) und CDR3 VRRSGPDRNSSPADF (SEQ ID NO:39) und Leichte-Kette-CDR-Sequenzen CDR1 QGIG (SEQ ID NO:40), CDR2 TTS (SEQ ID NO:41) und CDR3 QKYNSAPRT (SEQ ID NO:42), gemäß Figur 6,
zur Verwendung bei einem Verfahren zur Behandlung oder Linderung von zerebraler Ischämie oder Schlaganfall, Verbesserung der Nervenfunktion bei einem Tier nach Schlaganfall oder zerebraler Ischämie oder beim Schützen von Neuronen oder Nervenzellen vor Schäden oder Zelltod nach Schlaganfall oder zerebraler Ischämie,
wobei das Verfahren Verabreichen des isolierten menschlichen IgM-Antikörpers bzw. des Fragments davon in Kombination mit einem remyelinisierenden Antikörper umfasst und wobei der remyelinisierende Antikörper Folgendes umfasst:
(i) Schwere-Kette-variable-Region-CDR, die CDR1-Sequenz SSGMH (SEQ ID NO: 47), CDR2-Sequenz V(I)ISYDGSRKYYADSVKG (SEQ ID NO: 48) und CDR3-Sequenz GVTGSPTLDY (SEQ ID NO: 49) umfassen, und Leichte-Kette-variable-Region-CDR, die CDR1-Sequenz SGSSSNIGNNFVS (SEQ ID NO: 50), CDR2-Sequenz DITKRPS (SEQ ID NO: 51) und CDR3-Sequenz G (E) TWDSSLSAW (SEQ ID NO: 52) umfassen; oder
(ii)Schwere-Kette-variable-Region-CDR, die CDR1-Sequenz SGFTFSSYW (SEQ ID NO: 53), CDR2-Sequenz IKKDGSEK (SEQ ID NO: 54) und CDR3-Sequenz ARPNCGGDCYLPWYFD (SEQ ID NO: 55) umfassen, und Leichte-Kette-variable-Region-CDR, die CDR1-Sequenz QSVLYSSNNKNY (SEQ ID NO: 56), CDR2-Sequenz YWAS (SEQ ID NO: 57) und CDR3-Sequenz QQYYNTPQA (SEQ ID NO: 58) umfassen.

2. Remyelinisierender Antikörper, umfassend:
(i) Schwere-Kette-variable-Region-CDR, die CDR1-Sequenz SSGMH (SEQ ID NO: 47), CDR2-Sequenz V(I)ISYDGSRKYYADSVKG (SEQ ID NO: 48) und CDR3-Sequenz GVTGSPTLDY (SEQ ID NO: 49) umfassen, und Leichte-Kette-variable-Region-CDR, die CDR1-Sequenz SGSSSNIGNNFVS (SEQ ID NO: 50), CDR2-Sequenz DITKRPS (SEQ ID NO: 51) und CDR3-Sequenz G (E) TWDSSLSAW (SEQ ID NO: 52) umfassen; oder
(ii)Schwere-Kette-variable-Region-CDR, die CDR1-Sequenz SGFTFSSYW (SEQ ID NO: 53), CDR2-Sequenz IKKDGSEK (SEQ ID NO: 54) und CDR3-Sequenz ARPNCGGDCYLPWYFD (SEQ ID NO: 55) umfassen, und Leichte-Kette-variable-Region-CDR, die CDR1-Sequenz QSVLYSSNNKNY (SEQ ID NO: 56), CDR2-Sequenz YWAS (SEQ ID NO: 57) und CDR3-Sequenz QQYYNTPQA (SEQ ID NO: 58) umfassen,
zur Verwendung bei einem Verfahren zur Behandlung oder Linderung von zerebraler Ischämie oder Schlaganfall, Verbesserung der Nervenfunktion bei einem Tier nach Schlaganfall oder zerebraler Ischämie oder beim Schützen von Neuronen oder Nervenzellen vor Schäden oder Zelltod nach Schlaganfall oder zerebraler Ischämie,
wobei das Verfahren Verabreichen des remyelinisierenden Antikörpers in Kombination mit einem isolierten menschlichen IgM-Antikörper oder Fragment davon, der bzw. das Neuronen spezifisch bindet und Neuronen vor Zelltod schützt und keine Remyelinisierung fördert, umfasst und wobei der isolierte menschliche IgM-Antikörper bzw. das Fragment die folgende Sequenz umfasst:
(a) die Variable-schwere-Kette-Aminosäure-CDR-Domäne-Sequenzen CDR1 GGSVSLYY (SEQ ID NO:31), CDR2 GYIYSSGST (SEQ ID NO:32) und CDR3 ARSASIRGWFD (SEQ ID NO:33) und Leichte-Kette-CDR-Sequenzen CDR1 QSISSY (SEQ ID NO:34), CDR2 AAS (SEQ ID NO:35) und CDR3 QQSYHTPW (SEQ ID NO:36), gemäß Figur 5; oder
(b) die Variable-schwere-Kette-Aminosäure-CDR-Domäne-Sequenzen CDR1 GFTFSTYA (SEQ ID NO:37), CDR2 INVGGVTT (SEQ ID NO:38) und CDR3 VRRSGPDRNSSPADF (SEQ ID NO:39) und Leichte-Kette-CDR-Sequenzen CDR1 QGIG (SEQ ID NO:40), CDR2 TTS (SEQ ID NO:41) und CDR3 QKYNSAPRT (SEQ ID NO:42), gemäß Figur 6.

3. Isolierter menschlicher IgM-Antikörper oder Fragment davon zur Verwendung nach Anspruch 1 oder remyelinisierender Antikörper zur Verwendung nach Anspruch 2, wobei der isolierte menschliche IgM-Antikörper bzw. das Fragment davon die Variable-schwere-Kette-Aminosäuresequenz gemäß SEQ ID NO: 1 und die Variable-leichte-Kette-Aminosäuresequenz gemäß SEQ ID NO: 11 oder die Variable-schwere-Kette-Aminosäuresequenz gemäß SEQ ID NO: 17 und die Variable-leichte-Kette-Aminosäuresequenz gemäß SEQ ID NO: 27 oder hochhomologe Varianten davon umfasst, wobei bei den Varianten Neuronenbindung und nervenschützende oder nervenregenerierende Aktivität erhalten ist.

4. Isolierter menschlicher IgM-Antikörper oder Fragment davon zur Verwendung nach Anspruch 1 oder remyelinisierender Antikörper zur Verwendung nach Anspruch 2, wobei der isolierte menschliche IgM-Antikörper bzw. das Fragment davon ferner eine menschliche J-Kette-Sequenz umfasst.

5. Isolierter menschlicher IgM-Antikörper oder Fragment davon zur Verwendung oder remyelinisierender Antikörper zur Verwendung nach Anspruch 4, wobei die J-Kette die Aminosäuresequenz gemäß SEQ ID NO:15 umfasst.

6. Isolierter menschlicher IgM-Antikörper oder Fragment davon zur Verwendung oder remyelinisierender Antikörper zur Verwendung nach Anspruch 5, wobei es sich bei dem isolierten menschlichen IgM-Antikörper bzw. Fragment davon um einen vollständig menschlichen Antikörper bzw. ein Antikörperfragment davon handelt, der bzw. das eine Schwere-Kette- und eine Leichte-Kette-variable-Region, die eine aus den Aminosäuresequenzen SEQ ID NO:1 und 11 ausgewählte Aminosäuresequenz umfasst, oder hochhomologe Varianten davon umfasst, wobei bei den Varianten Neuronenbindung und nervenschützende oder nervenregenerierende Aktivität erhalten ist.

7. Isolierter menschlicher IgM-Antikörper oder Fragment davon zur Verwendung oder remyelinisierender Antikörper zur Verwendung nach einem der Ansprüche 1 bis 6, wobei der remyelinisierende Antikörper Folgendes umfasst:
(i) die Aminosäuresequenz gemäß SEQ ID NO: 43 und 44 oder
(ii)die Aminosäuresequenz gemäß SEQ ID NO: 45 und und 46.

8. Isolierter menschlicher IgM-Antikörper oder Fragment davon zur Verwendung oder remyelinisierender Antikörper zur Verwendung nach einem der Ansprüche 1 bis 7, wobei die Kombination von Antikörpern wie folgt verabreicht wird:
(i) kollektiv oder
(ii)in Reihe.

9. Isolierter menschlicher IgM-Antikörper oder Fragment davon zur Verwendung oder remyelinisierender Antikörper zur Verwendung nach einem der Ansprüche 1 bis 8, wobei das Verfahren Verabreichen des isolierten menschlichen IgM-Antikörpers oder Fragments davon und des remyelinisierenden Antikörpers in Kombination mit einem oder mehreren Mitteln, bei denen es sich um ein Thrombolytikum, einen Thrombozytenaggregationshemmer, einen blutdrucksenkenden Arzneistoff oder ein Statin handelt, umfasst.

10. Isolierter menschlicher IgM-Antikörper oder Fragment davon zur Verwendung oder remyelinisierender Antikörper zur Verwendung nach einem der Ansprüche 1 bis 9, wobei der isolierte menschliche IgM-Antikörper bzw. das Fragment davon und/oder der remyelinisierende Antikörper mit einer nachweisbaren oder funktionellen Markierung markiert ist.

11. Isolierter menschlicher IgM-Antikörper oder Fragment davon zur Verwendung oder remyelinisierender Antikörper zur Verwendung nach Anspruch 10, wobei es sich bei der Markierung um ein Enzym, einen spezifischen Bindungspartner, einen Liganden, einen Farbstoff, ein Fluoreszenz-Tag und/oder ein radioaktives Element handelt.

12. Pharmazeutische Zusammensetzung zur Verwendung mit der in einem der Ansprüche 1 bis 11 angegebenen Bedeutung, wobei die pharmazeutische Zusammensetzung einen isolierten menschlichen IgM-Antikörper bzw. ein Fragment mit der in einem der Ansprüche 1 bis 11 angegebenen Bedeutung und ein pharmazeutisch unbedenkliches Vehikel, Träger- oder Verdünnungsmittel umfasst.

13. Pharmazeutische Zusammensetzung zur Verwendung mit der in einem der Ansprüche 1 bis 11 angegebenen Bedeutung, wobei die pharmazeutische Zusammensetzung einen remyelinisierenden Antikörper mit der in einem der Ansprüche 1 bis 11 angegebenen Bedeutung und ein pharmazeutisch unbedenkliches Vehikel, Träger- oder Verdünnungsmittel umfasst.

14. Pharmazeutische Zusammensetzung zur Verwendung gemäß Anspruch 12 oder Anspruch 13, wobei das Verfahren Verabreichen der Zusammensetzung als Einzeldosis an das Tier, bei dem vermutet wird oder bestimmt wurde, dass es an Schlaganfall oder zerebraler Ischämie leidet, umfasst.

15. Kit zur Verwendung bei der Behandlung oder Linderung von Schlaganfall oder zerebraler Ischämie bei einem Tier, umfassend eine pharmazeutische Darreichungsform der pharmazeutischen Zusammensetzung nach Anspruch 12 und eine separate pharmazeutische Darreichungsform der pharmazeutischen Zusammensetzung nach Anspruch 13 und gegebenenfalls ferner umfassend ein oder mehrere zusätzliche neuroaktive Mittel oder Therapeutika, Thrombolytika, blutdrucksenkende Mittel, Thrombozytenaggregationshemmer, Entzündungshemmer, Neurotransmitterfreisetzung modulierende Mittel, Neurorezeptorliganden oder -agonisten oder -antagonisten, Calciumkanalmittel, Immunmodulatoren oder andere ZNS-reaktive Antikörper.

## Revendications

1. Anticorps IgM humain isolé ou fragment de celui-ci qui se lie spécifiquement à des neurones et protège les neurones de la mort cellulaire et qui ne favorise pas une remyélinisation, l'anticorps IgM humain isolé ou le fragment comprenant la séquence suivante :
(a) les séquences de domaines CDR d'acides aminés de chaînes lourdes variables CDR1 GGSVSLYY (SEQ ID n° : 31), CDR2 GYIYSSGST (SEQ ID n°: 32) et CDR3 ARSASIRGWFD (SEQ ID n° : 33), ainsi que les séquences de domaines CDR d'acides aminés de chaînes légères variables CDR1 QSISSY (SEQ ID n° : 34), CDR2 AAS (SEQ ID n°: 35) et CDR3 QQSYHTPW (SEQ ID n° : 36), telles qu'indiquées dans la Figure 5 ; ou
(b) les séquences de domaines CDR d'acides aminés de chaînes lourdes variables CDR1 GFTFSTYA (SEQ ID n° : 37), CDR2 INVGGVTT (SEQ ID n°: 38) et CDR3 VRRSGPDRNSSPADF (SEQ ID n°: 39), ainsi que les séquences de domaines CDR d'acides aminés de chaînes légères variables CDR1 QGIG (SEQ ID n° : 40), CDR2 TTS (SEQ ID n°: 41) et CDR3 QKYNSAPRT (SEQ ID n° : 42), telles qu'indiquées dans la Figure 6,
destiné à être utilisé dans un procédé de traitement ou d'amélioration d'une ischémie cérébrale ou d'un accident vasculaire cérébral, d'amélioration la fonction neuronale chez un animal après un accident vasculaire cérébral ou une ischémie cérébrale ou bien dans la protection des neurones ou des cellules neuronales contre un endommagement ou la mort cellulaire après un accident vasculaire cérébral ou une ischémie cérébrale,
dans lequel le procédé comprend l'étape d'administration de l'anticorps IgM humain isolé ou du fragment de celui-ci en combinaison avec un anticorps remyélinisant, et dans lequel l'anticorps remyélinisant comprend :
(i) des CDR d'une région variable de chaîne lourde comprenant la séquence de CDR1 SSGMH (SEQ ID n° : 47), la séquence de CDR2 V(I)ISYDGSRKYYADSVKG (SEQ ID n° : 48) et la séquence de CDR3 GVTGSPTLDY (SEQ ID n° : 49), ainsi que des CDR d'une région variable de chaîne légère comprenant la séquence de CDR1 SGSSSNIGNNFVS (SEQ ID n° : 50), la séquence de CDR2 DITKRPS (SEQ ID n° : 51) et la séquence de CDR3 G(E)TWDSSLSAVV (SEQ ID n° : 52) ; ou
(ii) des CDR d'une région variable de chaîne lourde comprenant la séquence de CDR1 SGFTFSSYW (SEQ ID n° : 53), la séquence de CDR2 IKKDGSEK (SEQ ID n° : 54) et la séquence de CDR3 ARPNCGGDCYLPWYFD (SEQ ID n° : 55), ainsi que des CDR d'une région variable de chaîne légère comprenant la séquence de CDR1 QSVLYSSNNKNY (SEQ ID n° : 56), la séquence de CDR2 YWAS (SEQ ID n° : 57) et la séquence de CDR3 QQYYNTPQA (SEQ ID n° : 58).

2. Anticorps remyélinisant comprenant :
(i) des CDR d'une région variable de chaîne lourde comprenant la séquence de CDR1 SSGMH (SEQ ID n° : 47), la séquence de CDR2 V(I)ISYDGSRKYYADSVKG (SEQ ID n° : 48) et la séquence de CDR3 GVTGSPTLDY (SEQ ID n° : 49), ainsi que des CDR d'une région variable de chaîne légère comprenant la séquence de CDR1 SGSSSNIGNNFVS (SEQ ID n° : 50), la séquence de CDR2 DITKRPS (SEQ ID n° : 51) et la séquence de CDR3 G(E)TWDSSLSAVV (SEQ ID n° : 52) ; ou
(ii) des CDR d'une région variable de chaîne lourde comprenant la séquence de CDR1 SGFTFSSYW (SEQ ID n° : 53), la séquence de CDR2 IKKDGSEK (SEQ ID n° : 54) et la séquence de CDR3 ARPNCGGDCYLPWYFD (SEQ ID n° : 55), ainsi que des CDR d'une région variable de chaîne légère comprenant la séquence de CDR1 QSVLYSSNNKNY (SEQ ID n° : 56), la séquence de CDR2 YWAS (SEQ ID n° : 57) et la séquence de CDR3 QQYYNTPQA (SEQ ID n° : 58),
destiné à être utilisé dans un procédé de traitement ou d'amélioration d'une ischémie cérébrale ou d'un accident vasculaire cérébral, d'amélioration la fonction neuronale chez un animal après un accident vasculaire cérébral ou une ischémie cérébrale ou bien dans la protection des neurones ou des cellules neuronales contre un endommagement ou la mort cellulaire après un accident vasculaire cérébral ou une ischémie cérébrale,
dans lequel le procédé comprend l'étape d'administration de l'anticorps remyélinisant en combinaison avec un anticorps IgM humain isolé ou un fragment de celui-ci qui se lie spécifiquement à des neurones et protège les neurones de la mort cellulaire et qui et qui ne favorise pas une remyélinisation, et dans lequel l'anticorps IgM humain isolé ou le fragment comprend la séquence suivante :
(a) les séquences de domaines CDR d'acides aminés de chaînes lourdes variables CDR1 GGSVSLYY (SEQ ID n° : 31), CDR2 GYIYSSGST (SEQ ID n° : 32) et CDR3 ARSASIRGWFD (SEQ ID n° : 33), ainsi que les séquences de domaines CDR d'acides aminés de chaînes légères variables CDR1 QSISSY (SEQ ID n° : 34), CDR2 AAS (SEQ ID n° : 35) et CDR3 QQSYHTPW (SEQ ID n° : 36), telles qu'indiquées dans la Figure 5 ; ou
(b) les séquences de domaines CDR d'acides aminés de chaînes lourdes variables CDR1 GFTFSTYA (SEQ ID n° : 37), CDR2 INVGGVTT (SEQ ID n° : 38) et CDR3 VRRSGPDRNSSPADF (SEQ ID n° : 39), ainsi que les séquences de domaines CDR d'acides aminés de chaînes légères variables CDR1 QGIG (SEQ ID n° : 40), CDR2 TTS (SEQ ID n° : 41) et CDR3 QKYNSAPRT (SEQ ID n° : 42), telles qu'indiquées dans la Figure 6.

3. Anticorps IgM humain isolé ou fragment de celui-ci destiné à l'utilisation de la revendication 1, ou anticorps remyélinisant destiné à l'utilisation de la revendication 2, l'anticorps IgM humain isolé ou le fragment de celui-ci comprenant la séquence d'acides aminés de chaîne lourde variable indiquée dans la SEQ ID n° : 1 et la séquence d'acides aminés de chaîne légère variable indiquée dans la SEQ ID n° : 11, ou la séquence d'acides aminés de chaîne lourde variable indiquée dans la SEQ ID n° : 17 et la séquence d'acides aminés de chaîne légère variable indiquée dans la SEQ ID n° : 27 ou bien des variants fortement homologues de celles-ci, dans lequel lesdits variants conservent une liaison aux neurones et une activité neuro-protectrice ou neuro-régénératrice.

4. Anticorps IgM humain isolé ou fragment de celui-ci destiné à l'utilisation de la revendication 1, ou anticorps remyélinisant destiné à l'utilisation de la revendication 2, l'anticorps IgM humain isolé ou le fragment de celui-ci comprenant en outre une séquence de chaîne J humaine.

5. Anticorps IgM humain isolé ou fragment de celui-ci destiné à l'utilisation ou anticorps remyélinisant destiné à l'utilisation de la revendication 4, dans lequel la chaîne J comprend la séquence d'acides aminés indiquée dans la SEQ ID n° : 15.

6. Anticorps IgM humain isolé ou fragment de celui-ci destiné à l'utilisation ou anticorps remyélinisant destiné à l'utilisation de la revendication 5, l'anticorps IgM humain isolé ou le fragment de celui-ci étant un anticorps totalement humain ou un fragment d'anticorps de celui-ci comprenant une région variable de chaîne lourde et de chaîne légère comprenant une séquence d'acides aminés choisie parmi les séquences d'acides aminés de SEQ ID n° : 1 et 11 ou des variants fortement homologues de celles-ci, dans lequel lesdits variants conservent une liaison aux neurones et une activité neuro-protectrice ou neuro-régénératrice.

7. Anticorps IgM humain isolé ou fragment de celui-ci destiné à l'utilisation ou anticorps remyélinisant destiné à l'utilisation de l'une quelconque des revendications 1 à 6, l'anticorps remyélinisant comprenant :
(i) la séquence d'acides aminés indiquée dans les SEQ ID n° : 43 et 44 ou
(ii) la séquence d'acides aminés indiquée dans les SEQ ID n° : 45 et 46.

8. Anticorps IgM humain isolé ou fragment de celui-ci destiné à l'utilisation ou anticorps remyélinisant destiné à l'utilisation de l'une quelconque des revendications 1 à 7, dans lequel la combinaison d'anticorps est administrée :
(i) collectivement ou
(ii) en série.

9. Anticorps IgM humain isolé ou fragment de celui-ci destiné à l'utilisation ou anticorps remyélinisant destiné à l'utilisation de l'une quelconque des revendications 1 à 8, dans lequel le procédé comprend l'étape d'administration de l'anticorps IgM humain isolé ou le fragment de celui-ci et de l'anticorps remyélinisant en combinaison avec un ou plusieurs agent(s) qui est/sont un agent thrombolytique, une substance médicamenteuse antiplaquettaire, une substance médicamenteuse hypertensive ou une statine.

10. Anticorps IgM humain isolé ou fragment de celui-ci destiné à l'utilisation ou anticorps remyélinisant destiné à l'utilisation de l'une quelconque des revendications 1 à 9, dans lequel l'anticorps IgM humain isolé ou le fragment de celui-ci et/ou de l'anticorps remyélinisant sont marqués avec un marqueur détectable ou fonctionnel.

11. Anticorps IgM humain isolé ou fragment de celui-ci destiné à l'utilisation ou anticorps remyélinisant destiné à l'utilisation de la revendication 10, dans lequel le marqueur est une enzyme, un partenaire spécifique de liaison, un ligand, un colorant, une étiquette fluorescente et/ou un élément radioactif.

12. Composition pharmaceutique destinée à l'utilisation telle que définie dans l'une quelconque des revendications 1 à 11, la composition pharmaceutique comprenant un anticorps IgM humain isolé ou un fragment, tel que défini dans l'une quelconque des revendications 1 à 11, et un véhicule, un transporteur ou un diluant acceptable d'un point de vue pharmaceutique.

13. Composition pharmaceutique destinée à l'utilisation telle que définie dans l'une quelconque des revendications 1 à 11, la composition pharmaceutique comprenant un anticorps remyélinisant, tel que défini dans l'une quelconque des revendications 1 à 11, et un véhicule, un transporteur ou un diluant acceptable d'un point de vue pharmaceutique.

14. Composition pharmaceutique destinée à être utilisée selon la revendication 12 ou la revendication 13, dans laquelle le procédé comprend une administration de la composition sous forme de dose unique au dit animal suspecté d'avoir, ou ayant été déterminé comme ayant, un accident vasculaire cérébral ou une ischémie cérébrale.

15. Trousse destinée à être utilisée dans le traitement ou l'amélioration d'un accident vasculaire cérébral ou d'une ischémie cérébrale chez un animal, comprenant une forme de dosage pharmaceutique de la composition pharmaceutique de la revendication 12 et une forme de dosage pharmaceutique séparée de la composition pharmaceutique de la revendication 13 et, en option, comprenant en outre un ou plusieurs agent(s) neuroactif(s) supplémentaire(s) ou agent(s) thérapeutique(s), thrombolytique(s), anti-hypertenseur(s), agent(s) antiplaquettaire(s), agent(s) anti-inflammatoire(s), agent(s) de modulation de la libération de neurotransmetteurs, ligand(s), agoniste(s) ou antagoniste(s) de neurorécepteur(s), agent(s) des canaux à calcium, immuno-modulateur(s) ou autre(s) anticorps réactif(s) avec le SNC.
